# EUROPEAN PATENT SPECIFICATION

(11) **EP 4 482 527 B1**
(45) Date of publication and mention of the grant of the patent: **08.04.2026**
(21) Application number: 23710443.5
(22) Date of filing: 24.02.2023
(51) Int. Cl.: A61K 47/54, A61P 35/00, A61K 39/395, A61P 29/00, A61P 31/00, A61P 37/00, C07D 471/04, C07K 16/16, C07K 16/44

(54) **CYTOTOXICITY TARGETING CHIMERAS FOR C-C CHEMOKINE RECEPTOR 2-EXPRESSING CELLS**
AUF ZYTOTOXIZITÄT ABZIELENDE CHIMÄRE FÜR C-C-CHEMOKINREZEPTOR-2-EXPRIMIERENDE ZELLEN
CHIMÈRES CIBLANT LA CYTOTOXICITÉ POUR DES CELLULES EXPRIMANT LE RÉCEPTEUR 2 DE LA CHIMIOKINE C-C

(30) Priority: 25.02.2022 US 202263314034 P
(43) Date of publication of application: 01.01.2025
(73) Proprietor: GlaxoSmithKline Intellectual Property Development Limited, Stevenage SG1 2NY (GB)
(72) Inventor: JEONG, Jae U., Collegeville, Pennsylvania 19426 (US); JESO, Valer, Collegeville, Pennsylvania 19426 (US); KNAPP-REED, Beth Anne, Collegeville, Pennsylvania 19426 (US); MARCUS, Andrew Peter, Collegeville, Pennsylvania 19426 (US); PHELAN, James P., Collegeville, Pennsylvania 19426 (US); SENDER, Matthew Robert, Collegeville, Pennsylvania 19426 (US); TURUNEN, Brandon, Collegeville, Pennsylvania 19426 (US)
(74) Representative: Graham Watt & Co
(86) International application number: PCT/IB2023/051743
(87) International publication number: WO 2023/161874

(56) References cited:
- WO-A1-2018/134731

## Description

### CROSS-REFERENCE TO RELATED APPLICATION

This application claims priority to U.S. Patent Application No. 63/314,034 filed on February 25, 2022.

### FIELD OF THE DISCLOSURE

The present disclosure relates to heterobifunctional molecules, referred to as cytotoxicity targeting chimeras (CyTaCs) or antibody recruiting molecules (ARMs) that are able to simultaneously bind a target cell-surface protein as well as an exogenous antibody protein. The present disclosure also relates to agents capable of binding to a receptor on a surface of a pathogenic cell and inducing the depletion of the pathogenic cell in a subject for use in the treatment of cancer, inflammatory diseases, autoimmune diseases, viral infection, or bacterial infection.

### BACKGROUND

Cell-surface proteins and their ligands play key roles in a range of inflammatory, infectious, and autoimmune diseases as well tumor initiation, growth and metastasis. Antibody-based therapeutics have promising properties as drug candidates for these indications due to their selectivity for pathogenic cell-surface targets and their ability to direct immune surveillance to target-expressing tissues or cells to induce depletion of the pathogenic cells. Examples of such depletion mechanisms include antibody-dependent cellular cytotoxicity (ADCC), antibody-dependent cellular phagocytosis (ADCP), and complement-dependant cytotoxicity (CDC). However, antibody-based therapeutics often suffer from a lack of bioavailability, high cost, thermal instability, and difficult manufacturing due to their size, complexity and peptide based structures. Conversely, small molecule therapeutics often provide affordability, stability, and the convenience of oral dosing, but may suffer from poor selectivity and off-target effects, while also lacking the immune control of therapeutic antibodies.

Accordingly, a need exists for improved therapeutic approaches that target pathogenic cells for use in the treatment of disease. Such compositions and related methods are provided in the present disclosure.

### SUMMARY

In one aspect, the present disclosure provides a heterobifunctional molecule referred to as a cytoxicity targeting chimera (CyTaC) or an antibody recruiting molecule (ARM), wherein the ARM comprises a moiety that binds a target cell-surface protein on a cell and a moiety that binds an exogenous antibody. In a further aspect, the ARM comprises a divalent linker that links the target-binding moiety to the antibody-binding moiety. In a further aspect, the target-binding moiety is a C-C chemokine receptor type 2 (CCR2)-binding moiety. In a further aspect, the exogenous antibody is an anti-cotinine antibody, or antigen-binding fragment thereof.

In a further aspect, the ARM is a compound of Formula (I): 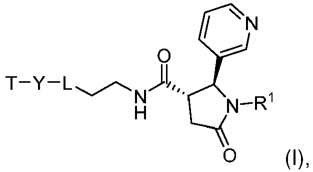
or a pharmaceutically acceptable salt thereof,
wherein:
T is 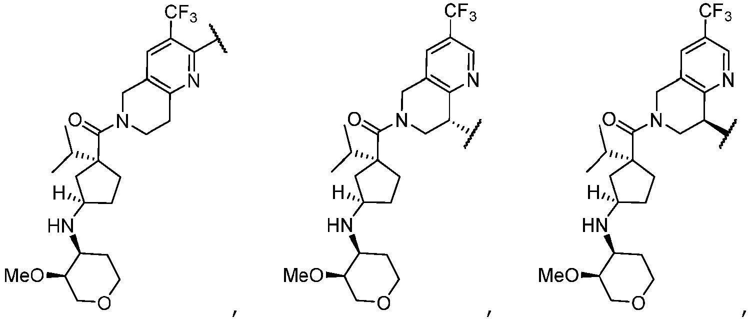 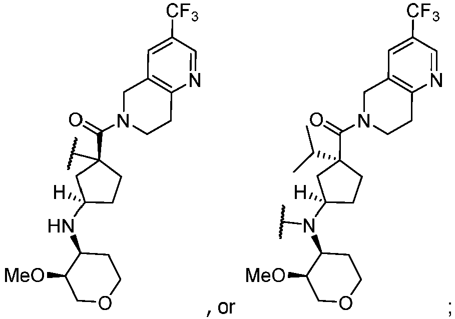
R¹ is C₁₋₄ alkyl or C₃₋₆ cycloalkyl;
Y is a bond or a divalent spacer moiety of one to twelve atoms in length; and
L is a divalent linker as described herein.

In one aspect, the present disclosure provides a method of treating and/or preventing a disease or disorder in a patient in need thereof, comprising: administering to the patient a compound of Formula (I) as disclosed herein and an anti-cotinine antibody, or antigen-binding fragment thereof.

In one aspect, the present disclosure provides a method of increasing antibody-dependent cell cytotoxicity (ADCC) of CCR2-expressing cells comprising: contacting the cells with a compound of Formula (I) as disclosed herein and an anti-cotinine antibody, or antigen-binding fragment thereof.

In one aspect, the present disclosure provides a method of depleting CCR2-expressing cells comprising: contacting the cells with a compound of Formula (I) as disclosed herein and an anti-cotinine antibody, or antigen-binding fragment thereof.

In one aspect, the present disclosure provides a compound of Formula (I) as disclosed herein for use in therapy. In a further aspect, the present disclosure provides a combination comprising a compound of Formula (I) as disclosed herein and an anti-cotinine antibody, or antigen-binding fragment thereof, for use in therapy.

In one aspect, the present disclosure provides a combination comprising a compound of Formula (I) as disclosed herein and an anti-cotinine antibody, or antigen-binding fragment thereof, for use in the treatment of a disease or disorder.

In one aspect, the present disclosure provides use of a compound of Formula (I) as disclosed herein in the manufacture of a medicament for the treatment of a disease or disorder. In a further aspect, the present disclosure provides use of a combination comprising a compound of Formula (I) as disclosed herein and an anti-cotinine antibody, or antigen-binding fragment thereof, in the manufacture of a medicament for the treatment of a disease or disorder.

In one aspect, the present disclosure provides a combination comprising a compound of Formula (I) as disclosed herein and an anti-cotinine antibody, or antigen-binding fragment thereof.

### BRIEF DESCRIPTION OF THE FIGURES

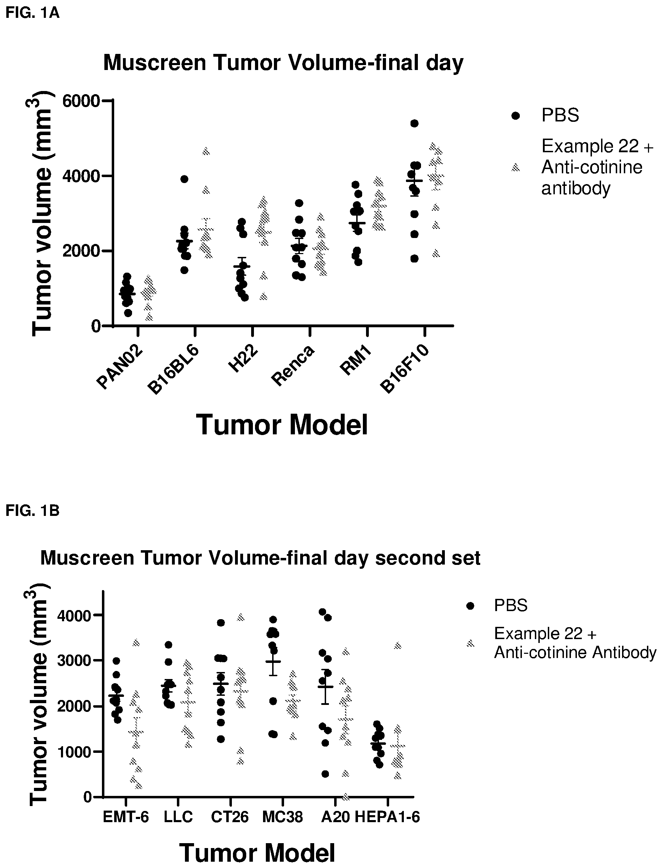

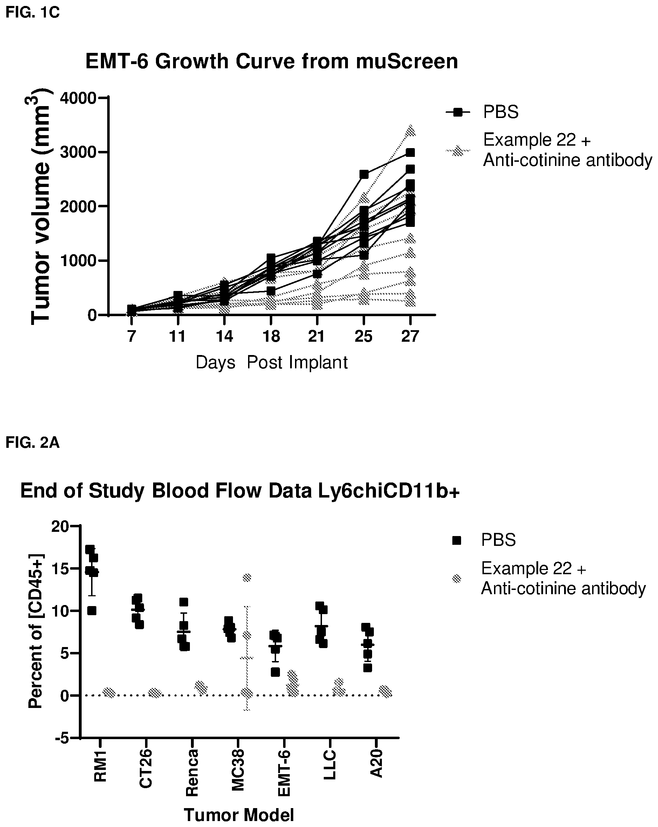

**FIG. 1A, FIG. 1B****, and** **FIG. 1C** show analysis of various syngeneic mouse tumors dosed with CyTaCs compounds (compound of Example 22) in the presence of anti-cotinine antibody; **FIG. 1A and FIG. 1B** show tumor volume of tumor bearing mice treated with the CyTaC compound of Example 22 and anti-cotinine antibody or PBS as control; tumor volume on the final day of the study for each tumor model is shown; **FIG. 1C** shows tumor volume of EMT-6 tumor bearing mice when dosed with PBS or with the CyTaC compound of Example 22 in combination with anti-cotinine antibody; each line represents an individual mouse in the same experiment.

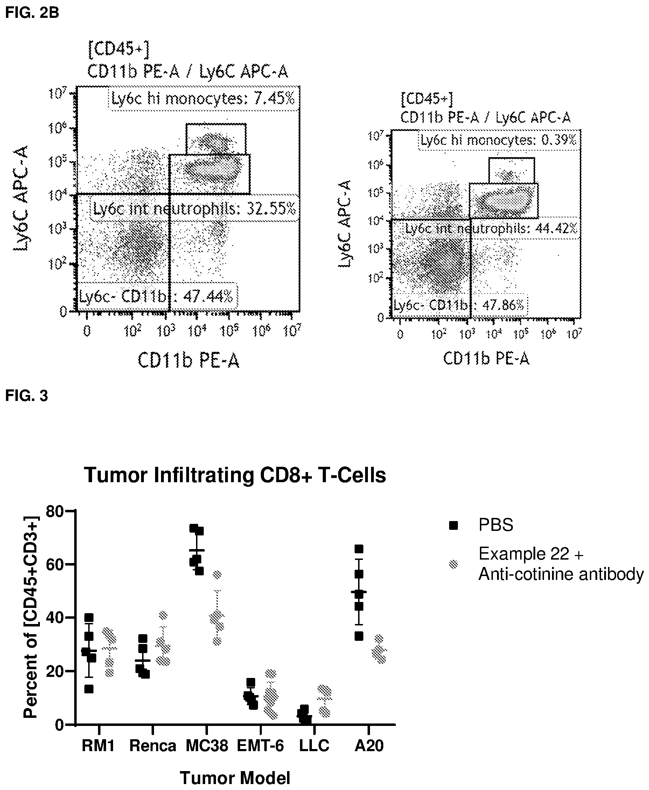

**FIG. 2A** **and** **FIG. 2B** show depletion of monocytes from the blood of tumor bearing animals following treatment with CyTaC compound (compound of Example 22) and anti-cotinine antibody; **FIG. 2A** shows the depletion of monocytes (Ly6Chi/CD11b+) cells across multiple tumor models; **FIG. 2B** shows an example of the flow gating of CD11b and Ly6C in the blood from MC38 tumor bearing animals for mice dosed with PBS (left) and mice dosed with CyTaC compound and antibody (right).
**FIG. 3** shows the effect of treatment with CyTaC compound (compound of Example 22) in combination with anti-cotinine antibody on the intratumoral levels of CD8+ Tcells determined by flow cytometry of lymphocytes from tumors excised from syngeneic models.

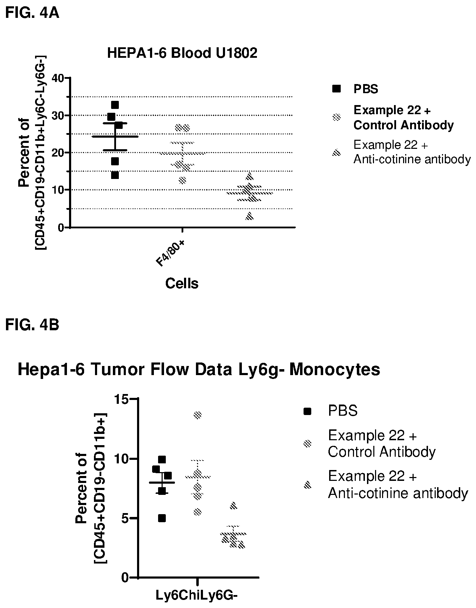

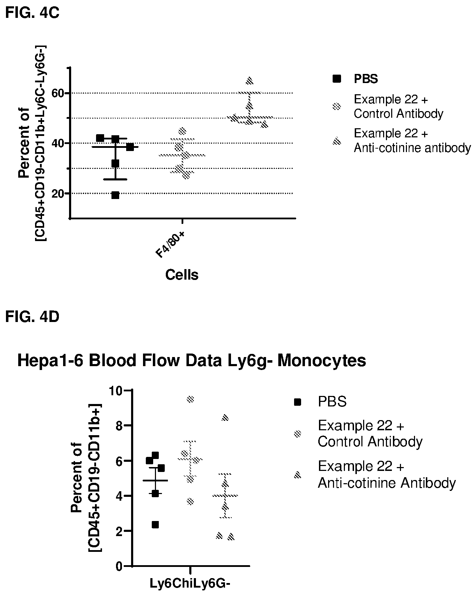

**FIG. 4A, FIG. 4B****,** **FIG. 4C, and FIG. 4D** show characterization by flow cytometry of various cell populations in the blood and tumor of HEPA1-6 bearing mice; **FIG. 4A** and **FIG. 4C** show the analysis of F480+ positive cells from the blood and intratumoral lymphocytes; **FIG. 4B** and **FIG. 4D** show the analysis of Ly6G- monocytes from the blood and intratumoral lymphocytes.

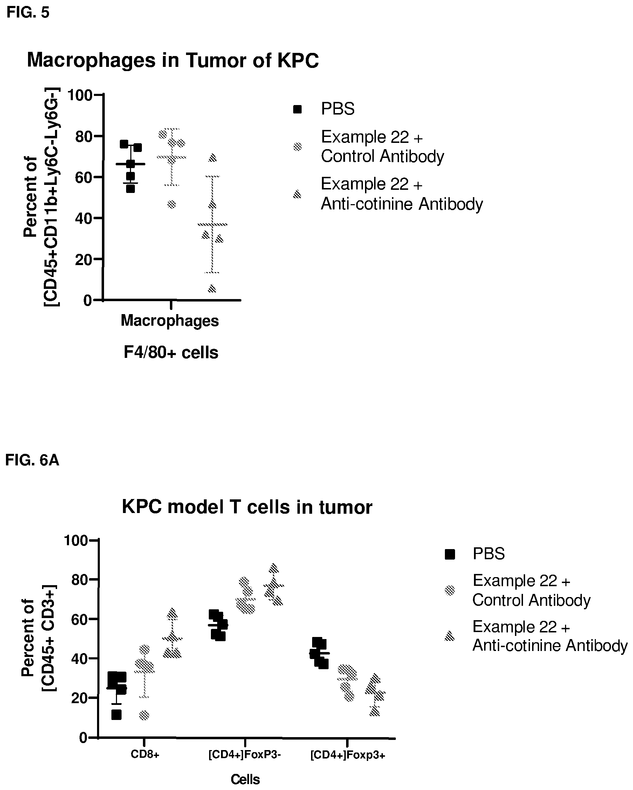

**FIG. 5** shows flow cytometry analysis of macrophages in tumor samples excised from KPC tumors grown subcutaneously.

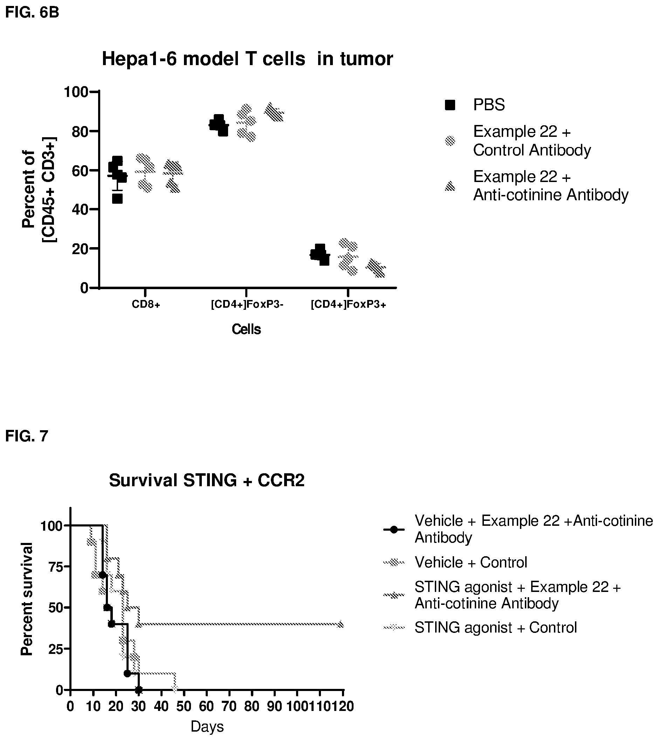

**FIG. 6A** **and** **FIG. 6B** show flow cytometry analysis of tumor samples from Hepa1-6 or KPC tumors grown subcutaneously; **FIG. 6A** shows the effect of treatment with CyTaC compound of Example 22 in combination with anti-cotinine antibody on the percentage of T cell populations present in KPC tumors; **FIG. 6B** shows the effect treatment with CyTaC compound of Example 22 in combination with anti-cotinine antibody on the percentage of T cell subpopulations present in Hepa1-6 tumors.
**FIG. 7** shows the efficacy of treatment with CyTaC compound of Example 22 in combination with anti-cotinine antibody, alone or further in combination with STING agonist treatment.

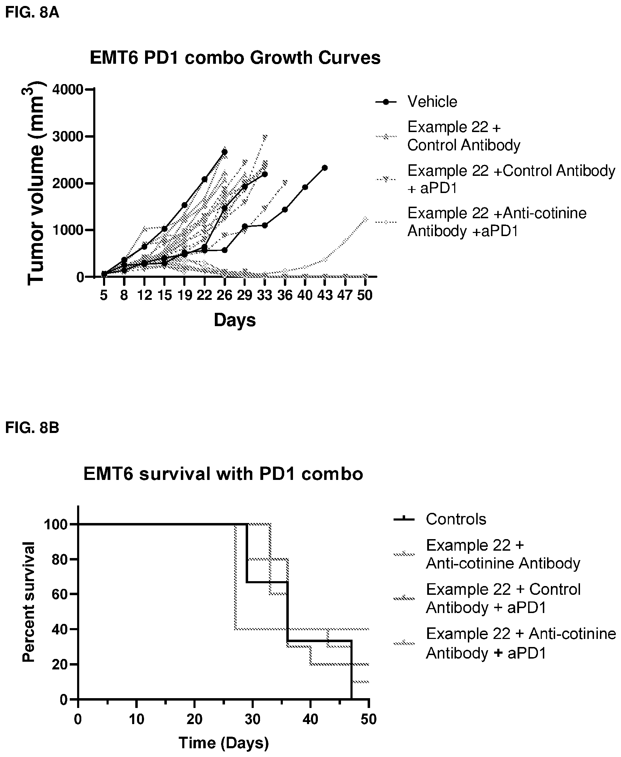

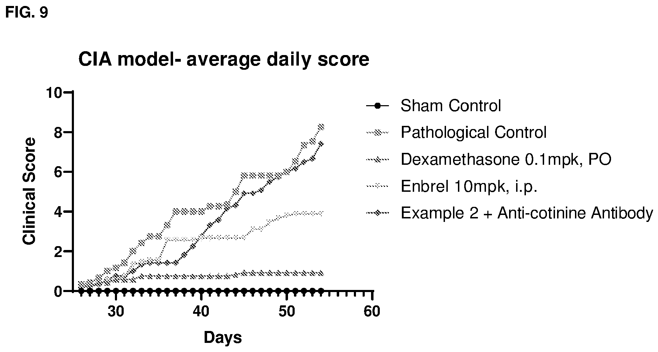

**FIG. 8A and FIG.** 8B show the efficacy of treatment with CyTaC compound of Example 22 in combination with anti-cotinine antibody, alone or further in combination with anti-PD1 treatment; **FIG. 8A** shows the tumor volume for each individual animal that was treated over time; **FIG. 8B** shows overall survival of the animals across treatment group. **FIG. 9** shows the efficacy of treatment with CyTaC compound of Example 22 in combination with anti-cotinine antibody in a collagen induced arthritis model; animals were dosed as described in Example 32 and assessed for inflammation to generate a conglomerate clinical score which is plotted.

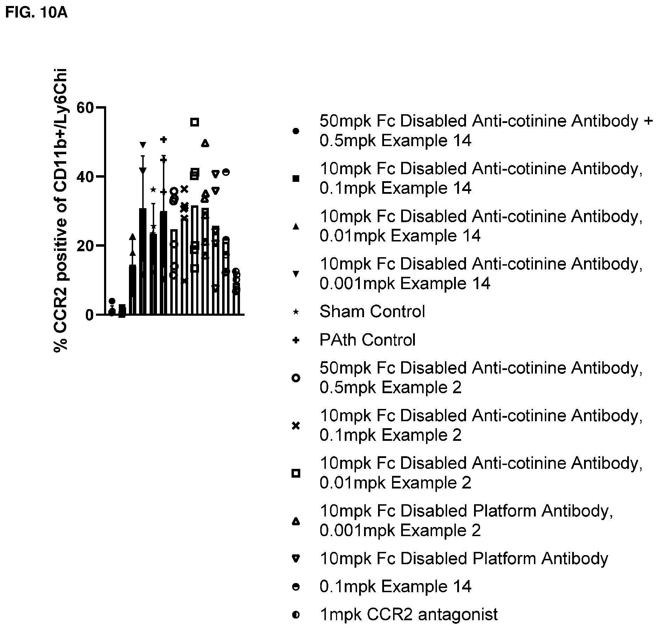

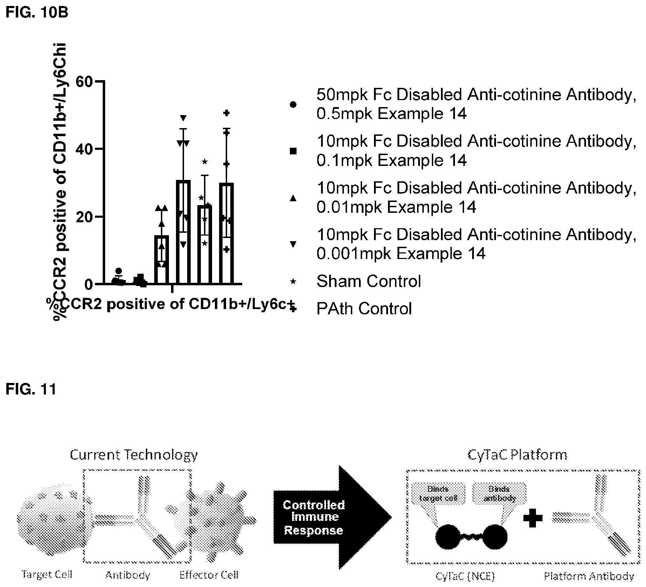

**FIG. 10A** **and** **FIG.** 10B show antagonism of monocyte chemotaxis by CyTaC compounds when dosed with a non-ADCC active anti-cotinine antibody; thioglyocollate was used to induce the chemotaxis of immune cells; intraperitoneal lavage was used to isolate cells and the cells were subjected to flow cytometry to identify monocytes (Ly6Chi/CD11b+); **FIG. 10A** shows a comparison between the activity of the compounds of Examples 2 and 14; **FIG. 10B** shows the activity of the compound of Example 14.
**FIG. 11****:** Schematic representation of cytotoxicity targeting chimeras (CyTaCs) technology compared to current antibody technology.

### DETAILED DESCRIPTION

In one aspect, the present disclosure provides a compound of Formula (I): 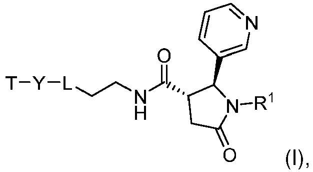
or a pharmaceutically acceptable salt thereof,
wherein:
T is 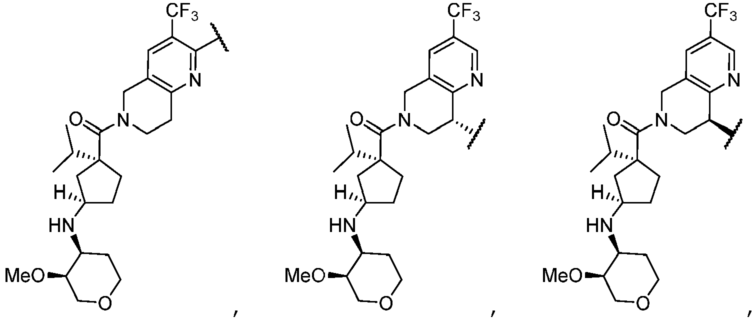 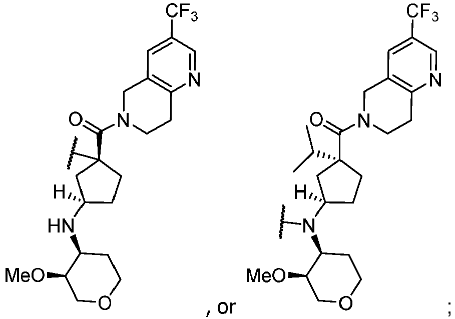
R¹ is C₁₋₄ alkyl or C₃₋₆ cycloalkyl;
Y is a bond or a divalent spacer moiety of one to twelve atoms in length; and
L is a divalent linker of Formula (L-a), (L-b), (L-c), (L-d), (L-e), (L-f), (L-g), (L-h), (L-i), (L-j), (L-k), (L-m), (L-n-i), (L-n-ii), (L-n-iii), or (L-n-iv).

In one embodiment of the disclosure L is a divalent linker of Formula (L-a): 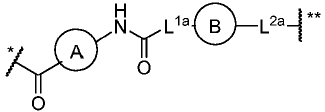 (L-a), or a stereoisomer thereof, wherein:
Ring A and Ring B are each independently C₄₋₆ cycloalkylene;
L^{1a} is C₃₋₅ linear alkylene, wherein 1 or 2 methylene units are replaced with -O- or -NR^{a}-; each R^{a} is independently hydrogen or C₁₋₃ alkyl; and
L^{2a} is -O-, -NHC(O)-, or -CH₂-O-;
wherein 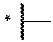 represents a covalent bond to the Y group of Formula (I), or when Y is a bond, a covalent bond to the T group of Formula (I), and 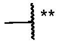 represents a covalent bond to the methylene group of Formula (I).

In another embodiment, Ring A and Ring B of Formula (L-a) are each independently 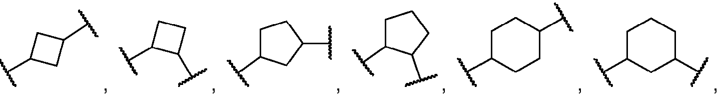 or

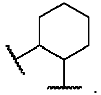

In another embodiment, L is a divalent linker of Formula (L-a-i): 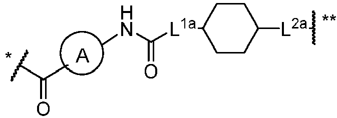 (L-a-i), or a stereoisomer thereof, wherein:
Ring A is C₄₋₆ cycloalkylene;
L^{1a} is C₃₋₅ linear alkylene, wherein 1 or 2 methylene units are replaced with -O- or -NR^{a}-;
each R^{a} is independently hydrogen or C₁₋₃ alkyl; and
L^{2a} is -O-, -NHC(O)-, or -CH₂-O-;
wherein 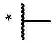 represents a covalent bond to the Y group of Formula (I), or when Y is a bond, a covalent bond to the T group of Formula (I), and 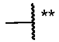 represents a covalent bond to the methylene group of Formula (I).

In another embodiment, Ring A of Formula (L-a-i) is

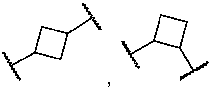

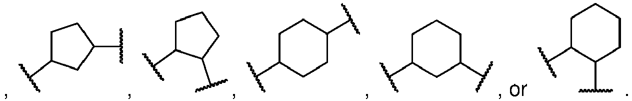

In another embodiment, L is a divalent linker of Formula (L-a-ii): 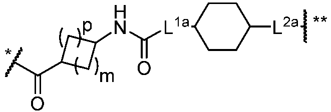 (L-a-ii), or a stereoisomer thereof, wherein:
L^{1a} is C₃₋₅ linear alkylene, wherein 1 or 2 methylene units are replaced with -O- or -NR^{a}-;
each R^{a} is independently hydrogen or C₁₋₃ alkyl;
L^{2a} is -O-, -NHC(O)-, or -CH₂-O-;
p is 1 or 2; and
m is 1 or 2;
wherein 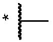 represents a covalent bond to the Y group of Formula (I), or when Y is a bond, a covalent bond to the T group of Formula (I), and 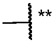 represents a covalent bond to the methylene group of Formula (I).

In another embodiment, L^{1a} of Formula (L-a), (L-a-i), or (L-a-ii) is selected from 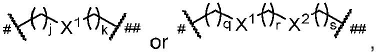 wherein:
j is 1, 2, 3, or 4;
k is 0, 1, 2, or 3;
the sum of j and k is 2, 3, or 4;
q is 1 or 2;
r is 1 or 2;
s is 0 or 1;
the sum of q, r, and s is 2 or 3;
X¹ and X² are independently -O- or NR^{a}; and
each R^{a} is independently hydrogen or C₁₋₃ alkyl;
wherein 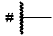 represents a covalent bond to the C(O) group of Formula (L-a), (L-a-i), or (L-a-ii), and 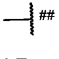 represents a covalent bond to Ring B of Formula (L-a) or to the cyclohexylene group of Formula (L-a-i) or (L-a-ii).

In another embodiment, L^{1a} of Formula (L-a), (L-a-i), or (L-a-ii) is selected from -(CH₂)₂O-, -(CH₂)₃O-, -(CH₂)₄O-, -(CH₂)₂OCH₂-, -(CH₂)₃OCH₂-, -(CH₂)₂O(CH₂)₂-, - CH₂OCH₂-, -CH₂O(CH₂)₂-, -CH₂O(CH₂)₃-, -CH₂OCH₂O-, or -CH₂OCH₂OCH₂-. In another embodiment, L^{1a} of Formula (L-a), (L-a-i), or (L-a-ii) is selected from -(CH₂)₂O-, -(CH₂)₃O-, - (CH₂)₂OCH₂-, or -(CH₂)₃OCH₂-. In another embodiment, L^{1a} of Formula (L-a), (L-a-i), or (L-a-ii) is selected from -(CH₂)₂NR^{a}-, -(CH₂)₃NR^{a}-, -(CH₂)₄NR^{a}-, -(CH₂)₂NR^{a}CH₂-, -(CH₂)₃NR^{a}CH₂-, -(CH₂)₂NR^{a}(CH₂)₂-, -CH₂NR^{a}CH₂-, -CH₂NR^{a}(CH₂)₂-, -CH₂NR^{a}(CH₂)₃-, -CH₂NR^{a}CH₂NR^{a}-, or - CH₂NR^{a}CH₂NR^{a}CH₂-, wherein each R^{a} is independently hydrogen or C₁₋₃ alkyl. In another embodiment, L^{1a} of Formula (L-a), (L-a-i), or (L-a-ii) is selected from -(CH₂)₂NR^{a}-, -(CH₂)₃NR^{a}-, -(CH₂)₂NR^{a}CH₂-, or -(CH₂)₃NR^{a}CH₂-, wherein R^{a} is hydrogen or C₁₋₃ alkyl. In another embodiment, L^{1a} of Formula (L-a), (L-a-i), or (L-a-ii) is selected from -(CH₂)₂NH-, -(CH₂)₃NH-, -(CH₂)₄NH-, -(CH₂)₂NHCH₂-, -(CH₂)₃NHCH₂-, -(CH₂)₂NH(CH₂)₂-, -CH₂NHCH₂-, -CH₂NH(CH₂)₂-, -CH₂NH(CH₂)₃-, -CH₂NHCH₂NH-, or -CH₂NHCH₂NHCH₂-. In another embodiment, L^{1a} of Formula (L-a), (L-a-i), or (L-a-ii) is selected from -(CH₂)₂NH-, -(CH₂)₃NH-, -(CH₂)₂NHCH₂-, or - (CH₂)₃NHCH₂-. In another embodiment, L^{1a} of Formula (L-a), (L-a-i), or (L-a-ii) is selected from -CH₂OCH₂NR^{a}-, -CH₂NR^{a}CH₂O-, -CH₂OCH₂NR^{a}CH₂-, -CH₂NR^{a}CH₂OCH₂-, wherein R^{a} is independently hydrogen or C₁₋₃ alkyl. In another embodiment, L^{1a} of Formula (L-a), (L-a-i), or (L-a-ii) is selected from -CH₂OCH₂NH-, -CH₂NHCH₂O-, -CH₂OCH₂NHCH₂-, - CH₂NHCH₂OCH₂-.

In another embodiment, L is a divalent linker of Formula (L-a-iii): 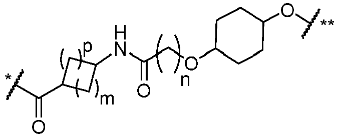 (L-a-iii), or a stereoisomer thereof, wherein:
p is 1 or 2;
m is 1 or 2; and
n is 1, 2, or 3;
wherein 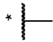 represents a covalent bond to the Y group of Formula (I), or when Y is a bond, a covalent bond to the T group of Formula (I), and 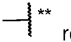 epresents a covalent bond to the methylene group of Formula (I).

In another embodiment, L is a divalent linker of Formula (L-a) selected from the group consisting of:

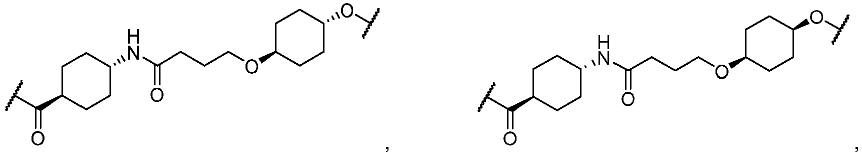

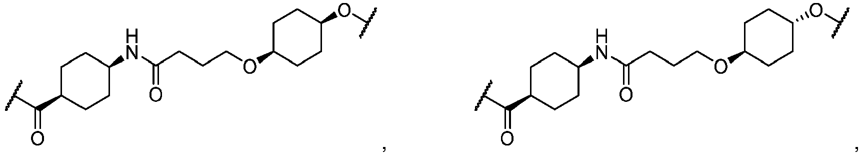

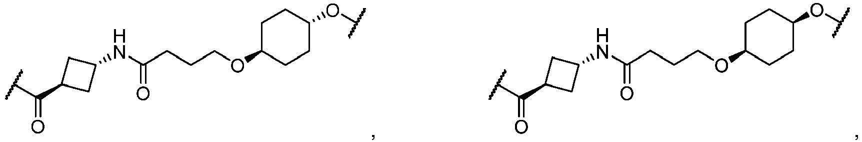

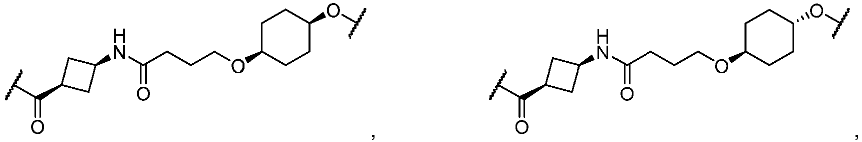

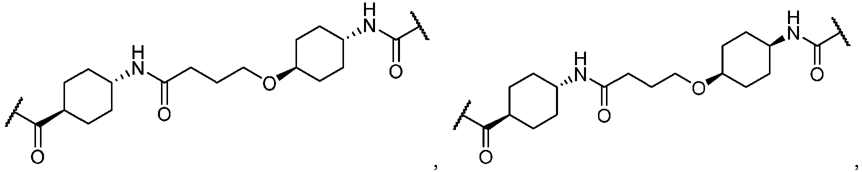

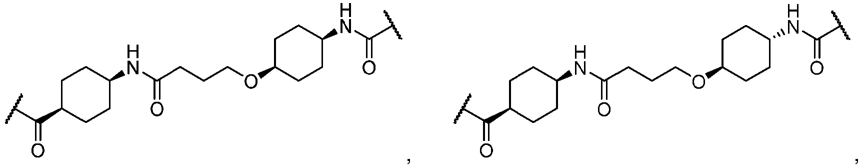

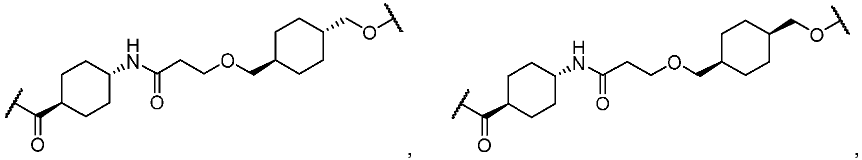

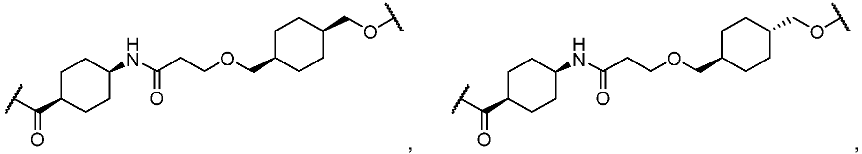

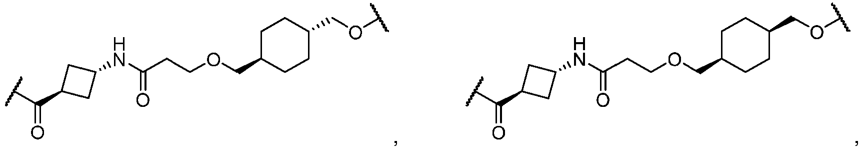

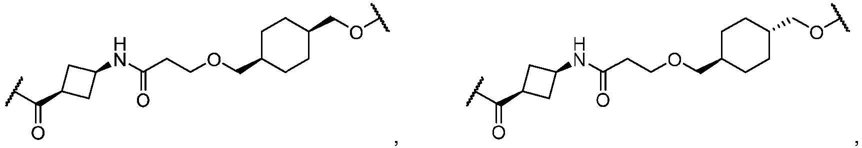

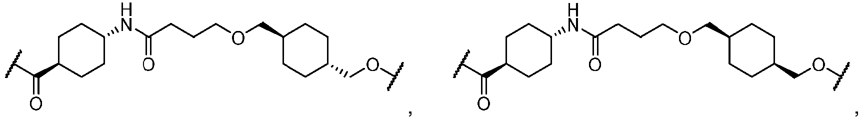

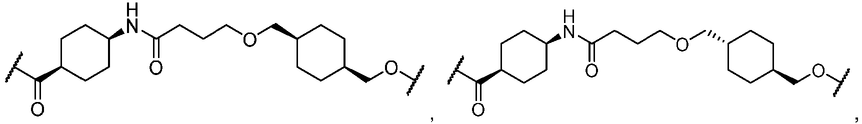

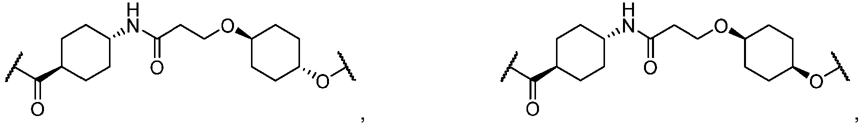

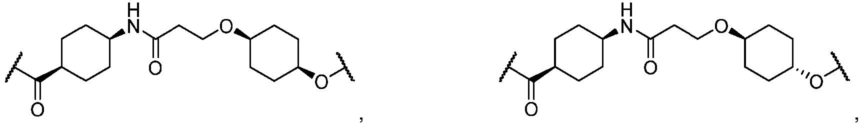

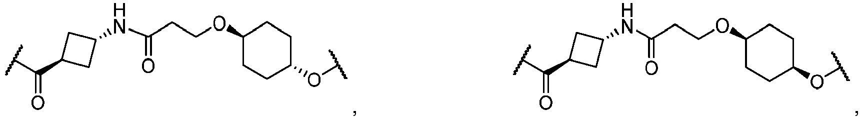

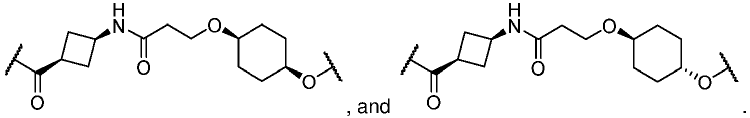

In another embodiment, L is a divalent linker of Formula (L-b): 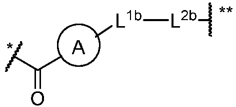 (L-b), or a stereoisomer thereof, wherein:
Ring A is C₄₋₆ cycloalkylene or C₇₋₉ bridged bicyclic cycloalkylene;
L^{1b} is -CH₂-NH-C(O)-, -NHC(O)-, or -C(O)NH-;
L^{2b} is C₆₋₁₂ linear alkylene, wherein 1, 2, 3, or 4 methylene units are replaced with -O-, -NR^{1b}-, -C(O)NR^{1b}-, or -NR^{1b}C(O)-; or
L^{2b} is 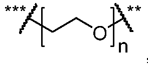 , wherein n is 1, 2, 3, or 4, and 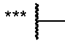 represents a covalent bond to L^{1b}; and
each R^{1b} is independently hydrogen or C₁₋₃ alkyl;
wherein 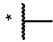 represents a covalent bond to the Y group of Formula (I), or when Y is a bond, a covalent bond to the T group of Formula (I), and 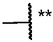 represents a covalent bond to the methylene group of Formula (I).

In another embodiment, Ring A of Formula (L-b) is 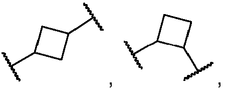 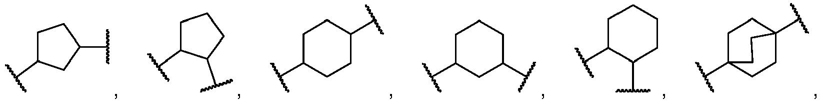 or

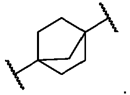

In another embodiment, L is a divalent linker of Formula (L-b-i): 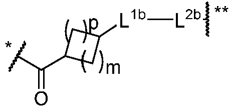 (L-b-i), or a stereoisomer thereof, wherein:
L^{1b} is -CH₂-NH-C(O)-, -NHC(O)-, or -C(O)NH-;
L^{2b} is C₆₋₁₂ linear alkylene, wherein 1, 2, 3, or 4 methylene units are replaced with -O-, -NR^{1b}-, -C(O)NR^{1b}-, or -NR^{1b}C(O)-; or
L^{2b} is 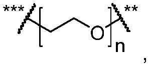 wherein n is 1, 2, 3, or 4, and 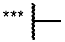 represents a covalent bond to L^{1b};
each R^{1b} is independently hydrogen or C₁₋₃ alkyl;
p is 1 or 2; and
m is 1 or 2;
wherein 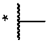 represents a covalent bond to the Y group of Formula (I), or when Y is a bond, a covalent bond to the T group of Formula (I), and 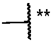 represents a covalent bond to the methylene group of Formula (I).

In another embodiment, L^{2b} of Formula (L-b) or (L-b-i) is selected from 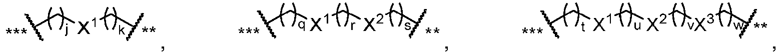 or 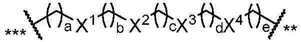 wherein:
j is 1, 2, 3, 4, 5, 6, 7, 8, 9, or 10;
k is 1, 2, 3, 4, 5, 6, 7, 8, 9, or 10;
the sum of j and k is 5, 6, 7, 8, 9, 10, or 11;
q is 1, 2, 3, 4, 5, 6, 7, 8, or 9;
r is 1, 2, 3, 4, 5, 6, 7, 8, or 9;
s is 0, 1, 2, 3, 4, 5, 6, 7, or 8;
the sum of q, r, and s is 4, 5, 6, 7, 8, 9, or 10;
t is 1, 2, 3, 4, 5, 6, or 7;
u is 1, 2, 3, 4, 5, 6, or 7;
v is 1, 2, 3, 4, 5, 6, or 7;
w is 0, 1, 2, 3, 4, 5, or 6;
the sum of t, u, v, and w is 3, 4, 5, 6, 7, 8, or 9;
a is 1, 2, 3, 4, or 5;
b is 1, 2, 3, 4, or 5;
c is 1, 2, 3, 4, or 5;
d is 1, 2, 3, 4, or 5;
e is 0, 1, 2, 3, or 4;
the sum of a, b, c, d, and e is 4, 5, 6, 7, or 8;
X¹, X², X³, and X⁴ are independently -O-, -NR^{1b}-, -C(O)NR^{1b}-, or -NR^{1b}C(O)-; and
each R^{1b} is independently hydrogen or C₁₋₃ alkyl;
wherein 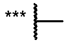 represents a covalent bond to L^{1b} of Formula (L-b) or (L-b-i), and 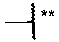 represents a covalent bond to the methylene group of Formula (I).

In another embodiment, L is a divalent linker of Formula (L-b) selected from the group consisting of: 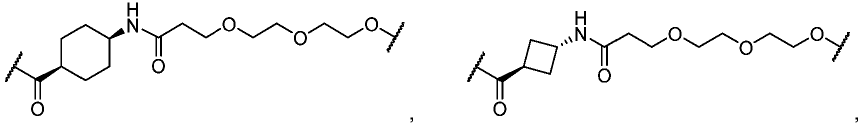 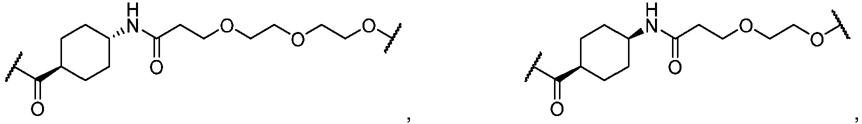 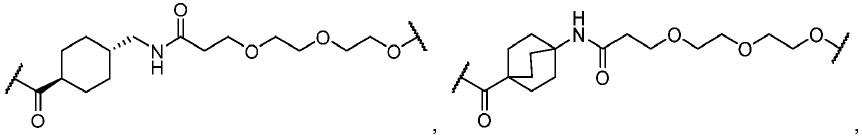 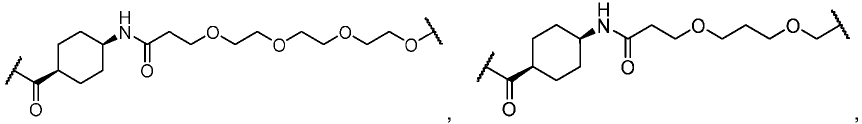 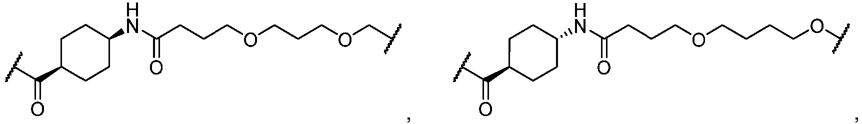 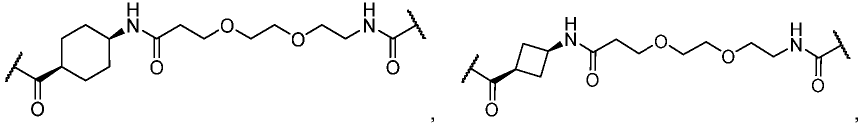 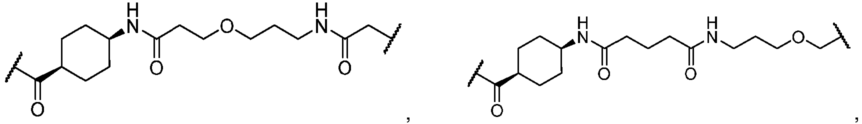 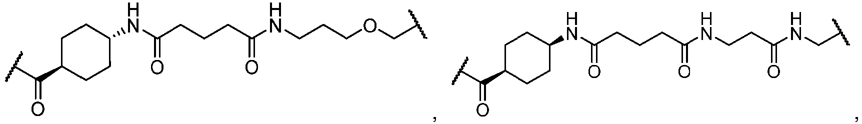 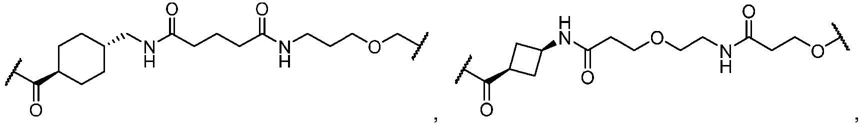 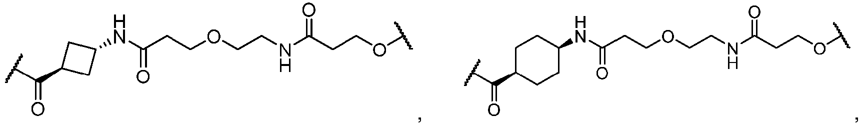 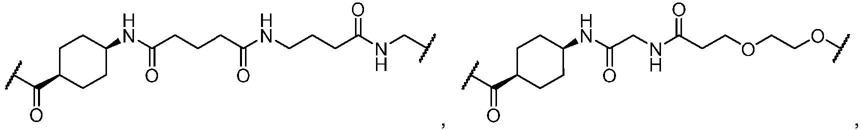 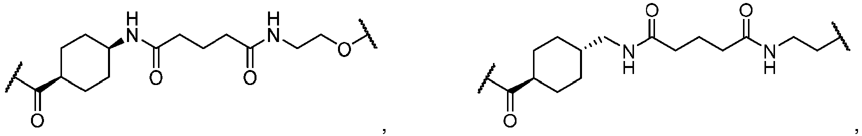 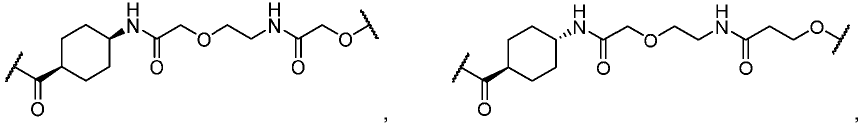 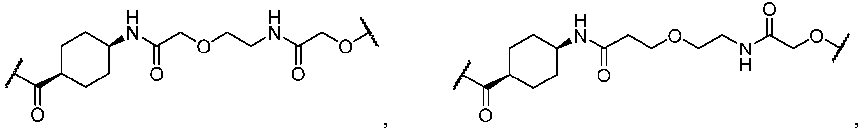 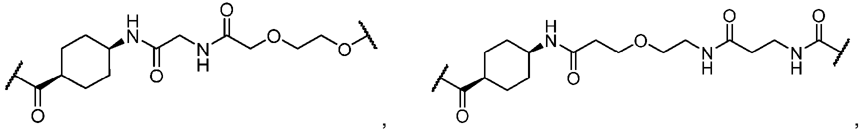 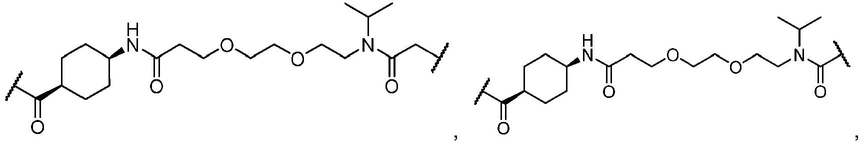 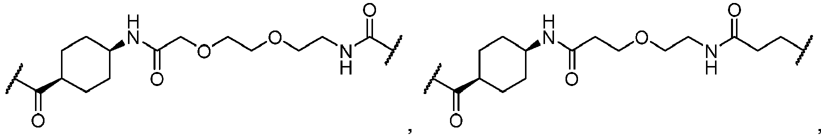 and

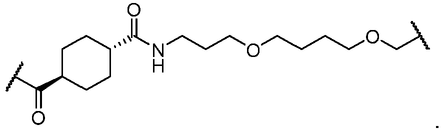

In another embodiment, L is a divalent linker of Formula (L-c): 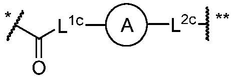 (L-c), or a stereoisomer thereof, wherein:
L^{1c} is C₂₋₁₀ linear alkylene, wherein 1, 2, or 3 methylene units are replaced with -O-, -NH-, - NHC(O)-, or -C(O)NH-;
Ring A is C₄₋₆ cycloalkylene or C₇₋₉ bridged bicyclic cycloalkylene; and
L^{2c} is -O- or a saturated C₂₋₁₀ linear alkylene, wherein 1, 2, or 3 methylene units are replaced with -O-, -NH-, -NHC(O)-, or -C(O)NH-;
wherein 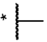 represents a covalent bond to the Y group of Formula (I), or when Y is a bond, a covalent bond to the T group of Formula (I), and 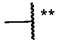 represents a covalent bond to the methylene group of Formula (I).

In another embodiment, Ring A of Formula (L-c) is 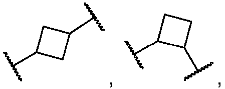 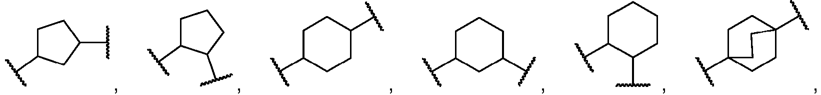 or

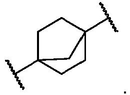

In another embodiment, L is a divalent linker of Formula (L-c-i): 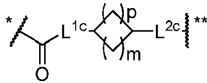 (L-c-i), or a stereoisomer thereof, wherein:
L^{1c} is C₂₋₁₀ linear alkylene, wherein 1, 2, or 3 methylene units are replaced with -O-, -NH-, - NHC(O)-, or -C(O)NH-;
L^{2c} is -O- or a saturated C₂₋₁₀ linear alkylene, wherein 1, 2, or 3 methylene units are replaced with -O-, -NH-, -NHC(O)-, or -C(O)NH-;
p is 1 or 2; and
m is 1 or 2;
wherein 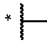 represents a covalent bond to the Y group of Formula (I), or when Y is a bond, a covalent bond to the T group of Formula (I), and 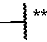 represents a covalent bond to the methylene group of Formula (I).

In another embodiment, L^{1c} of Formula (L-c) or (L-c-i) is selected from 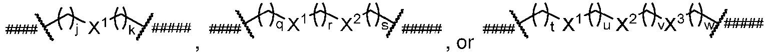 wherein:
j is 1, 2, 3, 4, 5, 6, 7, 8, or 9;
k is 0, 1, 2, 3, 4, 5, 6, 7, or 8;
the sum of j and k is 1, 2, 3, 4, 5, 6, 7, 8, or 9;
q is 1, 2, 3, 4, 5, 6, or 7;
r is 1, 2, 3, 4, 5, 6, or 7;
s is 0, 1, 2, 3, 4, 5, or 6;
the sum of q, r, and s is 2, 3, 4, 5, 6, 7, or 8;
t is 1, 2, 3, 4, or 5;
u is 1, 2, 3, 4, or 5;
v is 1, 2, 3, 4, or 5;
w is 0, 1, 2, 3, or 4;
the sum of t, u, v, and w is 3, 4, 5, 6, or 7; and
X¹, X² and X³ are independently -O-, -NH-, -NHC(O)-, or -C(O)NH-;
wherein 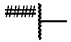 represents a covalent bond to the C(O) group of Formula (L-c) or (L-c-i), and 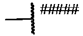 represents a covalent bond to the ring of Formula (L-c) or (L-c-i).

In another embodiment, L^{2c} of Formula (L-c) or (L-c-i) is selected from 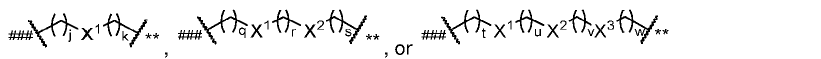 wherein:
j is 0, 1, 2, 3, 4, 5, 6, 7, 8, or 9;
k is 0, 1, 2, 3, 4, 5, 6, 7, 8, or 9;
the sum of j and k is 1, 2, 3, 4, 5, 6, 7, 8, or 9;
q is 0, 2, 3, 4, 5, 6, or 7;
r is 1, 2, 3, 4, 5, 6, 7, or 8;
s is 0, 1, 2, 3, 4, 5, 6, or 7;
the sum of q, r, and s is 1, 2, 3, 4, 5, 6, 7, or 8;
t is 0, 1, 2, 3, 4, or 5;
u is 1, 2, 3, 4, 5, or 6;
v is 1, 2, 3, 4, 5, or 6;
w is 0, 1, 2, 3, 4, or 5;
the sum of t, u, v, and w is 2, 3, 4, 5, 6, or 7; and
X¹, X² and X³ are independently -O-, -NH-, -NHC(O)-, or -C(O)NH-;
wherein 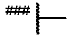 represents a covalent bond to the ring of Formula (L-c) or (L-c-i), and 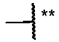 represents a covalent bond to the methylene group of Formula (I).

In another embodiment, L is a divalent linker of Formula (L-c) selected from the group consisting of:

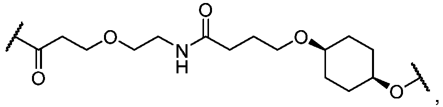

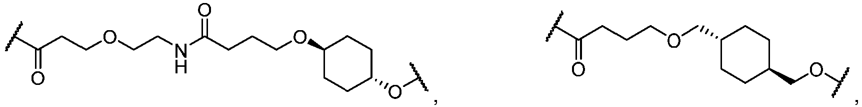

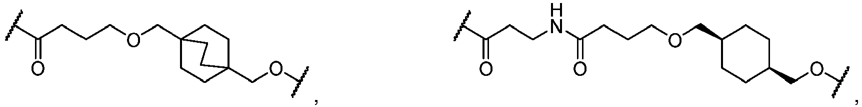

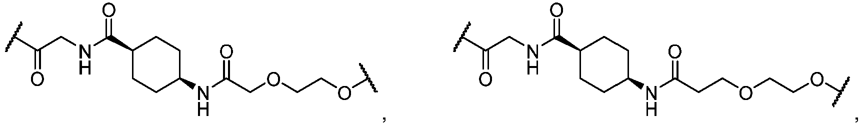

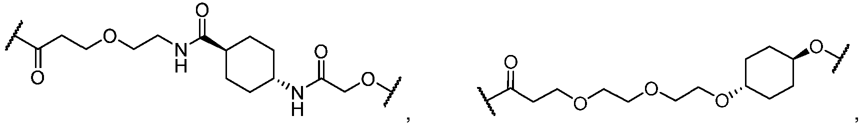

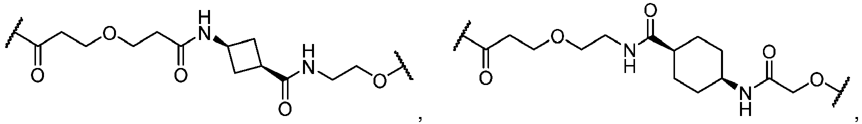

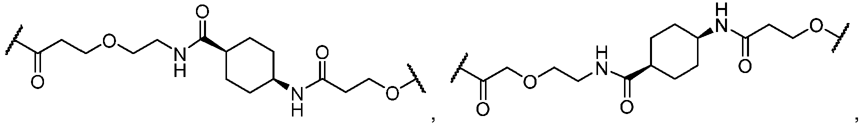

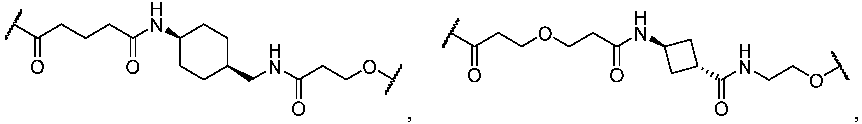

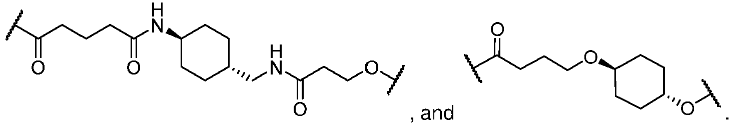

In another embodiment, L is a divalent linker of Formula (L-d): 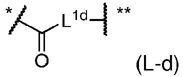 wherein:
L^{1d} is C₁₂₋₃₁ linear alkylene, wherein 1, 2, 3, 4, 5, 6, 7, 8, 9, 10, or 11 methylene units are replaced with -NH-, -O-, -C(O)NH-, -NHC(O)-, or -NHC(O)-NH-;
wherein 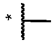 represents a covalent bond to the Y group of Formula (I), or when Y is a bond, a covalent bond to the T group of Formula (I), and 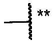 represents a covalent bond to the methylene group of Formula (I). In another embodiment, L^{1d} is a C₁₂, C₁₃, C₁₄, C₁₅, C₁₆, C₁₇, C₁₈, C₁₉, C₂₀, C₂₁, C₂₂, C₂₃, C₂₄, C₂₅, C₂₆, C₂₇, C₂₈, C₂₉, C₃₀, or C₃₁ linear alkylene, wherein 1, 2, 3, 4, 5, 6, 7, 8, 9, 10, or 11 methylene units are replaced with -NH-, -O-, -C(O)NH-, -NHC(O)-, or -NHC(O)-NH-. In another embodiment, L^{1d} is C₁₂₋₂₂ linear alkylene, for example, C₁₂, C₁₃, C₁₄, C₁₅, C₁₆, C₁₇, C₁₈, C₁₉, C₂₀, C₂₁, or C₂₂, wherein 1, 2, 3, 4, or 5 methylene units are replaced with -NH-, -O-, -C(O)NH-, -NHC(O)-, or -NHC(O)-NH-.

In another embodiment, L^{1d} of Formula (L-d) is selected from 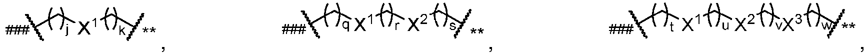 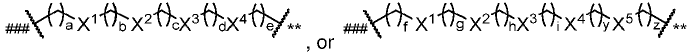 wherein:
j is 1, 2, 3, 4, 5, 6, 7, 8, 9, 10, 11, 12, 13, 14, 15, 16, 17, 18, 19, or 20;
k is 1, 2, 3, 4, 5, 6, 7, 8, 9, 10, 11, 12, 13, 14, 15, 16, 17, 18, 19, or 20;
the sum of j and k is 11, 12, 13, 14, 15, 16, 17, 18, 19, 20, or 21;
q is 1, 2, 3, 4, 5, 6, 7, 8, 9, 10, 11, 12, 13, 14, 15, 16, 17, 18, or 19;
r is 1, 2, 3, 4, 5, 6, 7, 8, 9, 10, 11, 12, 13, 14, 15, 16, 17, 18, or 19;
s is 0, 1, 2, 3, 4, 5, 6, 7, 8, 9, 10, 11, 12, 13, 14, 15, 16, 17, or 18;
the sum of q, r, and s is 10, 11, 12, 13, 14, 15, 16, 17, 18, 19, or 20;
t is 1, 2, 3, 4, 5, 6, 7, 8, 9, 10, 11, 12, 13, 14, 15, 16, or 17;
u is 1, 2, 3, 4, 5, 6, 7, 8, 9, 10, 11, 12, 13, 14, 15, 16, or 17;
v is 1, 2, 3, 4, 5, 6, 7, 8, 9, 10, 11, 12, 13, 14, 15, 16, or 17;
w is 0, 1, 2, 3, 4, 5, 6, 7, 8, 9, 10, 11, 12, 13, 14, 15, or 16;
the sum of t, u, v, and w is 9, 10, 11, 12, 13, 14, 15, 16, 17, 18, or 19;
a is 1, 2, 3, 4, 5, 6, 7, 8, 9, 10, 11, 12, 13, 14, or 15;
b is 1, 2, 3, 4, 5, 6, 7, 8, 9, 10, 11, 12, 13, 14, or 15;
c is 1, 2, 3, 4, 5, 6, 7, 8, 9, 10, 11, 12, 13, 14, or 15;
d is 1, 2, 3, 4, 5, 6, 7, 8, 9, 10, 11, 12, 13, 14, or 15;
e is 0, 1, 2, 3, 4, 5, 6, 7, 8, 9, 10, 11, 12, 13, or 14;
the sum of a, b, c, d, and e is 8, 9, 10, 11, 12, 13, 14, 15, 16, 17, or 18;
f is 1, 2, 3, 4, 5, 6, 7, 8, 9, 10, 11, 12, or 13;
g is 1, 2, 3, 4, 5, 6, 7, 8, 9, 10, 11, 12, or 13;
h is 1, 2, 3, 4, 5, 6, 7, 8, 9, 10, 11, 12, or 13;
i is 1, 2, 3, 4, 5, 6, 7, 8, 9, 10, 11, 12, or 13;
y is 1, 2, 3, 4, 5, 6, 7, 8, 9, 10, 11, 12, or 13;
z is 0, 1, 2, 3, 4, 5, 6, 7, 8, 9, 10, 11, or 12;
the sum of f, g, h, i, y, and z is 7, 8, 9, 10, 11, 12, 13, 14, 15, 16, or 17; and
X¹, X², X³, X⁴, and X⁵ are independently -NH-, -O-, -C(O)NH-, -NHC(O)-, or -NHC(O)-NH-;
wherein 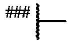 represents a covalent bond to the C(O) group of Formula (L-d), and 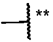 represents a covalent bond to the methylene group of Formula (I).

In another embodiment, L^{1d} of Formula (L-d) is 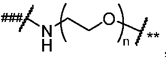 wherein n is 4, 5, 6, 7, 8, 9, or 10; wherein 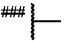 represents a covalent bond to the C(O) group of Formula (L-d), and 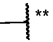 represents a covalent bond to the methylene group of Formula (I).

In another embodiment, L is a divalent linker of Formula (L-d) selected from the group consisting of: 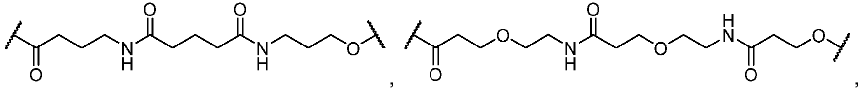 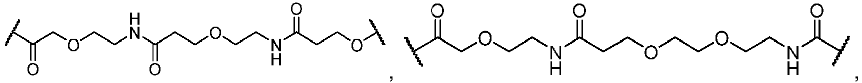 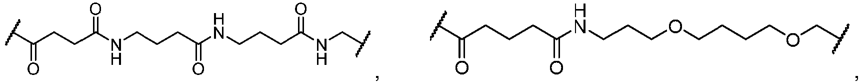 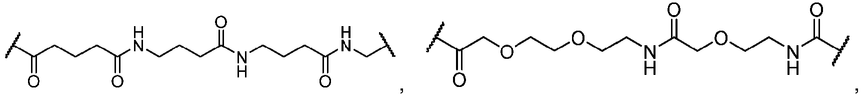 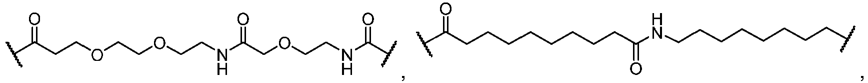 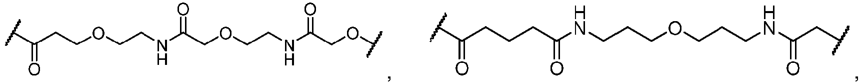 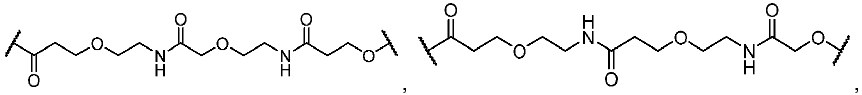 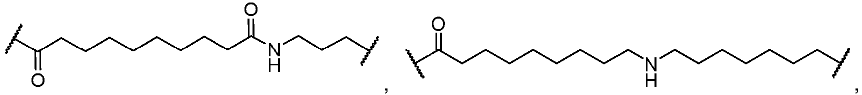 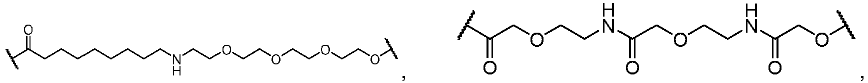 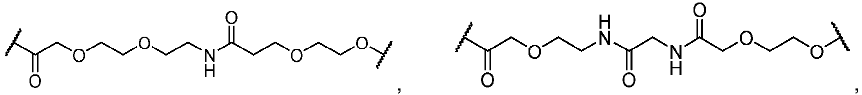 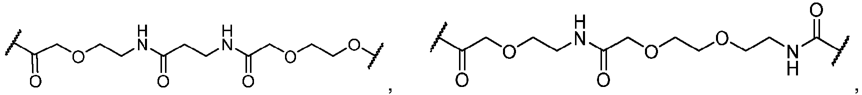 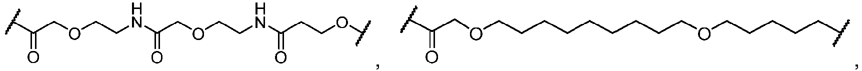 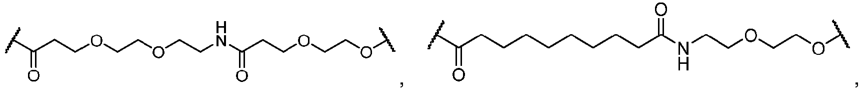 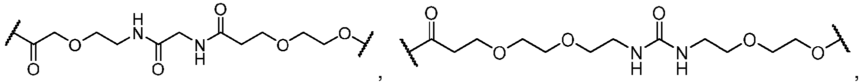 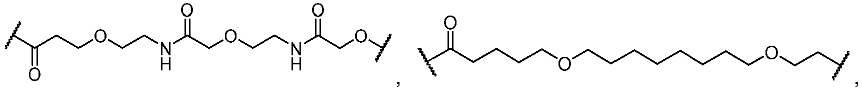 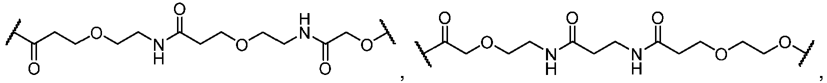 and

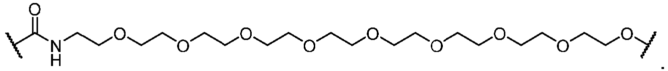

In another embodiment, L is a divalent linker of Formula (L-e): 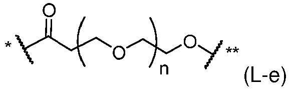 wherein:
n is an integer of 3 to 50;
wherein 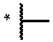 represents a covalent bond to the Y group of Formula (I), or when Y is a bond, a covalent bond to the T group of Formula (I), and 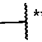 represents a covalent bond to the methylene group of Formula (I).

In another embodiment, n of Formula (L-e) is 3 to 25, 3 to 10, 3 to 8, 3 to 7, 3 to 5, or 3 to 4. In another embodiment, n of Formula (L-e) is 5 to 22, 7 to 15, or 9 to 13. In another embodiment, n of Formula (L-e) is 3, 4, 5, 7, 8, 11, 22, or 50.

In another embodiment, L is a divalent linker of Formula (L-f): 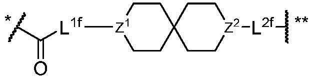 (L-f), or a stereoisomer thereof, wherein:
L^{1f} is a bond; C₁₋₆ linear alkylene, wherein 0, 1, or 2 methylene units are replaced with -O-, - NH-, or -C(O)-; or -(C₃₋₆ cycloalkylene)-NHC(O)-;
L^{2f} is a bond, -NHC(O)-, -C(O)NH-, or a C₁₋₆ linear alkylene, wherein 0, 1, or 2 methylene units are replaced with -O-; and
each of Z¹ and Z² is independently N or CH;
wherein 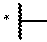 represents a covalent bond to the Y group of Formula (I), or when Y is a bond, a covalent bond to the T group of Formula (I), and 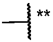 represents a covalent bond to the methylene group of Formula (I).

In another embodiment, L^{1f} of Formula (L-f) is selected from 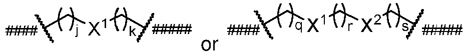 wherein:
j is 1, 2, 3, 4, or 5;
k is 0, 1, 2, 3, or 4;
the sum of j and k is 1, 2, 3, 4, or 5;
q is 1, 2, or 3;
r is 1, 2, or 3;
s is 0, 1, 2;
the sum of q, r, and s is 2, 3, or 4; and
X¹ and X² are independently -O-, -NH-, or -C(O)-; or -(C₃₋₆ cycloalkylene)-NHC(O)-;
wherein 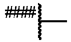 represents a covalent bond to the C(O) group of Formula (L-f), and 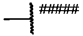 represents a covalent bond to the ring of Formula (L-f).

In another embodiment, L^{2f} of Formula (L-f) is selected from 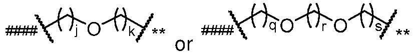 wherein:
j is 1, 2, 3, 4, or 5;
k is 0, 1, 2, 3, or 4;
the sum of j and k is 1, 2, 3, 4, or 5;
q is 1, 2, or 3;
r is 1, 2, or 3;
s is 0, 1, 2; and
the sum of q, r, and s is 2, 3, or 4;
wherein 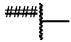 represents a covalent bond to the ring of Formula (L-f), and 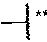 represents a covalent bond to the methylene group of Formula (I).

In another embodiment, L is a divalent linker of Formula (L-f) selected from the group consisting of: 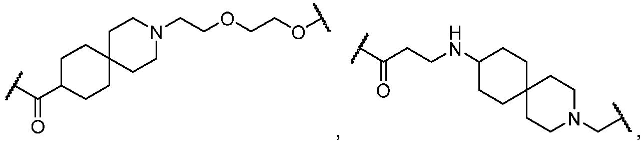 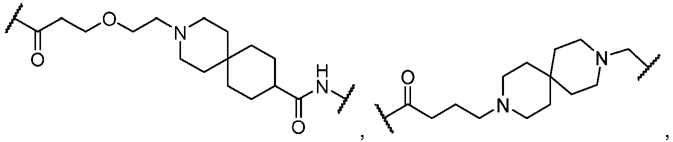 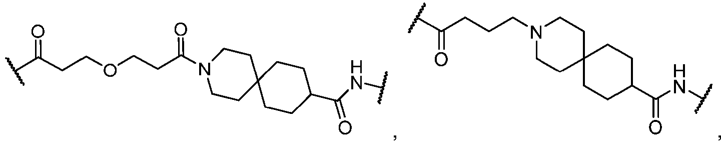 and

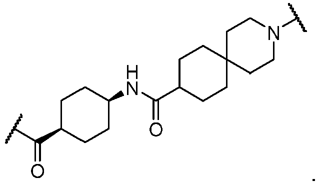

In another embodiment, L is a divalent linker of Formula (L-g): 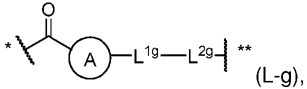 wherein:
Ring A is a 5 to 6 membered heteroarylene having 1 or 2 nitrogen ring atoms;
L^{1g} is a bond, -CH₂-, -NH-, or -O-; and
L^{2g} is 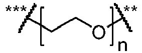 wherein n is 1, 2, 3, 4, or 5, and 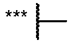 represents a covalent bond to L^{1g};
wherein 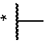 represents a covalent bond to the Y group of Formula (I), or when Y is a bond, a covalent bond to the T group of Formula (I), and 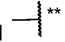 represents a covalent bond to the methylene group of Formula (I).

In another embodiment, L is a divalent linker of Formula (L-g-i): 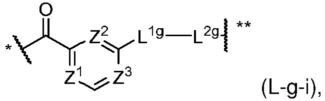 wherein:
L^{1g} is a bond, -CH₂-, -NH-, or -O-;
L^{2g} is 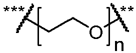 wherein n is 1, 2, 3, 4, or 5, and 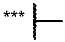 represents a covalent bond to L^{1g};
Z¹, Z², and Z³ are each independently selected from N or CH, provided that one or two of Z¹, Z², and Z³ is N;
wherein 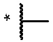 represents a covalent bond to the Y group of Formula (I), or when Y is a bond, a covalent bond to the T group of Formula (I), and 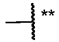 represents a covalent bond to the methylene group of Formula (I).

In another embodiment, L is a divalent linker of Formula (L-g) selected from the group consisting of: 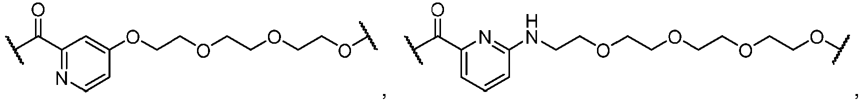 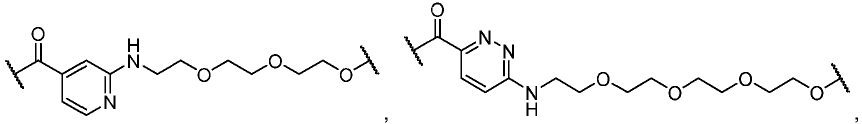 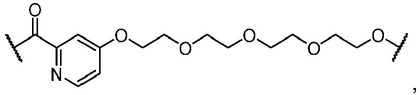 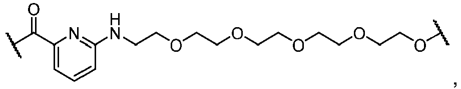 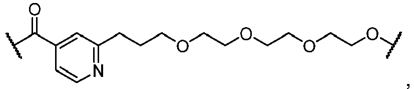 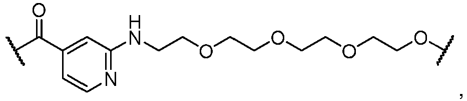 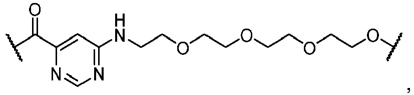 and

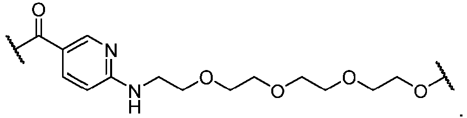

In another embodiment, L is a divalent linker of Formula (L-h): 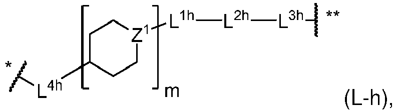 or a stereoisomer thereof, wherein:
each Z¹ is independently N or CH;
L^{1h} is a bond, -C(O)-, -C(O)-NH-, or -NHC(O)-;
L^{2h} is C₂₋₁₀ linear alkylene or 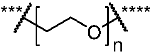 , wherein n is 1, 2, 3, or 4, and 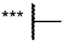 represents a covalent bond to L^{1h} and 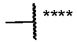 represents a covalent bond to L^{3h};
L^{3h} is a bond, -C(O)CH₂-, -O-(C₃₋₆ cycloalkylene)-O-, or -C(O)NH(CH₂)₃OCH₂-;
L^{4h} is a bond, -C(O)-, -CH₂C(O)-, or -C(O)CH₂-; and
m is 1, 2, or 3;
wherein 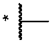 represents a covalent bond to the Y group of Formula (I), or when Y is a bond, a covalent bond to the T group of Formula (I), and 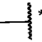 represents a covalent bond to the methylene group of Formula (I).

In another embodiment, L is a divalent linker of Formula (L-h) selected from the group consisting of: 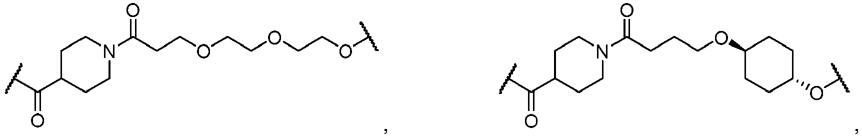 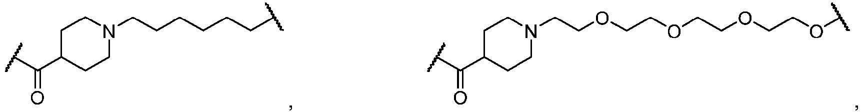 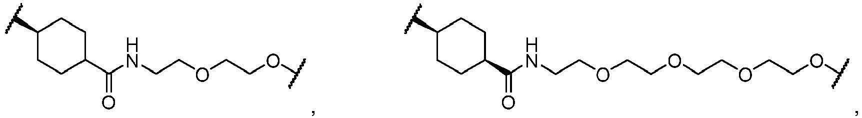 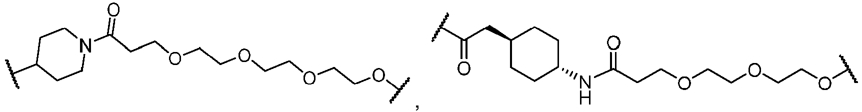 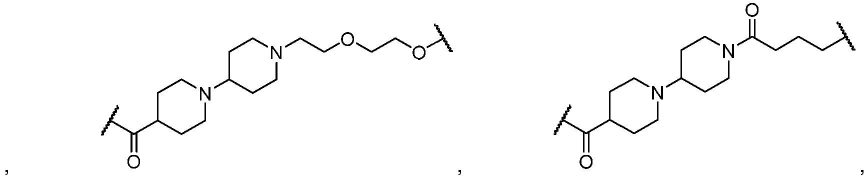 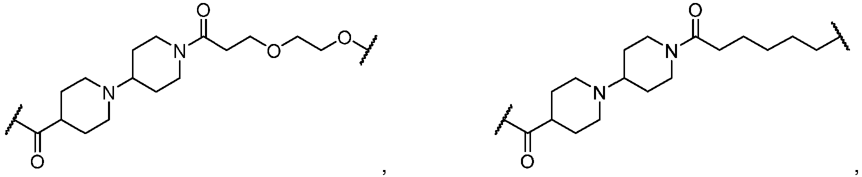 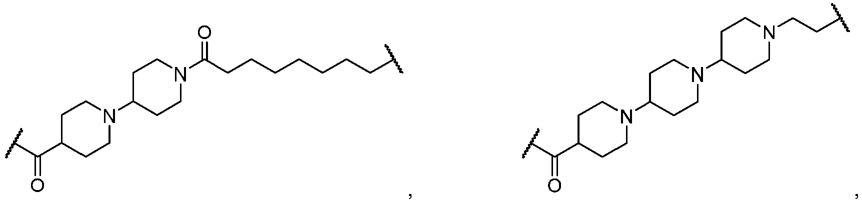 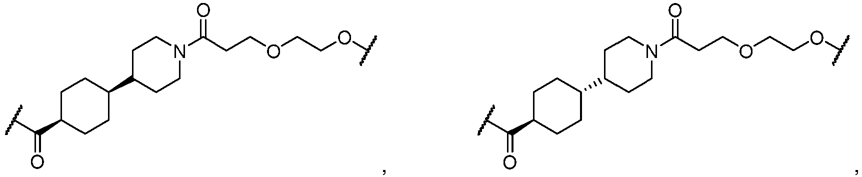 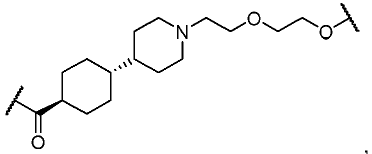 and

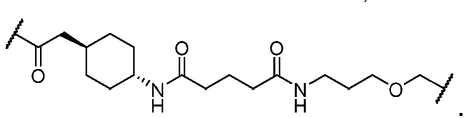

In another embodiment, L is a divalent linker of Formula (L-i): 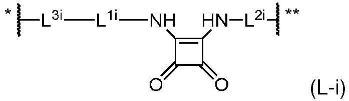 wherein:
L¹ⁱ is a bond, C₁₋₁₂ linear alkylene, or 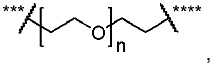 wherein n is 1, 2, 3, 4, or 5, and 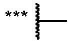 represents a covalent bond to L³ⁱ and 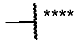 represents a covalent bond to NH;
L²ⁱ is a bond, C₁₋₁₂ linear alkylene, or 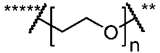 , wherein n is 1, 2, 3, 4, or 5, and 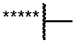 represents a covalent bond to HN; and
L³ⁱ is a bond or -C(O)-;
wherein 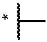 represents a covalent bond to the Y group of Formula (I), or when Y is a bond, a covalent bond to the T group of Formula (I), and 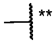 represents a covalent bond to the methylene group of Formula (I).

In another embodiment, L is a divalent linker of Formula (L-i) selected from the group consisting of: 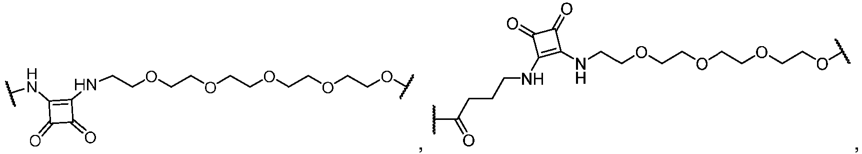 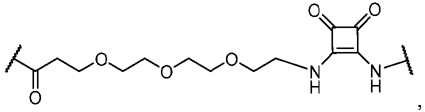 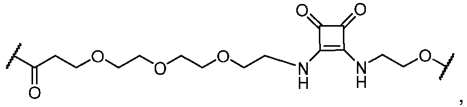 and

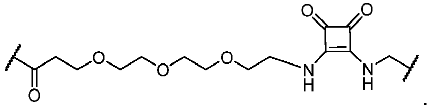

In another embodiment, L is a divalent linker of Formula (L-j): 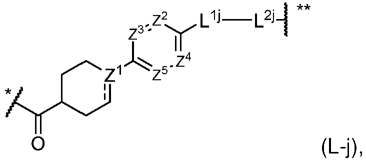 or a stereoisomer thereof, wherein:
Z¹ is C, CH, or N;
each of Z², Z³, Z⁴ and Z⁵ is independently CH or N, provided that no more than two of Z², Z³, Z⁴ and Z⁵ are N;
L^{1j} is -NH-, -C(O)NH-, -NHC(O)-, or -O-;
L^{2j} is C₁₋₆ linear alkylene or 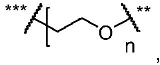 wherein n is 1 or 2, and 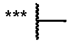 represents a covalent bond to L^{1j}; and 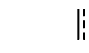 represents a single bond or a double bond;
wherein 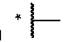 represents a covalent bond to the Y group of Formula (I), or when Y is a bond, a covalent bond to the T group of Formula (I), and 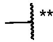 represents a covalent bond to the methylene group of Formula (I).

In another embodiment, L is a divalent linker of Formula (L-j) selected from the group consisting of:

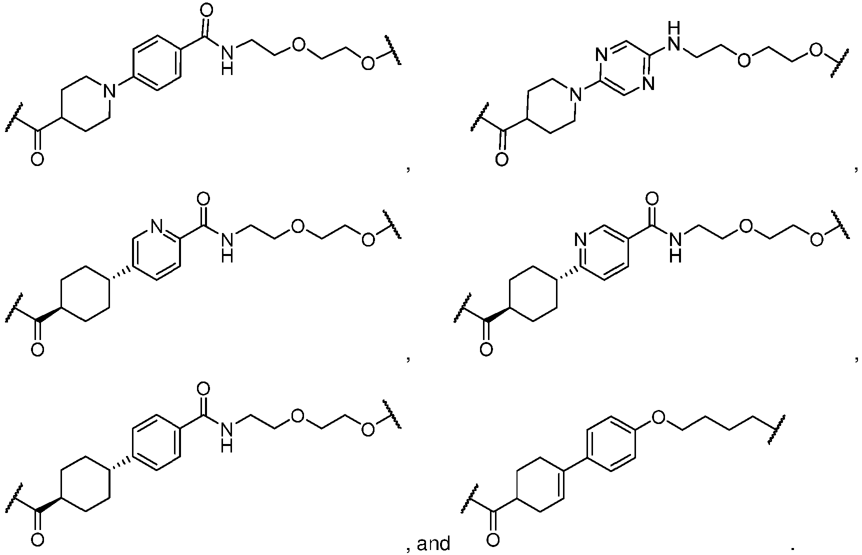

In another embodiment, L is a divalent linker of Formula (L-k): 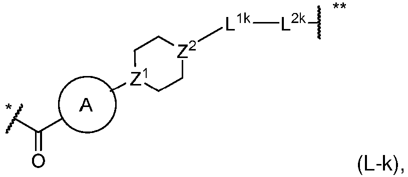 or a stereoisomer thereof, wherein:
Ring A is phenyl or a 5 or 6 membered heteroarylene having 1 or 2 nitrogen ring atoms;
each of Z¹ and Z² is independently CH or N;
L^{1k} is a bond, -C(O)-, -C(O)NH- or -NHC(O)-; and
L^{2k} is a C₃₋₈ straight chain alkylene or 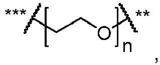 wherein n is 1, 2, or 3, and 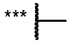 **represents** a covalent bond to L^{1k};
wherein 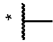 represents a covalent bond to the Y group of Formula (I), or when Y is a bond, a covalent bond to the T group of Formula (I), and 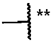 represents a covalent bond to the methylene group of Formula (I).

In another embodiment, L is a divalent linker of Formula (L-k) selected from the group consisting of:

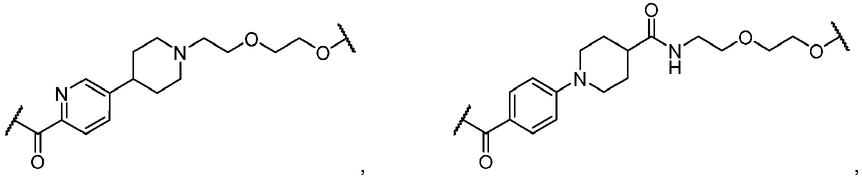

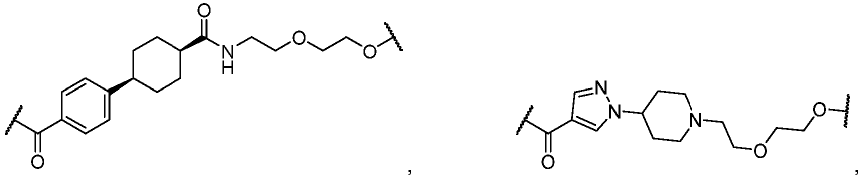

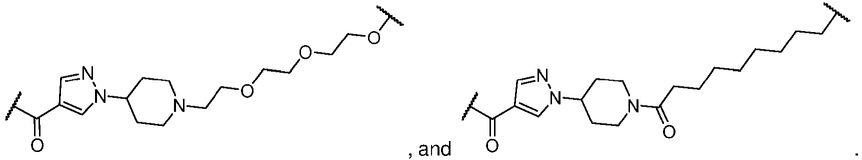

In another embodiment, L is a divalent linker of Formula (L-m): 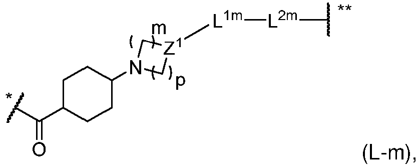 or a stereoisomer thereof, wherein:
Z¹ is CH or N;
m is 1 or 2;
p is 1 or 2;
0, 1, or 2 hydrogen atoms of 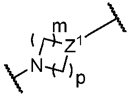 are replaced with F;
L^{1m} is a bond, -C(O)-, -C(O)NH-, -NHC(O)-, -S(O)₂NH- or -NHS(O)₂-; and
L^{2m} is C₃₋₆ linear alkylene, C₃₋₆ cycloalkylene, or 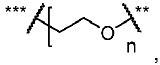 wherein n is 1 or 2, and 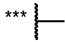 represents a covalent bond to L^{1m};
wherein 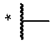 represents a covalent bond to the Y group of Formula (I), or when Y is a bond, a covalent bond to the T group of Formula (I), and 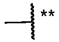 represents a covalent bond to the methylene group of Formula (I).

In another embodiment, L is a divalent linker of Formula (L-m) selected from the group consisting of: 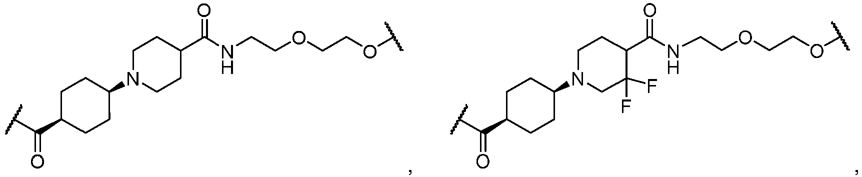 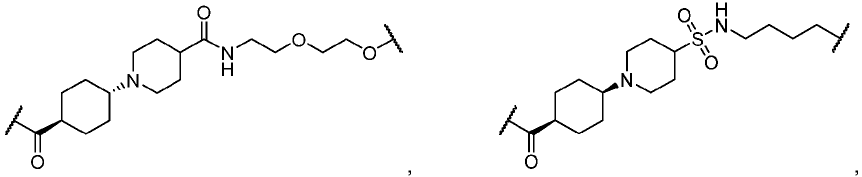 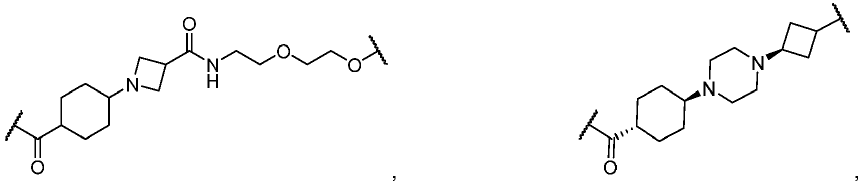 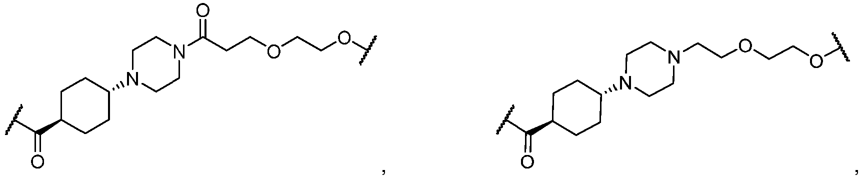 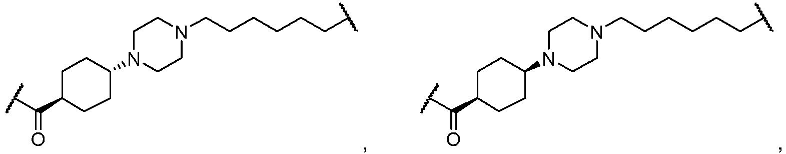 and

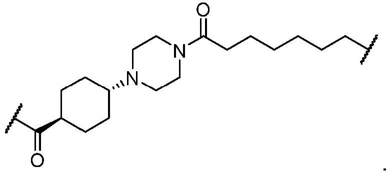

In another embodiment, L is a divalent linker of Formula (L-n-i): 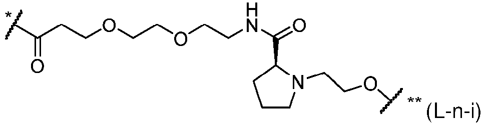 wherein 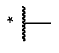 represents a covalent bond to the Y group of Formula (I), or when Y is a bond, a covalent bond to the T group of Formula (I), and 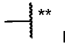 represents a covalent bond to the methylene group of Formula (I).

In another embodiment, L is a divalent linker of Formula (L-n-ii): 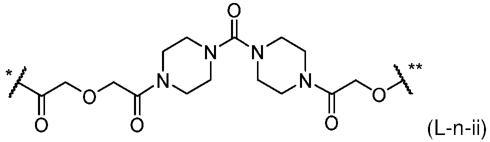 wherein 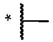 represents a covalent bond to the Y group of Formula (I), or when Y is a bond, a covalent bond to the T group of Formula (I), and 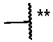 represents a covalent bond to the methylene group of Formula (I).

In another embodiment, L is a divalent linker of Formula (L-n-iii): 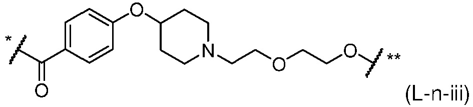 wherein 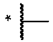 represents a covalent bond to the Y group of Formula (I), or when Y is a bond, a covalent bond to the T group of Formula (I), and 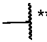 represents a covalent bond to the methylene group of Formula (I).

In another embodiment, L is a divalent linker of Formula (L-n-iv): 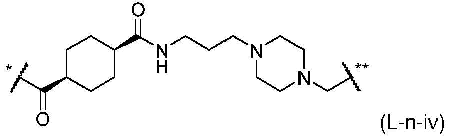 wherein 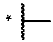 represents a covalent bond to the Y group of Formula (I), or when Y is a bond, a covalent bond to the T group of Formula (I), and 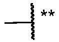 represents a covalent bond to the methylene group of Formula (I).

In one embodiment of the disclosure, Y is selected from a bond; -NH-; -(C₁₋₁₂ alkylene)-, wherein 1, 2, or 3 methylene units are replaced with -O-, -NH-, -C(O)-, -NHC(O)-, -C(O)NH-, -(C₃₋₆ cycloalkylene)-, -(C₃₋₆ cycloalkenylene)-, 3- to 6-membered heterocycloalkylene, arylene, or heteroarylene; or -(C₂₋₁₂ alkenylene)-, wherein 1, 2, or 3 methylene units are replaced with -O-, -NH-, -C(O)-, -NHC(O)-, -C(O)NH-, -(C₃₋₆ cycloalkylene)-, -(C₃₋₆ cycloalkenylene)-, 3- to 6-membered heterocycloalkylene, arylene, or heteroarylene.

In another embodiment, Y is selected from a bond; -NH-; -(C₁₋₆ alkylene)-O-; -(C₂₋₆ alkenylene)-O-; -(C₁₋₆ alkylene)-C(O)-; -(C₂₋₆ alkenylene)-C(O)-; phenylene; piperidinylene; -(C₁₋₆ alkylene)-O-phenylene-; -(C₂₋₆ alkenylene)-O-piperidinylene; -(C₁₋₅ alkylene)-NH-, wherein 0, 1, or 2 methylene units are replaced with -O-; -NH-(C₁₋₅ alkylene)-**NH-;** -(C₃₋₆ cycloalkylene)-NH-; -(C₃₋₆ cycloalkenylene)-NH-; or 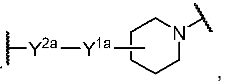 wherein Y^{1a} is a bond, -O-, -NH-, -NHC(O)-, -C(O)NH-, or C₁₋₃ alkylene; and Y^{2a} is a bond, -O-, -NH-, -NHC(O)-, -C(O)NH-, or C₁₋₃ alkylene. In another embodiment, Y is -NH-.

In another embodiment, Y is selected from the group consisting of:

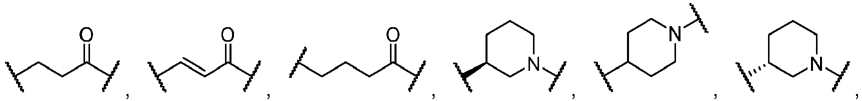

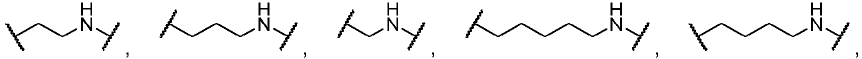

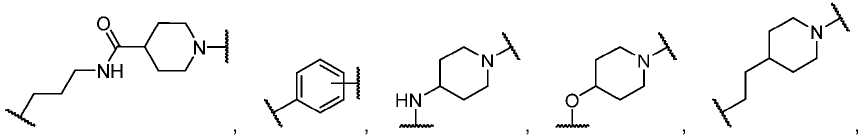

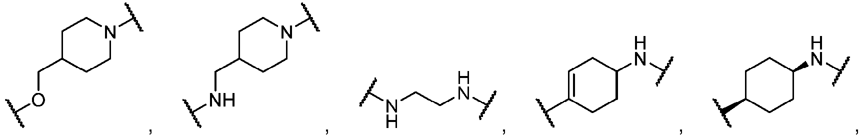

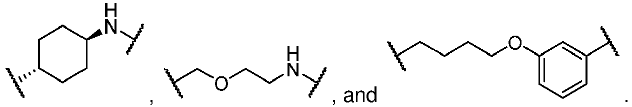

In another embodiment, R¹ is methyl, ethyl, 1-propyl, 2-propyl, 1-butyl, 2-butyl, or t-butyl. In another embodiment, R¹ is methyl. In another embodiment, R¹ is cyclopropyl, cyclobutyl, cyclopentyl, or cyclohexyl.

In another embodiment, the compound of Formula (I) is selected from a compound as listed in Table 1:

**Table 1**

| Ex. No. | Structure |
|---|---|
| 1 | 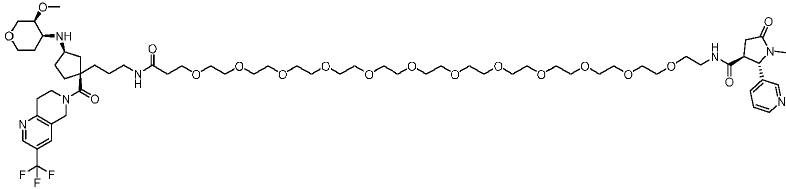 |
| 2 | 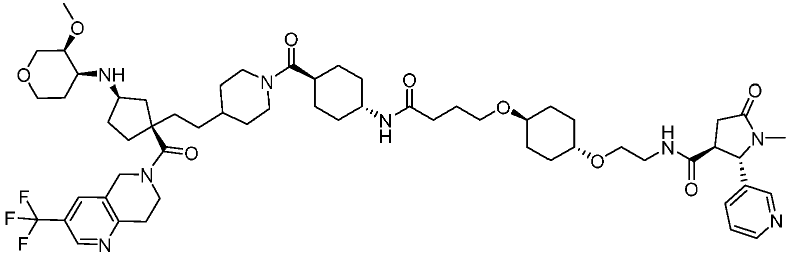 |
| 3 | 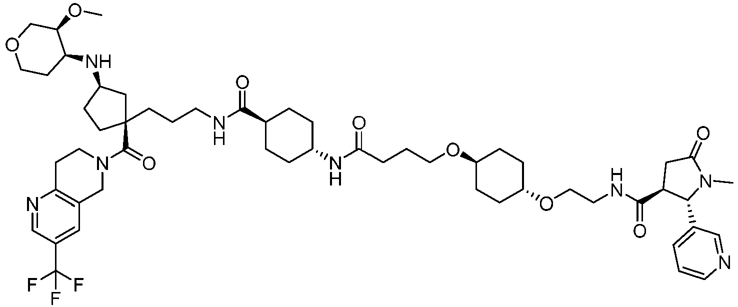 |
| 4 | 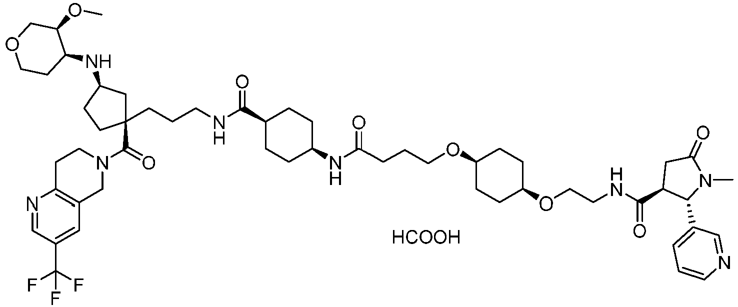 |
| 5 | 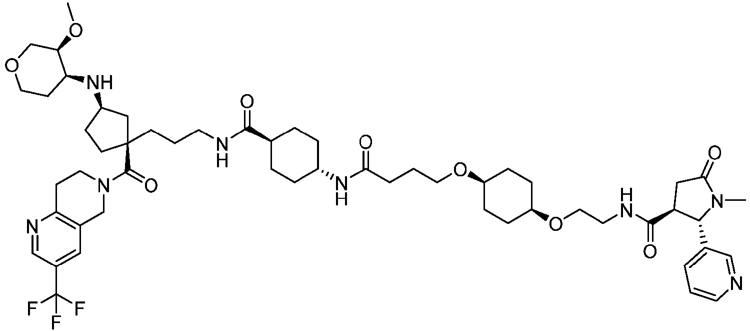 |
| 6 | 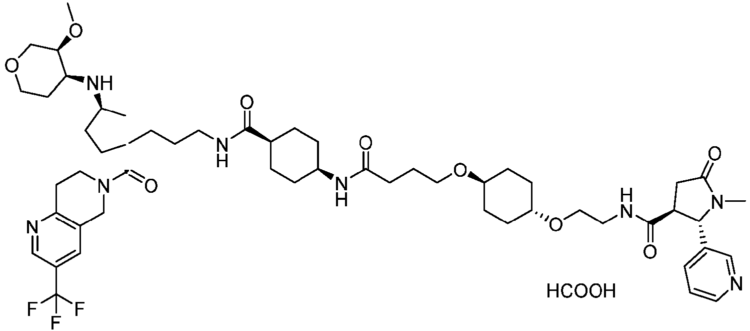 |
| 7 | 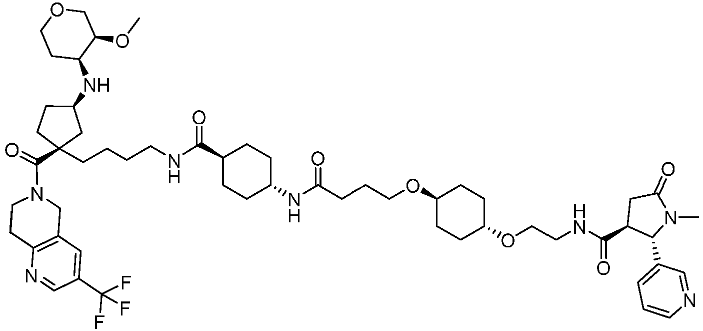 |
| 8 | 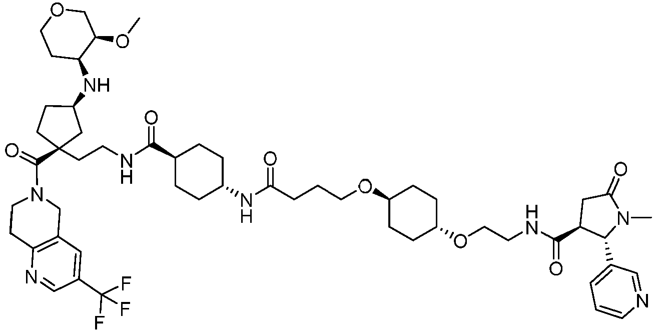 |
| 9 | 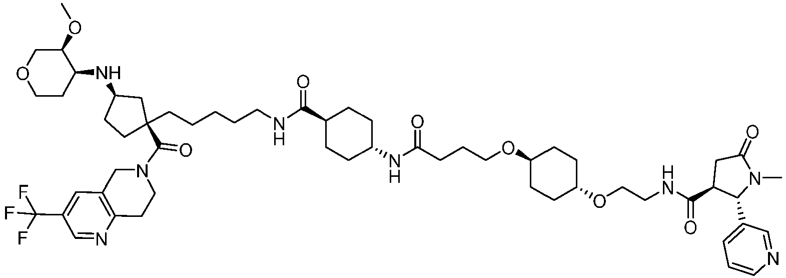 |
| 10 | 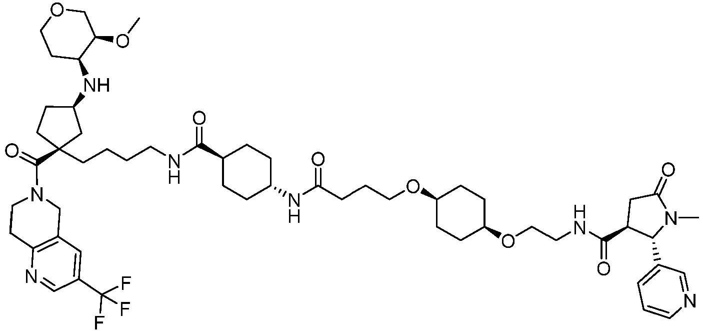 |
| 11 | 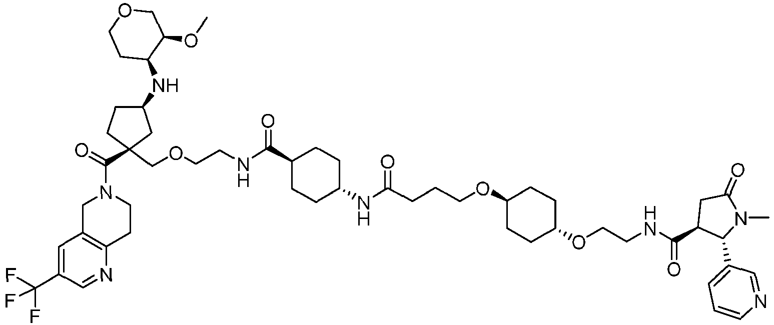 |
| 12 | 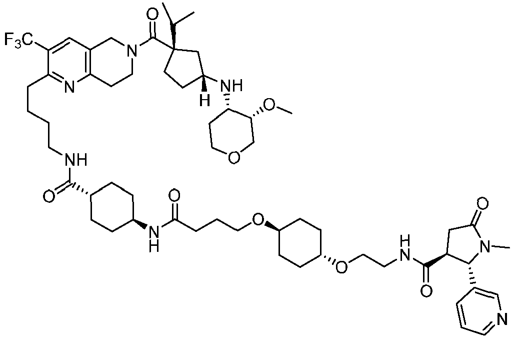 |
| 13 | 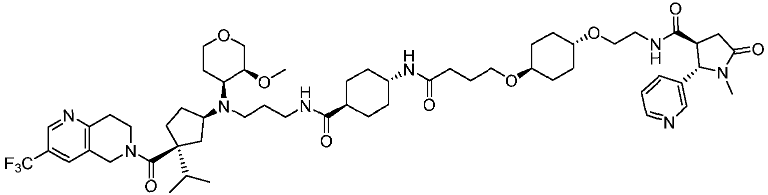 |
| 14 | 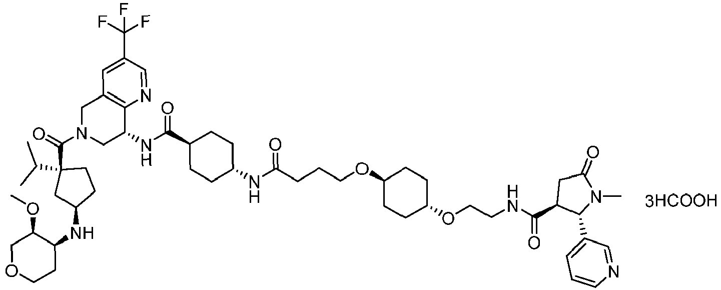 |
| 15 | 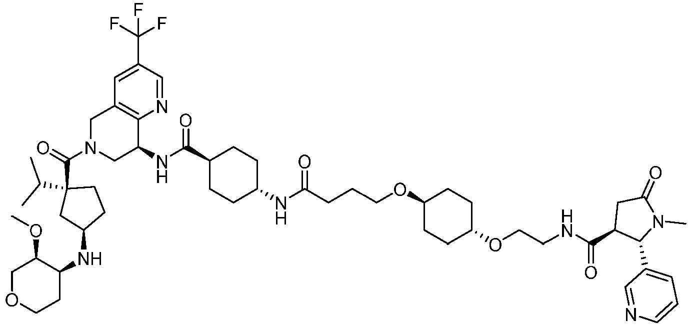 |
| 16 | 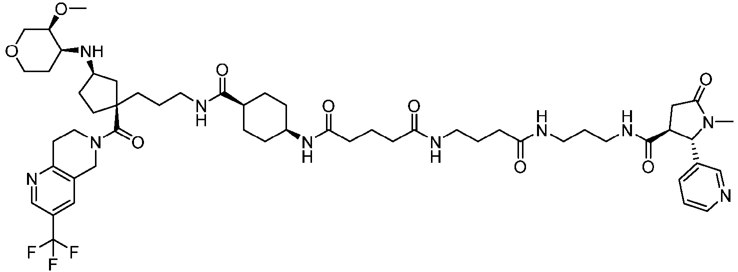 |
| 17 | 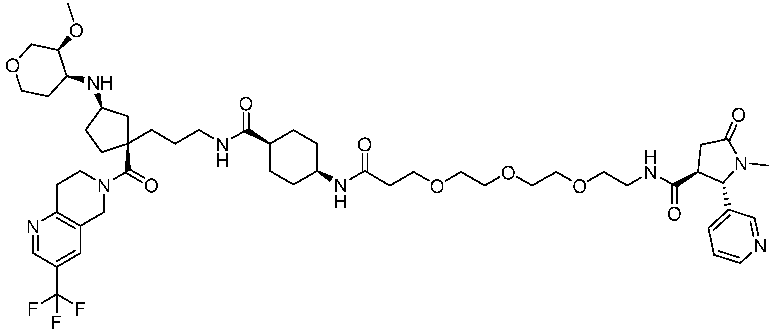 |
| 18 | 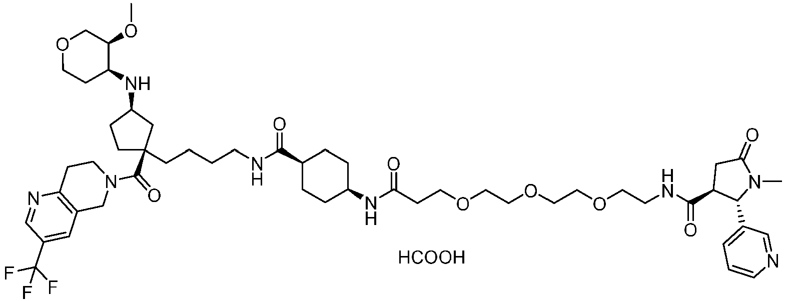 |
| 19 | 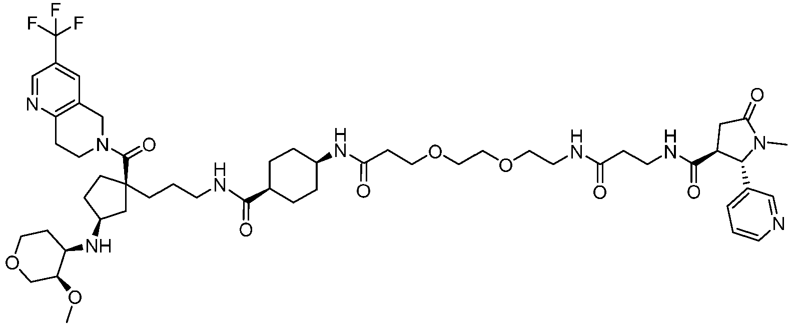 |
| 20 | 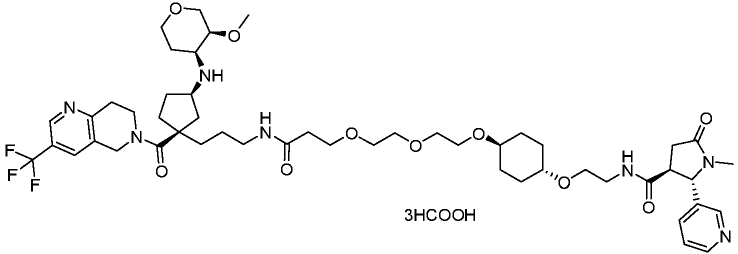 |
| 21 | 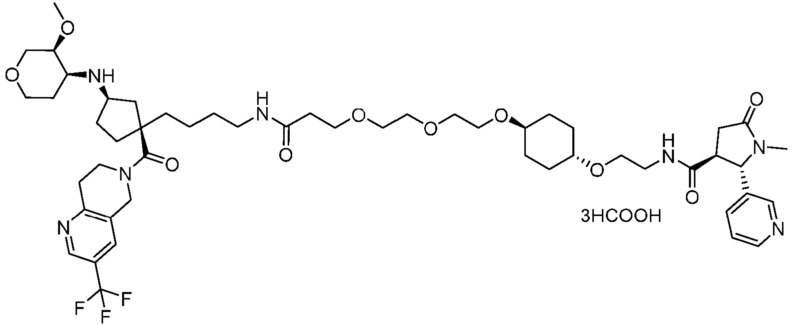 |
| 22 | 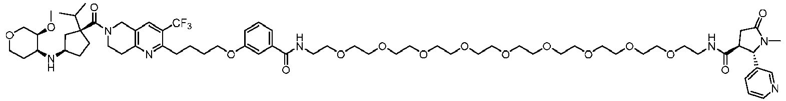 |
| 23 | 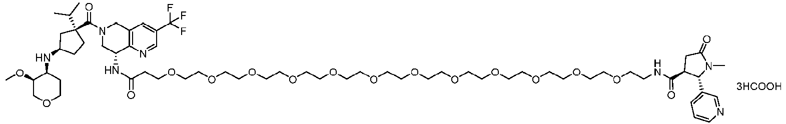 |
| 24 | 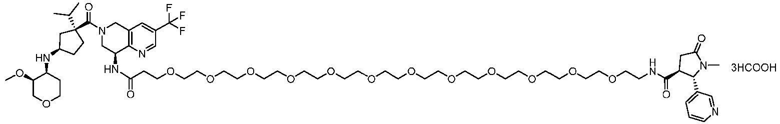 |

### Definitions

As used herein and in the claims, the singular forms "a" and "the" include plural reference unless the context clearly dictates otherwise.

As used herein and in the claims , the term "comprising" encompasses "including" or "consisting" e.g. a composition "comprising" X may consist exclusively of X or may include something additional, e.g., X + Y.

The term "consisting essentially of" limits the scope of the feature to the specified materials or steps and those that do not materially affect the basic characteristic(s) of the claimed feature.

The term "consisting of" excludes the presence of any additional component(s).

The term "pathogenic cells" includes a cell subset that causes or is capable of causing disease. Examples of pathogenic cells include, but are not limited to, pathogenic immune cells, cancer or tumor cells, and stromal cells. A pathogenic cell can also be a pathogenic agent capable of causing an infection, such as a virus or a bacterial cell.

The term "pathogenic immune cells" includes a particular immune cell subset that causes or is capable of causing disease. These cellular subsets are resident cells or are recruited to particular locations and secrete cytokines, chemokines and other mediators and contribute to the persistence and progression of disease such as cancer in the case of a tumor microenvironment or chronic inflammation of the lung in the case of asthma. Examples of pathogenic immune cells include, but are not limited to myeloid-derived suppressor cells (MDSCs), T regulatory cells (Tregs), neutrophils, macrophages, B regulatory cells (Bregs), CD8 regulatory cells, (CD8regs), and exhausted T cells.

The term "pharmaceutical composition" refers to a formulation of a compound of the invention and a medium generally accepted in the art for the delivery of the biologically active compound to mammals, e.g., humans. Such a medium includes all pharmaceutically acceptable carriers, diluents or excipients therefor.

The terms "effective amount" and "therapeutically effective amount" refer to an amount of a compound, or antibody, or antigen-binding portion thereof, according to the invention, which when administered to a patient in need thereof, is sufficient to effect treatment for disease-states, conditions, or disorders for which the compounds have utility. Such an amount would be sufficient to elicit the biological or medical response of a tissue system, or patient that is sought by a researcher or clinician. The amount of a compound according to the invention which constitutes a therapeutically effective amount will vary depending on such factors as the compound and its biological activity, the composition used for administration, the time of administration, the route of administration, the rate of excretion of the compound, the duration of the treatment, the type of disease-state or disorder being treated and its severity, drugs used in combination with or coincidentally with the compounds of the invention, and the age, body weight, general health, sex and diet of the patient. Such a therapeutically effective amount can be determined routinely by one of ordinary skill in the art having regard to their own knowledge, the state of the art, and this disclosure.

The term "alkyl" represents a saturated, linear or branched hydrocarbon moiety having the specified number of carbon atoms. The term "C₁₋₃ alkyl" refers to an unsubstituted alkyl moiety containing 1, 2 or 3 carbon atoms; exemplary alkyls include methyl, ethyl and propyl.

The term "alkylene" represents a saturated, linear or branched hydrocarbon moiety having the specified number of carbon atoms, with two points of attachment. The two points of attachment can be from the same or different carbon atoms. The term "C₁₋₃ alkylene" refers to an unsubstituted alkyl moiety containing 1, 2 or 3 carbon atoms with two points of attachment; exemplary C₁₋₃ alkylene groups include methylene, ethylene and propylene.

The term "alkenyl" represents an unsaturated, linear or branched hydrocarbon moiety having the specified number of carbon atoms. The term "C₂₋₆ alkenyl" refers to an unsubstituted alkenyl moiety containing 2, 3, 4, 5, or 6 carbon atoms; exemplary alkenyls include propenyl, butenyl, pentenyl and hexenyl.

The term "alkenylene" represents an unsaturated, linear or branched hydrocarbon moiety having the specified number of carbon atoms, with two points of attachment. The two points of attachment can be from the same or different carbon atoms. The term "C₂₋₆ alkenylene" refers to an unsubstituted alkenyl moiety containing 2, 3, 4, 5, or 6 carbon atoms with two points of attachment; exemplary C₂₋₆ alkenylene groups include propenylene, butenylene, pentenylene and hexenylene.

The term "cycloalkyl" represents a saturated cyclic hydrocarbon moiety having the specified number of carbon atoms. The term "C₃₋₆ cycloalkyl" refers to an unsubstituted cycloalkyl moiety containing 3, 4, 5 or 6 carbon atoms; exemplary cycloalkyls include cyclopropyl, cyclobutyl, cyclopentyl, and cyclohexyl.

The term "cycloalkylene" represents a saturated cyclic hydrocarbon moiety having the specified number of carbon atoms, with two points of attachment. The two points of attachment can be from the same or different carbon atoms. The term "C₄₋₆ cycloalkylene" refers to an unsubstituted cycloalkylene moiety containing 4, 5, or 6 carbon atoms with two points of attachment. Exemplary cycloalkylene groups include cyclobutane-1,3-diyl, cyclopentane-1,3-diyl, cyclohexane-1,3-diyl, or cyclohexane-1,4-diyl.

The term "cycloalkenylene" represents an unsaturated cyclic hydrocarbon moiety having the specified number of carbon atoms, with two points of attachment. The two points of attachment can be from the same or different carbon atoms. The term "C₃₋₆ cycloalkenylene" refers to an unsubstituted cycloalkenylene moiety containing 3, 4, 5, or 6 carbon atoms with two points of attachment.

The term "heterocycloalkylene" refers to a saturated cyclic hydrocarbon moiety containing 1 or 2 heteroatoms independently selected from oxygen, sulphur or nitrogen atoms, with two points of attachment. The two points of attachment can be from the same or different carbon atoms. The term "3- to 6-membered heterocycloalkylene" refers to a 3- to 6-membered saturated cyclic moiety containing 2, 3, 4 or 5 carbon atoms in addition to 1 or 2 oxygen, sulphur or nitrogen atoms, with two points of attachment. Suitably, the 3- to 6-membered heterocycloalkylene group contains 1 oxygen or nitrogen atom. Suitably such group contains 3 carbon atoms and 1 oxygen or nitrogen atom, such as azetidindiyl or oxetandiyl. Suitably such group contains 4 or 5 carbon atoms and 1 oxygen or nitrogen atom, such as tetrahydrofurandiyl, tetrahydropyrandiyl, pyrrolidindiyl or piperidindiyl.

The term "bridged bicyclic cycloalkylene" refers to a saturated bicyclic hydrocarbon moiety having at least one bridge, with two points of attachment. A "bridge" is an unbranched chain of atoms or an atom or a valence bond connecting two bridgeheads, where a "bridgehead" is any skeletal atom of the ring system which is bonded to three or more skeletal atoms (excluding hydrogen). The two points of attachment can be from the same or different carbon atoms. The term "C₇₋₉ bridged bicyclic cycloalkylene" refers to an unsubstituted bridged bicyclic cycloalkylene moiety containing 7, 8, or 9 carbon atoms with two points of attachment.

The term "arylene" refers to a monocyclic or bicyclic ring system wherein at least one ring in the system is aromatic, with two points of attachment. Exemplary arylene groups include phenylene, biphenylene, naphthylene, and anthracylene.

The term "heteroarylene" refers to a monocyclic or bicyclic ring system wherein at least one ring in the system is aromatic, and having, in addition to carbon atoms, from one to five heteroatoms independently selected from oxygen, sulphur or nitrogen atoms, with two points of attachment. The term "5- to 6-membered heteroarylene" refers to a 5- to 6-membered cyclic aromatic moiety containing 2, 3, 4 or 5 carbon atoms in addition to 1, 2, or 3 heteroatoms independently selected from oxygen, sulphur or nitrogen atoms, with two points of attachment.

The skilled artisan will appreciate that salts, including pharmaceutically acceptable salts, of the compounds according to Formula (I) may be prepared. Indeed, in certain embodiments of the invention, salts including pharmaceutically-acceptable salts of the compounds according to Formula (I) may be preferred over the respective free or unsalted compound. Accordingly, the invention is further directed to salts, including pharmaceutically-acceptable salts, of the compounds according to Formula (I). The invention is further directed to free or unsalted compounds of Formula (I).

The salts, including pharmaceutically acceptable salts, of the compounds of the invention are readily prepared by those of skill in the art.

Representative pharmaceutically acceptable acid addition salts include, but are not limited to, 4-acetamidobenzoate, acetate, adipate, alginate, ascorbate, aspartate, benzenesulfonate (besylate), benzoate, bisulfate, bitartrate, butyrate, calcium edetate, camphorate, camphorsulfonate (camsylate), caprate (decanoate), caproate (hexanoate), caprylate (octanoate), cinnamate, citrate, cyclamate, digluconate, 2,5-dihydroxybenzoate, disuccinate, dodecylsulfate (estolate), edetate (ethylenediaminetetraacetate), estolate (lauryl sulfate), ethane-1,2-disulfonate (edisylate), ethanesulfonate (esylate), formate, fumarate, galactarate (mucate), gentisate (2,5-dihydroxybenzoate), glucoheptonate (gluceptate), gluconate, glucuronate, glutamate, glutarate, glycerophosphorate, glycolate, hexylresorcinate, hippurate, hydrabamine (N,N'-di(dehydroabietyl)-ethylenediamine), hydrobromide, hydrochloride, hydroiodide, hydroxynaphthoate, isobutyrate, lactate, lactobionate, laurate, malate, maleate, malonate, mandelate, methanesulfonate (mesylate), methylsulfate, mucate, naphthalene-1,5-disulfonate (napadisylate), naphthalene-2-sulfonate (napsylate), nicotinate, nitrate, oleate, palmitate, p-aminobenzenesulfonate, p-aminosalicyclate, pamoate (embonate), pantothenate, pectinate, persulfate, phenylacetate, phenylethylbarbiturate, phosphate, polygalacturonate, propionate, p-toluenesulfonate (tosylate), pyroglutamate, pyruvate, salicylate, sebacate, stearate, subacetate, succinate, sulfamate, sulfate, tannate, tartrate, teoclate (8-chlorotheophyllinate), thiocyanate, triethiodide, trifluoroacetate, undecanoate, undecylenate, and valerate.

Representative pharmaceutically acceptable base addition salts include, but are not limited to, aluminium, 2-amino-2-(hydroxymethyl)-1,3-propanediol (TRIS, tromethamine), arginine, benethamine (N-benzylphenethylamine), benzathine (N,N'-dibenzylethylenediamine), b/s-(2-hydroxyethyl)amine, bismuth, calcium, chloroprocaine, choline, clemizole (1-p chlorobenzyl-2-pyrrolidine-1'-ylmethylbenzimidazole), cyclohexylamine, dibenzylethylenediamine, diethylamine, diethyltriamine, dimethylamine, dimethylethanolamine, dopamine, ethanolamine, ethylenediamine, L-histidine, iron, isoquinoline, lepidine, lithium, lysine, magnesium, meglumine (N-methylglucamine), piperazine, piperidine, potassium, procaine, quinine, quinoline, sodium, strontium, t-butylamine, and zinc.

The compounds according to Formula (I) may contain one or more asymmetric centers (also referred to as a chiral center) and may, therefore, exist as individual enantiomers, diastereomers, or other stereoisomeric forms, or as mixtures thereof. Chiral centers, such as chiral carbon atoms, may be present in a substituent such as an alkyl group. Where the stereochemistry of a chiral center present in a compound of Formula (I), or in any chemical structure illustrated herein, if not specified the structure is intended to encompass all individual stereoisomers and all mixtures thereof. Thus, compounds according to Formula (I) containing one or more chiral centers may be used as racemic mixtures, enantiomerically enriched mixtures, or as enantiomerically pure individual stereoisomers.

A mixture of stereoisomers in which the relative configuration of all of the stereocenters is known may be depicted using the symbol "&" together with an index number (e.g., "&1"). For example, a group of two stereogenic centers labeled with the symbol "&1" represents a mixture of two possible stereoisomers in which the two stereogenic centers have a relative configuration as depicted.

Divalent groups are groups having two points of attachment. For all divalent groups, unless otherwise specified, the orientation of the group is implied by the direction in which the formula or structure of the group is written.

Unless defined otherwise, all technical and scientific terms used herein have the same meaning as commonly understood by one of ordinary skill in the art to which this invention belongs. Although any compositions and methods similar or equivalent to those described herein can be used in the practice or testing of the methods of the disclosure, exemplary compositions and methods are described herein. Any of the aspects and embodiments of the disclosure described herein may also be combined. For example, the subject matter of any dependent or independent claim disclosed herein may be multiply combined (e.g., one or more recitations from each dependent claim may be combined into a single claim based on the independent claim on which they depend).

Ranges provided herein include all values within a particular range described and values about an endpoint for a particular range.

Concentrations described herein are determined at ambient temperature and pressure. This may be, for example, the temperature and pressure at room temperature or in a particular portion of a process stream. Preferably, concentrations are determined at a standard state of 25 ºC and 1 bar of pressure.

### CCR2 Target and CCR2-Binding Moieties

The compounds of Formula (I) as disclosed herein are heterobifunctional synthetic agents designed such that one terminus interacts with a cell surface CCR2 target, while the other terminus binds a specific antibody. More specifically, the ARM simultaneously binds the cell surface CCR2 target as well as the specific antibody. This ternary complex directs immune surveillance to CCR2-expressing tissue/cells and unites the mechanisms of antibody function with the dose-control of small molecules. This mechanism may include antibody dependent cellular cytotoxicity (ADCC), antibody dependent cellular phagocytosis (ADCP), or complement dependant cytotoxicity (CDC), and preferably includes ADCC. The same Fc receptor expressing immune cells that initiate destruction of the ARM/antibody tagged cells also participate in presentation of endogenous antigens for the potential for long term cellular immunity.

The compounds of Formula (I) as disclosed herein include a CCR2-binding moiety that is capable of binding CCR2 present on the surface of a cell. In one embodiment, the CCR2 is expressed on a pathogenic cell.

In a further embodiment, the pathogenic cell is a pathogenic immune cell, a tumor cell or cancer cell, or a stromal cell (including stromal cells present in a tumor microenvironment).

In a further embodiment, the CCR2 target is present on the surface of a pathogenic agent selected from a virus or a bacterial cell. Examples of a virus expressing cell surface targets include, but are not limited to, influenza.

In a further embodiment, the pathogenic immune cells are monocytes, myeloid derived suppressor cells (MDSC), such as monocytic MDSCs (mMDSCs) and polymorphonuclear MDSCs (PMN_MDSCs), T regulatory cells (Tregs), neutrophils (e.g., N2 neutrophils), macrophages (e.g., M2 macrophages), B regulatory cells (Bregs, memory B cells), plasma cells, CD8 cells (e.g., CD8 regulatory cells (CD8regs), memory CD8 cells, effector CD8 cells, naïve CD8 Tcells, TEMRA), exhausted T cells, eosinophils, basophils, mast cells, dendritic cells, natural killer (NK cells), innate lymphoid cells, NK T cells (NKT), or γδT cells.

In a further embodiment, the pathogenic immune cells are myeloid derived suppressor cells (MDSC), such as monocytic MDSCs (mMDSCs) and polymorphonuclear MDSCs (PMN_MDSCs), T regulatory cells (Tregs), neutrophils (e.g., N2 neutrophils), macrophages (e.g., M2 macrophages), B regulatory cells (Bregs), CD8 regulatory cells (CD8regs), exhausted T cells.

In a further embodiment, the pathogenic immune cells expressing CCR2 are myeloid derived suppressor cells (MDSCs). In a further embodiment, the pathogenic immune cells expressing CCR2 are selected from monocytic MDSCs (mMDSCs) and polymorphonuclear MDSCs (PMN_MDSCs).

In a further embodiment, the tumor cells or cancer cells are solid tumor cells.

In a further embodiment, the tumor cells or cancer cells are non-small cell lung cancer (NSCLC) cells, hepatocellular carcinoma (HCC) cells, colorectal cancer (CRC) cells, cervical squamous cell carcinoma (CESC) cells, head and neck squamous cell carcinoma (HNSC) cells, pancreatic cancer cells, metastatic castration-resistant prostate cancer (mCRPC) cells, ovarian cancer cells, bladder cancer cells, or breast cancer cells, preferably NSCLC cells, HCC cells, or CRC cells.

In a further embodiment, the stromal cells are cancer associated fibroblasts (CAFs).

The present disclosure also provides a pharmaceutical composition comprising a compound of Formula (I) as disclosed herein, and a pharmaceutically acceptable excipient, carrier, or diluent.

### Anti-Cotinine Antibodies

The present disclosure provides an antibody, or antigen-binding fragment thereof, that binds to a cotinine moiety. As used herein, the term "anti-cotinine antibody or antigen-binding fragment thereof" refers to an antibody, or antigen binding fragment thereof that binds to a cotinine moiety. Cotinine has the following structure:

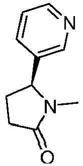

As used herein, the term "cotinine moiety" refers to cotinine or an analog of cotinine. Compounds of Formula (I) described herein comprise a cotinine moiety linked via a linker to a CCR2-binding moiety. The cotinine moiety has the following structure: 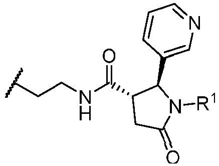 wherein R¹ is C₁₋₄ alkyl or C₃₋₆ cycloalkyl. In another embodiment, R¹ is methyl, ethyl, 1-propyl, 2-propyl, 1-butyl, 2-butyl, or t-butyl. In another embodiment, R¹ is methyl. In another embodiment, R¹ is cyclopropyl, cyclobutyl, cyclopentyl, or cyclohexyl.

The term "antibody" is used herein in the broadest sense to refer to molecules with an immunoglobulin-like domain (for example IgG, IgM, IgA, IgD or IgE) and includes monoclonal, recombinant, polyclonal, chimeric, human, humanised, multispecific antibodies, including bispecific antibodies, and heteroconjugate antibodies; a single variable domain (e.g., a domain antibody (DAB)), antigen binding antibody fragments, Fab, F(ab')2, Fv, disulphide linked Fv, single chain Fv, disulphide-linked scFv, diabodies, TANDABS, etc. and modified versions of any of the foregoing (for a summary of alternative "antibody" formats see Holliger and Hudson, Nature Biotechnology, 2005, 23(9): 1126-1136).

The term, full, whole or intact antibody, used interchangeably herein, refers to a heterotetrameric glycoprotein with an approximate molecular weight of 150,000 daltons. An intact antibody is composed of two identical heavy chains (HCs) and two identical light chains (LCs) linked by covalent disulphide bonds. This H2L2 structure folds to form three functional domains comprising two antigen-binding fragments, known as 'Fab' fragments, and a 'Fc' crystallisable fragment. The Fab fragment is composed of the variable domain at the amino-terminus, variable heavy (VH) or variable light (VL), and the constant domain at the carboxyl terminus, CH1 (heavy) and CL (light). The Fc fragment is composed of two domains formed by dimerization of paired CH2 and CH3 regions. The Fc may elicit effector functions by binding to receptors on immune cells or by binding C1q, the first component of the classical complement pathway. The five classes of antibodies IgM, IgA, IgG, IgE and IgD are defined by distinct heavy chain amino acid sequences, which are called µ, α, γ, ε and δ respectively, each heavy chain can pair with either a K or A light chain. The majority of antibodies in the serum belong to the IgG class, there are four isotypes of human IgG (IgG1, IgG2, IgG3 and IgG4), the sequences of which differ mainly in their hinge region.

"CDRs" are defined as the complementarity determining region amino acid sequences of an antibody or antigen binding fragment thereof. These are the hypervariable regions of immunoglobulin heavy and light chains. There are three heavy chain and three light chain CDRs (or CDR regions) in the variable portion of an immunoglobulin. Thus, "CDRs" as used herein refers to all three heavy chain CDRs, all three light chain CDRs, all heavy and light chain CDRs, or at least two CDRs.

Throughout this specification, amino acid residues in variable domain sequences and variable domain regions within full-length antigen binding sequences, e.g. within an antibody heavy chain sequence or antibody light chain sequence, are numbered according to the Kabat numbering convention. Similarly, the terms "CDR", "CDRL1", "CDRL2", "CDRL3", "CDRH1", "CDRH2", "CDRH3" used in the Examples follow the Kabat numbering convention. For further information, see Kabat et al., Sequences of Proteins of Immunological Interest, 4th Ed., U.S. Department of Health and Human Services, National Institutes of Health (1987).

It will be apparent to those skilled in the art that there are alternative numbering conventions for amino acid residues in variable domain sequences and full-length antibody sequences. There are also alternative numbering conventions for CDR sequences, for example those set out in Chothia et al., Nature, 1989, 342: 877-883. The structure and protein folding of the antigen binding protein may mean that other residues are considered part of the CDR sequence and would be understood to be so by a skilled person.

Other numbering conventions for CDR sequences available to a skilled person include "AbM" (University of Bath) and "contact" (University College London) methods.

Table 2 below represents one definition using each numbering convention for each CDR or binding unit. It should be noted that some of the CDR definitions may vary depending on the individual publication used.

**Table 2**

| | **Kabat CDR** | **Chothia CDR** | **AbM CDR** | **Contact CDR** |
|---|---|---|---|---|
| H1 | 31-35/35A/ 35B | 26-32/33/34 | 26-35/35A/35B | 30-35/35A/35B |
| H2 | 50-65 | 52-56 | 50-58 | 47-58 |
| H3 | 95-102 | 95-102 | 95-102 | 93-101 |
| L1 | 24-34 | 24-34 | 24-34 | 30-36 |
| L2 | 50-56 | 50-56 | 50-56 | 46-55 |
| L3 | 89-97 | 89-97 | 89-97 | 89-96 |

In a further embodiment, the anti-cotinine antibody is humanized. In a further embodiment, the Fc region of the anti-cotinine antibody is modified to increase ADCC activity, ADCP activity, and/or CDC activity, suitable modifications of which are provided below. In a further embodiment, the Fc region of the anti-cotinine antibody is modified to increase ADCC activity.

Fc engineering methods can be applied to modify the functional or pharmacokinetics properties of an antibody. Effector function may be altered by making mutations in the Fc region that increase or decrease binding to C1q or Fcγ receptors and modify CDC or ADCC activity respectively. Modifications to the glycosylation pattern of an antibody can also be made to change the effector function. The in vivo half-life of an antibody can be altered by making mutations that affect binding of the Fc to the FcRn (neonatal Fc receptor).

The term "effector function" as used herein refers to one or more of antibody-mediated effects including antibody-dependent cell-mediated cytotoxicity (ADCC), antibody-mediated complement activation including complement-dependent cytotoxicity (CDC), complement-dependent cell-mediated phagocytosis (CDCP), antibody dependent complement-mediated cell lysis (ADCML), and Fc-mediated phagocytosis or antibody-dependent cellular phagocytosis (ADCP).

The interaction between the Fc region of an antigen binding protein or antibody and various Fc receptors (FcR), including FcγRI (CD64), FcγRII (CD32), FcγRIII (CD16), FcRn, C1q, and type II Fc receptors is believed to mediate the effector functions of the antigen binding protein or antibody. Significant biological effects can be a consequence of effector functionality. Usually, the ability to mediate effector function requires binding of the antigen binding protein or antibody to an antigen and not all antigen binding proteins or antibodies will mediate every effector function.

Effector function can be assessed in a number of ways including, for example, evaluating ADCC effector function of antibody coated to target cells mediated by Natural Killer (NK) cells via FcγRIII, or monocytes/macrophages via FcγRI, or evaluating CDC effector function of antibody coated to target cells mediated by complement cascade via C1q. For example, an antibody, or antigen binding fragment thereof, of the present invention can be assessed for ADCC effector function in a Natural Killer cell assay. Examples of such assays can be found in Shields et al., The Journal of Biological Chemistry, 2001, 276: 6591-6604; Chappel et al., The Journal of Biological Chemistry, 1993, 268: 25124-25131; Lazar et al., PNAS, 2006, 103: 4005-4010.

Examples of assays to determine CDC function include those described in J Imm Meth, 1995, 184: 29-38.

The effects of mutations on effector functions (e.g., FcRn binding, FcγRs and C1q binding, CDC, ADCML, ADCC, ADCP) can be assessed, e.g., as described in Grevys et al., J Immunol., 2015,194(11): 5497-5508; Tam et al., Antibodies, 2017, 6(3): 12; or Monnet et al., mAbs, 2014, 6(2): 422-436.

Throughout this specification, amino acid residues in Fc regions, in antibody sequences or full-length antigen binding protein sequences, are numbered according to the EU index numbering convention.

Human IgG1 constant regions containing specific mutations have been shown to enhance binding to Fc receptors. In some cases these mutations have also been shown to enhance effector functions, such as ADCC and CDC, as described below. Antibodies, or antigen binding fragments thereof, of the present invention may include any of the following mutations.

Enhanced CDC: Fc engineering can be used to enhance complement-based effector function. For example (with reference to IgG1), K326W/E333S; S267E/H268F/S324T; and IgG1/IgG3 cross subclass can increase C1q binding; E345R (Diebolder et al., Science, 2014, 343: 1260-1293) and E345R/E430G/S440Y results in preformed IgG hexamers (Wang et al., Protein Cell, 2018, 9(1): 63-73).

Enhanced ADCC: Fc engineering can be used to enhance ADCC. For example (with reference to IgG1), F243L/R292P/Y300L/V305I/P396L; S239D/I332E; and S298A/E333A/K334A increase FcγRIIIa binding; S239D/I332E/A330L increases FcγRIIIa binding and decreases FcyRllb binding; G236A/S239D/I332E improves binding to FcyRlla, improves the FcγRIIa/FcγRIIb binding ratio (activating/inhibitory ratio), and enhances phagocytosis of antibody-coated target cells by macrophages. An asymmetric Fc in which one heavy chain contains L234Y/L235Q/G236W/S239M/H268D/D270E/S298A mutations and D270E/K326D/A330M/K334E in the opposing heavy chain, increases affinity for FcyRllla F158 (a lower-affinity allele) and FcγRIIIa V158 (a higher-affinity allele) with no increased binding affinity to inhibitory FcyRllb (Mimoto et al., mAbs, 2013, 5(2): 229-236).

Enhanced ADCP: Fc engineering can be used to enhance ADCP. For example (with reference to IgG1), G236A/S239D/I332E increases FcyRlla binding and increases FcγRIIIa binding (Richards, J. et al., Mol. Cancer Ther., 2008, 7: 2517-2527).

Increased co-engagement: Fc engineering can be used to increase co-engagement with FcRs. For example (with reference to IgG1), S267E/L328F increases FcyRllb binding; N325S/L328F increases FcyRlla binding and decreases FcγRIIIa binding Wang et al., Protein Cell, 2018, 9(1): 63-73).

In a further embodiment, an antibody, or antigen binding fragment thereof, of the present invention may comprise a heavy chain constant region with an altered glycosylation profile, such that the antibody, or antigen binding fragment thereof, has an enhanced effector function, e.g., enhanced ADCC, enhanced CDC, or both enhanced ADCC and CDC. Examples of suitable methodologies to produce an antibody, or antigen binding fragment thereof, with an altered glycosylation profile are described in WO 2003/011878, WO 2006/014679 and EP1229125.

The absence of the α1,6 innermost fucose residues on the Fc glycan moiety on N297 of IgG1 antibodies enhances affinity for FcyRIIIA. As such, afucosylated or low fucosylated monoclonal antibodies may have increased therapeutic efficacy (Shields et al., J Biol Chem., 2002, 277(30): 26733-40 and Monnet et al., mAbs, 2014, 6(2): 422-436).

In one embodiment there is provided an antibody, or antigen binding fragment thereof, comprising a chimeric heavy chain constant region. In an embodiment, the antibody, or antigen binding fragment thereof, comprises an IgG1/IgG3 chimeric heavy chain constant region, such that the antibody, or antigen binding fragment thereof, has an enhanced effector function, for example enhanced ADCC or enhanced CDC, or enhanced ADCC and CDC functions. For example, a chimeric antibody, or antigen binding fragment thereof, of the invention may comprise at least one CH2 domain from IgG3. In one such embodiment, the antibody, or antigen binding fragment thereof, comprises one CH2 domain from IgG3 or both CH2 domains may be from IgG3. In a further embodiment, the chimeric antibody, or antigen binding fragment thereof, comprises an IgG1 CH1 domain, an IgG3 CH2 domain, and an IgG3 CH3 domain. In a further embodiment, the chimeric antibody, or antigen binding fragment thereof, comprises an IgG1 CH1 domain, an IgG3 CH2 domain, and an IgG3 CH3 domain except for position 435 that is histidine.

In a further embodiment, the chimeric antibody, or antigen binding fragment thereof, comprises an IgG1 CH1 domain and at least one CH2 domain from IgG3. In an embodiment, the chimeric antibody, or antigen binding fragment thereof, comprises an IgG1 CH1 domain and the following residues, which correspond to IgG3 residues, in a CH2 domain: 274Q, 276K, 296F, 300F and 339T. In an embodiment, the chimeric antibody, or antigen binding fragment thereof, also comprises 356E, which corresponds to an IgG3 residue, within a CH3 domain. In an embodiment, the antibody, or antigen binding fragment thereof, also comprises one or more of the following residues, which correspond to IgG3 residues within a CH3 domain: 358M, 384S, 392N, 397M, 4221, 435R, and 436F.

Also provided is a method of producing an antibody, or antigen binding fragment thereof, according to the invention comprising the steps of:
a) culturing a recombinant host cell comprising an expression vector comprising a nucleic acid sequence encoding a chimeric Fc region having both IgG1 and IgG3 Fc region amino acid residues (e.g. as described above); and
b) recovering the antibody, or antigen binding fragment thereof.

Such methods for the production of antibody, or antigen binding fragment thereof, with chimeric heavy chain constant regions can be performed, for example, using the COMPLEGENT technology system available from BioWa, Inc. (Princeton, NJ) and Kyowa Hakko Kirin Co., Ltd. The COMPLEGENT system comprises a recombinant host cell comprising an expression vector in which a nucleic acid sequence encoding a chimeric Fc region having both IgG1 and IgG3 Fc region amino acid residues is expressed to produce an antibody, or antigen binding fragment thereof, having enhanced CDC activity, i.e. CDC activity is increased relative to an otherwise identical antibody, or antigen binding fragment thereof, lacking such a chimeric Fc region, as described in WO 2007/011041 and US 2007/0148165. In an alternative embodiment, CDC activity may be increased by introducing sequence specific mutations into the Fc region of an IgG chain. Those of ordinary skill in the art will also recognize other appropriate systems.

The present invention also provides a method of producing an antibody, or antigen binding fragment thereof, according to the invention comprising the steps of:
a) culturing a recombinant host cell comprising an expression vector comprising a nucleic acid encoding the antibody, or antigen binding fragment thereof, optionally wherein the FUT8 gene encoding alpha-1,6-fucosyltransferase has been inactivated in the recombinant host cell; and
b) recovering the antibody, or antigen binding fragment thereof.

Such methods for the production of an antibody, or antigen binding fragment thereof, can be performed, for example, using the POTELLIGENT technology system available from BioWa, Inc. (Princeton, NJ) in which CHOK1SV cells lacking a functional copy of the FUT8 gene produce monoclonal antibodies having enhanced ADCC activity that is increased relative to an identical monoclonal antibody produced in a cell with a functional FUT8 gene as described in US Patent No. 7,214,775, US Patent No. 6,946,292, WO 00/61739 and WO 02/31240. Those of ordinary skill in the art will also recognize other appropriate systems.

In one embodiment, the antibody, or antigen binding fragment thereof, is produced in a host cell in which the FUT8 gene has been inactivated. In a further embodiment, the antibody, or antigen binding fragment thereof, is produced in a -/- FUT8 host cell. In a further embodiment, the antibody, or antigen binding fragment thereof, is afucosylated at Asn297 (IgG1).

It will be apparent to those skilled in the art that such modifications may not only be used alone but may be used in combination with each other in order to further enhance effector function.

In one such embodiment, there is provided an antibody, or antigen binding fragment thereof, comprising a heavy chain constant region that comprises a both a mutated and chimeric heavy chain constant region, individually described above. For example, an antibody, or antigen binding fragment thereof, comprising at least one CH2 domain from IgG3 and one CH2 domain from IgG1, and wherein the IgG1 CH2 domain has one or more mutations at positions selected from 239, 332 and 330 (for example the mutations may be selected from S239D, I332E and A330L), such that the antibody, or antigen binding fragment thereof, has enhanced effector function, e.g. enhanced ADCC or enhanced CDC, or enhanced ADCC and enhanced CDC in comparison to an equivalent antibody, or antigen binding fragment thereof, with an IgG1 heavy chain constant region lacking said mutations. In one embodiment, the IgG1 CH2 domain has the mutations S239D and I332E. In another embodiment, the IgG1 CH2 domain has the mutations S239D, A330L, and I332E.

In an alternative embodiment, there is provided an antibody, or antigen binding fragment thereof, comprising both a chimeric heavy chain constant region and an altered glycosylation profile, as individually described above. In an embodiment, the antibody, or antigen binding fragment thereof, comprises an altered glycosylation profile such that the ratio of fucose to mannose is 0.8:3 or less. In one such embodiment, the heavy chain constant region comprises at least one CH2 domain from IgG3 and one CH2 domain from IgG1 and has an altered glycosylation profile such that the ratio of fucose to mannose is 0.8:3 or less, for example wherein the antibody, or antigen binding fragment thereof, is defucosylated. Said antibody, or antigen binding fragment thereof, has an enhanced effector function, e.g. enhanced ADCC or enhanced CDC, or enhanced ADCC and enhanced CDC, in comparison to an equivalent antibody, or antigen binding fragment thereof, with an IgG1 heavy chain constant region lacking said glycosylation profile.

In an alternative embodiment, the antibody, or antigen binding fragment thereof, has at least one IgG3 heavy chain CH2 domain and at least one heavy chain constant domain from IgG1 wherein both IgG CH2 domains are mutated in accordance with the limitations described herein.

In one aspect, there is provided a method of producing an antibody, or antigen binding fragment thereof, according to the invention described herein comprising the steps of:
a) culturing a recombinant host cell containing an expression vector comprising a nucleic acid sequence encoding a chimeric Fc domain having both IgG1 and IgG3 Fc domain amino acid residues (e.g. as described above); and wherein the FUT8 gene encoding alpha-1,6-fucosyltransferase has been inactivated in the recombinant host cell; and
b) recovering the antibody, or antigen binding fragment thereof.

Such methods for the production of an antibody, or antigen binding fragment thereof, can be performed, for example, using the ACCRETAMAB technology system available from BioWa, Inc. (Princeton, NJ) that combines the POTELLIGENT and COMPLEGENT technology systems to produce an antibody, or antigen binding fragment thereof, having both enhanced ADCC and CDC activity relative to an otherwise identical monoclonal antibody that lacks a chimeric Fc domain and that is fucosylated.

In another embodiment, there is provided an antibody, or antigen binding fragment thereof, comprising a mutated and chimeric heavy chain constant region wherein said antibody, or antigen binding fragment thereof, has an altered glycosylation profile such that the antibody, or antigen binding fragment thereof, has enhanced effector function, e.g. enhanced ADCC or enhanced CDC, or both enhanced ADCC and CDC. In one embodiment the mutations are selected from positions 239, 332 and 330, e.g. S239D, I332E and A330L. In a further embodiment the heavy chain constant region comprises at least one CH2 domain from IgG3 and one CH1 domain from IgG1. In one embodiment the heavy chain constant region has an altered glycosylation profile such that the ratio of fucose to mannose is 0.8:3 or less, e.g. the antibody, or antigen binding fragment thereof, is defucosylated, such that said antibody, or antigen binding fragment thereof, has an enhanced effector function in comparison with an equivalent non-chimeric antibody, or antigen binding fragment thereof, lacking said mutations and lacking said altered glycosylation profile.

In a further embodiment, the anti-cotinine antibody, or antigen binding fragment thereof, comprises a heavy chain CDR1 having SEQ ID NO: 1, a heavy chain CDR2 having SEQ ID NO: 2, a heavy chain CDR3 having SEQ ID NO: 3, a light chain CDR1 having SEQ ID NO: 4, a light chain CDR2 having SEQ ID NO: 5, and a light chain CDR3 having SEQ ID NO: 6. In a further embodiment, the anti-cotinine antibody has a heavy chain and a light chain, the heavy chain comprising a CDR1 having SEQ ID NO: 1, a CDR2 having SEQ ID NO: 2, and a CDR3 having SEQ ID NO: 3, and the light chain comprising a CDR1 having SEQ ID NO: 4, a CDR2 having SEQ ID NO: 5, and a CDR3 having SEQ ID NO: 6. In a further embodiment, the anti-cotinine antibody is of IgG1 isotype. In a further embodiment, the anti-cotinine antibody is of IgG1 isotype comprising a substitution in an Fc region to increase or enhance ADCC activity. In a further embodiment, the anti-cotinine antibody is of IgG1 isotype comprising a substitution in an Fc region to increase or enhance ADCC activity, wherein the substitution is S239D/I332E or S239D/I332E/A330L, wherein residue numbering is according to the EU Index. In a further embodiment, the anti-cotinine antibody is of IgG1 isotype comprising a substitution in an Fc region to increase or enhance ADCC activity, wherein the substitution is S239D/I332E, wherein residue numbering is according to the EU Index.

In a further embodiment, the anti-cotinine antibody, or antigen binding fragment thereof, comprises a heavy chain variable region (VH) having SEQ ID NO: 7, a light chain variable region (VL) having SEQ ID NO: 8. In a further embodiment, the anti-cotinine antibody has a heavy chain and a light chain, the heavy chain comprising a heavy chain variable region (VH) having SEQ ID NO: 7, and the light chain comprising a light chain variable region (VL) having SEQ ID NO: 8. In a further embodiment, the anti-cotinine antibody is of IgG1 isotype. In a further embodiment, the anti-cotinine antibody is of IgG1 isotype comprising a substitution in an Fc region to increase or enhance ADCC activity. In a further embodiment, the anti-cotinine antibody is of IgG1 isotype comprising a substitution in an Fc region to increase or enhance ADCC activity, wherein the substitution is S239D/I332E or S239D/I332E/A330L, wherein residue numbering is according to the EU Index. In a further embodiment, the anti-cotinine antibody is of IgG1 isotype comprising a substitution in an Fc region to increase or enhance ADCC activity, wherein the substitution is S239D/I332E, wherein residue numbering is according to the EU Index.

In a further embodiment, the anti-cotinine antibody has a heavy chain comprising SEQ ID NO: 9 and a light chain comprising SEQ ID NO: 10.

The present disclosure also provides a pharmaceutical composition comprising an anti-cotinine antibody, or antigen binding fragment thereof as disclosed herein, and a pharmaceutically acceptable excipient, carrier, or diluent.

The present disclosure also provides a combination comprising the compound of Formula (I) as disclosed herein, and an anti-cotinine antibody, or antigen-binding fragment thereof as disclosed herein. The compound of Formula (I) and anti-cotinine antibody, or antigen binding fragment thereof can be present in the same composition or in separate compositions. In one embodiment, a combination comprises a pharmaceutical composition comprising the compound of Formula (I) as disclosed herein and an anti-cotinine antibody, or antigen binding fragment thereof as disclosed herein, and a pharmaceutically acceptable carrier, diluent, or excipient. In another embodiment, a combination comprises a first pharmaceutical composition comprising a compound of Formula (I) as disclosed herein and a pharmaceutically acceptable carrier, diluent, or excipient; and a second pharmaceutical composition comprising an anti-cotinine antibody or antigen binding fragment thereof as disclosed herein, and a pharmaceutically acceptable carrier, excipient, or diluent.

### Statement of Use

The compounds of Formula (I) and pharmaceutically acceptable salts thereof are capable of simultaneously binding a cell surface-expressed CCR2 and an anti-cotinine antibody, or antigen binding fragment thereof to form a ternary complex for the treatment and/or prevention of diseases or disorders associated with CCR2-expressing cells.

In one embodiment, the present disclosure provides compounds for use in a method of treating and/or preventing a disease or disorder in a patient in need thereof comprising administering to the patient a therapeutically effective amount of the compound of Formula (I), or a pharmaceutically acceptable salt thereof, and an anti-cotinine antibody, or antigen-binding fragment thereof, wherein the disease or disorder is selected from a cancer, an inflammatory disease, an autoimmune disease, a viral infection, or a bacterial infection.

In a further embodiment, the compound and the antibody, or antigen-binding fragment thereof, are administered simultaneously. In a further embodiment, the compound and the antibody, or antigen-binding fragment thereof, are administered simultaneously from a single composition, including as a fixed-dose composition or by pre-mixing the compound and the antibody, or antigen-binding fragment thereof, prior to administration. For example, the compound and the antibody, or antigen-binding fragment thereof, can be pre-mixed about 2 seconds to about 30 seconds, about 30 seconds to about 2 minutes, about 2 minutes to about 10 minutes, about 10 minutes to about 30 minutes, or about 30 minutes to about 2 hours prior to administration. In a further embodiment, the compound and the antibody, or antigen-binding fragment thereof, are administered simultaneously from two separate compositions.

In a further embodiment, the compound and the antibody, or antigen-binding fragment thereof, are administered sequentially.

In certain embodiments, the compound and the antibody, or antigen-binding fragment thereof, whether administered simultaneously or sequentially, may be administered by the same route or may be administered by different routes. In one embodiment, the compound and the antibody, or antigen-binding fragment thereof, are both administered intraveneously or subcutaneously, in the same composition or in separate compositions. In another embodiment, the compound is administered orally and the antibody or antigen-binding fragment thereof is administered intravenously or subcutaneously.

In a further embodiment, the compound and the antibody, or antigen-binding fragment thereof, are administered in a molar ratio of compound to antibody, or antigen-binding fragment thereof, of about 2:1, about 1.8:1, about 1.6:1, about 1.5:1, about 1.4:1, about 1.3:1, about 1.2:1, about 1:1, about 1:1.2, about 1:1.3, about 1:1.4, about 1:1.5, about 1:1.6, about 1:1.8, about 1:2, about 2:1 to about 1.5:1, about 1.5:1 to about 1.2:1, about 1.2:1 to about 1:1, about 1:1 to about 1:1.2, about 1:1.2 to about 1:1.5, or about 1:1.5 to about 1:2.

In a further embodiment, the compound and the antibody, or antigen-binding fragment thereof, are present as a combination in a molar ratio of compound to antibody, or antigen-binding fragment thereof, of about 2:1, about 1.8:1, about 1.6:1, about 1.5:1, about 1.4:1, about 1.3:1, about 1.2:1, about 1:1, about 1:1.2, about 1:1.3, about 1:1.4, about 1:1.5, about 1:1.6, about 1:1.8, about 1:2, about 2:1 to about 1.5:1, about 1.5:1 to about 1.2:1, about 1.2:1 to about 1:1, about 1:1 to about 1:1.2, about 1:1.2 to about 1:1.5, or about 1:1.5 to about 1:2.

In a further embodiment, the compound and the antibody, or antigen-binding fragment thereof, are administered at a dosage of compound of 0.0001 mg/kg to 1 mg/kg and antibody of 0.01 mg/kg to 100 mg/kg. For example, in a further embodiment, the compound is administered at a dosage of about 0.0001 mg/kg to about 0.0002 mg/kg, about 0.0002 mg/kg to about 0.0003 mg/kg, about 0.0003 mg/kg to about 0.0004 mg/kg, about 0.0004 mg/kg to about 0.0005 mg/kg, about 0.0005 mg/kg to about 0.001 mg/kg, about 0.001 mg/kg to about 0.002 mg/kg, about 0.002 mg/kg to about 0.003 mg/kg, about 0.003 mg/kg to about 0.004 mg/kg, about 0.004 mg/kg to about 0.005 mg/kg, about 0.005 mg/kg to about 0.01 mg/kg, about 0.01 mg/kg to about 0.02 mg/kg, about 0.02 mg/kg to about 0.03 mg/kg, about 0.03 mg/kg to about 0.04 mg/kg, about 0.04 mg/kg to about 0.05 mg/kg, about 0.05 mg/kg to about 0.1 mg/kg, about 0.1 mg/kg to about 0.2 mg/kg, about 0.2 mg/kg to about 0.3 mg/kg, about 0.3 mg/kg to about 0.4 mg/kg, about 0.4 mg/kg to about 0.5 mg/kg, and/or about 0.5 mg/kg to about 1 mg/kg, and the antibody, or antigen-binding fragment thereof, is administered at a dosage of about 0.01 mg/kg to about 0.02 mg/kg, about 0.02 mg/kg to about 0.03 mg/kg, about 0.03 mg/kg to about 0.04 mg/kg, about 0.04 mg/kg to about 0.05 mg/kg, about 0.05 mg/kg to about 0.1 mg/kg, about 0.1 mg/kg to about 0.2 mg/kg, about 0.2 mg/kg to about 0.3 mg/kg, about 0.3 mg/kg to about 0.4 mg/kg, about 0.4 mg/kg to about 0.5 mg/kg, about 0.5 mg/kg to about 1 mg/kg, about 1 mg/kg to about 2 mg/kg, about 2 mg/kg to about 3 mg/kg, about 3 mg/kg to about 4 mg/kg, about 4 mg/kg to about 5 mg/kg, about 5 mg/kg to about 10 mg/kg, about 10 mg/kg to about 15 mg/kg, about 15 mg/kg to about 20 mg/kg, about 20 mg/kg to about 25 mg/kg, about 25 mg/kg to about 30 mg/kg, about 30 mg/kg to about 35 mg/kg, about 35 mg/kg to about 40 mg/kg, about 40 mg/kg to about 45 mg/kg, about 45 mg/kg to about 50 mg/kg, about 50 mg/kg to about 60 mg/kg, about 60 mg/kg to about 70 mg/kg, about 70 mg/kg to about 80 mg/kg, about 80 mg/kg to about 90 mg/kg, and/or about 90 mg/kg to about 100 mg/kg.

In a further embodiment, the compound and the antibody, or antigen-binding fragment thereof, are administered at a dosage of compound of 0.007 mg to 70 mg and antibody of 0.7 mg to 7000 mg. For example, in a further embodiment, the compound is administered at a dosage of about 0.007 mg to about 0.01 mg, about 0.01 mg to about 0.02 mg, about 0.02 mg to about 0.03 mg, about 0.03 mg to about 0.04 mg, about 0.04 mg to about 0.05 mg, about 0.05 mg to about 0.1 mg, about 0.1 mg to about 0.2 mg, about 0.2 mg to about 0.3 mg, about 0.3 mg to about 0.4 mg, about 0.4 mg to about 0.5 mg, about 0.5 mg to about 1 mg, about 1 mg to about 2 mg, about 2 mg to about 3 mg, about 3 mg to about 4 mg, about 4 mg to about 5 mg, about 5 mg to about 10 mg, about 10 mg to about 20 mg, about 20 mg to about 30 mg, about 30 mg to about 40 mg, about 40 mg to about 50 mg, about 50 mg to about 60 mg, and/or about 60 mg to about 70 mg, and the antibody, or antigen-binding fragment thereof, is administered at a dosage of about 0.7 mg to about 1 mg, about 1 mg to about 2 mg, about 2 mg to about 3 mg, about 3 mg to about 4 mg, about 4 mg to about 5 mg, about 5 mg to about 10 mg, about 10 mg to about 20 mg, about 20 mg to about 30 mg, about 30 mg to about 40 mg, about 40 mg to about 50 mg, about 50 mg to about 100 mg, about 100 mg to about 500 mg, about 500 mg to about 1000 mg, about 1000 mg to about 1500 mg, about 1500 mg to about 2000 mg, about 2000 mg to about 2500 mg, about 2500 mg to about 3000 mg, about 3000 mg to about 3500 mg, about 3500 mg to about 4000 mg, about 4000 mg to about 4500 mg, about 4500 mg to about 5000 mg, about 5000 mg to about 5500 mg, about 5500 mg to about 6000 mg, about 6000 mg to about 6500 mg, and/or about 6500 mg to about 7000 mg.

In a further embodiment, the compound and the antibody, or antigen-binding fragment thereof, are administered in a molar ratio and/or dosage as described herein once every week, once every two weeks, once every three weeks, once every four weeks, once every five weeks, or once every six weeks for a period of one week to one year, such as a period of one week, one month, two months, three months, four months, five months, six months, seven months, eight months, nine months, ten months, eleven months, or twelve months.

In a further embodiment, the present disclosure provides a therapeutically effective amount of the compound of Formula (I), or a pharmaceutically acceptable salt thereof, and an anti-cotinine antibody, or antigen-binding fragment thereof for use in therapy. The compound of Formula (I), or a pharmaceutically acceptable salt thereof, and anti-cotinine antibody, or antigen-binding fragment thereof can be used in treating or preventing a disease or disorder selected from a cancer, an inflammatory disease, an autoimmune disease, a viral infection, or a bacterial infection.

In a further embodiment, the present disclosure provides a therapeutically effective amount of the compound of Formula (I), or a pharmaceutically acceptable salt thereof, and an anti-cotinine antibody, or antigen-binding fragment thereof for the manufacture of a medicament. The medicament can be used in treating or preventing a disease or disorder selected from a cancer, an inflammatory disease, an autoimmune disease, a viral infection, or a bacterial infection.

In a further embodiment, the disease or disorder is mediated by chemokine receptor 2 (CCR2) and/or is associated with CCR2-positive pathogenic cells. In a further embodiment, CCR-positive cell types are identified by testing for expression of CCR by immunohistochemistry or flow cytometry.

In a further embodiment, the disease or disorder is a cancer selected from non-small cell lung cancer (NSCLC), hepatocellular carcinoma (HCC), colorectal cancer (CRC), cervical squamous cell carcinoma (CESC), head and neck squamous cell carcinoma (HNSC), pancreatic cancer, metastatic castration-resistant prostate cancer (mCRPC), ovarian cancer, bladder cancer, or breast cancer, preferably a cancer selected from NSCLC, HCC, or CRC.

In a further embodiment, the disease or disorder is a solid tumor. In a further embodiment, the disease or disorder is a solid tumor selected from NSCLC, HCC, CRC, CESC, HNSC, pancreatic cancer, mCRPC, ovarian cancer, bladder cancer, or breast cancer, preferably a solid tumor selected from NSCLC, HCC, or CRC.

In a further embodiment, the disease or disorder is a PD-1 relapsed or refractory cancer, such as a PD-1 relapsed or refractory NSCLC, HCC, CRC, CESC, HNSC, pancreatic cancer, mCRPC, ovarian cancer, bladder cancer, or breast cancer, preferably a PD-1 relapsed or refractory NSCLC, HCC, or CRC.

In a further embodiment, the disease or disorder is a non-solid cancer. In a further embodiment, the disease or disorder is a leukemia, a lymphoma, or a myeloma.

In a further embodiment, the disease or disorder is a viral infection. In a further embodiment, the viral infection is caused by an influenza virus, a coronavirus (e.g., COVID-19), or a hepatitis B virus.

In a further embodiment, the disease or disorder is a bacterial infection. In a further embodiment, the bacterial infection is a chronic bacterial infection.

In one embodiment, the present disclosure provides a method of increasing antibody-dependent cell cytotoxicity (ADCC) of CCR2-expressing cells comprising contacting the cells with an effective amount of the compound of Formula (I), or pharmaceutically acceptable salt thereof, and an anti-cotinine antibody, or antigen-binding fragment thereof, wherein the CCR2-binding moiety of the compound binds the CCR2 expressed on the cells.

In one embodiment, the present disclosure provides a method of increasing antibody dependent cellular phagocytosis (ADCP) of CCR2-expressing cells comprising contacting the cells with an effective amount of the compound of Formula (I), or pharmaceutically acceptable salt thereof, and an anti-cotinine antibody, or antigen-binding fragment thereof, wherein the CCR2-binding moiety of the compound binds the CCR2 expressed on the cells.

In one embodiment, the present disclosure provides a method of increasing complement dependant cytotoxicity (CDC) of CCR2-expressing cells comprising contacting the cells with an effective amount of the compound of Formula (I), or pharmaceutically acceptable salt thereof, and an anti-cotinine antibody, or antigen-binding fragment thereof, wherein the CCR2-binding moiety of the compound binds the CCR2 expressed on the cells.

In one embodiment, the present disclosure provides a method of conditioning a patient for therapy with a chimeric antigen receptor (CAR) T cell therapy, comprising administering to a patient an effective amount of the compound of Formula (I), or pharmaceutically acceptable salt thereof, and an anti-cotinine antibody, or antigen-binding fragment thereof. In some embodiments, the compound of Formula (I), or pharmaceutically acceptable salt thereof, and an anti-cotinine antibody, or antigen-binding fragment thereof are administered in combination with the CAR-T cell therapy. A compound of Formula (I), or pharmaceutically acceptable salt thereof, and an anti-cotinine antibody, or antigen-binding fragment thereof may be administered as a conditioning therapy or combination therapy to improve efficacy in treatment of solid tumor cancers. In other embodiments, a compound of Formula (I), or pharmaceutically acceptable salt thereof, and an anti-cotinine antibody, or antigen-binding fragment thereof may be administered as a neoadjuvant treatment for other therapies, including but not limited to immunotherapy, surgical resection, radiation, and/or chemotherapy.

In one embodiment, the present disclosure provides a method of depleting CCR2-expressing cells comprising contacting the cells with the compound of Formula (I), or pharmaceutically acceptable salt thereof, and an anti-cotinine antibody, or antigen-binding fragment thereof, wherein the CCR2-binding moiety of the compound binds the CCR2 expressed on the cells.

In a further embodiment, the CCR2-expressing cells are pathogenic cells.

In a further embodiment, the pathogenic cell is a pathogenic immune cell, a tumor cell or cancer cell, or a stromal cell.

In a further embodiment, the pathogenic immune cells are monocytes, myeloid derived suppressor cells (MDSC), such as monocytic MDSCs (mMDSCs) and polymorphonuclear MDSCs (PMN_MDSCs), T regulatory cells (Tregs), neutrophils (e.g., N2 neutrophils), macrophages (e.g., M2 macrophages), B regulatory cells (Bregs, memory B cells), plasma cells, CD8 cells (e.g., CD8 regulatory cells (CD8regs), memory CD8 cells, effector CD8 cells, naïve CD8 Tcells, TEMRA), exhausted T cells, eosinophils, basophils, mast cells, dendritic cells, natural killer (NK cells), innate lymphoid cells, NK T cells (NKT), or γδT cells.

In a further embodiment, the pathogenic immune cells are myeloid derived suppressor cells (MDSC), such as monocytic MDSCs (mMDSCs) and polymorphonuclear MDSCs (PMN_MDSCs), T regulatory cells (Tregs), neutrophils (e.g., N2 neutrophils), macrophages (e.g., M2 macrophages), B regulatory cells (Bregs), CD8 regulatory cells (CD8regs), exhausted T cells.

In a further embodiment, the tumor cells or cancer cells are non-small cell lung cancer (NSCLC) cells, hepatocellular carcinoma (HCC) cells, colorectal cancer (CRC) cells, cervical squamous cell carcinoma (CESC) cells, head and neck squamous cell carcinoma (HNSC) cells, pancreatic cancer cells, metastatic castration-resistant prostate cancer (mCRPC) cells, ovarian cancer cells, bladder cancer cells, or breast cancer cells, preferably NSCLC cells, HCC cells, or CRC cells.

In a further embodiment, the stromal cells are cancer associated fibroblasts (CAFs).

### Combination Therapies

The compounds of the invention may be employed alone or in combination with other therapeutic agents. Combination therapies according to the present invention thus comprise the administration of at least one compound of Formula (I) or a pharmaceutically acceptable salt thereof, and the use of at least one other pharmaceutically active agent. The compounds of the invention and the other pharmaceutically active agents may be administered together in a single pharmaceutical composition or separately and, when administered separately this may occur simultaneously or sequentially in any order. The amounts of the compounds of the invention and the other pharmaceutically active agents and the relative timings of administration will be selected in order to achieve the desired combined therapeutic effect.

It will be appreciated that when the compound of the present invention is administered in combination with one or more other therapeutically active agents normally administered by the inhaled, intravenous, oral, intranasal, ocular topical or other route, that the resultant pharmaceutical composition may be administered by the same route. Alternatively, the individual components of the composition may be administered by different routes.

In one embodiment, the compounds and pharmaceutical composition disclosed herein are used in combination with, or include, one or more additional therapeutic agents. In a further embodiment, the additional therapeutic agent is a checkpoint inhibitor or an immune modulator.

In a further embodiment, the checkpoint inhibitor is selected from a PD-1 inhibitor (e.g., an anti-PD-1 antibody including, but not limited to, pembrolizumab, nivolumab, cemiplimab, or dostarlimab), a PD-L1 inhibitor (e.g., an anti-PD-L1 antibody including, but not limited to, atezolizumab, avelumab, or durvalumab), or a CTLA-4 inhibitor (e.g., an anti-CTLA-4 antibody including, but not limited to, ipilimumab or tremilumumab).

In a further embodiment, the checkpoint inhibitor is selected from a CD226 axis inhibitor, including but not limited to a TIGIT inhibitor (e.g., an anti-TIGIT antibody), a CD96 inhibitor (e.g., an anti-CD96 antibody), and/or a PVRIG inhibitor (e.g., an anti-PVRIG antibody).

In a further embodiment, the immune modulator is an ICOS agonist (e.g., an anti-ICOS antibody including, but not limited to feladilimab), a PARP inhibitor (e.g., niraparib, olaparib), or a STING agonist.

### Pharmaceutical Compositions, Dosages, and Dosage Forms

For the purposes of administration, in certain embodiments, the ARMs described herein are administered as a raw chemical or are formulated as pharmaceutical compositions. Pharmaceutical compositions disclosed herein include an ARM and one or more of: a pharmaceutically acceptable carrier, diluent or excipient. An ARM is present in the composition in an amount which is effective to treat a particular disease, disorder or condition of interest. The activity of the ARM can be determined by one skilled in the art, for example, as described in the biological assays described below. Appropriate concentrations and dosages can be readily determined by one skilled in the art. In certain embodiments, the ARM is present in the pharmaceutical composition in an amount from about 25 mg to about 500 mg. In certain embodiments, the ARM is present in the pharmaceutical composition in an amount of about 0.01 mg to about 300 mg. In certain embodiments, ARM is present in the pharmaceutical composition in an amount of about 0.01 mg, 0.1 mg, 1 mg, 5 mg, 10 mg, 25 mg, 50 mg, 100 mg, 200 mg, 300 mg, 400 mg or about 500 mg.

Administration of the compounds of the invention, or their pharmaceutically acceptable salts, in pure form or in an appropriate pharmaceutical composition, is carried out via any of the accepted modes of administration of agents for serving similar utilities. The pharmaceutical compositions of the invention are prepared by combining a compound of the invention with an appropriate pharmaceutically acceptable carrier, diluent or excipient, and in specific embodiments are formulated into preparations in solid, semi-solid, liquid or gaseous forms, such as tablets, capsules, powders, granules, ointments, solutions, suppositories, injections, inhalants, gels, microspheres, and aerosols. Exemplary routes of administering such pharmaceutical compositions include, without limitation, oral, topical, transdermal, inhalation, parenteral (e.g., intramuscular, subcutaneous, intravenous, or intradermal), sublingual, buccal, rectal, vaginal, and intranasal. Pharmaceutical compositions of the invention are formulated so as to allow the active ingredients contained therein to be bioavailable upon administration of the composition to a patient.

Compositions that will be administered to a subject or patient take the form of one or more dosage units, where for example, a tablet may be a single dosage unit, and a container of a compound of the invention in aerosol form may hold a plurality of dosage units. Actual methods of preparing such dosage forms are known, or will be apparent, to those skilled in this art; for example, see Remington: The Science and Practice of Pharmacy, 20th Edition (Philadelphia. College of Pharmacy and Science, 2000). The composition to be administered will, in any event, contain a therapeutically effective amount of a compound of the invention, or a pharmaceutically acceptable salt thereof, for treatment of a disease or condition of interest in accordance with the teachings described herein.

The pharmaceutical compositions disclosed herein are prepared by methodologies well known in the pharmaceutical art. For example, in certain embodiments, a pharmaceutical composition intended to be administered by injection is prepared by combining a compound of the invention with sterile, distilled water so as to form a solution. In some embodiments, a surfactant is added to facilitate the formation of a homogeneous solution or suspension. Surfactants are compounds that non-covalently interact with the compound of the invention so as to facilitate dissolution or homogeneous suspension of the compound in the aqueous delivery system.

Traditional antibody therapeutics have several disadvantages that are addressed by the ARMs approach described herein including difficulties in managing adverse events via adjusting dose and dose frequency of administration, challenges in generating antibodies to certain classes of drug targets (e.g., GPCRs, ion channels, and enzymes), and a new cell line for development is required for each new antibody which can be slow and costly. Moreover, different formats of biologics (e.g., bispecifics) can be challenging to manufacture. In contrast, the ARMs approach provides the following advantages: uniting the pharmacology of antibodies with the dose-control of small molecules, dose controlled PK/PD allowing temporal cell depletion, simpler multimerization, and rapid reversal of cell depletion through dosing of the antibody-binding component (e.g., cotinine hapten) which can uncouple therapeutic effects from potential adverse events.

### EXAMPLES

The following examples illustrate the invention. These Examples are not intended to limit the scope of the invention, but rather to provide guidance to the skilled artisan to prepare and use the compounds, compositions, and methods of the invention. While particular embodiments of the invention are described, the skilled artisan will appreciate that various changes and modifications can be made. References to preparations carried out in a similar manner to, or by the general method of, other preparations, may encompass variations in routine parameters such as time, temperature, workup conditions, minor changes in reagent amounts etc. Chemical names for all title compounds were generated using ChemDraw Plug-in version 16.0.1.13c (90) or ChemDraw desktop version 16.0.1.13 (90). A person of ordinary skill in the art will recognize that compounds of the invention may have alternative names when different naming software is used.

### COMPOUND SYNTHESIS

The compounds according to Formula (I) are prepared using conventional organic synthetic methods. A suitable synthetic route is depicted below in the following general reaction schemes. All the starting materials are commercially available or are readily prepared from commercially available starting materials by those of skill in the art.

The skilled artisan will appreciate that if a substituent described herein is not compatible with the synthetic methods described herein, the substituent may be protected with a suitable protecting group that is stable to the reaction conditions. The protecting group may be removed at a suitable point in the reaction sequence to provide a desired intermediate or target compound. Suitable protecting groups and the methods for protecting and de-protecting different substituents using such suitable protecting groups are well known to those skilled in the art; examples of which may be found in T. Greene and P. Wuts, Protecting Groups in Organic Synthesis (4th ed.), John Wiley & Sons, NY (2006). In some instances, a substituent may be specifically selected to be reactive under the reaction conditions used. Under these circumstances, the reaction conditions convert the selected substituent into another substituent that is either useful as an intermediate compound or is a desired substituent in a target compound.

### Scheme 1

### INTERMEDIATES

### Intermediate 1: Methyl (1R,4S)-4-(2,5-dimethyl-1H-pyrrol-1-yl)cyclopent-2-ene-1-carboxylate.

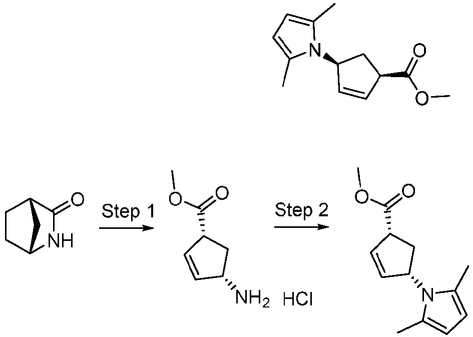

### Step 1: Methyl (1R,4S)-4-aminocyclopent-2-ene-1-carboxylate, hydrochloric acid salt.

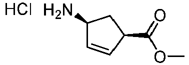

SOCl₂ (14.60 mL, 200 mmol) was added dropwise to methanol (100 mL) at 0 °C, commercially-available (1*S*,4*R*)-2-azabicyclo[2.2.1]hept-5-en-3-one (10.91 g, 100 mmol) was added and the mixture was stirred for 2 h at 0 °C. The mixture was concentrated using a steady stream of nitrogen at 50 °C to afford the title compound as a white solid (25 g, 99 mmol, 99 % yield). LC/MS *m*/*z* 142.2 (M+H)⁺.

### Step 2: Methyl (1R,4S)-4-(2,5-dimethyl-1 H-pyrrol-1-yl)cyclopent-2-ene-1-carboxylate.

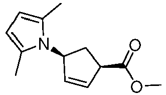

Methyl (1*R*,4*S*)-4-aminocyclopent-2-ene-1-carboxylate, hydrochloric acid salt (17.76 g, 100 mmol) was dissolved in methanol (50.0 mL) under an atmosphere of nitrogen at rt. DIPEA (20 mL, 115 mmol) was added, followed by hexane-2,5-dione (12.90 mL, 110 mmol). The reaction was stirred at rt overnight and was diluted with ethyl acetate. The organic layer was washed with saturated aqueous NH₄Cl (2 x 50 mL), with brine (3 x 50 mL), was dried over Na₂SO₄, was filtered, and the filtrate was concentrated to afford the title compound as a red oil (21.835 g, 90 mmol, 90 % yield). LC/MS *m*/*z* 220.2 (M+H)⁺.

### Intermediate 2: (R)-3-methoxytetrahydro-4H-pyran-4-one

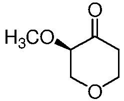

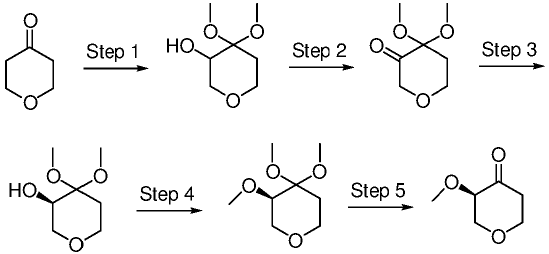

### Step 1: 4,4-Dimethoxytetrahydro-2H-pyran-3-ol.

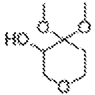

Potassium hydroxide (80 g, 1428 mmol) was dissolved in methanol (1 L) with stirring at rt. The homogeneous mixture was cooled to 0° C. Commercially available tetrahydro-4H-pyran-4-one (50 g, 499 mmol) dissolved in methanol (250 mL) and was added dropwise to the solution of potassium hydroxide while maintaining temperature at < 5° C. After stirring for an additional 1.5 h, iodine (139 g, 549 mmol) was added portionwise over 1.5 h while maintaining the temperature at < 5° C. The reaction mixture was stirred at rt for 18 h. The reaction was concentrated, toluene (500 mL) was added, and the mixture was stirred for 30 min. A small amount of precipitate was removed by filtration and the filtrate was evaporated to provide the title compound as a maroon oil (60.8 g, 375 mmol, 76 % yield). ¹H NMR (400 MHz, CHLOROFORM-d) δ ppm 1.74 - 1.82 (m, 1 H) 1.96 (ddd, J=14, 12, 4.9 Hz, 1 H) 2.37 (s, 1H) 3.26 (s, 3 H) 3.28 (s, 3 H) 3.50 (td, J=12, 2.7 Hz, 1 H) 3.66 - 3.72 (m, 2 H) 3.77 - 3.88 (m, 2 H).

### Step 2: 4,4-Dimethoxydihydro-2H-pyran-3(4H)-one.

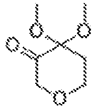

A solution of 4,4-dimethoxytetrahydro-2H-pyran-3-ol (60.8 g, 375 mmol) in dichloromethane (DCM) (500 mL) and was stirred under an atmosphere of nitrogen at rt. 4Å molecular sieves (60.8 g, 375 mmol) were added, followed by 4-methylmorpholine N-oxide (110 g, 937 mmol) and tetra-n-propylammonium perruthenate(vii) (5.47 g, 15.57 mmol). The mixture was stirred for 13 h. The reaction was filtered through Celite^{®} and was concentrated. Purification by normal-phase chromatography eluting with 10 % ethyl acetate in hexane provided the title compound as a yellow oil (57.73 g, 360 mmol, 96 % yield). ¹H NMR (400 MHz, CHLOROFORM-d) δ ppm 2.11 - 2.15 (m, 2 H) 3.16 (s, 6 H) 3.84 - 3.88 (m, 2 H) 3.91 - 3.98 (m, 2 H).

### Step 3: (R)-4,4-Dimethoxytetrahydro-2H-pyran-3-ol.

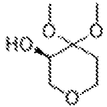

To a solution 4,4-dimethoxydihydro-2H-pyran-3(4H)-one (45 g, 281 mmol) in potassium phosphate buffer (7.0 pH) (1.125L) at rt was added NADPH⁺ (7.92 g, 281 mmol), D-glucose (67.5 g, 375 mmol), KRED-101 (2.25 g, 281 mmol), and GDH (2.25 g, 281 mmol). The mixture was stirred for at 25 °C for 6 h. At 2 h intervals, saturated KHCO₃ solution was used to adjust the pH to 7. Potassium chloride (50 g) was added and the mixture was stirred for 10 min. The mixture was extracted with acetonitrile (3 x 500 mL) and the organic extract was concentrated to near dryness. The residue was dissolved in dichloromethane (DCM) (250 mL) and the residual water was separated. The organic layer was dried over anhydrous Na₂SO₄, was filtered, and was concentrated. Purification by normal-phase chromatography eluting with a gradient of 10 to 50 % ethyl acetate in hexane provided the title compound as a colorless liquid (35 g, 209 mmol, 74.5 % yield). Specific Optical Rotation[α]^{25 589} : -16.2º (1% in methanol). GC-MS m/z 162.1 (M)⁺. ¹H NMR (400 MHz, CHLOROFORM-d) δ ppm 1.74 - 1.82 (m, 1 H) 1.90-1.99 (m, 1 H) 2.15 (d, 6 Hz, 1H) 3.26 (s, 3 H) 3.28 (s, 3 H) 3.50 (td, J=12, 2.7 Hz, 1 H) 3.66 - 3.72 (m, 2 H) 3.77 - 3.88 (m, 2 H).

### Step 4: (R)-3,4,4-Trimethoxytetrahydro-2H-pyran.

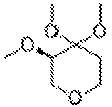

A 60% mineral oil dispersion of sodium hydride (17.26 g, 432 mmol) in tetrahydrofuran (THF) (220 mL) was stirred under an atmosphere of nitrogen and was cooled to 0 °C. (R)-4,4-dimethoxytetrahydro-2H-pyran-3-ol (35g, 216 mmol) was added dropwise over 15 min. The mixture was stirred at 25 °C for 1 h, methyl iodide (27.0 mL, 432 mmol) was added dropwise over 15 min, and the mixture was stirred at 25 °C for 4 h. The mixture was slowly poured into ice cold water (500 mL) and was extracted with ethyl acetate (500 mL). The aqueous phase was extracted with dichloromethane (DCM) (2 x 500 mL), the combined organic extracts were dried over anhydrous Na₂SO₄, were filtered, and the filtrate was concentrated. Purification by normal-phase chromatography eluting with 30 % ethyl acetate in hexane provided the title compound as a light brown liquid (34 g, 191 mmol, 89 % yield). Specific Optical Rotation[α]^{25 589} : -29.400º (1 % in methanol). GC-MS m/z 176.2 (M⁺). ¹H NMR (400 MHz, CHLOROFORM-d) δ ppm 1.74 - 1.79 (m, 1 H) 1.90 - 2.05 (m, 1 H) 3.19 - 3.28 (m, 9 H) 3.45 - 3.51 (m, 2 H) 3.55 - 3.60 (m, 1 H) 3.75 - 3.83 (m, 1 H) 3.95 - 4.05 (m, 1H).

### Step 5: (R)-3-Methoxytetrahydro-4H-pyran-4-one.

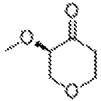

To a mixture solution of (R)-3,4,4-trimethoxytetrahydro-2H-pyran (35g, 199 mmol) in tetrahydrofuran (THF) (105 mL) and water (35.0 mL) at 0 °C was added dropwise concentrated aqueous HCl (280 mL, 3248 mmol) over 30 min. The mixture was stirred at 0 °C for 1 h and was concentrated to remove tetrahydrofuran (THF). Water (100 mL) was added and the mixture was extracted with dichloromethane (DCM) (2 x 1 L). The combined organic extracts were washed with saturated NaHCO₃ solution (100 mL), were dried over anhydrous Na₂SO₄, were filtered, and the filtrate was concentrated to provide the title compound as an off-white solid (25 g, 184 mmol, 93 % yield). Specific Optical Rotation[α]^{25 589} : +85.000º (1 % in methanol). GC-MS m/z 130.1 (M)⁺. ¹H NMR (400 MHz, CHLOROFORM-d) δ ppm 2.58 - 2.62 (m, 1 H) 3.50 (s, 3 H) 3.55 - 3.58 (m, 1 H) 3.70 - 3.82 (m, 2 H) 4.08 - 4.17 (m, 1 H) 4.19 - 4.23 (m, 1H).

### Intermediate 3: (2S,3S)-1-Methyl-5-oxo-2-(pyridin-3-yl)pyrrolidine-3-carboxylic acid

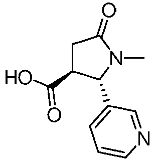

Commercially available, racemic trans-4-cotininecarboxylic acid (304 g, 1.38 mol) was purified by chiral prep HPLC (61 injections) on Chiralpak 1A 20u 101 x 210 mm at 500 mL/min eluting with 50 % acetonitrile in methanol containing 0.1 % formic acid. The desired fractions were collected and were concentrated at 45 °C. The solid residue was stirred in acetonitrile, was filtered, and was dried under reduced pressure for 18 h to provide the title compound as a white solid (143.6 g, 652 mmol, 94.5 % yield). Analytical chiral HPLC: 95 % ee at ret. time 2.5 min, Chiralpak AD-H 5u 4.6 x 150 mm, methanol with 0.1 % formic acid at 1.0 mL/min; Alpha D = + 58 deg (c=0.3, CH₃OH); VCD analysis was used to assign absolute stereochemistry. LC-MS m/z 221.1 (M+H)⁺.

### Intermediate 4: ((1S,3R)-3-(((3S,4S)-3-Methoxytetrahydro-2H-pyran-4-yl)amino)-1-(2-(piperidin-4-yl)ethyl)cyclopentyl)(3-(trifluoromethyl)-7,8-dihydro-1,6-naphthyridin-6(5H)-yl)methanone, 3hydrochloric acid salt.

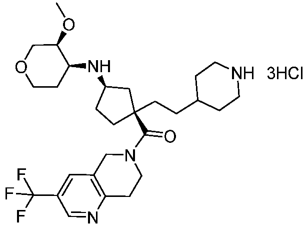

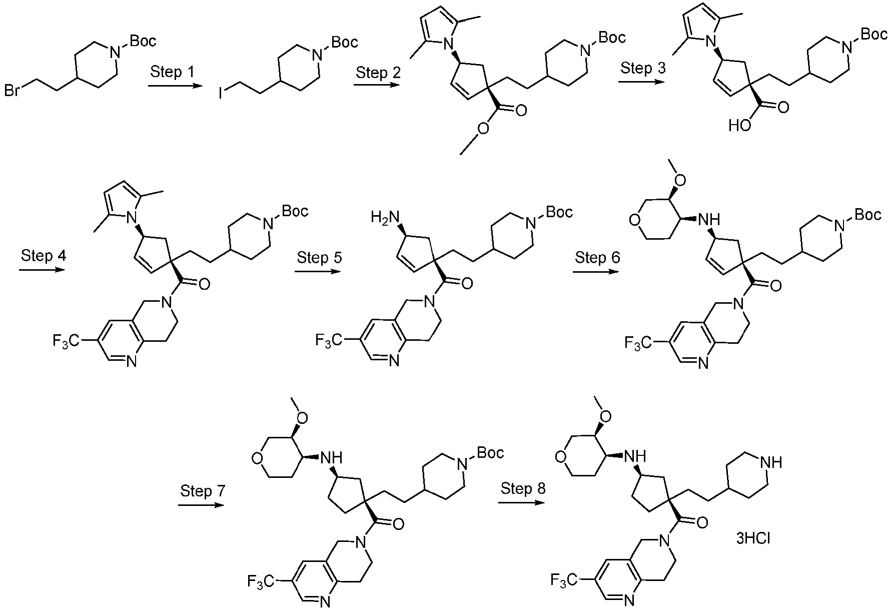

### Step 1: tert-Butyl 4-(2-iodoethyl)piperidine-1-carboxylate.

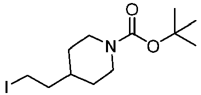

A mixture of commercially-available tert-butyl 4-(2-bromoethyl)piperidine-1-carboxylate (20.65 g, 70.7 mmol), sodium iodide (37.1 g, 247 mmol), and sodium bicarbonate (2.97 g, 35.3 mmol) in acetone (236 mL) was heated at 60 °C for 20 h. The mixture was cooled to rt and was concentrated under reduced vacuum. The residue was partitioned between ethyl acetate and water, and the organic phase was washed with 50 % (v/v) Na₂S₂O₅ in water, with brine, was dried over MgSO₄, was filtered, and the filtrate was concentrated under reduced pressure. Purification by ISCO CombiFlash^{®} chromatography (220 g silica Gold column) eluting with a gradient of 0 to 50 % ethyl acetate in heptane provided the title compound as a colorless oil (23.49 g, 65.8 mmol, 93 % yield). LCMS (m/z) 284.0 (M-(t-Bu)+H)⁺.

### Step 2: tert-Butyl 4-(2-((1R,4S)-4-(2,5-dimethyl-1H-pyrrol-1-yl)-1-(methoxycarbonyl)cyclopent-2-en-1-yl)ethyl)piperidine-1-carboxylate.

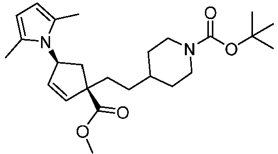

To a solution of 1 M lithium bis(trimethylsilyl)amide (LiHMDS) in tetrahydrofuran (THF) (70.2 mL, 70.2 mmol) in tetrahydrofuran (THF) (70 mL) under an atmosphere of nitrogen at -78 °C was added dropwise a solution of methyl (1*R*,4*S*)-4-(2,5-dimethyl-1H-pyrrol-1-yl)cyclopent-2-ene-1-carboxylate **(Intermediate 1)** (14.0 g, 63.8 mmol) in tetrahydrofuran (THF) (30.0 mL), and the mixture was stirred for 40 min. tert-Butyl 4-(2-iodoethyl)piperidine-1-carboxylate (22.74 g, 67.0 mmol) in tetrahydrofuran (THF) (30 mL) was added dropwise and the mixture was stirred at -78 °C for 30 min. The mixture was warmed up to 0 °C over 1.5 h and stirred at 0 °C for 40 min. Saturated aqueous NH₄Cl (250 mL) and water (150 mL) were added and the aqueous layer was extracted with ethyl acetate (2 x). The combined organic phases were washed with saturated aqueous NaHCO₃, with brine, were dried over MgSO₄, were filtered, and the filtrate was concentrated. The residue was purified in two portions by ISCO Combiflash^{®} chromatography (330 g silica Gold cartridge) eluting with a stepwise gradient of 0 to 15 % (15 min), 15% (6 min), and 15 to 50 % (10 min) ethyl acetate in heptane to provide the title compound as a light-brown oil (19.75 g, 45.0 mmol, 70.4 % yield). LCMS (m/z) 431.4 (M+H)⁺.

### Step 3: (1R,4S)-1-(2-(1-(tert-Butoxycarbonyl)piperidin-4-yl)ethyl)-4-(2,5-dimethyl-1H-pyrrol-1-yl)cyclopent-2-ene-1-carboxylic acid.

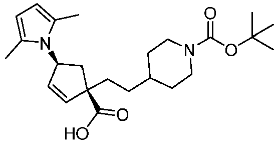

To a suspension of tert-butyl 4-(2-((1*R*,4*S*)-4-(2,5-dimethyl-1H-pyrrol-1-yl)-1-(methoxycarbonyl)cyclopent-2-en-1-yl)ethyl)piperidine-1-carboxylate (21.05 g, 48.9 mmol) in methanol (120 mL) and water (100 mL) was added 5 M sodium hydroxide (98 mL, 489 mmol) and the mixture was stirred at 60 °C for 1.5 h. Methanol was added (20 mL) and the mixture was stirred for 80 °C for 1 h. The mixture was cooled to rt, was poured into water (500 mL), and the pH was adjusted to 3 with 6 N HCl. The precipitate was collected by filtration, was washed with water (x 5), and was dried in a vacuum oven at 45 °C for 2 days to provide the title compound as a light-brown solid (19.26 g, 43.9 mmol, 90 % yield). LCMS (m/z) 417.5 (M+H)⁺.

### Step 4: tert-Butyl 4-(2-((1R,4S)-4-(2,5-dimethyl-1H-pyrrol-1-yl)-1-(3-(trifluoromethyl)-5,6,7,8-tetrahydro-1,6-naphthyridine-6-carbonyl)cyclopent-2-en-1-yl)ethyl)piperidine-1-carboxylate.

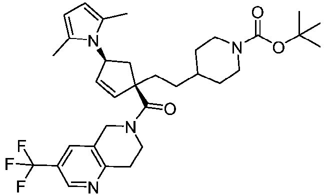

To a solution of (1*R*,4*S*)-1-(2-(1-(tert-butoxycarbonyl)piperidin-4-yl)ethyl)-4-(2,5-dimethyl-1H-pyrrol-1-yl)cyclopent-2-ene-1-carboxylic acid (4.0 g, 9.03 mmol) in N,N-dimethylacetamide (DMA) (19 mL) was added dropwise HATU (3.78 g, 9.93 mmol) and DIPEA (6.31 mL, 36.1 mmol). The mixture was stirred at rt for 15 min, commercially available 3-(trifluoromethyl)-5,6,7,8-tetrahydro-1,6-naphthyridine, 2hydrochloric acid salt (2.61 g, 9.48 mmol) was added and the mixture was stirred at rt for 30 min. The mixture was slowly added to ice water (150 mL), saturated NHCl₄ (30 mL) was added, and the precipitate was collected by filtration. The precipitate was washed with water and was dried under reduced pressure to provide the title compound (7.23 g, 9.03 mmol, 100 % yield). LCMS (m/z) 601.2 (M+H)⁺.

### Step 5: tert-Butyl 4-(2-((1R,4S)-4-amino-1-(3-(trifluoromethyl)-5,6,7,8-tetrahydro-1,6-naphthyridine-6-carbonyl)cyclopent-2-en-1-yl)ethyl)piperidine-1-carboxylate.

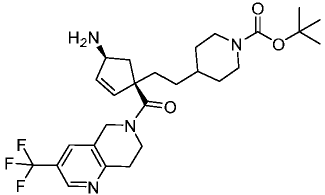

To a solution of tert-butyl 4-(2-((1 *R*,4*S*)-4-(2,5-dimethyl-1H-pyrrol-1-yl)-1-(3-(trifluoromethyl)-5,6,7,8-tetrahydro-1,6-naphthyridine-6-carbonyl)cyclopent-2-en-1-yl)ethyl)piperidine-1-carboxylate (7.23 g, 9.03 mmol) in methanol (60 mL) under an atmosphere of nitrogen was added hydroxylamine hydrochloride (3.76 g, 54.2 mmol), hydroxylamine (50 wt % in water) (3.60 mL, 58.7 mmol), and water (30.0 mL), and the suspension was stirred at 95 °C for 30 min. Methanol (30 mL) was added and the mixture was stirred at 95 °C for 18 h. The mixture was cooled to rt and was concentrated to remove the methanol. Water was added (50 mL) and the pH was adjusted to 11 with 1.0 N sodium hydroxide. The aqueous phase was extracted with ethyl acetate (3 x), the combined extracts were washed with brine, were dried over MgSO₄, were filtered, and the filtrate was concentrated to provide the title compound (6.29 g, 9.03 mmol, 100 % yield). LCMS (m/z) 523.4 (M+H)⁺.

### Step 6: tert-Butyl 4-(2-((1R,4S)-4-(((3S,4S)-3-methoxytetrahydro-2H-pyran-4-yl)amino)-1-(3-(trifluoromethyl)-5,6,7,8-tetrahydro-1,6-naphthyridine-6-carbonyl)cyclopent-2-en-1-yl)ethyl)piperidine-1-carboxylate.

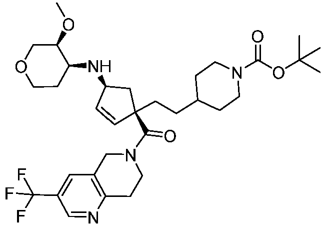

To a solution of tert-butyl 4-(2-((1R,4S)-4-amino-1-(3-(trifluoromethyl)-5,6,7,8-tetrahydro-1,6-naphthyridine-6-carbonyl)cyclopent-2-en-1-yl)ethyl)piperidine-1-carboxylate (5.80 g, 11.09 mmol) in isopropyl acetate (55.5 mL) under a nitrogen atmosphere at 0 °C was added 2-propanol (2.56 mL, 33.3 mmol) and sodium triacetoxyborohydride (7.05 g, 33.3 mmol), and the mixture was stirred at 0 °C for 40 min. (R)-3-methoxytetrahydro-4H-pyran-4-one **(Intermediate 2)** (1.804 g, 13.86 mmol) in isopropyl acetate (5 mL) was added, and the mixture was stirred at 0 °C for 1.5 h. Saturated aqueous NaHCO₃ (30 mL) and water (50 mL) were added, the pH was adjusted to 10 with 1 N sodium hydroxide (75 mL), and the aqueous phase was extracted with ethyl acetate (x 2). The combined organic extracts were washed with saturated NaHCO₃ (100 mL), with water (100 mL), and with brine (100 mL), were dried over MgSO₄, were filtered, and the filtrate was concentrated. Purification by ISCO Combiflash^{®} Rf chromatography (220 g silica Gold column) eluting with a stepwise gradient of 0 to 5 %, 5 %, and 5 to 20 % methanol in dichloromethane (DCM) provided the title compound as a light-beige solid (4.75 g, 6.94 mmol, 62.6 % yield). LCMS (m/z) 637.2 (M+H)⁺.

### Step 7: tert-Butyl 4-(2-((1S,3R)-3-(((3S,4S)-3-methoxytetrahydro-2H-pyran-4-yl)amino)-1-(3-(trifluoromethyl)-5,6,7,8-tetrahydro-1,6-naphthyridine-6-carbonyl)cyclopentyl)ethyl)piperidine-1-carboxylate.

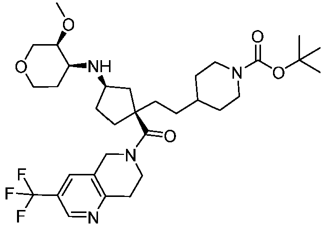

A mixture of tert-butyl 4-(2-((1*R*,4*S*)-4-(((3S,4S)-3-methoxytetrahydro-2H-pyran-4-yl)amino)-1-(3-(trifluoromethyl)-5,6,7,8-tetrahydro-1,6-naphthyridine-6-carbonyl)cyclopent-2-en-1-yl)ethyl)piperidine-1-carboxylate (1.64 g, 2.58 mmol) and Pd/C (0.548 g, 0.258 mmol) (wet) in methanol (13.0 mL) was stirred under a balloon atmosphere of hydrogen at rt for 90 min, and the 18 h. The mixture was filtered through Celite^{®}, the catalyst was washed with methanol, and the combined filtrates were concentrated under reduced pressure to provide the title compound as a pale-beige solid (1.653 g, 2.381 mmol, 92 % yield). LCMS (m/z) 639.3 (M+H)⁺.

### Step 8: ((1S,3R)-3-(((3S,4S)-3-Methoxytetrahydro-2H-pyran-4-yl)amino)-1-(2-(piperidin-4-yl)ethyl)cyclopentyl)(3-(trifluoromethyl)-7,8-dihydro-1,6-naphthyridin-6(5H)-yl)methanone, 3Hydrochloric acid salt.

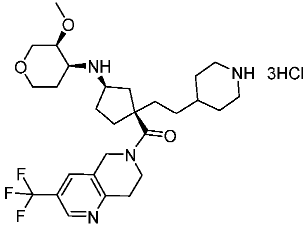

To a solution of tert-butyl 4-(2-((1*S*,3*R*)-3-(((3*S*,4*S*)-3-methoxytetrahydro-2H-pyran-4-yl)amino)-1-(3-(trifluoromethyl)-5,6,7,8-tetrahydro-1,6-naphthyridine-6-carbonyl)cyclopentyl)ethyl)piperidine-1-carboxylate (1.58 g, 2.473 mmol) in dichloromethane (DCM) (12.37 mL) was slowly added 4 M HCl (5.00 mL, 20.00 mmol). The mixture was stirred at rt for 1 h and was concentrated under reduced pressure to provide the title compound as a light-brown solid (1.73 g, 2.483 mmol, 100 % yield). LCMS (m/z) 539.4 (M+H)⁺.

The following intermediates were or could be prepared using procedures analogous to those described for **Intermediate 4:**

| **Intermediate** | **Compound** | **Structure** | **LC-MS m/z (M+H)⁺** |
|---|---|---|---|
| 5 | ((1S,3R)-1-(3-aminopropyl)-3-(((3S,4S)-3-methoxytetrahydro-2H-pyran-4-yl)amino)cyclopentyl)(3-(trifluoromethyl)-7,8-dihydro-1,6-naphthyridin-6(5H)-yl)methanone trihydrochloride salt | 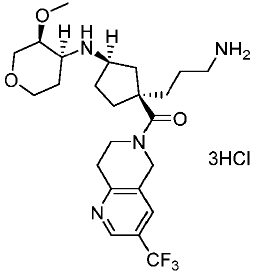 | 485.4 |
| 6 | ((1S,3R)-1-(4-aminobutyl)-3-(((3S,4S)-3-methoxytetrahydro-2H-pyran-4-yl)amino)cyclopentyl)(3-(trifluoromethyl)-7,8-dihydro-1,6-naphthyridin-6(5H)-yl)methanone, 3Hydrochloride | 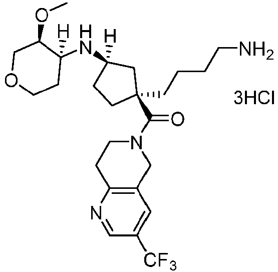 | 499.5 |
| 7 | ((1*S*,3*R*)-1-(5-Aminopentyl)-3-(((3*S*,4*S*)-3-methoxytetrahydro-2H-pyran-4-yl)amino)cyclopentyl)(3-(trifluoromethyl)-7,8-dihydro-1,6-naphthyridin-6(5H)-yl)methanone, 3Hydrochloric acid salt. | 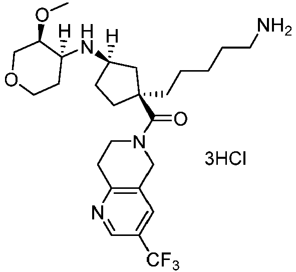 | 513.4 |

### Intermediate 8: ((1R,3R)-1-(2-Aminoethyl)-3-(((3S,4S)-3-methoxytetrahydro-2H-pyran-4-yl)amino)cyclopentyl)(3-(trifluoromethyl)-7,8-dihydro-1,6-naphthyridin-6(5H)-yl)methanone, 3Hydrochloric acid salt.

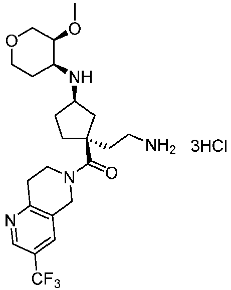

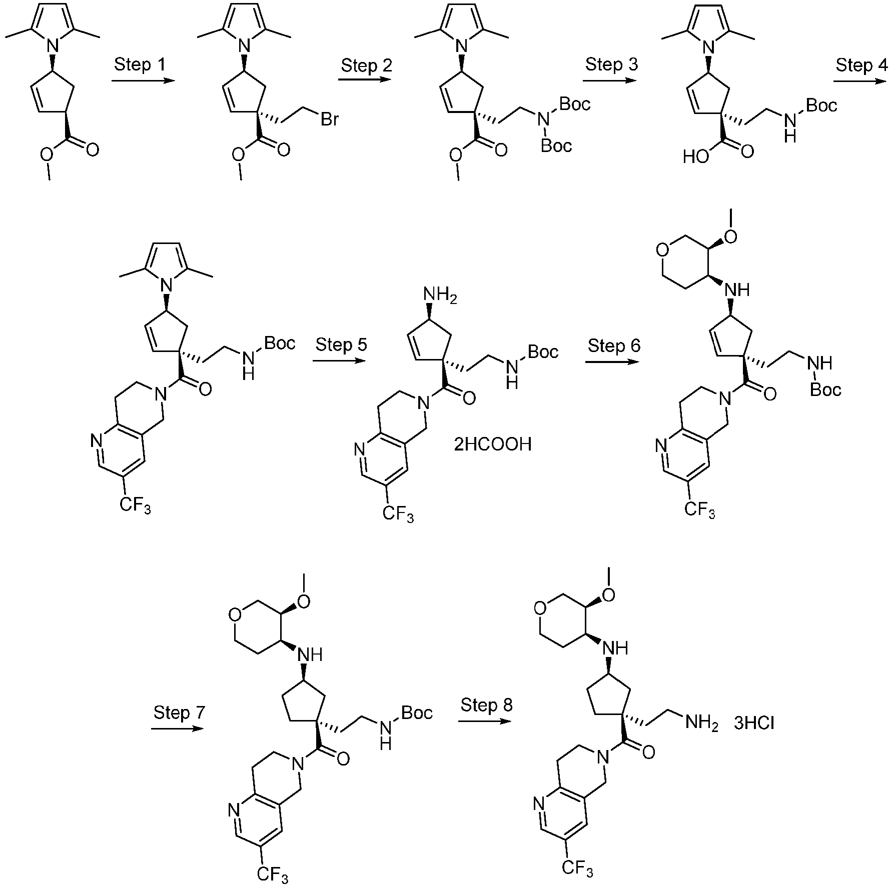

### Step 1: Methyl (1S,4S)-1-(2-bromoethyl)-4-(2,5-dimethyl-1H-pyrrol-1-yl)cyclopent-2-ene-1-carboxylate.

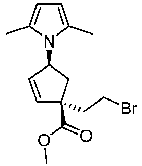

To a solution of 1 M lithium bis(trimethylsilyl)amide (LiHMDS) in (tetrahydrofuran) THF (25 mL, 25.00 mmol) at -78 °C was added dropwise a solution of methyl (1*R*,4*S*)-4-(2,5-dimethyl-1H-pyrrol-1-yl)cyclopent-2-ene-1-carboxylate **(Intermediate 1)** (4.39 g, 20 mmol) in tetrahydrofuran (THF) (20.00 mL). The mixture was stirred at -78 °C for 1 h and was added dropwise to a solution of 1,2-dibromoethane (5.17 mL, 60.0 mmol) in tetrahydrofuran (THF) (20.00 mL) at -78 °C. The mixture was stirred at -78 °C for 30 minutes, was warmed to 0 °C over 3.5 h, and was stirred at 0 °C for 60 min. Saturated aqueous NH₄Cl (100 mL) and ethyl acetate were added, and the aqueous phase was extracted with ethyl acetate (3 x). The combined organic phases were washed with saturated aqueous NaHCO₃ (1 x), with brine (1 x), were dried over MgSO₄, were filtered, and the filtrate was concentrated to provide the title compound as a brown oil (8.09 g, 11.66 mmol, 58.3 % yield). LCMS (m/z) 326.1 (M+H)⁺.

### Step 2: Methyl (1R,4S)-1-(2-(bis(tert-butoxycarbonyl)amino)ethyl)-4-(2,5-dimethyl-1H-pyrrol-1-yl)cyclopent-2-ene-1-carboxylate.

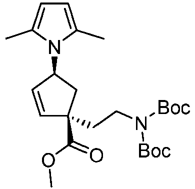

To a solution of methyl (1*S*,4*S*)-1-(2-bromoethyl)-4-(2,5-dimethyl-1H-pyrrol-1-yl)cyclopent-2-ene-1-carboxylate (8.09 g, 11.66 mmol) in N,N-dimethylformamide (DMF) (100 mL) was added di-tert-butyl iminodicarboxylate (10.86 g, 50 mmol) and Cs₂CO₃ (20.36 g, 62.5 mmol). The mixture was heated to 80 °C for 2 h. The mixture was cooled to rt, water was added, and the aqueous phase was extracted with diethyl ether (5 x). The combined organic extracts were concentrated. Purification by ISCO CombiFlash^{®} chromatography (120 g silica Gold column, 150 mL/min) eluting with a gradient of 0 to 100 % ethyl acetate in heptane provided the title compound (2.65 g, 1.375 mmol, 11.80 % yield). LCMS (m/z) 463.4 (M+H)⁺.

### Step 3: (1R,4S)-1-(2-((tert-Butoxycarbonyl)amino)ethyl)-4-(2,5-dimethyl-1H-pyrrol-1-yl)cyclopent-2-ene-1-carboxylic acid

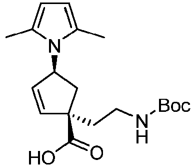

To a solution of methyl (1*R*,4*S*)-1-(4-((bis-tert-butoxycarbonyl)amino)ethyl)-4-(2,5-dimethyl-1H-pyrrol-1-yl)cyclopent-2-ene-1-carboxylate (2.65 g, 1.375 mmol) in methanol (10.31 mL) and water (3.44 mL) was added 5 M aqueous sodium hydroxide (5.50 mL, 27.5 mmol). The mixture stirred at 90 °C for 2 h and was concentrated under a steady stream of nitrogen. Water (150 mL) was added. The mixture was cooled in an ice-bath and was adjusted to pH 4 with 1 N HCl. The white solid precipitate was collected by filtration, was washed with cold water (100 mL), with hexanes (100 mL), and was chased with toluene (3 x) to provide the title compound as an orange solid (797 mg, 0.549 mmol, 39.9 % yield). LCMS (m/z) 349.3 (M+H)⁺.

### Step 4: tert-Butyl (2-((1R,4S)-4-(2,5-dimethyl-1H-pyrrol-1-yl)-1-(3-(trifluoromethyl)-5,6,7,8-tetrahydro-1,6-naphthyridine-6-carbonyl)cyclopent-2-en-1-yl)ethyl)carbamate

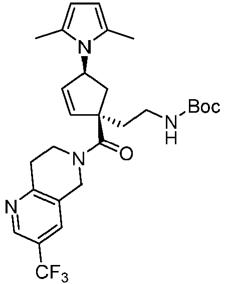

A mixture of (1*R*,4*S*)-1-(2-((tert-butoxycarbonyl)amino)ethyl)-4-(2,5-dimethyl-1H-pyrrol-1-yl)cyclopent-2-ene-1-carboxylic acid (1.996 g, 1.375 mmol) and HATU (0.784 g, 2.063 mmol) under an atmosphere of nitrogen was dissolved in N,N-dimethylacetamide (DMA) (13.75 mL). DIPEA (0.961 mL, 5.50 mmol) was added dropwise and the mixture was stirred at rt for 15 min. Commercially-available 3-(trifluoromethyl)-5,6,7,8-tetrahydro-1,6-naphthyridine, 2hydrochloric acid salt (0.416 g, 1.513 mmol) was added and the mixture was stirred at rt for 30 min. Ice water (30 mL) and saturated aqueous NH₄Cl (30 mL) were added. The precipitate was collected by filtration, was washed with water, with saturated aqueous NaHCO₃, and with water to provide the title compound as brown solid (3.85 g, 1.375 mmol, 100 % yield). LCMS (m/z) 455.4 (M+H)⁺.

### Step 5: tert-Butyl (2-((1R,4S)-4-amino-1-(3-(trifluoromethyl)-5,6,7,8-tetrahydro-1,6-naphthyridine-6-carbonyl)cyclopent-2-en-1-yl)ethyl)carbamate

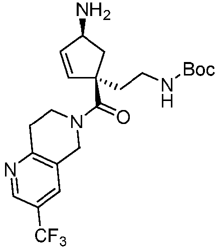

To solution of tert-butyl (2-((1R,4S)-4-(2,5-dimethyl-1H-pyrrol-1-yl)-1-(3-(trifluoromethyl)-5,6,7,8-tetrahydro-1,6-naphthyridine-6-carbonyl)cyclopent-2-en-1-yl)ethyl)carbamate (732 mg, 1.375 mmol) was in methanol (10 mL) was added hydroxylamine hydrochloride (562 mg, 8.09 mmol), hydroxylamine (50 wt% in water) (0.550 mL, 9.14 mmol), and water (5.00 mL) and the mixture was stirred at 75 °C for 18.5 h. The mixture was cooled to rt and was concentrated. Purification by CombiFlash^{®} EZ Prep chromatography (C18 100 g Gold column, 60 mL/min) eluting with a gradient of 10 to 50 % acetonitrile in water containing formic acid (0.1 %) provided the title compound as a yellow oil (266.5 mg, 0.516 mmol, 37.5 % yield). LCMS (m/z) 455.4 (M+H)⁺.

### Step 6: tert-Butyl (2-((1R,4S)-4-(((3S,4S)-3-methoxytetrahydro-2H-pyran-4-yl)amino)-1-(3-(trifluoromethyl)-5,6,7,8-tetrahydro-1,6-naphthyridine-6-carbonyl)cyclopent-2-en-1-yl)ethyl)carbamate

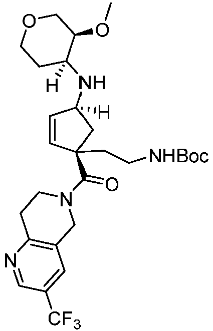

To a mixture of tert-butyl (2-((1*R*,4*S*)-4-amino-1-(3-(trifluoromethyl)-5,6,7,8-tetrahydro-1,6-naphthyridine-6-carbonyl)cyclopent-2-en-1-yl)ethyl)carbamate (0.2665 g, 0.488 mmol), isopropyl acetate (10 mL), triethylamine (0.204 mL, 1.463 mmol), and 2-propanol (0.113 mL, 1.463 mmol) at 5 °C was added sodium triacetoxyborohydride (0.310 g, 1.463 mmol) and the mixture was stirred for 1 h. The reaction was cooled to 1 °C, (R)-3-methoxytetrahydro-4H-pyran-4-one (0.105 g, 0.807 mmol) in isopropyl acetate (1 mL) was added, and the mixture was stirred for 1 h. Saturated aqueous NaHCO₃ (5mL), water (10 mL), and ethyl acetate were added (10 mL). The aqueous phase was extracted with ethyl acetate (5 x). The combined organic phases were washed with 1 M sodium hydroxide (1 x), with brine (1 x), were dried over Na₂SO₄, were filtered, and the filtrate was concentrated. The residue was placed under high vacuum overnight to provide the title compound as a tan solid (257.6 mg, 0.367 mmol, 75 % yield). LCMS (m/z) 569.5 (M+H)⁺.

### Step 7: tert-Butyl (2-((1R,4S)-4-(((3S,4S)-3-methoxytetrahydro-2H-pyran-4-yl)amino)-1-(3-(trifluoromethyl)-5,6,7,8-tetrahydro-1,6-naphthyridine-6-carbonyl)cyclopent-2-en-1-yl)ethyl)carbamate

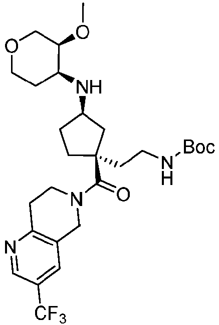

To a solution of tert-butyl (2-((1*R*,4*S*)-4-(((3*S*,4*S*)-3-methoxytetrahydro-2H-pyran-4-yl)amino)-1-(3-(trifluoromethyl)-5,6,7,8-tetrahydro-1,6-naphthyridine-6-carbonyl)cyclopent-2-en-1-yl)ethyl)carbamate (0.2576 g, 0.453 mmol) in methanol (4.53 mL) was added 10 % Pd/C (0.121 g, 0.113 mmol) and the mixture was stirred under an atmosphere hydrogen at rt for 1 h. The reaction was filtered through Celite^{®}, was washed with methanol, and the filtrate was concentrated to provide the title compound as a white solid (0.1493 g, 0.235 mmol, 52.0 % yield). LCMS (m/z) 571.5 (M+H)⁺.

### Step 8: ((1R,3R)-1-(2-Aminoethyl)-3-(((3S,4S)-3-methoxytetrahydro-2H-pyran-4-yl)amino)cyclopentyl)(3-(trifluoromethyl)-7,8-dihydro-1,6-naphthyridin-6(5H)-yl)methanone, 3Hydrochloric acid salt

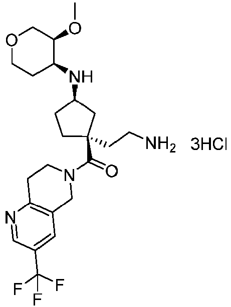

To a mixture of tert-butyl (2-((1*R*,3*R*)-3-(((3*S*,4*S*)-3-methoxytetrahydro-2H-pyran-4-yl)amino)-1-(3-(trifluoromethyl)-5,6,7,8-tetrahydro-1,6-naphthyridine-6-carbonyl)cyclopentyl)ethyl)carbamate (0.114 g, 0.200 mmol) in dichloromethane (DCM) (2.000 mL) was added 4 M HCl in 1,4-dioxane (1 mL, 4.00 mmol). The mixture was stirred at rt for 15 minutes. Methanol (1 mL) was added and the mixture was concentrated under a steady stream of nitrogen to provide the title compound as a white solid (0.147 g, 0.2 mmol, 100 % yield). LCMS (m/z) 471.4 (M+H)⁺.

### Intermediate 9: ((1S,3R)-1-((2-Aminoethoxy)methyl)-3-(((3S,4S)-3-methoxytetrahydro-2H-pyran-4-yl)amino)cyclopentyl)(3-(trifluoromethyl)-7,8-dihydro-1,6-naphthyridin-6(5H)-yl)methanone, 3Acetic acid salt

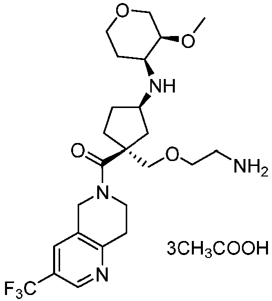

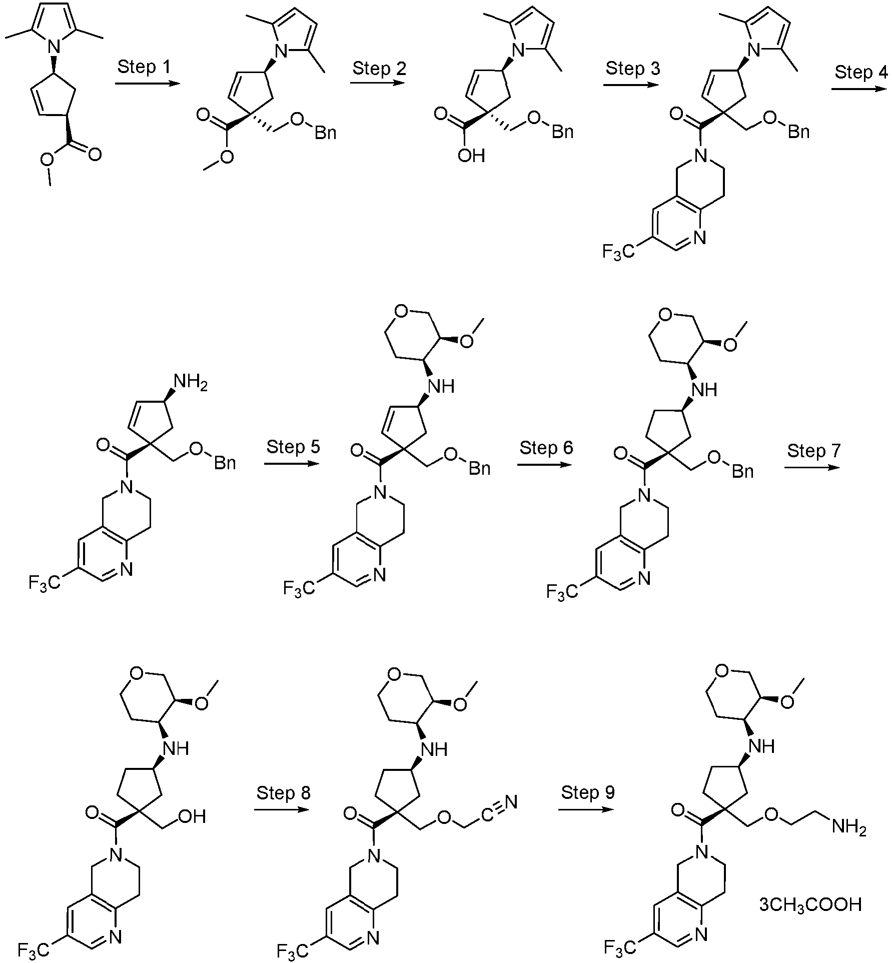

### Step 1: Methyl (1R,4S)-1-((benzyloxy)methyl)-4-(2,5-dimethyl-1H-pyrrol-1-yl)cyclopent-2-ene-1-carboxylate

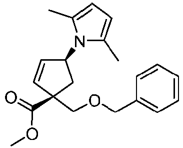

To a solution of 1 M LHMDS in tetrahydrofuran (THF) (25 mL, 25.00 mmol) at -78 °C was added dropwise a solution of methyl (1R,4S)-4-(2,5-dimethyl-1H-pyrrol-1-yl)cyclopent-2-ene-1-carboxylate (4.39 g, 20 mmol) in 2-methyltetrahydrofuran (2-MeTHF) (20.00 mL) and the mixture was stirred at -78 °C for 1 h. A solution of commercially available ((chloromethoxy)methyl)benzene (5.56 mL, 40.0 mmol) in 2-methyltetrahydrofuran (2-MeTHF) (20.00 mL) was added dropwise and the mixture was stirred at -78 °C for 30 min. The mixture was warmed to 0 °C over 3.5 h and was stirred at 0 °C for 60 min. Saturated aqueous NH₄Cl (100 mL) and ethyl acetate were added, and the aqueous phase was extracted with ethyl acetate (3 x). The combined organic extracts were washed with saturated aqueous NaHCO₃ (1 x), with brine (1 x), were dried over MgSO₄, were filtered, and the filtrate was concentrated. Purification by ISCO CombiFlash^{®} chromatography (120 g silica Gold column, 85 mL/min) eluting with a gradient of 0 to 20 % ethyl acetate in heptane provided the title compound as a colorless oil (4.64 g, 12.44 mmol, 62.2 % yield). LCMS (m/z) 340.3 (M+H)⁺.

### Step 2: (1R,4S)-1-((Benzyloxy)methyl)-4-(2,5-dimethyl-1H-pyrrol-1-yl)cyclopent-2-ene-1-carboxylic acid

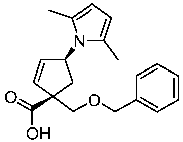

To a mixture of methyl (1*R*,4*S*)-1-((benzyloxy)methyl)-4-(2,5-dimethyl-1H-pyrrol-1-yl)cyclopent-2-ene-1-carboxylate (4.64 g, 13.67 mmol) in methanol (34.2 mL) and water (11.39 mL) was added 5 M aqueous sodium hydroxide (27.3 mL, 137 mmol), and the mixture was heated at 90 °C for 1.5 h. The mixture was concentrated under a steady stream of nitrogen. Water (200 mL) was added and the mixture was placed in an ice bath. The pH was adjusted to 4 with 1 N HCl. The precipitate was collected by filtration and was dried under reduced pressure to provide the title compound as an orange solid (3.48 g, 9.84 mmol, 72.0 % yield). LCMS (m/z) 326.3 (M+H)⁺.

### Step 3: ((1R,4S)-1-((Benzyloxy)methyl)-4-(2,5-dimethyl-1H-pyrrol-1-yl)cyclopent-2-en-1-yl)(3-(trifluoromethyl)-7,8-dihydro-1,6-naphthyridin-6(5H)-yl)methanone

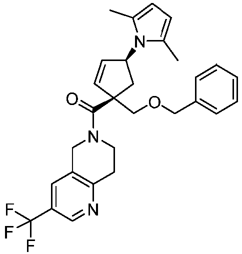

To a solution of (1*R*,4*S*)-1-((benzyloxy)methyl)-4-(2,5-dimethyl-1H-pyrrol-1-yl)cyclopent-2-ene-1-carboxylic acid (3.48 g, 10.69 mmol) and HATU in N,N-dimethylacetamide (DMA) (26.7 mL). (4.47 g, 11.76 mmol) under an atmosphere of nitrogen was added dropwise DIPEA (7.47 mL, 42.8 mmol) and the mixture was stirred at rt for 15 min. Commercially-available 3-(trifluoromethyl)-5,6,7,8-tetrahydro-1,6-naphthyridine, 2hydrochloric acid salt (2.94 g, 10.69 mmol) was added and the mixture was stirred at rt for 30 minutes. The mixture was poured into a solution of water and saturated aqueous NH₄Cl (1:1) at 0 °C. The precipitate was collected by filtration and was washed with water to provide the title compound as a red-tan, wet solid (6.05 g, 10.69 mmol, 100 % yield). LCMS (m/z) 510.4 (M+H)⁺.

### Step 4: ((1R,4S)-1-((Benzyloxy)methyl)-4-(((3S,4S)-3-methoxytetrahydro-2H-pyran-4-yl)amino)cyclopent-2-en-1-yl)(3-(trifluoromethyl)-7,8-dihydro-1,6-naphthyridin-6(5H)-yl)methanone

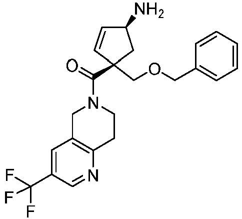

A mixture of ((1*R*,4*S*)-1-((benzyloxy)methyl)-4-(2,5-dimethyl-1H-pyrrol-1-yl)cyclopent-2-en-1-yl)(3-(trifluoromethyl)-7,8-dihydro-1,6-naphthyridin-6(5H)-yl)methanone (5.45 g, 10.69 mmol), methanol (71.3 mL), hydroxylamine hydrochloride (4.37 g, 62.9 mmol), hydroxylamine (50 wt % in water ) (4.28 mL, 71.1 mmol) and water (35.6 mL) was stirred at 75 °C for 22.5 h. The mixture was cooled to rt, was diluted with water, the pH was adjusted to 11 with 1 M sodium hydroxide, and the aqueous phase was extracted with ethyl acetate (5 x). The combined organic extracts were washed with brine, were concentrated under reduced pressure. The residue was chased with dichloromethane (DCM) to provide the title compound as a tan solid (6.15 g, 7.84 mmol, 73.3 % yield). LCMS (m/z) 432.4 (M+H)⁺.

### Step 5: ((1R,4S)-1-((Benzyloxy)methyl)-4-(((3S,4S)-3-methoxytetrahydro-2H-pyran-4-yl)amino)cyclopent-2-en-1-yl)(3-(trifluoromethyl)-7,8-dihydro-1,6-naphthyridin-6(5H)-yl)methanone

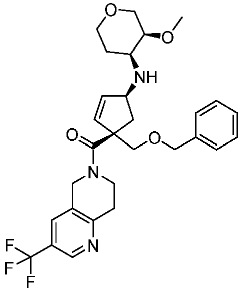

To a mixture of ((1*R*,4*S*)-4-amino-1-((benzyloxy)methyl)cyclopent-2-en-1-yl)(3-(trifluoromethyl)-7,8-dihydro-1,6-naphthyridin-6(5H)-yl)methanone (2.08 g, 4.82 mmol), isopropyl acetate (48.2 mL), and 2-propanol (1.114 mL, 14.46 mmol). at 0 °C was added sodium triacetoxyborohydride (3.07 g, 14.46 mmol), and the mixture was stirred for 1 hr. (*R*)-3-methoxytetrahydro-4H-pyran-4-one (0.941 g, 7.23 mmol) in isopropyl acetate (6 mL) was added and the mixture was stirred at 0 °C for 1 h. Saturated aqueous NaHCO₃ (20 mL), water (30 mL), and ethyl acetate (20 mL) were added and the aqueous phase was extracted with ethyl acetate (5 x). The combined organic extracts were washed with 1 M sodium hydroxide (1 x), with brine (1 x), were dried over Na₂SO₄, were filtered, and the filtrate was concentrated under reduced pressure to provide the title compound a tan solid (3.46 g, 4.82 mmol, 100 % yield). LCMS (m/z) 546.3 (M+H)⁺.

### Step 6: ((1S,3R)-1-((Benzyloxy)methyl)-3-(((3S,4S)-3-methoxytetrahydro-2H-pyran-4-yl)amino)cyclopentyl)(3-(trifluoromethyl)-7,8-dihydro-1,6-naphthyridin-6(5H)-yl)methanone.

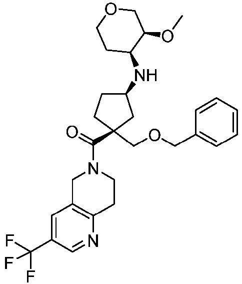

A mixture of ((1*R*,4*S*)-1-((benzyloxy)methyl)-4-(((3*S*,4*S*)-3-methoxytetrahydro-2H-pyran-4-yl)amino)cyclopent-2-en-1-yl)(3-(trifluoromethyl)-7,8-dihydro-1,6-naphthyridin-6(5H)-yl)methanone (2.63 g, 4.82 mmol) and Pd/C (0.513 g, 0.482 mmol) in methanol (48.2 mL) was stirred under an atmosphere hydrogen at rt for 2.5 days. The mixture was filtered through Celite^{®}, the catalyst was washed with methanol, and the combined filtrates were concentrated to provide the title compound as a yellow solid (1.674 g, 2.109 mmol, 43.8 % yield). LCMS (m/z) 548.3 (M+H)⁺.

### Step 7: ((1S,3R)-1-(Hydroxymethyl)-3-(((3S,4S)-3-methoxytetrahydro-2H-pyran-4-yl)amino)cyclopentyl)(3-(trifluoromethyl)-7,8-dihydro-1,6-naphthyridin-6(5H)-yl)methanone.

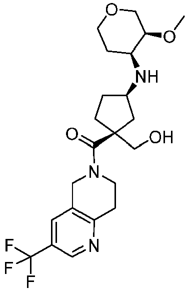

To a solution of ((1*S*,3*R*)-1-((benzyloxy)methyl)-3-(((3*S*,4*S*)-3-methoxytetrahydro-2H-pyran-4-yl)amino)cyclopentyl)(3-(trifluoromethyl)-7,8-dihydro-1,6-naphthyridin-6(5H)-yl)methanone (1.479 g, 2.7 mmol) in methanol (26 mL) was added Pd(OH)₂ (2.085 g, 2.97 mmol), hydrogen gas was bubbled into the mixture for 5 min, and the mixture was stirred at rt for 1 h. Acetic acid (3.25 mL) was added and the mixture was stirred under an atmosphere of hydrogen for 5.5 hours. The mixture was opened to the atmosphere for 15 min and was filtered through Celite^{®} with methanol. This solution was combined with similar reaction, beginning with ((1*S*,3*R*)-1-((benzyloxy)methyl)-3-(((3*S*,4*S*)-3-methoxytetrahydro-2H-pyran-4-yl)amino)cyclopentyl)(3-(trifluoromethyl)-7,8-dihydro-1,6-naphthyridin-6(5H)-yl)methanone (0.290 g, 0.530 mmol). Purification by ISCO CombiFlash^{®} chromatography (24 g silica Gold column, 35 mL/min) eluting with a gradient of 0 to 100 % methanol containing ammonium hydroxide (10 %) in dichloromethane (DCM) provided the title compound as a white solid (265 mg, 0.441 mmol, 16.32 % yield). LCMS (m/z) 458.4 (M+H)⁺.

### Step 8: 2-(((1S,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)methoxy)acetonitrile

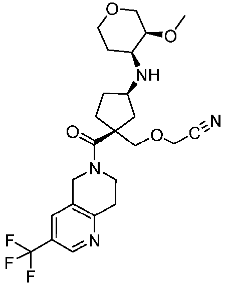

A mixture of sodium hydride (60% in mineral oil) (0.060 g, 1.500 mmol) and ((1*S*,3*R*)-1-(hydroxymethyl)-3-(((4*S*)-3-methoxytetrahydro-2H-pyran-4-yl)amino)cyclopentyl)(3-(trifluoromethyl)-7,8-dihydro-1,6-naphthyridin-6(5H)-yl)methanone (0.107 g, 0.234 mmol) in tetrahydrofuran (THF) (2.339 mL) was stirred at 0 °C for 10 min. Commercially-available 2-bromoacetonitrile (0.050 mL, 0.718 mmol) was added dropwise and the mixture was stirred at 0 °C for 30 min. Saturated aqueous NH₄Cl (10 mL) and dichloromethane (DCM) were added and the organic phase was concentrated under a steady stream of nitrogen. Purification by ISCO CombiFlash^{®} chromatography (12 g silica Gold column, 30 mL/min) eluting with a gradient of 0 to 100 % methanol in dichloromethane (DCM) provided the title compound as a brown oil (51 mg, 0.075 mmol, 32.1 % yield). LCMS (m/z) 497.7 (M+H)⁺.

### Step 9: ((1S,3R)-1-((2-Aminoethoxy)methyl)-3-(((3S,4S)-3-methoxytetrahydro-2H-pyran-4-yl)amino)cyclopentyl)(3-(trifluoromethyl)-7,8-dihydro-1,6-naphthyridin-6(5H)-yl)methanone, 3acetic acid salt.

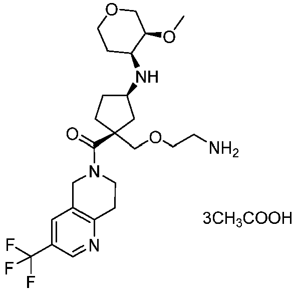

To a solution of 2-(((1*S*,3*R*)-3-(((3*S*,4*S*)-3-methoxytetrahydro-2H-pyran-4-yl)amino)-1-(3-(trifluoromethyl)-5,6,7,8-tetrahydro-1,6-naphthyridine-6-carbonyl)cyclopentyl)methoxy)acetonitrile (0.250 g, 0.503 mmol) in isopropanol (4 mL) and acetic acid (2.000 mL) was added Pd/C (0.268 g, 0.252 mmol) and the reaction was stirred under an atmosphere of hydrogen for 1 h. The mixture was stirred open to air for 10 minutes This mixture was combined with a similar reaction, beginning with 2-(((1*S*,3*R*)-3-(((3S,4S)-3-methoxytetrahydro-2H-pyran-4-yl)amino)-1-(3-(trifluoromethyl)-5,6,7,8-tetrahydro-1,6-naphthyridine-6-carbonyl)cyclopentyl)methoxy)acetonitrile (0.050 g, 0.100 mmol). The combined mixtures were filtered through Celite^{®} and were concentrated to provide the title compound as an off-white solid (314 mg, 0.302 mmol, 100% yield). LCMS (m/z) 501.4 (M+H)⁺.

### Intermediate 10: (1r,4r)-4-(2-(Dibenzylamino)ethoxy)cyclohexan-1-ol

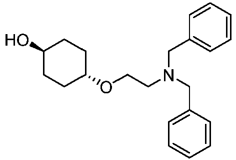

A mixture of 5 N sodium hydroxide (67.5 mL, 338 mmol), solid sodium hydroxide (13.50 g, 338 mmol), 1,4-dioxane (100 mL), toluene (50 mL), tetrabutylammonium iodide (1.247 g, 3.38 mmol), commercially-available (1r,4r)-cyclohexane-1,4-diol (5.88 g, 50.6 mmol), and commercially-available N,N-dibenzyl-2-chloroethan-1-amine, hydrochloric acid salt (10 g, 33.8 mmol) was stirred at 55 °C for 19.5 h, at 60 °C for 24.5 h, and at 65 °C for 24 h. NH₄Cl (10 g) was added and the mixture was extracted with ethyl acetate (3 x 150 mL). The combined organic extracts were washed with brine (100 mL), were dried over MgSO₄, were filtered, and the filtrate was concentrated under reduced pressure. Dichloromethane (DCM) (100 mL) was added, the mixture was filtered, and the filtrate was purified by ISCO CombiFlash^{®} chromatography (120 g Gold column, 85 mL/min) eluting with a gradient of 30 to 70 % ethyl acetate in heptane to provide the title compound (5.8227 g, 17.15 mmol, 50.8 % yield). LCMS (m/z) 340.4 (M+H)⁺.

### Intermediate 11: tert-Butyl (4-(6-((1S,3R)-1-isopropyl-3-(((3S,4S)-3-methoxytetrahydro-2H-pyran-4-yl)amino)cyclopentane-1-carbonyl)-3-(trifluoromethyl)-5,6,7,8-tetrahydro-1,6-naphthyridin-2-yl)butyl)carbamate.

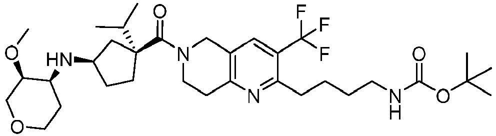

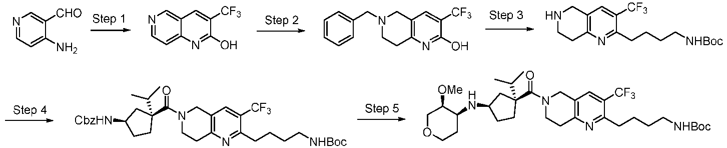

### Step 1: 3-(Trifluoromethyl)-1,6-naphthyridin-2-ol.

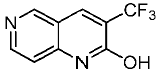

A solution of 4-aminonicotinaldehyde (5.1 g, 41.8 mmol) and DIPEA (10.94 mL, 62.6 mmol) in dichloromethane (DCM) (100 mL was added dropwise to a solution of 3,3,3-trifluoropropanoyl chloride (9.18 g, 62.6 mmol) in dichloromethane (DCM) (200 mL) at 20 °C over 20 min. The mixture was stirred for 1.5 h, saturated aqueous NaHCO₃ (200 mL) was added, and the mixture was stirred vigorously at rt for 5 min. The organic phase was dried over Na₂SO₄, was filtered, and the filtrate was concentrated under reduced pressure. The residue was dissolved in N,N-dimethylformamide (DMF) (100 mL), K₂CO₃ (5.77 g, 41.8 mmol) was added, and the mixture was heated to 70 °C for 15 min and was cooled to rt. Water (100 mL) was added followed by slow addition of 1.0 M hydrochloric acid (84 mL, 84 mmol). The mixture was stirred at rt for 1 h and was concentrated under reduced pressure. Water (200 mL) was added to the residue. The resulting precipitate was collected by filtration, was rinsed with water, and was air-dried to provide the title compound as a dark yellow solid (7.24 g, 33.8 mmol, 81 % yield). LC-MS m/z 215.1 (M+H)⁺.

### Step 2: 6-Benzyl-3-(trifluoromethyl)-5,6,7,8-tetrahydro-1,6-naphthyridin-2-ol.

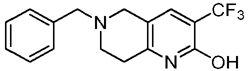

To a suspension of 3-(trifluoromethyl)-1,6-naphthyridin-2-ol (6.66 g, 31.1 mmol) in acetonitrile (50 mL) was added benzyl bromide (4.07 mL, 34.2 mmol). The mixture was refluxed for 2 h and was concentrated under reduced pressure. The residue was dissolved in methanol (200 mL) and water (60 mL) was added. The flask was purged with nitrogen, sodium borohydride (5.88 g, 156 mmol) was added in portions over 30 min, and the mixture was stirred under an atmosphere of nitrogen at rt for 1 h. Methanol (200 mL) was added, followed by the addition of sodium borohydride (5.88 g, 156 mmol) over the course of 30 minutes. The reaction mixture was stirred under an atmosphere of nitrogen for 30 minutes, and additional sodium borohydride (5.88 g, 156 mmol) was added in portions over the course of 30 minutes. The reaction mixture was stirred at rt for 1 h and was concentrated under reduced pressure. The residue was suspended in water (300 mL) and the resulting precipitate was collected by filtration. Purification by ISCO CombiFlash^{®} (120 g RediSep Gold^{®} column, 85 mL/min) eluting with a gradient of 0 to 75 % dichloromethane (DCM):methanol (4:1) in dichloromethane (DCM) provided the title compound as a yellow-orange solid (3.75 g, 12.16 mmol, 39 % yield). LC-MS m/z 309.3 (M+H)⁺.

### Step 3: tert-Butyl (4-(3-(trifluoromethyl)-5,6,7,8-tetrahydro-1,6-naphthyridin-2-yl)butyl)carbamate.

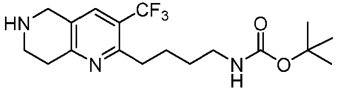

To a solution of 6-benzyl-3-(trifluoromethyl)-5,6,7,8-tetrahydro-1,6-naphthyridin-2-ol (3.75 g, 12.16 mmol) in dichloromethane (DCM) (200 mL) at 0 °C was added a solution of trifluoromethanesulfonic anhydride (2.260 mL, 13.38 mmol) in dichloromethane (DCM) (20 mL) dropwise over 5 min. The mixture was stirred at rt for 1 h and was concentrated under reduced pressure. Bis(triphenylphosphine)palladium(II) chloride (0.171 g, 0.243 mmol) and copper(I) iodide (0.069 g, 0.365 mmol) were added to the residue. The mixture was purged with nitrogen and degassed N,N-dimethylformamide (DMF) (100 mL), tert-butyl but-3-yn-1-ylcarbamate (3.09 g, 18.24 mmol), and DIPEA (6.37 mL, 36.5 mmol) were added sequentially. The mixture was stirred at 80 °C for 15 h and was concentrated under reduced pressure. 20 % palladium hydroxide on carbon (8.54 g, 12.16 mmol) and methanol (100 mL) were added to the residue, hydrogen was bubbled into the mixture for 10 min, and the mixture was stirred for 5.5 h The mixture was stirred opened to air for 15 min and was filtered. The catalyst was washed with 2 M NH₃ in methanol (100 mL) and with methanol (100 mL). and the combined filtrates were concentrated under reduced pressure. The residue was partitioned between dichloromethane (DCM) (200 mL) and 1.0 M NaOH (100 mL) and the aqueous layer was extracted with dichloromethane (DCM) (200 mL). The combined organic extracts were dried over Na₂SO₄, were filtered and the filtrate was concentrated under reduced pressure. Purification by ISCO CombiFlash^{®} (220 g RediSep Gold^{®} column, 150 mL/min) eluting with a 5 % methanol (containing ammonium hydroxide) in dichloromethane (DCM) provided the title compound as a pale-yellow oil (3.0 g, 8.03 mmol, 66 % yield). LC-MS m/z 374.1 (M+H)⁺.

### Step 4: Benzyl ((1R,3S)-3-(2-(4-((tert-butoxycarbonyl)amino)butyl)-3-(trifluoromethyl)-5,6,7,8-tetrahydro-1,6-naphthyridine-6-carbonyl)-3-isopropylcyclopentyl)carbamate.

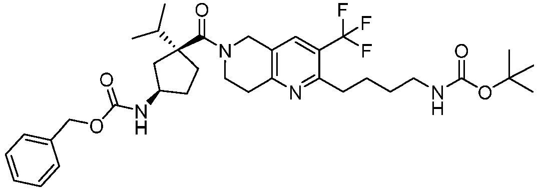

To (1S,3R)-3-(((benzyloxy)carbonyl)amino)-1-isopropylcyclopentane-1-carboxylic acid (2.62 g, 8.57 mmol) (dried prior to use by azeotroping with toluene) was added sequentially dichloromethane (DCM) (15 mL), HATU (1.751 g, 4.61 mmol), and DIPEA (5.99 mL, 34.3 mmol) and the mixture was stirred at rt for 3 h. A solution of tert-butyl (4-(3-(trifluoromethyl)-5,6,7,8-tetrahydro-1,6-naphthyridin-2-yl)butyl)carbamate (1.60 g, 4.28 mmol) in dichloromethane (DCM) (7.50 mL) was added dropwise and the mixture was stirred at rt for 67 h. The reaction mixture was split evenly into two 20 mL sealed tubes and was heated at 80 °C for 25 h. The mixtures were combined, dichloromethane (DCM) (100 mL), water (50 mL), and 1.0 M NaOH (50 mL) were added, and the mixture was stirred vigorously for 5 min. the organic phase was dried over Na₂SO₄, was filtered, and the filtrate was concentrated under reduced pressure. Purification by ISCO CombiFlash^{®} (80 g RediSep Gold^{®} column, 22 mL/min) eluting with a gradient of 0 to 22 % ethyl acetate:ethanol (3:1) in hexanes provided the title compound as a pale-yellow foam (2.61 g, 3.95 mmol, 92 % yield). LC-MS m/z 661.4 (M+H)⁺.

### Step 5: tert-Butyl (4-(6-((1S,3R)-1-isopropyl-3-(((3S,4S)-3-methoxytetrahydro-2H-pyran-4-yl)amino)cyclopentane-1-carbonyl)-3-(trifluoromethyl)-5,6,7,8-tetrahydro-1,6-naphthyridin-2-yl)butyl)carbamate.

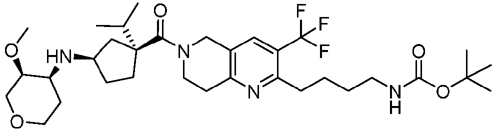

A mixture of benzyl ((1R,3S)-3-(2-(4-((tert-butoxycarbonyl)amino)butyl)-3-(trifluoromethyl)-5,6,7,8-tetrahydro-1,6-naphthyridine-6-carbonyl)-3-isopropylcyclopentyl)carbamate (2.61 g, 3.95 mmol) and 20 % palladium hydroxide on carbon (0.832 g, 1.185 mmol) in methanol (100 mL) was purged with nitrogen and by hydrogen. Hydrogen was bubbled through the mixture for 2 min, and the reaction mixture was stirred under a balloon atmosphere of hydrogen for 30 min. The mixture was opened to air, 7.0 M ammonia in methanol (50 mL) was added, and was stirred for 15 min. The mixture was filtered, the catalyst was washed with methanol, and the combined filtrates were concentrated under reduced pressure. isopropyl acetate (50 mL), 2-propanol (0.761 mL, 9.87 mmol), and triethylamine (0.551 mL, 3.95 mmol) were added to the residue, the mixture was cooled to 0 °C and sodium triacetoxyborohydride (1.842 g, 8.69 mmol) was added followed by dropwise addition of a solution of (R)-3-methoxytetrahydro-4H-pyran-4-one (0.900 g, 6.91 mmol) in isopropyl acetate (20 mL). The mixture was stirred at 0 °C for 10 min, the cooling bath was removed and the mixture was stirred at rt for 1 h. Ethyl acetate was added and the organic phase was washed with 1.0 M NaOH. The aqueous layer was extracted with ethyl acetate and the combined organic extracts were dried over Na₂SO₄, were filtered, and the filtrate was concentrated under reduced pressure. Purification by ISCO CombiFlash^{®} (120 g RediSep Gold^{®} column, 85 mL/min) eluting with a gradient of 0 to 2 % methanol in dichloromethane (DCM) provided the title compound as a foam. LC-MS m/z 641.2 (M+H)⁺.

### Intermediate 12: (2-(4-Aminobutyl)-3-(trifluoromethyl)-7,8-dihydro-1,6-naphthyridin-6(5H)-yl)((1S,3R)-1-isopropyl-3-(((3S,4S)-3-methoxytetrahydro-2H-pyran-4-yl)amino)cyclopentyl)methanone, 2hydrochloride, 1,4-dioxane

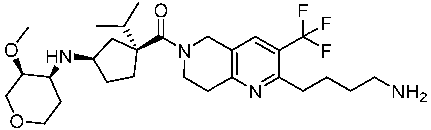

To the mixture of tert-butyl (4-(6-((1S,3R)-1-isopropyl-3-(((3S,4S)-3-methoxytetrahydro-2H-pyran-4-yl)amino)cyclopentane-1-carbonyl)-3-(trifluoromethyl)-5,6,7,8-tetrahydro-1,6-naphthyridin-2-yl)butyl)carbamate (210 mg, 0.328 mmol) in 1,4-dioxane (3 mL) was added HCl (4 M in p-dioxane) (0.819 mL, 3.28 mmol). The result reaction mixture was stirred at ambient temperature for 3 h. The reaction mixture was evaporated down under vacuum to give the title product (223.2 mg, 0.318 mmol, 97 % yield) LC/MS (M+H)⁺ 541.4 m/z.

### Intermediate 13: 3-(4-(6-((1S,3R)-1-isopropyl-3-(((3S,4S)-3-methoxytetrahydro-2H-pyran-4-yl)amino)cyclopentane-1-carbonyl)-3-(trifluoromethyl)-5,6,7,8-tetrahydro-1,6-naphthyridin-2-yl)butoxy)benzoic acid.

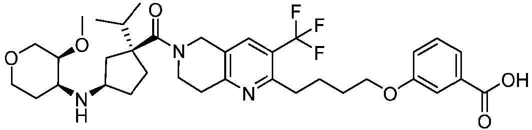

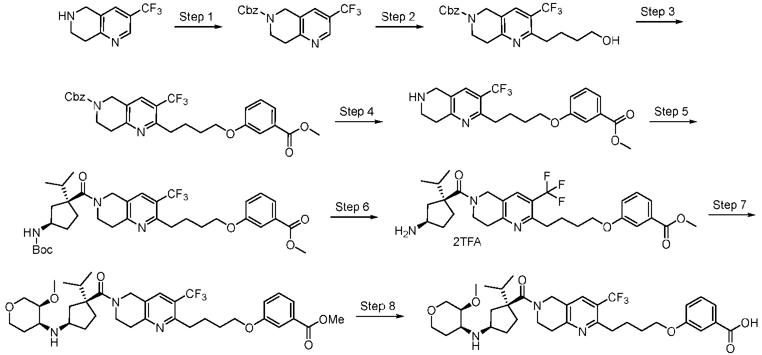

### Step 1: Benzyl 3-(trifluoromethyl)-7,8-dihydro-1,6-naphthyridine-6(5H)-carboxylate.

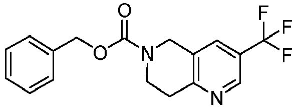

A mixture of 3-(trifluoromethyl)-5,6,7,8-tetrahydro-1,6-naphthyridine, 2hydrochloric acid salt (1000 mg, 3.64 mmol) and DIPEA (1.905 mL, 10.91 mmol) in N,N-dimethylformamide (DMF) (20 mL) was stirred under an atmosphere of nitrogen at rt for 5 minutes and benzyl chloroformate (0.519 mL, 3.64 mmol) was added dropwise over 5 min. The mixture was stirred at rt for 3 h and water was added. The organic phase was washed with brine (2 x 30 mL). The aqueous phase was extracted with ethyl acetate (2 x 30 mL). The combined organic extracts were dried over MgSO₄, were filtered, and the filtrate was concentrated under reduced pressure. Purification by normal-phase silica gel chromatography (40 g RediSep Gold^{®} column) eluting with a stepwise gradient of 30 % and 50 % ethyl acetate in hexanes provided the title compound as a yellow, waxy solid (1100 mg, 3.27 mmol, 90 % yield). LC-MS m/z 337.1 (M+H)⁺.

### Step 2: Benzyl 2-(4-hydroxybutyl)-3-(trifluoromethyl)-7,8-dihydro-1,6-naphthyridine-6(5H)-carboxylate.

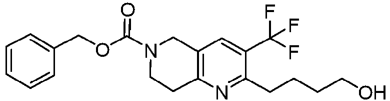

To a mixture of benzyl 3-(trifluoromethyl)-7,8-dihydro-1,6-naphthyridine-6(5H)-carboxylate (2.5 g, 7.43 mmol), [4,4'-bis(1,1-dimethylethyl)-2,2'-bipyridine-N1,N1']bis[3,5-difluoro-2-[5-(trifluoromethyl)-2-pyridinyl-N]phenyl-C]Iridium(III) hexafluorophosphate, [Ir{dF(CF3)ppy}2(dtbpy)]PF6 (0.125 g, 0.112 mmol), 6 M HCl (1.363 mL, 8.18 mmol), and trifluoroacetic acid (4.01 mL, 52.0 mmol) in methanol (9 mL) was added benzoyl peroxide, Luperox^{®} A75 (5.40 g, 16.73 mmol) and tetrahydrofuran (THF) (45.0 mL).

The mixture was irradiated with an H150-Blue Kessil lamp at 1 inch while encased in aluminum foil. House air was blown on the reaction vessel and the mixture was stirred at rt for 48 h. The light was removed and the mixture was stirred overnight. The mixture was cooled in an ice bath and saturated aqueous NaHCO₃ (100 mL) was added. The aqueous phase was extracted with ethyl acetate. The organic extract was washed with brine (50 mL). The aqueous phase was extracted again with ethyl acetate and the combine organic extracts were dried over Na₂SO₄, were filtered, and the filtrate was concentrated under reduced pressure. Purification by ISCO CombiFlash^{®} (120 g RediSep Gold^{®} column) eluting with a stepwise gradient of 30 to 50 % and 75 to 100 % ethyl acetate in hexanes provided the title compound as a light orange oil that partially solidified (1780 mg, 4.361 mmol, 58.7 % yield). LC-MS m/z 409.2 (M+H)⁺.

### Step 3: Benzyl 2-(4-(3-(methoxycarbonyl)phenoxy)butyl)-3-(trifluoromethyl)-7,8-dihydro-1,6-naphthyridine-6(5H)-carboxylate.

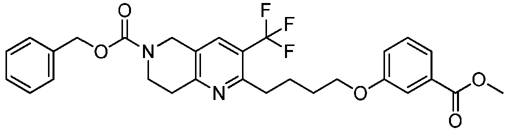

To a solution of benzyl 2-(4-hydroxybutyl)-3-(trifluoromethyl)-7,8-dihydro-1,6-naphthyridine-6(5H)-carboxylate (1780 mg, 4.36 mmol), methyl 3-hydroxybenzoate (995 mg, 6.54 mmol) and triphenylphosphine (1715 mg, 6.54 mmol) in tetrahydrofuran (THF) (40mL) was added diisopropyl (E)-diazene-1,2-dicarboxylate (1.271 mL, 6.54 mmol) The mixture was stirred overnight and was concentrated. Ethyl acetate was added and the organic phase was washed with water and with brine. The organic phase was dried over MgSO₄ and was concentrated. Purification by ISCO CombiFlash^{®} (80 g RediSep Gold^{®} column) eluting with a gradient of 10 to 50 % ethyl acetate in heptane provided the title compound (1580 mg, 2.91 mmol, 66.8 % yield). LC-MS m/z 543.2 (M+H)⁺.

### Step 4: Methyl 3-(4-(3-(trifluoromethyl)-5,6,7,8-tetrahydro-1,6-naphthyridin-2-yl)butoxy)benzoate.

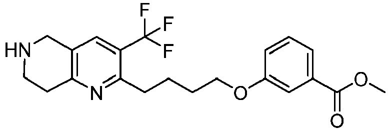

33 % HBr in acetic acid (2.64 mL, 14.56 mmol) was added to a solution of benzyl 2-(4-(3-(methoxycarbonyl)phenoxy)butyl)-3-(trifluoromethyl)-7,8-dihydro-1,6-naphthyridine-6(5H)-carboxylate (1580 mg, 2.91 mmol) in dichloromethane (DCM) (20 mL), the mixture was stirred at rt for 2 h, and was concentrated. Purification by ISCO CombiFlash^{®} (40 g RediSep Gold^{®} column) eluting with a gradient of 30 to 70 % methanol in dichloromethane (DCM) containing ammonium hydroxide (1 %) provided the title compound (1150 mg, 2.017 mmol, 69.3 % yield). LC-MS m/z 409.2 (M+H)⁺.

### Step 5: Methyl 3-(4-(6-((1S,3R)-3-((tert-butoxycarbonyl)amino)-1-isopropylcyclopentane-1-carbonyl)-3-(trifluoromethyl)-5,6,7,8-tetrahydro-1,6-naphthyridin-2-yl)butoxy)benzoate.

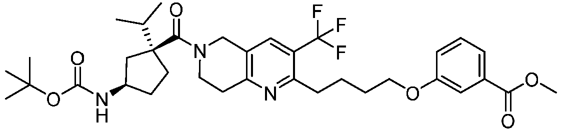

To a mixture of methyl 3-(4-(3-(trifluoromethyl)-5,6,7,8-tetrahydro-1,6-naphthyridin-2-yl)butoxy)benzoate (1150 mg, 2.82 mmol) in dichloromethane (DCM) (2 mL) was added DIPEA (2.459 mL, 14.08 mmol). The solution was stirred for 5 minutes, then DMAP (275 mg, 2.253 mmol), (1S,3R)-3-((tert-butoxycarbonyl)amino)-1-isopropylcyclopentane-1-carboxylic acid (1375 mg, 5.07 mmol) were added, followed by PyBroP (3282 mg, 7.04 mmol) and the mixture was stirred at rt for 4 days. Saturated aqueous NaHCO₃ (15 mL) was added and the aqueous phase was extracted with dichloromethane (DCM) (2 x 150 mL). The combined organic extracts were dried over MgSO₄, were filtered, and the filtrate was concentrated under reduced pressure. Purification by ISCO CombiFlash^{®} (40 g RediSep Gold^{®} column) eluting with a gradient of 15 to 70 % ethyl acetate in heptane provided the title compound (1100 mg, 1.496 mmol, 53.1 % yield). LC-MS m/z 662.5 (M+H)⁺.

### Step 6: Methyl 3-(4-(6-((1S,3R)-3-amino-1-isopropylcyclopentane-1-carbonyl)-3-(trifluoromethyl)-5,6,7,8-tetrahydro-1,6-naphthyridin-2-yl)butoxy)benzoate, 2Trifluoroacetic acid salt.

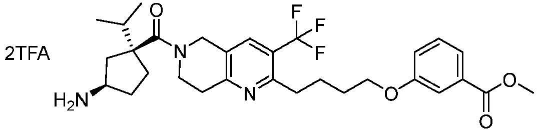

To a solution of methyl 3-(4-(6-((1S,3R)-3-((tert-butoxycarbonyl)amino)-1-isopropylcyclopentane-1-carbonyl)-3-(trifluoromethyl)-5,6,7,8-tetrahydro-1,6-naphthyridin-2-yl)butoxy)benzoate (1.14 g, 1.723 mmol) in dichloromethane (DCM) (10 mL) was added trifluoracetic acid (2.65 mL, 34.5 mmol). The reaction mixture was stirred at rt for 2 h and was concentrated under reduced pressure. The residue was chased with dichloromethane (DCM) 3x 3 mL) and dried under high vacuum to provide the title compound as an orange oil (1.35 g, 1.710 mmol, 99 % yield). LC-MS m/z 562.3 (M+H)⁺.

### Step 7: Methyl 3-(4-(6-((1S,3R)-1-isopropyl-3-(((3S,4S)-3-methoxytetrahydro-2H-pyran-4-yl)amino)cyclopentane-1-carbonyl)-3-(trifluoromethyl)-5,6,7,8-tetrahydro-1,6-naphthyridin-2-yl)butoxy)benzoate.

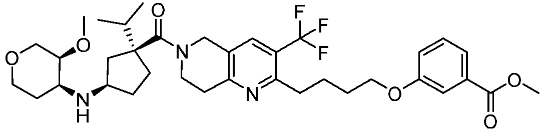

To a solution of methyl 3-(4-(6-((1S,3R)-3-amino-1-isopropylcyclopentane-1-carbonyl)-3-(trifluoromethyl)-5,6,7,8-tetrahydro-1,6-naphthyridin-2-yl)butoxy)benzoate, 2trifluoroacetic acid salt (1.34 g, 1.701 mmol) in dichloromethane (DCM) (17 mL) was added DIPEA (0.891 mL, 5.10 mmol) and The mixture was stirred for 5 min. 4 Å powder, activated, molecular sieves (~325 mesh, Aldrich) (1 g, 1.701 mmol), (R)-3-methoxytetrahydro-4H-pyran-4-one (0.266 g, 2.041 mmol) followed by sodium triacetoxyborohydride (0.541 g, 2.55 mmol) were added and the mixture was stirred under an atmosphere of nitrogen at rt for 22 h. Dichloromethane (DCM) (20 mL) was added. The organic phase was washed with saturated aqueous NaHCO₃ (25 mL) and with brine (25 mL). The combined aqueous phase was extracted with dichloromethane (DCM) (20 mL). The combined organic extracts were dried over MgSO₄, were filtered, and the filtrate was concentrated under reduced pressure. Purification by ISCO Lumen CombiFlash^{®} (80 g RediSep Gold^{®} column, 60 mL/min) eluting with a gradient of 10 to 90 % dichloromethane (DCM):methanol (4:1) in dichloromethane (DCM) provided the title compound as a colorless oil (505 mg, 0.747 mmol, 43.9 % yield) as a colorless oil. LC-MS m/z 676.4 (M+H)⁺.

### Step 8: 3-(4-(6-((1S,3R)-1-Isopropyl-3-(((3S,4S)-3-methoxytetrahydro-2H-pyran-4-yl)amino)cyclopentane-1-carbonyl)-3-(trifluoromethyl)-5,6,7,8-tetrahydro-1,6-naphthyridin-2-yl)butoxy)benzoic acid.

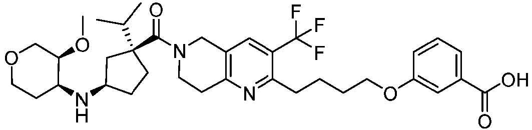

To a solution of methyl 3-(4-(6-((1S,3R)-1-isopropyl-3-(((3S,4S)-3-methoxytetrahydro-2H-pyran-4-yl)amino)cyclopentane-1-carbonyl)-3-(trifluoromethyl) 5,6,7,8-tetrahydro-1,6-naphthyridin-2-yl)butoxy)benzoate (496 mg, 0.734 mmol) in ethanol (5 mL) was added a solution of lithium hydroxide (92 mg, 2.202 mmol) in water (5.00 mL). The reaction mixture was stirred at rt for 68 h and most of the ethanol was removed under reduced pressure. The resulting aqueous mixture was adjusted to pH ~7.2 with 1 N HCl (~2.1 mL) and was extracted with dichloromethane (DCM) (3 x 20 mL). The combined organic extracts were dried over MgSO₄, were filtered, and the filtrate was concentrated under reduced pressure. The residue was placed under high vacuum to provide the title compound as a foam (380 mg, 0.574 mmol, 78 % yield). LC-MS m/z 662.4 (M+H)⁺.

### Intermediate 14: ((1S,3S)-3-((3-aminopropyl)((3S,4S)-3-methoxytetrahydro-2H-pyran-4-yl)amino)-1-isopropylcyclopentyl)(3-(trifluoromethyl)-7,8-dihydro-1,6-naphthyridin-6(5H)-yl)methanone

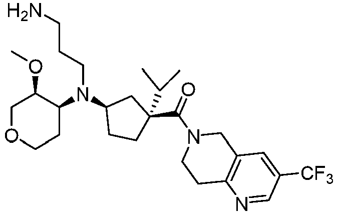

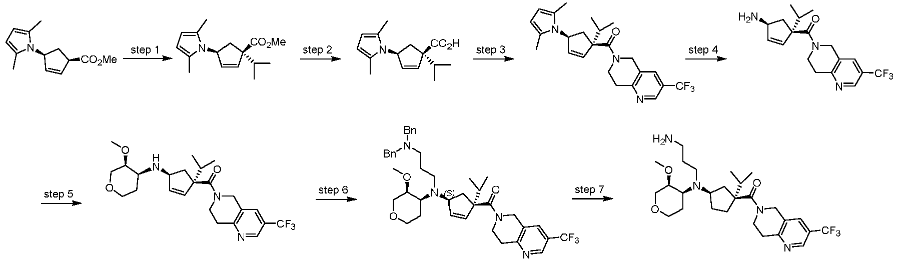

### Step 1: methyl (1S,4S)-4-(2,5-dimethyl-1H-pyrrol-1-yl)-1-isopropylcyclopent-2-ene-1-carboxylate

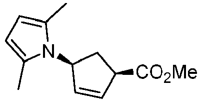

To a mixture of methyl (1R,4S)-4-(2,5-dimethyl-1H-pyrrol-1-yl)cyclopent-2-ene-1-carboxylate (1000 mg, 4.56 mmol) in THF (10 mL) at -20 °C was added LHMDS in THF (7.30 mL, 7.30 mmol) dropwise. The resulting mixture was stirred at -20 °C for 30 mins, followed by the addition of 2-iodopropane (0.912 mL, 9.12 mmol) in THF (10 mL). The mixture was stirred at rt for 1 hour, then warmed to room temperature until LC-MS showed consumption of starting material. The reaction was quenched with 6% aqueous solution of NH₄Cl (50 mL) and diluted with ethyl acetate. The organic layer was separated, then washed with water (1X 50 mL), dried over sodium sulfate and concentrated in vacuo. The crude material was then purified via column chromatograph (RediSepRf high performance GOLD 24g HP Silica with CH₂Cl₂ / 20%MeOH in CH₂Cl₂ as the mobile phase) to yield methyl (1S,4S)-4-(2,5-dimethyl-1H-pyrrol-1-yl)-1-isopropylcyclopent-2-ene-1-carboxylate in 90% purity (1.06 g, 80% yield) as light brown oil. LCMS MS (m/z) 262.2 (M+H)⁺

### Step 2: (1S,4S)-4-(2,5-dimethyl-1H-pyrrol-1-yl)-1-isopropylcyclopent-2-ene-1-carboxylic acid

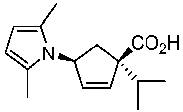

Into 20 mL microwave vial was added methyl (1S,4S)-4-(2,5-dimethyl-1H-pyrrol-1-yl)-1-isopropylcyclopent-2-ene-1-carboxylate (2000 mg, 7.65 mmol), Methanol (3 mL), and potassium hydroxide (3.06 mL, 30.6 mmol). The resulting mixture was heated to 120 °C for 3 hours. LC-MS showed the completed formation of the product. MeOH was removed under vacuo. The reaction mixture was then placed on an ice bath. The pH of the solution was adjusted to ~2 by 12N HCl. The light brown precipitate was hard to be filtered. The reaction solution was then extracted by ethyl acetate (2X 30 mL). It was then purified on column chromatograph using RediSepRf high performance GOLD 24g HP Silica with DCM / 20%MeOH in DCM as the mobile phase with gradient 0-100% to yield the desired product (1S,4S)-4-(2,5-dimethyl-1H-pyrrol-1-yl)-1-isopropylcyclopent-2-ene-1-carboxylic acid (1600 mg, 5.82 mmol, 76 % yield), as a light brown solid. LCMS (m/z) 248.2 (M+H)⁺.

### Step 3: ((1S,4S)-4-(2,5-dimethyl-1H-pyrrol-1-yl)-1-isopropylcyclopent-2-en-1-yl)(3-(trifluoromethyl)-7,8-dihydro-1,6-naphthyridin-6(5H)-yl)methanone

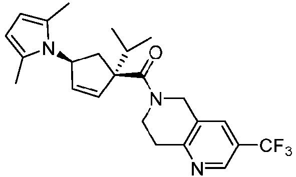

To a 100 mL round bottom flask, (1S,4S)-4-(2,5-dimethyl-1H-pyrrol-1-yl)-1-isopropylcyclopent-2-ene-1-carboxylic acid (1 g, 4.04 mmol) was added as a red amber syrup. The syrup was dissolved in Tetrahydrofuran (THF) (10 mL). To the dark solution, DIEA (1.412 mL, 8.09 mmol) was added. The reaction mixture was cooled to 0°C . Ms-Cl (0.347 mL, 4.45 mmol) was added dropwise over 2 minutes. The reaction was allowed to warm to room temperature. It was stirred at room temperature for 4 hours. The reaction mixture was cooled in ice for 3 minutes, which was followed by the addition of 3-(trifluoromethyl)-5,6,7,8-tetrahydro-1,6-naphthyridine, 2Hydrochloride (1.008 g, 3.66 mmol). The reaction was warmed gradually to ambient temperatures. Another volume of DIEA (0.706 mL, 4.04 mmol) was added. The reaction was allowed to stir at room temperature for 45 minutes. The reaction mixture was added to a separatory funnel, along with 50 mL of 5% NaHCO₃. The two layers were partitioned and the resulting aqueous layer was extracted twice with ethyl acetate. The organic layers were combined and washed with water. The resulting organic solutions were combined, and concentrated under vacuum to afford a deep brown oil. The oil was pumped overnight, purified using a gold 40 g silica gel column cartridge on the ISCO with a 10% MeOH/DCM mobile phase. LCMS analysis indicated contamination with the starting carboxylic acid. In order remove the carboxylic acid starting material, the fractions were combined and washed with saturated bicarbonate 5 times. The resulting organic layers were washed with brine, and dried over sodium sulfate, then filtered, concentrated, and purified using the ISCO, 40 g silica gel column gold, with a 5% MeOH/DCM mobile phase to afford ((1S,4S)-4-(2,5-dimethyl-1H-pyrrol-1-yl)-1-isopropylcyclopent-2-en-1-yl)(3-(trifluoromethyl)-7,8-dihydro-1,6-naphthyridin-6(5H)-yl)methanone. LCMS (m/z) 432.35 (M+H)⁺.

### Step 4: ((1S,4S)-4-amino-1-isopropylcyclopent-2-en-1-yl)(3-(trifluoromethyl)-7,8-dihydro-1,6-naphthyridin-6(5H)-yl)methanone, 2Hydrochloride

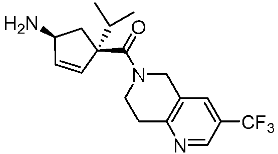

To a 50 mL round bottom flask, hydroxylamine hydrochloride (1.025 g, 14.75 mmol) was added and dissolved in water. To the solution, hydroxylamine 50% w/w (aq) (6.56 mL, 2.508 mmol) was added. ((1S,4S)-4-(2,5-dimethyl-1H-pyrrol-1-yl)-1-isopropylcyclopent-2-en-1-yl)(3-(trifluoromethyl)-7,8-dihydro-1,6-naphthyridin-6(5H)-yl)methanone (1.0823 g, 2.508 mmol) was dissolved in Methanol (10.35 mL) and added via pipette to the solution of hydroxylamine. The reaction was refluxed at 71.5°C for 16 hours. The reaction mixture was cooled to room temperature. The pH was adjusted to 11 using 10 N NaOH. Thereafter, the solution was diluted with water and transferred to a 250 mL separatory funnel. The aqueous layer was exhaustively extracted 4 times with 10 mL of EtOAc each. The organic layers were combined and concentrated. The resulting orange oil was purified on the HPH reverse phase ISCO to afford ((1S,4S)-4-amino-1-isopropylcyclopent-2-en-1-yl)(3-(trifluoromethyl)-7,8-dihydro-1,6-naphthyridin-6(5H)-yl)methanone, 2Hydrochloride (.717 g, 1.682 mmol, 67.1 % yield) as an orange oil. LCMS (m/z) 354.28 (M+H)⁺.

### Step 5: ((1S,4S)-1-isopropyl-4-(((3S,4S)-3-methoxytetrahydro-2H-pyran-4-yl)amino)cyclopent-2-en-1-yl)(3-(trifluoromethyl)-7,8-dihydro-1,6-naphthyridin-6(5H)-yl)methanone

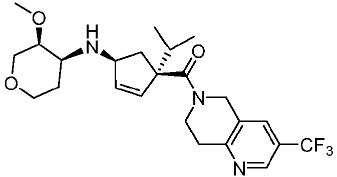

To a 100 mL round bottom flask was added ((1S,4S)-4-amino-1-isopropylcyclopent-2-en-1-yl)(3-(trifluoromethyl)-7,8-dihydro-1,6-naphthyridin-6(5H)-yl)methanone, 2Hydrochloride (0.350 g, 0.821 mmol) dissolved in Isopropyl acetate (3.40 ml). The solution was cooled to 0 °C. 2-propanol (0.278 ml, 3.61 mmol) was then added followed by sodium triacetoxyborohydride (0.766 g, 3.61 mmol). The reaction was stirred at 0 °C for 1 h, (R)-3-methoxytetrahydro-4H-pyran-4-one (0.46 g, 3.53 mmol) was added as an oil. The reaction was stirred at 0 °C for 2 h. Upon completion, the reaction was quenched with ammonium chloride, diluted with 5 mL of water, and extracted with DCM (2x20 mL). The resulting organic solutions were combined, concentrated, and purified on the ISCO using a 0-5% DCM/MeOH ramping elution. ((1S,4S)-1-isopropyl-4-(((3S,4S)-3-methoxytetrahydro-2H-pyran-4-yl)amino)cyclopent-2-en-1-yl)(3-(trifluoromethyl)-7,8-dihydro-1,6-naphthyridin-6(5H)-yl)methanone (312 mg, 0.667 mmol, 81 % yield) as an orange solid. LCMS (m/z) 468.44 (M+H)⁺.

### Step 6: ((1S,4S)-4-((3-(dibenzylamino)propyl)((4S)-3-methoxytetrahydro-2H-pyran-4-yl)amino)-1-isopropylcyclopent-2-en-1-yl)(3-(trifluoromethyl)-7,8-dihydro-1,6-naphthyridin-6(5H)-yl)methanone

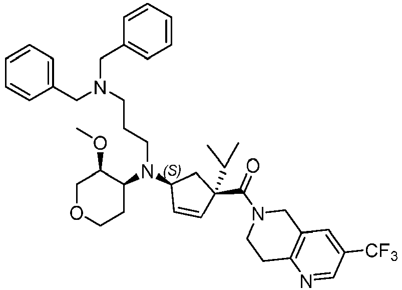

To a small 2-5 mL microwave vial was added ((1S,4S)-1-isopropyl-4-(((4S)-3-methoxytetrahydro-2H-pyran-4-yl)amino)cyclopent-2-en-1-yl)(3-(trifluoromethyl)-7,8-dihydro-1,6-naphthyridin-6(5H)-yl)methanone (450 mg, 0.962 mmol) dissolved in a solution of Methanol (9021 µl) and Acetic Acid (902 µl). 2-(dibenzylamino)acetaldehyde (2073 mg, 8.66 mmol) was added as an oil. alpha-picoline borane (759 mg, 6.74 mmol) was added. The reaction was capped and stirred at room temperature for 16 hours. Upon completion, the reaction was concentrated and suspended in 1 N HCl. The suspension was stirred for 30 min. It was then basified with 2.5 g of sodium carbonate dissolved in 10 mL of water. The solution was extracted with DCM (3x15 mL), washed with brine, dried over sodium sulfate, filtered, and concentrated. The crude oil was combined with another batch and was purified on a 40 g ISCO cartridge eluting with a ramping elution method (0-20% MeOH/DCM). Desired fractions were combined and concentrated to afford ((1S,4S)-4-((3-(dibenzylamino)propyl)((4S)-3-methoxytetrahydro-2H-pyran-4-yl)amino)-1-isopropylcyclopent-2-en-1-yl)(3-(trifluoromethyl)-7,8-dihydro-1,6-naphthyridin-6(5H)-yl)methanone (30 mg, 0.043 mmol, 4.42 % yield) as a orange solid. LCMS (m/z) 705.57 (M+H)⁺.

### Step 7: ((1S,3S)-3-((3-aminopropyl)((3S,4S)-3-methoxytetrahydro-2H-pyran-4-yl)amino)-1-isopropylcyclopentyl)(3-(trifluoromethyl)-7,8-dihydro-1,6-naphthyridin-6(5H)-yl)methanone

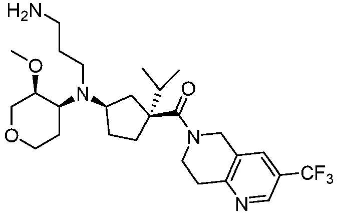

To a 50 mL round bottom flask was added Pd-C (85 mg, 0.040 mmol). The catalyst was wet with 0.1 mL of Methanol. To the catalyst containing round bottom flask was added ((1S,4R)-4-((3-(dibenzylamino)propyl)((3S,4S)-3-methoxytetrahydro-2H-pyran-4-yl)amino)-1-isopropylcyclopent-2-en-1-yl)(3-(trifluoromethyl)-7,8-dihydro-1,6-naphthyridin-6(5H)-yl)methanone (28.2 mg, 0.040 mmol) dissolved in 0.4 mL methanol. The reaction flask was degassed and exposed to hydrogen gas via balloon. The reaction was allowed to stir at room temperature for 16 h. Upon completion, the reaction mixture was passed through a celite pad, and concentrated to afford the product ((1S,3S)-3-((3-aminopropyl)((3S,4S)-3-methoxytetrahydro-2H-pyran-4-yl)amino)-1-isopropylcyclopentyl)(3-(trifluoromethyl)-7,8-dihydro-1,6-naphthyridin-6(5H)-yl)methanone (13 mg, 0.025 mmol, 61.7 % yield) as a clear oil. LCMS (m/z) 527.44 (M+H)⁺.

### Intermediate 15: 3-(dibenzylamino)propan-1-ol

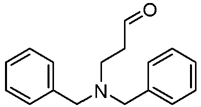

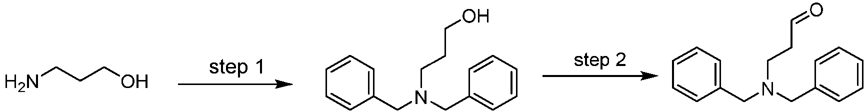

### Step 1: 3-(dibenzylamino)propan-1-ol

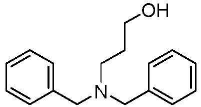

To a 250 mL round bottom flask was added 3-aminopropan-1-ol (5.06 ml, 66.6 mmol) dissolved in Acetone (119 ml). To the solution was added (bromomethyl)benzene (16.23 ml, 136 mmol) followed by K2CO3 (19.32 g, 140 mmol). The reaction was allowed to stir at room temperature for 16 hours. Upon completion, the reaction mixture was filtered. The solid was washed with acetone. The filtrate was concentrated to afford a dark brown oil, and purified on a 330 g silica gel cartridge on the ISCO with a ramping 10-50% EtOAc/Heptane mobile phase, affording 3-(dibenzylamino)propan-1-ol (15.016 g, 58.8 mmol, 88 % yield) as a yellow oil. LCMS (m/z) 256.6 (M+H)⁺.

### Step 2: 3-(dibenzylamino)propanal

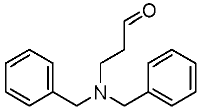

To a oven dried 100 mL round bottom flask was added oxalyl chloride DCM solution (6.31 mL, 3.16 mmol, 0.5M) at -78°C. dimethyl sulfoxide (0.466 mL, 6.57 mmol) was then added dropwise over 1 minute. The reaction was allowed to stir for 15 minutes. Upon activation, 3-(dibenzylamino)propan-1-ol (4.90 mL, 1.958 mmol) dissolved in 4.9 mL of DCM was added dropwise over 5 minutes. The reaction was allowed to stir for 15 minutes. Monitoring with TLC (30% EtOAc:Heptanes) indicates complete formation of the sulfur oxygen bond via loss of alcohol spot. To the reaction was added triethylamine (0.718 mL, 5.15 mmol) dropwise over 1 minute. The reaction was allowed to stir for 15 minutes. Upon completion, the reaction was quenched with saturated sodium bicarbonate. The bicarbonate layer was extracted three times with DCM. The DCM layers were combined, dried over sodium sulfate, filtered, and concentrated to afford 3-(dibenzylamino)propanal (412 mg, 1.626 mmol, 83 % yield) as a crude product. ¹H NMR (400 MHz, DMSO-*d*₆) δ ppm 2.55 - 2.65 (m, 2 H) 2.69 - 2.79 (m, 2 H) 3.52 (s, 4 H) 7.21 - 7.39 (m, 10 H) 9.50 (t, *J*=2.20 Hz, 1 H)

### Intermediate 16: (1S,3R)-3-(((Benzyloxy)carbonyl)amino)-1-isopropylcyclopentane-1-carboxylic acid

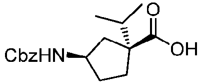

To a solution of methyl (1*S*,3*R*)-3-((tert-butoxycarbonyl)amino)-1-isopropylcyclopentane-1-carboxylate (3.64 g, 12.75 mmol, J. Med. Chem., 2013, 56(19), 7706) in methanol (75 mL) was added a solution of lithium hydroxide (1.527 g, 63.8 mmol) in water (15 mL), followed by tetrahydrofuran (THF) (7.5 mL). The mixture was refluxed for 24 h and was cooled to rt. 1.0 M HCl (63.8 mL, 63.8 mmol) was added slowly and the mixture was extracted with dichloromethane (DCM) (3 x 150 mL). The combined organic extracts were dried over Na₂SO₄, were filtered, and the filtrate was concentrated under reduced pressure. The residue was dissolved in 2,2,2-trifluoroacetic acid (20 mL, 12.75 mmol). After 1 h, 12 M hydrochloric acid (50.0 mL, 600 mmol) was added, the mixture was heated at 100 °C for 16 h and was concentrated under reduced pressure. The residue was dissolved in a mixture of 1,4-dioxane (50 mL) and 1.0 M sodium hydroxide (50.0 mL, 50.0 mmol), was cooled to 0 °C, and Cbz-Cl (2.367 mL, 16.58 mmol) was added dropwise. After stirring at 0 °C for 20 h, the mixture was extracted with ether (100 mL). The aqueous layer was acidified with 1.0 M HCl (50.0 mL, 50.0 mmol) and was extracted with diethyl ether (150 mL). The combined organic extracts were dried over Na₂SO₄, were filtered, and the filtrate was concentrated under reduced pressure to provide the title compound as a brown oil (3.32 g, 10.87 mmol, 85 % yield). LC-MS *m*/*z* 306.3 (M+H)⁺.

### Intermediate 17A: Benzyl (R)-8-amino-3-(trifluoromethyl)-7,8-dihydro-1,6-naphthyridine-6(5H)-carboxylate; and Intermediate 17B: Benzyl (S)-8-amino-3-(trifluoromethyl)-7,8-dihydro-1,6-naphthyridine-6(5H)-carboxylate.

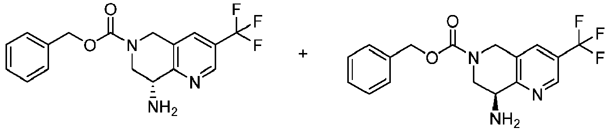

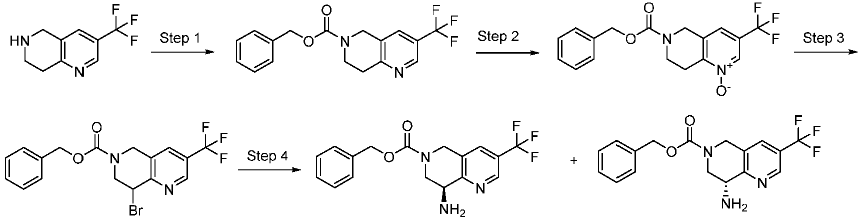

### Step 1: Benzyl 3-(trifluoromethyl)-7,8-dihydro-1,6-naphthyridine-6(5H)-carboxylate.

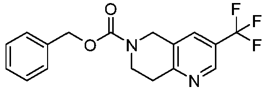

A mixture of commercially-available 3-(trifluoromethyl)-5,6,7,8-tetrahydro-1,6-naphthyridine, dihydrochloric acid salt (5.0 g, 18.18 mmol) in 2-methyltetrahydrofuran (2-MeTHF) (100 mL) was stirred at rt for 1 min. 1.0 M sodium hydroxide (72.7 mL, 72.7 mmol) was added slowly and the mixture was stirred for 5 min. Cbz-Cl (2.72 mL, 19.08 mmol) was added dropwise at rt and the mixture was stirred for 1 h. 2-Methyltetrahydrofuran (2-MeTHF) was removed under reduced pressure and the resulting emulsion stirred until the oil solidified into a yellow solid. The solid was filtered off, was crushed with a spatula into a fine powder, was washed with water, and was air-dried overnight under vacuum filtration to afford the title compound as a yellow solid (6.06 g, 18.02 mmol, 99 % yield). LC-MS *m*/*z* 337.3 (M+H)⁺.

### Step 2: 6-((Benzyloxy)carbonyl)-3-(trifluoromethyl)-5,6,7,8-tetrahydro-1,6-naphthyridine 1-oxide.

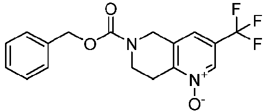

To a solution of benzyl 3-(trifluoromethyl)-7,8-dihydro-1,6-naphthyridine-6(5H)-carboxylate (6.0 g, 17.84 mmol) in dichloromethane (DCM) (10.0 mL) at rt was added methyltrioxorhenium(vii) (0.160 g, 0.642 mmol) followed by dropwise addition of 30 % hydrogen peroxide (3.64 mL, 35.7 mmol) and the mixture was stirred at rt for 20 h. The excess hydrogen peroxide was destroyed by addition of manganese(IV) oxide (1.0 mg, 0.012 mmol) and the mixture was stirred for 30 min. Dichloromethane (DCM) (50 mL) and 1.0 M NaOH (50 mL) were added and the aqueous phase was extracted with dichloromethane (DCM) (50 mL). The combined organic extracts were dried over Na₂SO₄ and were filtered. The filtrate was diluted with toluene (50 mL) and was concentrated under reduced pressure to provide the title compound as a white solid (6.20 g, 17.6 mmol, 99 % yield). LC-MS *m*/*z* 353.3 (M+H)⁺.

### Step 3: Benzyl 8-bromo-3-(trifluoromethyl)-7,8-dihydro-1,6-naphthyridine-6(5H)-carboxylate.

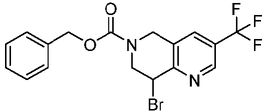

To a solution of 6-((benzyloxy)carbonyl)-3-(trifluoromethyl)-5,6,7,8-tetrahydro-1,6-naphthyridine 1-oxide (6.20 g, 17.60 mmol) in chloroform (30 mL) were simultaneously added at rt over 7 min the following two solutions dropwise from two separate syringes: a solution of phosphorus oxybromide (10.09 g, 35.2 mmol) in chloroform (10.0 mL) and a solution of triethylamine (1.385 mL, 9.93 mmol) in chloroform (10.0 mL). The mixture was stirred at rt for 45 min, was poured on ice, and was basified with 2.0 M Na₂CO₃. The mixture was extracted with dichloromethane (DCM) (3 x 200 mL). The combined organic extracts were dried over Na₂SO₄, were filtered, and the filtrate was concentrated under reduced pressure. Purification by normal-phase chromatography (330 g silica, 200 mL/min) eluting with a gradient of 0 to 40 % ethyl acetate in heptane provided the title compound as a white crystalline solid (2.83 g, 6.82 mmol, 38.7 % yield). LC-MS *m*/*z* 417.1 (M+H)⁺.

### Step 4: Benzyl (R)-8-amino-3-(trifluoromethyl)-7,8-dihydro-1,6-naphthyridine-6(5H)-carboxylate; and Benzyl (S)-8-amino-3-(trifluoromethyl)-7,8-dihydro-1,6-naphthyridine-6(5H)-carboxylate.

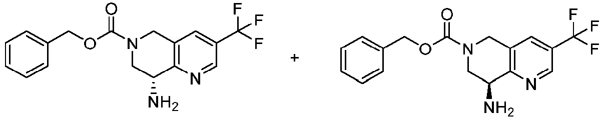

To a solution of benzyl 8-bromo-3-(trifluoromethyl)-7,8-dihydro-1,6-naphthyridine-6(5H)-carboxylate (2.70 g, 6.50 mmol) in N,N-dimethylformamide (DMF) (25.0 mL) was added sodium azide (1.268 mg, 19.51 mmol) and the mixture was stirred at rt for 18 h. The flask was purged with nitrogen, freshly degassed tetrahydrofuran (THF), (30.000 mL) and water (6.000 mL) were added followed by 1.0 M trimethylphosphine in toluene (32.5 mL, 32.5 mmol). The mixture was stirred at rt for 20 h and was concentrated under reduced pressure. The residue was partitioned between diethyl ether and water, the organic phase was separated, was dried over Na₂SO₄, and was concentrated under reduced pressure. Preparative chiral resolution using a Lux Amylose 2 column (5 µm, 21 x 250 mm, 20 mL/min) and eluting with 30 % heptane in ethanol containing 0.1% isopropylamine provided the title compound as a white solid (990.1 mg, 2.82 mmol, 43.3 % yield). LC-MS *m*/*z* 352.2 (M+H)⁺. Benzyl (S)-8-amino-3-(trifluoromethyl)-7,8-dihydro-1,6-naphthyridine-6(5H)-carboxylate was also isolated as a white solid (888.7 mg, 2.53 mmol, 38.9 % yield). LC-MS *m*/*z* 352.3 (M+H)⁺. The absolute stereochemistry of both enantiomers was determined by VCD using the following procedure:

Samples were dissolved in CD₃CN (16.8 mM) and placed in a 100 µm path length cell with BaF₂ windows. IR and VCD spectra were recorded on a ChiralIR2X^{™} VCD spectrometer (BioTools Inc., Jupiter, FL) equipped with dual PEM accessory, with 4 cm⁻¹ resolution, 6-hour collection for one isomer-E1 and 12-hour collection for the other isomer-E2, and the instrument optimized at 1400 cm⁻¹. A conformational search on the modeled (R)-structure was carried out using MOE at LowMode using MMFF94x forcefield with Born solvation, dielectric constant set at 20 and exterior dielectric constant set at 47. The geometry optimization, frequency, and IR and VCD intensity calculations of the 10 conformers resulted from the conformational search were carried out at the DFT level b3lyp/6-31G(d) scrf = (solvent = dimethyl sulfoxide) with Gaussian 16 (Gaussian Inc., Wallingford, CT). The Gaussian output files were converted to VCD and IR spectra using BLAIR. The calculated frequencies were scaled by 0.981 and the IR and VCD intensities were converted to Lorentzian bands with 8-cm-1 half-width for comparison to experimental spectra. The assignment was evaluated by CompareVOA program (BioTools Inc., Jupiter, FL) to generate a confidence level based on current database that includes 88 previous correct assignments for different chiral structures.

### Intermediate 18: ((R)-8-Amino-3-(trifluoromethyl)-7,8-dihydro-1,6-naphthyridin-6(5H)-yl)((1S,3R)-1-isopropyl-3-(((3S,4S)-3-methoxytetrahydro-2H-pyran-4-yl)amino)cyclopentyl)methanone

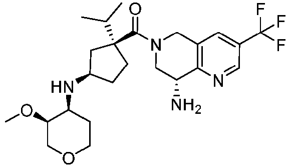

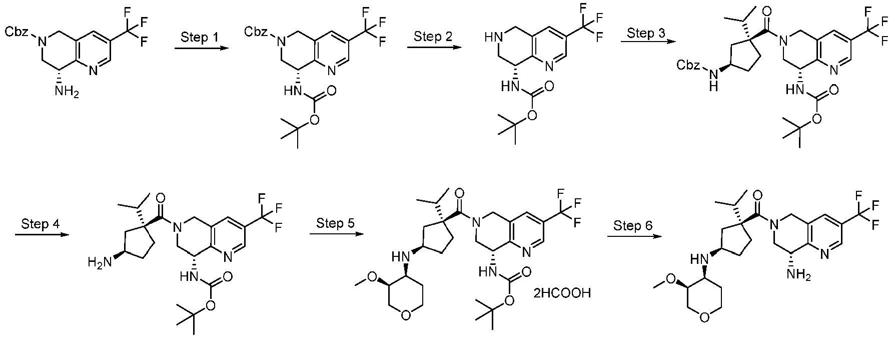

### Step 1: Benzyl (R)-8-((tert-butoxycarbonyl)amino)-3-(trifluoromethyl)-7,8-dihydro-1,6-naphthyridine-6(5H)-carboxylate.

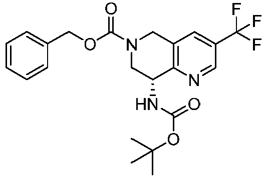

To benzyl (*R*)-8-amino-3-(trifluoromethyl)-7,8-dihydro-1,6-naphthyridine-6(5H)-carboxylate **(Step 3-4)** (4.25 g, 12.10 mmol) was added di-tert-butyl dicarbonate (2.90 g, 13.31 mmol) and water (24.00 mL) and the mixture was stirred for 5 min. Acetone (24.00 mL) was added slowly. The mixture was stirred for 2 h, the precipitate was filtered, was washed with water, and was air-dried to provide the title compound as a pale-yellow solid (4.70 g, 10.41 mmol, 86 % yield). LC-MS *m*/*z* 452.1 (M+H)⁺.

### Step 2: tert-Butyl (R)-(3-(trifluoromethyl)-5,6,7,8-tetrahydro-1,6-naphthyridin-8-yl)carbamate.

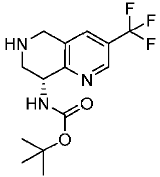

A mixture of benzyl (*R*)-8-((tert-butoxycarbonyl)amino)-3-(trifluoromethyl)-7,8-dihydro-1,6-naphthyridine-6(5H)-carboxylate (4.60 g, 10.19 mmol) and 20 % palladium hydroxide on carbon (2.216 g, 3.06 mmol) in methanol (100.000 mL) was sealed in a flask and was purged with nitrogen. Hydrogen was bubbled into the mixture and the mixture was stirred for 1.5 h under a balloon atmosphere of hydrogen. The mixture was open to air and was stirred for 10 min. 7.0 M ammonia in methanol (100 mL) was added, the mixture was filtered, and the catalyst was washed with methanol. The combined filtrates were concentrated under reduced pressure. Purification by reverse-phase HPLC chromatography (C18 Aq 275 g Gold column, 125 mL/min) eluting with a gradient of 30 to 60 % acetonitrile in water containing ammonium bicarbonate (10 mM) and ammonia hydroxide (0.075 %) provided the title compound as a pale-yellow foam (2.66 g, 8.38 mmol, 82 % yield). LC-MS *m*/*z* 318.1 (M+H)⁺.

### Step 3: tert-Butyl ((R)-6-((1S,3R)-3-(((benzyloxy)carbonyl)amino)-1-isopropylcyclopentane-1-carbonyl)-3-(trifluoromethyl)-5,6,7,8-tetrahydro-1,6-naphthyridin-8-yl)carbamate.

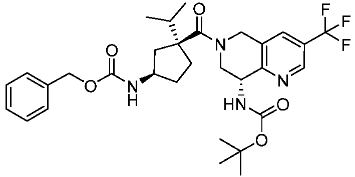

To a solution of (1*S*,3*R*)-3-(((benzyloxy)carbonyl)amino)-1-isopropylcyclopentane-1-carboxylic acid (5.12 g, 16.77 mmol) in dichloromethane (DCM) was added DIPEA (9.15 mL, 52.4 mmol) and HATU (5.90 g, 15.51 mmol) and the mixture was stirred for 24 h. The mixture was added to tert-butyl (*R*)-(3-(trifluoromethyl)-5,6,7,8-tetrahydro-1,6-naphthyridin-8-yl)carbamate (2.66 g, 8.38 mmol), was stirred for 2 min, and the resulting solution was divided into two 20 mL vials. The vials were sealed and were stirred at 80 °C for 22 h. The mixtures were cooled to rt, water was added (1.000 mL each), the vials were sealed, and the mixtures were stirred at 80 °C for 50 min. The two mixtures were cooled to rt, were combined, and water (100 mL) and dichloromethane (DCM) (100 mL) were added. The organic phase was dried over Na₂SO₄, was filtered, and the filtrate was concentrated under reduced pressure. Purification by ISCO CombiFlash^{®} chromatography (330 g silica, 200 mL/min) eluting with a gradient of 0 to 10 % methanol in dichloromethane (DCM) provided the title compound as a white foam (2.95 g, 4.88 mmol, 58.2 % yield). LC-MS *m*/*z* 605.2 (M+H)⁺.

### Step 4: tert-Butyl ((R)-6-((1S,3R)-3-amino-1-isopropylcyclopentane-1-carbonyl)-3-(trifluoromethyl)-5,6,7,8-tetrahydro-1,6-naphthyridin-8-yl)carbamate.

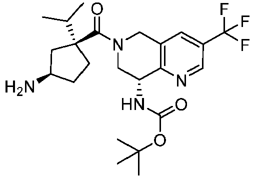

A mixture of tert-butyl ((*R*)-6-((1*S*,3*R*)-3-(((benzyloxy)carbonyl)amino)-1-isopropylcyclopentane-1-carbonyl)-3-(trifluoromethyl)-5,6,7,8-tetrahydro-1,6-naphthyridin-8-yl)carbamate (2.95 g, 4.88 mmol) and 20 % palladium hydroxide on carbon (1.028 g, 1.464 mmol) in methanol (50.000 mL) was purged with nitrogen. Hydrogen was bubbled into the mixture and the mixture was stirred for 1 h under a balloon atmosphere of hydrogen. The mixture was opened to air and the mixture was stirred for 15 min. 2.0 M ammonia in methanol (100 mL) was added and the mixture was filtered. The catalyst was washed with methanol and the combined filtrates were concentrated under reduced pressure to provide the title compound as a white foam (2.24 g, 4.76 mmol, 98 % yield). LC-MS *m*/*z* 471.4 (M+H)⁺.

### Step 5: tert-Butyl ((R)-6-((1S,3R)-1-isopropyl-3-(((3S,4S)-3-methoxytetrahydro-2H-pyran-4-yl)amino)cyclopentane-1-carbonyl)-3-(trifluoromethyl)-5,6,7,8-tetrahydro-1,6-naphthyridin-8-yl)carbamate, 2Formic acid salt.

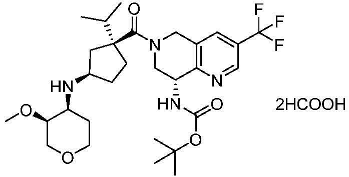

To a solution of the tert-butyl ((*R*)-6-((1*S*,3*R*)-3-amino-1-isopropylcyclopentane-1-carbonyl)-3-(trifluoromethyl)-5,6,7,8-tetrahydro-1,6-naphthyridin-8-yl)carbamate (2.24 g, 4.76 mmol) in isopropyl acetate (50.00 mL) was added 2-propanol (0.917 mL, 11.90 mmol) and triethylamine (0.664 mL, 4.76 mmol). The mixture was cooled to 0 °C, sodium triacetoxyborohydride (2.52 g, 11.90 mmol) was added followed by dropwise addition of a solution of (*R*)-3-methoxytetrahydro-4H-pyran-4-one (0.929 g, 7.14 mmol) in isopropyl acetate (50.00 mL). The mixture was stirred at 0 °C for 20 min, was warmed to rt, and was stirred at rt for 2 h. 1.0 M NaOH (200 mL) and isopropyl acetate (200 mL) were added, the organic phase was dried over Na₂SO₄, was filtered, and the filtrate was concentrated under reduced pressure. Purification by reverse-phase HPLC chromatography (C18 275g Gold column; 125 mL/min) eluting with 35 % acetonitrile in water containing formic acid (0.1 %) provided the title compound as a white foam (2.73 g, 4.03 mmol, 85 % yield). The compound was used as an intermediate in the next step. LC-MS *m*/*z* 585.2 (M+H)⁺.

### Step 6: ((R)-8-Amino-3-(trifluoromethyl)-7,8-dihydro-1,6-naphthyridin-6(5H)-yl)((1S,3R)-1-isopropyl-3-(((3S,4S)-3-methoxytetrahydro-2H-pyran-4-yl)amino)cyclopentyl)methanone.

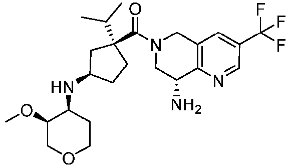

A mixture of tert-butyl ((*R*)-6-((1*S*,3*R*)-1-isopropyl-3-(((3*S*,4*S*)-3-methoxytetrahydro-2H-pyran-4-yl)amino)cyclopentane-1-carbonyl)-3-(trifluoromethyl)-5,6,7,8-tetrahydro-1,6-naphthyridin-8-yl)carbamate, 2formic acid salt (2.73 g, 4.03 mmol) and trifluoroacetic acid (10 mL, 130 mmol) was stirred at rt for 15 min and was concentrated under reduced pressure. Dichloromethane (DCM) (100 mL) and 1.0 M NaOH (100 mL) were added and the aqueous phase was extracted with dichloromethane (DCM) (100 mL). The combined organic extracts were dried over Na₂SO₄, were filtered, and the filtrate were concentrated under reduced pressure to provide the title compound as a white foam (1.77 g, 3.65 mmol, 91 % yield). LC-MS *m*/*z* 485.4 (M+H)⁺.

The following intermediates were or could be prepared using procedures analogous to those described for **Intermediate 18:**

| **Intermediate** | **Compound** | **Structure** | **LC-MS m/z (M+H)⁺** |
|---|---|---|---|
| **19** | **((*S*)-8-Amino-3-(trifluoromethyl)-7,8-dihydro-1,6-naphthyridin-6(5H)-yl)((1*S*,3*R*)-1-isopropyl-3-(((3*S*,4*S*)-3-methoxytetrahydro-2H-pyran-4-yl)amino)cyclopentyl)methan one.** | 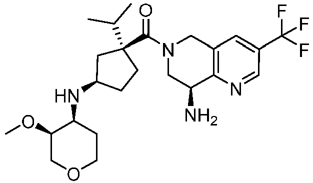 | 485.2 (M+H)⁺ |

### Example 1

### (2S,3S)-N-(43-((1S,3R)-3-(((3S,4S)-3-methoxytetrahydro-2H-pyran-4-yl)amino)-1-(3-(trifluoromethyl)-5,6,7,8-tetrahydro-1,6-naphthyridine-6-carbonyl)cyclopentyl)-39-oxo-3,6,9,12,15,18,21,24,27,30,33,36-dodecaoxa-40-azatritetracontyl)-1-methyl-5-oxo-2-(pyridin-3-yl)pyrrolidine-3-carboxamide

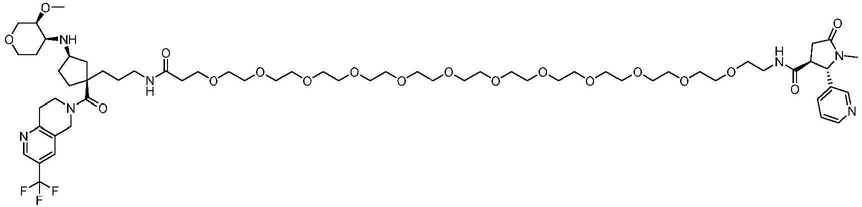

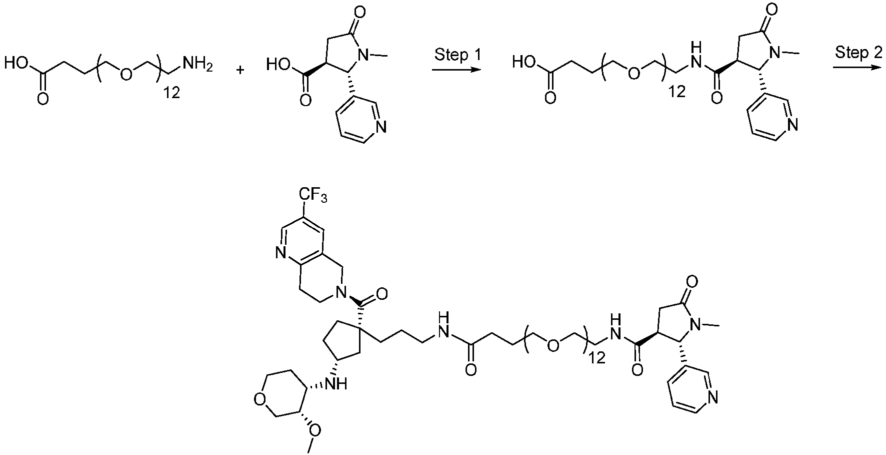

### 1-((2S,3S)-1-Methyl-5-oxo-2-(pyridin-3-yl)pyrrolidin-3-yl)-1-oxo-5,8,11,14,17,20,23,26,29,32,35,38-dodecaoxa-2-azahentetracontan-41-oic acid, Ammonia salt

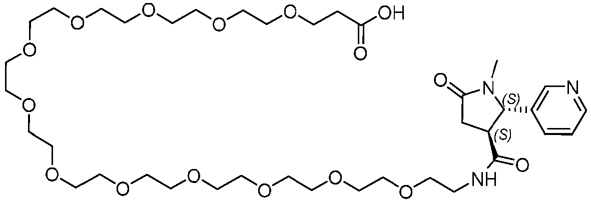

In a round bottom flask, (2S,3S)-1-methyl-5-oxo-2-(pyridin-3-yl)pyrrolidine-3-carboxylic acid (0.357 g, 1.619 mmol) was dissolved in acetonitrile (10 ml). TSTU (0.487 g, 1.619 mmol) and DIPEA (1.131 ml, 6.48 mmol) were then added and the reaction was stirred at RT. Upon activation of the acid, 1-amino-3,6,9, 12, 15, 18,21,24,27,30,33,36-dodecaoxanonatriacontan-39-oic acid (1 g, 1.619 mmol) was added as a solid and N,N-dimethylformamide (DMF) (3 ml) was added to aid in the solubility of the acid. The reaction was stirred at RT and monitored by LCMS. Upon completion, the reaction was concentrated to afford a viscous oil. The crude material was purified by reverse phase chromatography (loading as a solution in ~6 mL of 10mM aqueous ammonium bicarbonate in H20 adjusted to pH 10 with ammonia, C18 100g Gold column, 60mL/min, gradient 5-50% 10mM aqueous ammonium bicarbonate in H20 adjusted to pH 10 with ammonia to MeCN over 23 min) to afford the title compound (1.3 g, 1.351 mmol, 83 % yield) as a colorless oil (87% purity by NMR, the material contains CH₂Cl₂). LC-MS m/z 820.1 (M+H)⁺.

### Step 2: ((2S,3S)-N-(43-((1S,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)-39-oxo-3,6,9,12,15,18,21,24,27,30,33,36-dodecaoxa-40-azatritetracontyl)-1-methyl-5-oxo-2-(pyridin-3-yl)pyrrolidine-3-carboxamide

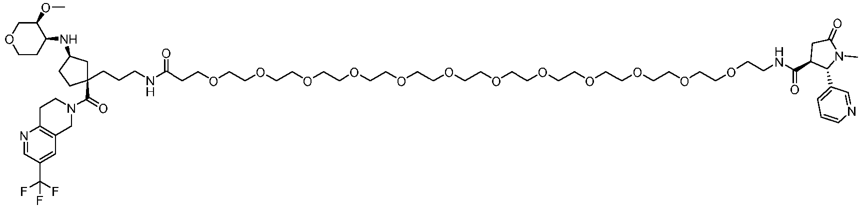

To a solution of ((1S,3R)-1-(3-aminopropyl)-3-(((3S,4S)-3-methoxytetrahydro-2H-pyran-4-yl)amino)cyclopentyl)(3-(trifluoromethyl)-7,8-dihydro-1,6-naphthyridin-6(5H)-yl)methanone, 3hydrochloric acid salt (89 mg, 0.15 mmol) in dichloromethane (DCM) (1500 µL) was added a solution of 1-((2S,3S)-1-methyl-5-oxo-2-(pyridin-3-yl)pyrrolidin-3-yl)-1-oxo-5,8,11,14,17,20,23,26,29,32,35,38-dodecaoxa-2-azahentetracontan-41-oic acid, ammonium salt (123 mg, 0.150 mmol) in dichloromethane (DCM) (1500 µL), HATU (86 mg, 0.225 mmol), and DIPEA (262 µL, 1.500 mmol). The mixture was stirred at rt for 2 h, methanol was added, and the mixture was concentrated. The residue was purified by CombiFlash^{®} EZ Prep chromatography (30 g C18 column, 35 mL/min) eluting with a gradient 10 to 70 % acetonitrile in water containing ammonium bicarbonate (10 mM) and adjusted to pH 10 with ammonia. Further purification by MDAP (XSelect^{™} CSH C18 column, 40 mL/min) eluting with a gradient of 15 to 55 % acetonitrile in water containing ammonium bicarbonate (10 mM) adjusted to pH 10 with ammonia provided the title compound as a clear, viscous oil (79.2 mg, 0.061 mmol, 40.6 % yield). LCMS *m*/*z* (M/2+H)⁺ 644.1. ¹H NMR (400 MHz, DMSO-d₆) δ ppm 1.11 - 1.28 (m, 4H), 1.29 - 1.39 (m, 1H), 1.41 - 1.53 (m, 3H), 1.53 - 1.68 (m, 2H), 1.69 - 1.86 (m, 2H), 1.99 (br dd, J = 12.7, 6.8 Hz, 1H), 2.03 - 2.14 (m, 1H), 2.22 (br t, J = 6.4 Hz, 2H), 2.44 (dd, J = 16.9, 7.6 Hz, 2H), 2.50 (br s, 4H), 2.70 (br dd, J = 16.6, 9.3 Hz, 2H), 2.86 - 3.04 (m, 5H), 3.12 - 3.28 (m, 8H), 3.38 (td, J = 5.7, 1.7 Hz, 2H), 3.42 - 3.57 (m, 44H), 3.66 - 3.76 (m, 2H), 3.86 (dt, J = 7.7, 3.7 Hz, 3H), 4.65 (d, J = 5.9 Hz, 1H), 4.79 (br s, 2H), 7.45 (dd, J = 7.8, 4.9 Hz, 1H), 7.68 (dt, J = 7.8, 2.0 Hz, 1H), 7.74 (br s, 1H), 8.07 (t, J = 5.4 Hz, 1H), 8.18 (br s, 1H), 8.44 - 8.52 (m, 1H), 8.56 (dd, J = 4.6, 1.7 Hz, 1H), 8.77 (s, 1H)

### Example 2: (2S,3S)-N-(2-(((1R,4S)-4-(4-(((1S,4R)-4-(4-(2-((1S,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)ethyl)piperidine-1-carbonyl)cyclohexyl)amino)-4-oxobutoxy)cyclohexyl)oxy)ethyl)-1-methyl-5-oxo-2-(pyridin-3-yl)pyrrolidine-3-carboxamide

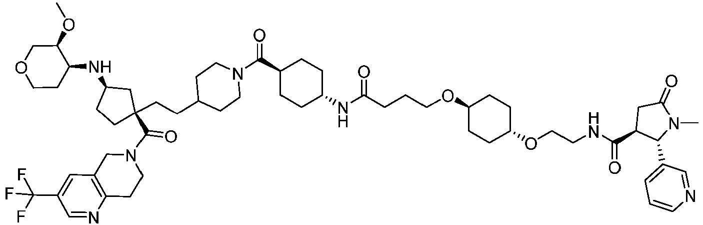

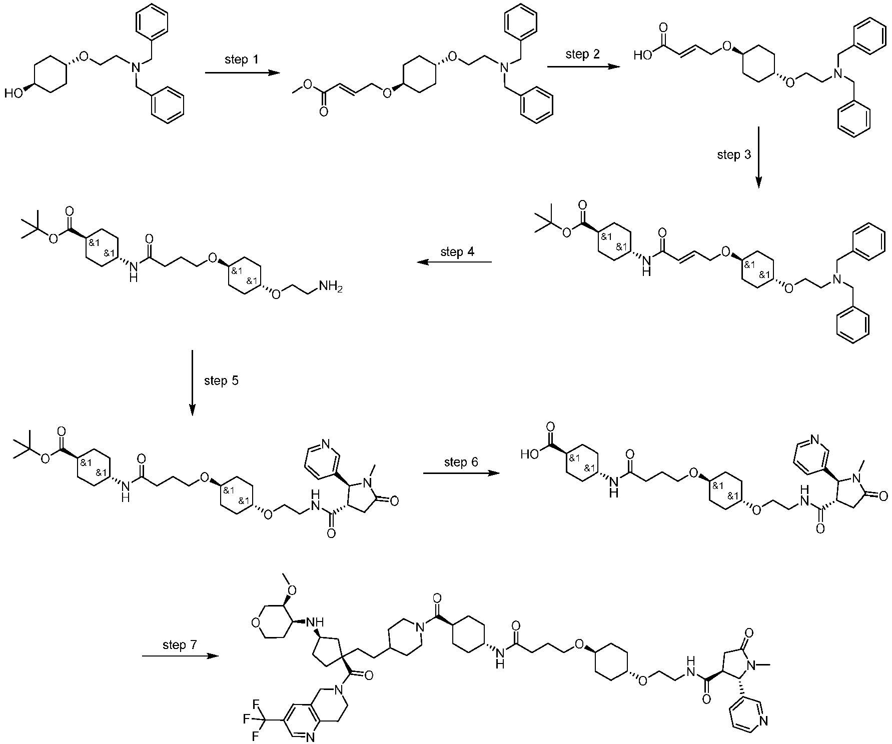

### Step 1

### methyl (E)-4-(((1r,4r)-4-(2-(Dibenzylamino)ethoxy)cyclohexyl)oxy)but-2-enoate.

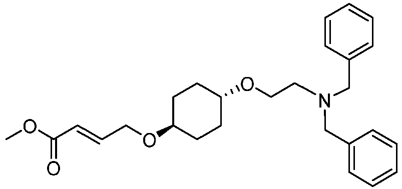

To a stirred solution of (1r,4r)-4-(2-(dibenzylamino)ethoxy)cyclohexan-1-ol (250 g, 736 mmol) in toluene (2500 mL) were added methyl but-2-ynoate (140 g, 1423 mmol), triphenylphosphine (19.32 g, 73.6 mmol), and acetic acid (16.86 mL, 295 mmol) at RT and the resulting solution was stirred at 115 ⁰C for 16 h. The reaction mixture was cooled to room temperature and concentrated under reduced pressure to the crude compound. The crude compound was adsorbed on silicagel (500 g, 60-120mesh), and purified by manual column chromatography(1.5 kg , 100-200 mesh) eluted with 15 % EtOAc in pet-ether to afford methyl (E&Z)-4-(((1r,4r)-4-(2-(dibenzylamino)ethoxy)cyclohexyl)oxy)but-2-enoate (350 g) as a mixture of E/Z isomers (52.48 % and 21.15 %). To separate both isomers, the compound was adsorbed on silica gel (500 g, 100-200 mesh), and purified by manual column chromatography(1.5 kg , 100-200 mesh) eluted with 15 % EtOAc in pet-ether to afford the title compound (240 g, 463 mmol, 62.9 % yield, 84.45% purity) as a pale yellow liquid. LC-MS m/z 438.3 (M+H)⁺.

### Step 2

### (E)-4-(((1,4-trans)-4-(2-(Dibenzylamino)ethoxy)cyclohexyl)oxy)but-2-enoic acid

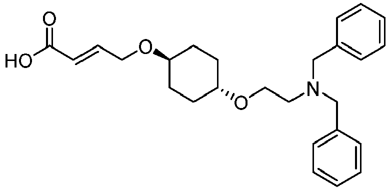

Methyl (E)-4-(((1,4-trans)-4-(2-(dibenzylamino)ethoxy)cyclohexyl)oxy)but-2-enoate (9.03 g, 20.64 mmol) was dissolved in tetrahydrofuran (THF) (25 mL) and aqueous 5.089 Molar sodium hydroxide (4.87 mL, 24.76 mmol) was added. The homogenous pale-yellow reaction was heated at reflux for 1 hour. Additional 5.089 M sodium hydroxide (1.217 mL, 6.19 mmol) was added and the reaction was refluxed for 50 minutes. The reaction was cooled over 60 minutes and concentrated in vacuo. The residue was azeotroped twice with toluene in order to aid in removal of water. The residue was pumped under high vacuum to afford the title compound (9.9 g, 22.17 mmol, 107% yield, 82% purity, E/Z mixture) as a yellow solid. LC-MS m/z 424.4 (M+H)⁺.

### Step 3

### tert-butyl (1R,4r)-4-((E)-4-(((1 r,4R)-4-(2-(Dibenzylamino)ethoxy)cyclohexyl)oxy)but-2-enamido)cyclohexane-1-carboxylate.

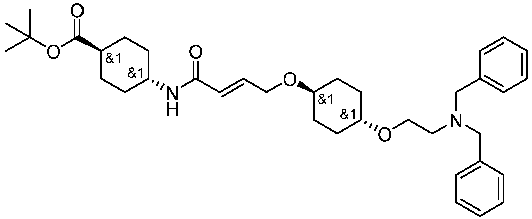

(E)-4-(((1,4-trans)-4-(2-(dibenzylamino)ethoxy)cyclohexyl)oxy)but-2-enoic acid, sodium salt (9.2 g, 20.60 mmol) was suspended in dry DMF (40 ml) with stirring. HATU (8.62 g, 22.66 mmol) was added as a solid and a partially dissolved mixture was observed. The mixture was stirred for 30 minutes to give a partially dissolved greenish solution. tert-butyl (1,4-trans)-4-aminocyclohexane-1-carboxylate (4.11 g, 20.60 mmol) was added as a solution in DMF (10 ml) followed by addition of a solution of DIEA (10.80 mL, 61.8 mmol) in DMF (10 ml). An additional 10 ml of DMF was added and the heterogeneous mixture was stirred for 15 hours at room temperature. Additional HATU (1.724 g, 4.53 mmol) was added and the almost homogeneous reaction was stirred for 60 minutes. The cloudy reaction was stirred for an additional 60 minutes. The reaction was diluted with 200 ml of EtOAc and 200 ml of water and stirred for 10 minutes. The resulting homogeneous biphasic mixture was transferred to a separatory funnel and the layers were separated. The aqueous layer was extracted twice more with 150 ml EtOAc and the combined EtOAc layers were washed 4 x with water and 2 x with saturated NaCl in order to remove DMF. The EtOAc extracts were dried over sodium sulfate, filtered, and concentrated in vacuo, and pumped under high vacuum to give an orange syrup which was purified via silica-gel chromatography (Isco Combiflash, 330 gram gold column, 0-80% EtOAc:heptane over 45 minutes, 150 ml/min flow rate, loaded as a solution in DCM) to give the title compound (4.55 g, 7.52 mmol, 36.5% yield) as a white foamy solid. LC-MS m/z 605.5 (M+H)⁺.

### Step 4

### tert-butyl (1R,4r)-4-(4-(((1r,4R)-4-(2-Aminoethoxy)cyclohexyl)oxy)butanamido)cyclohexane-1-carboxylate

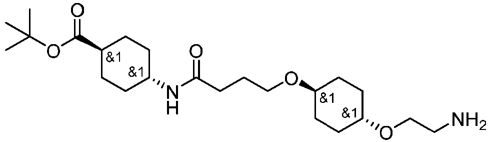

A 500 ml 3-necked RB flask was charged with tert-butyl (1R,4r)-4-((E)-4-(((1r,4R)-4-(2-(dibenzylamino)ethoxy)cyclohexyl)oxy)but-2-enamido)cyclohexane-1-carboxylate (6.40 g, 10.58 mmol) and isopropanol (120 mL) and the suspension was stirred until a homogeneous solution was obtained. 10% wet Pd-C (0.640 g, 6.01 mmol) was added and the flask was evacuated and placed under 2 balloons of hydrogen attached to the end necks of the flask. The middle neck was covered with a rubber septum. The reaction was stirred at room temperature for 16 hours. The reaction was degassed and filtered 2 X through celite. The filtrate was concentrated in vacuo and pumped under high vacuum to give a waxy grey solid with a slight odor of isopropanol. The waxy solid was dissolved in DCM and concentrated in vacuo at 54 degrees C for 20 minutes in order to aid in removal of isopropanol. The residue was pumped under high vacuum to afford the title compound (4.44 g, 10.41 mmol, 98% yield) as a waxy grey solid. LC-MS m/z 427.4 (M+H)⁺.

### Step 5\

### ert-butyl (1R,4r)-4-(4-(((1S,4R)-4-(2-((2S,3S)-1-Methyl-5-oxo-2-(pyridin-3-yl)pyrrolidine-3-carboxamido)ethoxy)cyclohexyl)oxy)butanamido)cyclohexane-1-carboxylate.

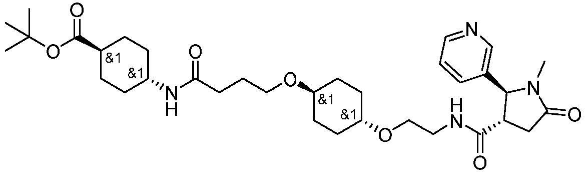

(2S,3S)-1-Methyl-5-oxo-2-(pyridin-3-yl)pyrrolidine-3-carboxylic acid (2.287 g, 10.38 mmol) was suspended in 30 ml DCM with stirring in a 500 ml RB flask at room temperature. HATU (4.34 g, 11.42 mmol) was added and the suspension was stirred for 15 minutes. A solution of tert-butyl (1R,4r)-4-(4-(((1r,4R)-4-(2-aminoethoxy)cyclohexyl)oxy)butanamido)cyclohexane-1-carboxylate (4.43 g, 10.38 mmol) in DCM (20 ml) was added dropwise via pipet over 15 minutes. After addition was complete, a solution of DIEA (5.44 mL, 31.2 mmol) in DCM (10 ml) was added dropwise over 10 minutes and the resulting homogeneous dark solution was stirred at room temperature for 16 hours. The reaction was concentrated in vacuo in order to remove DCM and DIEA. The residue was dissolved in 100 ml DCM and transferred to a separatory funnel. 20 ml of saturated sodium bicarbonate was added The layers were separated and the DCM layer was washed with saturated NaCl, dried over sodium sulfate, filtered, concentrated in vacuo, and pumped under high vacuum to give an orange syrup which was purified via silica gel chromatography (Isco Combiflash, 0-10% MeOH:DCM over 60 minutes, 330 gram gold column, 150 ml/min flow rate, loaded as a solution in 30 ml DCM) to afford the title compound (4.13 g, 6.57 mmol, 63.2% yield) as a white solid. LC-MS m/z 629.3 (M+H)⁺.

### Step 6

### (1R,4r)-4-(4-(((1S,4R)-4-(2-((2S,3S)-1-Methyl-5-oxo-2-(pyridin-3-yl)pyrrolidine-3-carboxamido)ethoxy)cyclohexyl)oxy)butanamido)cyclohexane-1-carboxylic acid, Hydrochloride salt.

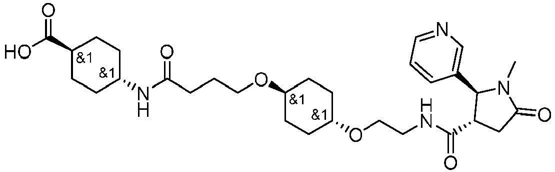

tert-Butyl (1R,4r)-4-(4-(((1S,4R)-4-(2-((2S,3S)-1-methyl-5-oxo-2-(pyridin-3-yl)pyrrolidine-3-carboxamido)ethoxy)cyclohexyl)oxy)butanamido)cyclohexane-1-carboxylate (4.13 g, 6.57 mmol) was dissolved in dry 1,4-dioxane (13 ml) with stirring in a 250 ml RB flask. 4 M anhydrous HCl (39 mL, 156 mmol) in 1,4-dioxane was added and the mixture was stirred at room temperature. Upon HCl addition, an insoluble oil was observed. The mixture was stirred for 90 minutes at room temperature. The reaction was concentrated in vacuo (at 60 degrees bath temperature) and pumped under high vacuum for 15 hours to afford the title compound (4.187 g, 6.87 mmol, 105% yield) as a white solid. LC-MS m/z 573.4 (M+H)⁺.

### Step 7: (2S,3S)-N-(2-(((1R,4S)-4-(4-(((1S,4R)-4-(4-(2-((1S,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)ethyl)piperidine-1-carbonyl)cyclohexyl)amino)-4-oxobutoxy)cyclohexyl)oxy)ethyl)-1-methyl-5-oxo-2-(pyridin-3-yl)pyrrolidine-3-carboxamide

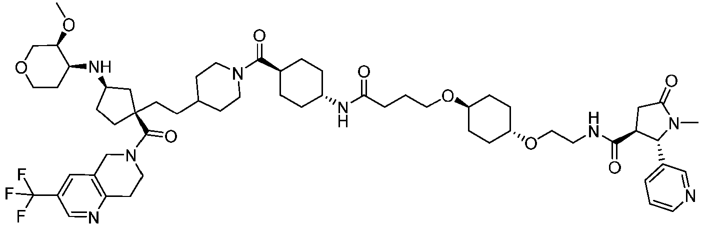

A mixture of (1R,4r)-4-(4-(((1S,4R)-4-(2-((2S,3S)-1-methyl-5-oxo-2-(pyridin-3-yl)pyrrolidine-3-carboxamido)ethoxy)cyclohexyl)oxy)butanamido)cyclohexane-1-carboxylic acid, hydrochloric acid salt (54.7 mg, 0.090 mmol) and HATU (37.6 mg, 0.099 mmol) in dichloromethane (DCM) (1.0 mL) was stirred at rt for 15 minutes. ((1*S*,3*R*)-3-(((3*S*,4*S*)-3-methoxytetrahydro-2H-pyran-4-yl)amino)-1-(2-(piperidin-4-yl)ethyl)cyclopentyl)(3-(trifluoromethyl)-7,8-dihydro-1,6-naphthyridin-6(5H)-yl)methanone, 3hydrochloric acid salt (58.2 mg, 0.090 mmol) was added as a solid. DIPEA (0.125 mL, 0.718 mmol) and dichloromethane (DCM) (1.5 mL) were added and mixture was stirred at rt for 16 h. Dichloromethane (DCM) (6 mL) and saturated NaHCO₃ (6 mL) were added and the mixture was stirred for 10 min. The organic phase was washed with saturated NaCl, was dried over Na₂SO₄, was filtered, and the filtrate was concentrated under reduced pressure. Purification by ISCO CombiFlash^{®} chromatography (12 g silica Gold column, 30 mL/min) eluting with a gradient of 0 to 30 % methanol in dichloromethane (DCM) provided the title compound as a white solid (63.5 mg, 0.0508 mmol, 64.7 % yield). LCMS (m/z) 1093.7 (M+H)⁺. ¹H NMR (400 MHz, METHANOL-*d*₄) δ ppm 1.22 - 1.43 (m, 8 H) 1.50 - 1.77 (m, 8 H) 1.83 (dt, *J*=14, 6.7 Hz, 4 H) 1.90 - 2.05 (m, 8 H) 2.11 - 2.20 (m, 1 H) 2.25 (t, *J*=7.3 Hz, 2 H) 2.60 (br d, *J*=11 Hz, 2 H) 2.66 (s, 3 H) 2.68 - 2.76 (m, 2 H) 2.80 - 2.91 (m, 2 H) 2.83 - 2.83 (m, 1 H) 2.96 (br s, 1 H) 3.03 - 3.16 (m, 2 H) 3.24 - 3.31 (m, 2 H) 3.35 - 3.53 (m, 17 H) 3.56 - 3.78 (m, 2 H) 3.83 - 4.10 (m, 6 H) 4.15 (br d, *J*=13 Hz, 2 H) 4.43 (br s, 2 H) 4.80 - 4.85 (m, 1 H) 7.54 (dd, *J*=8.8, 4.9 Hz, 1 H) 7.81 (dt, *J*=8.3, 2.0 Hz, 1 H) 8.09 (br s, 1 H) 8.52 (d, *J*=2.0 Hz, 1 H) 8.59 (dd, *J*=4.9, 1.5 Hz, 1 H) 8.72 (s, 1 H).

The following compounds were or could be prepared with procedures analogous to that described in Example 2:

| **Ex.** | **Compound** | **LC-MS m/z** | **NMR** |
|---|---|---|---|
| 3 | 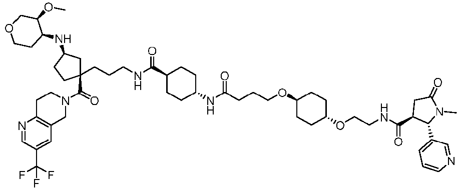 (2S,3S)-N-(2-(((1R,4S)-4-(4-(((1S,4R)-4-((3-((1S,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)propyl)carbamoyl)cyclohexy I)amino)-4-oxobutoxy)cyclohexyl)oxy)ethyl)-1-methyl-5-oxo-2-(pyridin-3-yl)pyrrolidine-3-carboxamide, | 1039.7 (M+H)⁺ | ¹H NMR (400 MHz, METHANOL-d4) δ 1.17 - 1.47 (m, 8H) 1.47 -1.60 (m, 2H) 1.60 - 1.76 (m, 4H) 1.76 - 1.90 (m, 6H) 1.90 - 2.03 (m, 8H) 2.03 - 2.13 (m, 1H) 2.14 - 2.20 (m, 1H) 2.24 (t, *J* = 7.6 Hz, 2H) 2.52 - 2.63 (m, 1H) 2.67 (s, 3H) 2.68 - 2.75 (m, 2H) 2.83 (s, 2H) 3.01 - 3.18 (m, 5H) 3.24 - 3.31 (m, 3H) 3.34 - 3.43 (m, 8H) 3.43 - 3.54 (m, 5H) 3.62 (tt, *J =* 12, 3.9 Hz, 1H) 3.91 (br d, *J* = 12 Hz, 2H) 3.94 - 4.08 (m, 2H) 4.10 - 4.23 (m, 1H) 4.82 - 4.87 (m, 2H) 7.51 - 7.58 (m, 1H) 7.81 (dt, *J* = 7.8, 2.0 Hz, 1H) 8.06 (br s, 1H) 8.47 - 8.55 (m, 1H) 8.59 (dd, *J* = 4.9, 1.5 Hz, 1H) 8.72 (s, 1H). |
| 4 | 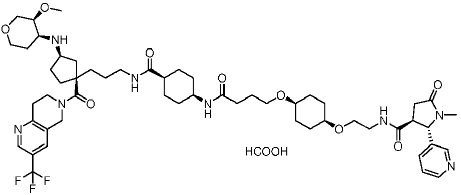 (2S,3S)-N-(2-(((1S,4R)-4-(4-(((1R,4S)-4-((3-((1S,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)propyl)carbamoyl)cyclohexy I)amino)-4-oxobutoxy)cyclohexyl)oxy)ethyl)-1-methyl-5-oxo-2-(pyridin-3-yl)pyrrolidine-3-carboxamide, Formic acid salt. | 1039.9 (M+H)⁺ | ¹H NMR (400 MHz, DMSO-*d*₆) δ 1.07 - 1.35 (m, 4H), 1.35 - 1.51 (m, 8H), 1.56 (br dd, J = 17.9, 13.4 Hz, 9H), 1.66 (dt, J = 14.3, 6.8 Hz, 4H), 1.76 - 1.97 (m, 2H), 2.01 - 2.10 (m, 2H), 2.13 (t, J = 7.6 Hz, 2H), 2.43 (br dd, J = 16.6, 7.3 Hz, 2H), 2.64 - 2.77 (m, 2H), 2.87 - 3.05 (m, 6H), 3.08 - 3.41 (m, 16H), 3.68 - 3.80 (m, 3H), 3.86 (br t, J = 5.6 Hz, 2H), 3.93 (br dd, J = 12.7, 3.4 Hz, 1H), 4.66 (d, J = 6.4 Hz, 1H), 4.79 (br s, 2H), 7.44 (dd, J = 7.8, 4.9 Hz, 1H), 7.63 (br s, 1H), 7.65 - 7.72 (m, 2H), 8.06 (t, J = 5.9 Hz, 1H), 8.18 (s, 1H), 8.23 (s, 1H), 8.48 (d, J = 2.4 Hz, 1H), 8.56 (dd, J = 4.9, 1.5 Hz, 1H), 8.77 (s, 1H) |
| 5 | 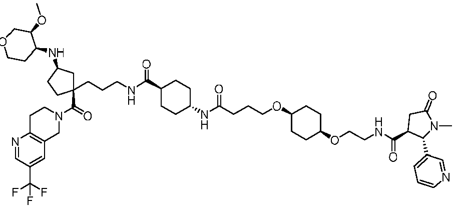 (2S,3S)-N-(2-(((1S,4R)-4-(4-(((1S,4R)-4-((3-((1S,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)propyl)carbamoyl)cyclohexy I)amino)-4-oxobutoxy)cyclohexyl)oxy)ethyl)-1-methyl-5-oxo-2-(pyridin-3-yl)pyrrolidine-3-carboxamide | 1040.1 (M+H)⁺ | ¹H NMR (400 MHz, METHANOL-*d*₄) δ 1.18 - 1.30 (m, 2H), 1.30 - 1.47 (m, 3H), 1.47 - 1.61 (m, 6H), 1.61 - 1.76 (m, 8H), 1.76 - 1.88 (m, 5H), 1.90 - 2.02 (m, 4H), 2.03 - 2.21 (m, 2H), 2.25 (t, J = 7.6 Hz, 2H), 2.58 (td, J = 7.9, 4.2 Hz, 1H), 2.65 - 2.75 (m, 4H), 2.78 - 2.91 (m, 2H), 3.02 - 3.18 (m, 5H), 3.26 - 3.32 (m, 1H), 3.34 - 3.53 (m, 13H), 3.56 - 3.70 (m, 2H), 3.87 - 4.08 (m, 4H), 4.09 - 4.22 (m, 2H), 4.84 (d, J = 6.8 Hz, 1H), 7.51 - 7.59 (m, 1H), 7.81 (dt, J = 8.1, 1.8 Hz, 1H), 8.06 (br s, 1H), 8.45 - 8.55 (m, 1H), 8.59 (dd, J = 4.9, 1.5 Hz, 1H), 8.71 (s, 1H) |
| 6 | 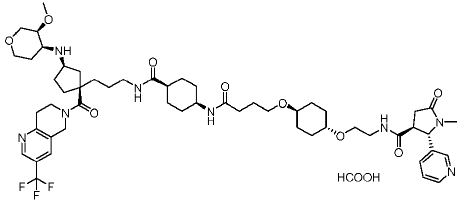 (2S,3S)-N-(2-(((1R,4S)-4-(4-(((1R,4S)-4-((3-((1S,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)propyl)carbamoyl)cyclohexy l)amino)-4-oxobutoxy)cyclohexyl)oxy)ethyl)-1-methyl-5-oxo-2-(pyridin-3-yl)pyrrolidine-3-carboxamide, Formic acid salt | 1039.9 (M+H)⁺ | ¹H NMR (400 MHz, METHANOL-*d*₄) δ ppm 1.13 - 1.52 (m, 6 H) 1.55 - 1.68 (m, 4 H) 1.68 - 2.09 (m, 14 H) 2.19 - 2.33 (m, 4 H) 2.36 - 2.62 (m, 2 H) 2.63 - 2.73 (m, 4 H) 2.80 - 2.88 (m, 1 H) 2.99 - 3.20 (m, 5 H) 3.21 - 3.31 (m, 4 H) 3.35 - 3.59 (m, 12 H) 3.74 (br t, *J*=7.6 Hz, 1 H) 3.92 (br s, 1 H) 3.95 - 4.11 (m, 5 H) 4.29 (br d, *J*=13 Hz, 2 H) 4.84 (d, *J*=6.9 Hz, 2 H) 7.54 (dd, *J*=7.8, 4.9 Hz, 1 H) 7.79 - 7.86 (m, 2 H) 8.07 (br s, 1 H) 8.48 (s, 1 H) 8.52 (d, *J*=2.0 Hz, 1 H) 8.59 (dd, *J*=4.9, 1.5 Hz, 2 H) 8.74 (s, 1 H) |
| 7 | 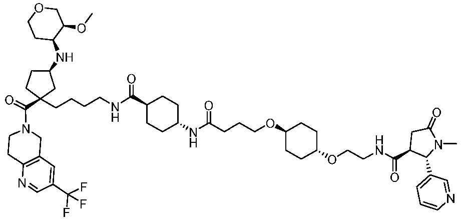 (2S,3S)-N-(2-(((1R,4S)-4-(4-(((1S,4R)-4-((4-((1S,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)butyl)carbamoyl)cyclohexyl) amino)-4-oxobutoxy)cyclohexyl)oxy)ethyl)-1-methyl-5-oxo-2-(pyridin-3-yl)pyrrolidine-3-carboxamide. | 1053.9 (M+H)⁺ | ¹H NMR (400 MHz, DMSO-*d*₆) δ 0.91 - 1.25 (m, 11H), 1.25 - 1.39 (m, 3H), 1.39 - 1.70 (m, 10H), 1.70 - 1.89 (m, 8H), 1.89 - 2.03 (m, 2H), 2.06 (t, J = 7.3 Hz, 2H), 2.42 (dd, J = 16.4, 7.6 Hz, 2H), 2.52 (br s, 2H), 2.65 - 2.78 (m, 2H), 2.81 - 2.92 (m, 2H), 2.92 - 3.01 (m, 3H), 3.05 - 3.26 (m, 10H), 3.27 - 3.33 (m, 3H), 3.35 - 3.48 (m, 3H), 3.67 - 3.78 (m, 1H), 3.85 (br dd, J = 7.1, 3.2 Hz, 3H), 4.66 (d, J = 6.4 Hz, 1H), 4.79 (br s, 2H), 7.45 (dd, J = 8.1, 4.6 Hz, 1H), 7.58 (br t, J = 5.4 Hz, 1H), 7.63 (d, J = 7.8 Hz, 1H), 7.68 (dt, J = 7.8, 2.0 Hz, 1H), 8.04 (s, 1H), 8.19 (br s, 1H), 8.48 (d, J = 2.0 Hz, 1H), 8.57 (dd, J = 4.9, 1.5 Hz, 1H), 8.75 (s, 1H) |
| 8 | 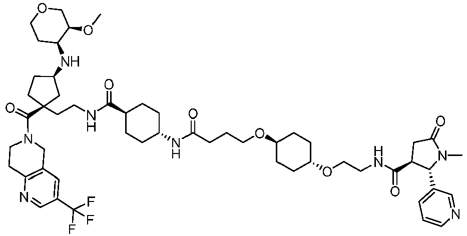 (2S,3S)-N-(2-(((1R,4S)-4-(4-(((1R,4R)-4-((2-((1R,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)ethyl)carbamoyl)cyclohexyl) amino)-4-oxobutoxy)cyclohexyl)oxy)ethyl)-1-methyl-5-oxo-2-(pyridin-3-yl)pyrrolidine-3-carboxamide. | 1025.9 (M+H)⁺ | ¹H NMR (400 MHz, DMSO-*d*₆) δ 1.00 - 1.35 (m, 10H), 1.42 - 1.57 (m, 3H), 1.57 - 1.70 (m, 5H), 1.70 - 1.79 (m, 5H), 1.79 - 1.93 (m, 6H), 1.99 - 2.09 (m, 3H), 2.38 - 2.47 (m, 2H), 2.65 - 2.74 (m, 2H), 2.74 - 2.92 (m, 3H), 2.92 - 3.00 (m, 1H), 3.04 (br s, 1H), 3.06 - 3.14 (m, 2H), 3.14 - 3.21 (m, 4H), 3.21 - 3.26 (m, 5H), 3.26 - 3.33 (m, 2H), 3.35 (br s, 3H), 3.58 - 3.79 (m, 2H), 3.80 - 4.05 (m, 4H), 4.59 - 4.72 (m, 1H), 4.72 - 4.89 (m, 2H), 7.45 (dd, J = 7.8, 4.9 Hz, 1H), 7.62 (br d, J = 7.8 Hz, 2H), 7.68 (dt, J = 7.8, 2.0 Hz, 1H), 8.04 (t, J = 5.6 Hz, 1H), 8.17 (s, 1H), 8.48 (d, J = 2.0 Hz, 1H), 8.56 (dd, J = 4.6, 1.7 Hz, 1H), 8.76 (s, 1H) |
| 9 | 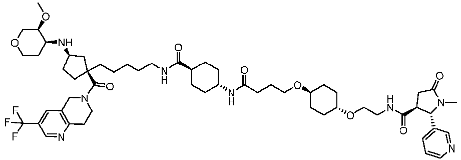 (2S,3S)-N-(2-(((1R,4S)-4-(4-(((1S,4R)-4-((5-((1S,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)pentyl)carbamoyl)cyclohexy I)amino)-4-oxobutoxy)cyclohexyl)oxy)ethyl)-1-methyl-5-oxo-2-(pyridin-3-yl)pyrrolidine-3-carboxamide | 1067.9 (M+H)⁺ | ¹H NMR (400 MHz, DMSO-*d*₆) δ ppm 0.80 - 1.56 (m, 16 H) 1.58 - 1.71 (m, 4 H) 1.71 - 1.89 (m, 6 H) 1.90 - 2.02 (m, 1 H) 2.06 (t, *J*=7.3 Hz, 2 H) 2.42 (dd, *J*=17, 7.8 Hz, 2 H) 2.62 - 2.79 (m, 4 H) 2.87 (br s, 2 H) 2.90 - 3.02 (m, 4 H) 3.05 - 3.33 (m, 18 H) 3.37 - 3.51 (m, 4 H) 3.72 (dt, *J*=11, 4.3 Hz, 2 H) 3.81 - 3.92 (m, 3 H) 4.66 (d, *J*=6.7 Hz, 1 H) 4.80 (br s, 2 H) 7.41 - 7.48 (m, 1 H) 7.45 - 7.45 (m, 1 H) 7.57 (br s, 1 H) 7.64 (d, *J*=7.9 Hz, 1 H) 7.68 (dt, *J*=7.9, 2.0 Hz, 1 H) 8.04 (br t, *J*=5.6 Hz, 1 H) 8.19 (br s, 1 H) 8.47 (d, *J=*2.0 Hz, 1 H) 8.56 (dd, *J*=4.7, 1.7 Hz, 1 H) 8.76 (s, 1 H) |
| 10 | 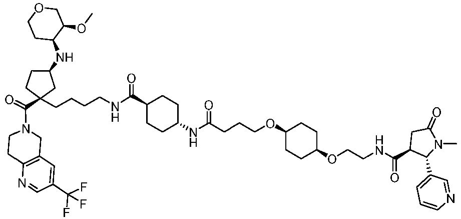 (2S,3S)-N-(2-(((1S,4R)-4-(4-(((1S,4R)-4-((4-((1S,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)butyl)carbamoyl)cyclohexyl)amino)-4-oxobutoxy)cyclohexyl)oxy)ethyl)-1-methyl-5-oxo-2-(pyridin-3-yl)pyrrolidine-3-carboxamide | 1053.9 (M+H)⁺ | ¹H NMR (400 MHz, METHANOL-*d*₄) δ ppm -0.12 - 0.16 (m, 1 H) 1.08 - 1.32 (m, 5 H) 1.38 (br d, *J*=8.8 Hz, 3 H) 1.46 - 1.60 (m, 6 H) 1.61 - 1.75 (m, 8 H) 1.75 - 1.89 (m, 6 H) 1.95 (br d, *J*=12 Hz, 4 H) 2.04 - 2.21 (m, 3 H) 2.25 (t, *J*=7.3 Hz, 2 H) 2.58 (br s, 1 H) 2.67 (s, 3 H) 2.68 - 2.74 (m, 1 H) 2.79 - 2.91 (m, 2 H) 2.97 - 3.14 (m, 5 H) 3.23 - 3.31 (m, 1 H) 3.35 - 3.53 (m, 12 H) 3.56 - 3.76 (m, 2 H) 3.87 - 4.09 (m, 4 H) 4.15 (br d, *J*=11 Hz, 1 H) 4.75 - 4.86 (m, 1 H) 7.55 (dd, *J*=7.8, 4.9 Hz, 1 H) 7.81 (dt, *J*=7.8, 2.0 Hz, 2 H) 8.07 (br s, 1 H) 8.52 (d, *J=*2.0 Hz, 1 H) 8.59 (dd, *J*=4.9, 1.47 Hz, 1 H) 8.72 (s, 1 H) |
| 11 | 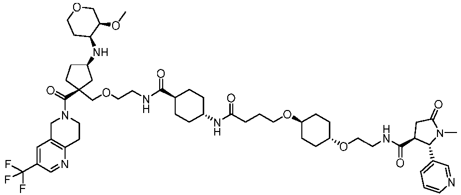 (2S,3S)-N-(2-(((1R,4S)-4-(4-(((1S,4R)-4-((2-(((1S,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)methoxy)ethyl)carbamoyl)c yclohexyl)amino)-4-oxobutoxy)cyclohexyl)oxy)ethyl)-1-methyl-5-oxo-2-(pyridin-3-yl)pyrrolidine-3-carboxamide | 1055.7 (M+H)⁺ | ¹H NMR (400 MHz, DMSO-*d*₆) δ 0.98 - 1.20 (m, 7H), 1.20 - 1.41 (m, 4H), 1.41 - 1.57 (m, 2H), 1.60 - 1.90 (m, 14H), 1.94 - 2.13 (m, 4H), 2.25 - 2.37 (m, 1H), 2.42 (dd, J = 16.9, 7.6 Hz, 1H), 2.70 (br dd, J = 16.9, 9.5 Hz, 2H), 2.92 - 3.01 (m, 3H), 3.01 - 3.30 (m, 15H), 3.30 - 3.33 (m, 3H), 3.35 - 3.38 (m, 2H), 3.42 (s, 3H), 3.72 (dt, J = 11.1, 3.7 Hz, 1H), 3.81 - 3.91 (m, 3H), 4.66 (d, J = 6.4 Hz, 1H), 4.79 (br s, 2H), 7.45 (dd, J = 7.8, 4.9 Hz, 1H), 7.56 - 7.75 (m, 3H), 8.04 (t, J = 5.6 Hz, 1H), 8.17 (s, 1H), 8.48 (d, J = 2.0 Hz, 1H), 8.57 (dd, J = 4.9, 1.5 Hz, 1H), 8.76 (s, 1H) |
| 12 | 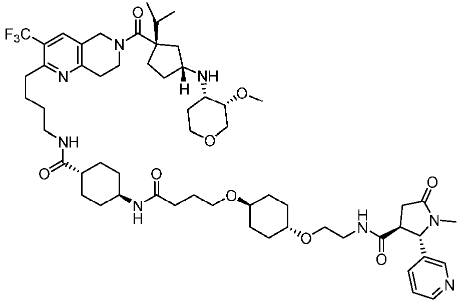 (2S,3S)-N-(2-(((1R,4S)-4-(4-(((1r,4R)-4-((4-(6-((1S,3R)-1-Isopropyl-3-(((3S,4S)-3-methoxytetrahydro-2H-pyran-4-yl)amino)cyclopentane-1-carbonyl)-3(trifluoromethyl)-5,6,7,8-tetrahydro-1,6-naphthyridin-2-yl)butyl)carbamoyl)cyclohexyl)amino)-4 oxobutoxy)cyclohexyl)oxy)ethyl)-1-methyl-5-oxo-2-(pyridin-3-yl)pyrrolidine-3 carboxamide. | 1095.6 (M+H)⁺ | ¹H NMR (600 MHz, DMSO-*d*₆) δ ppm 8.55 (dd, *J=* 1.6, 4.7 Hz, 1 H) 8.47 (d, *J=* 2.0 Hz, 1 H) 8.05 - 8.08 (m, 1 H) 8.04 (t, *J=* 5.5 Hz, 1 H) 7.70 (d, *J=* 7.4 Hz, 1 H) 7.66 (td, *J=* 2.0, 7.9 Hz, 1 H) 7.64 (t, *J=* 5.6 Hz, 1 H) 7.43 (dd, *J=* 4.7, 7.8 Hz, 1 H) 4.69 - 4.81 (m, 2 H) 4.64 (d, *J=* 6.1 Hz, 1 H) 3.79 - 3.91 (m, 3 H) 3.66 - 3.77 (m, 2 H) 3.57 (t, *J=* 6.5 Hz, 2 H) 3.42 - 3.49 (m, 8 H) 3.33 - 3.41 (m, 2 H) 3.12 - 3.29 (m, 11 H) 2.98 - 3.07 (m, 3 H) 2.86 - 2.98 (m, 3 H) 2.79 (br t, *J=* 7.6 Hz, 2 H) 2.66 - 2.74 (m, 2 H) 2.50 (s, 3 H) 2.43 (dd, J= 7.4, 16.8 Hz, 1 H) 2.33 (t, *J=* 6.5 Hz, 3 H) 2.08 - 2.18 (m, 2 H) 2.03 (br s, 1 H) 1.55 - 1.77 (m, 8 H) 1.42 - 1.47 (m, 4 H) 1.36 - 1.55 (m, 6 H) 1.24 - 1.36 (m, 1 H) 1.08 (br d, *J= 6.1* Hz, 1 H) 0.83 (br d, *J*= 6.3 Hz, 3 H) 0.70 (br d, *J=* 6.3 Hz, 3 H). |
| 13 | 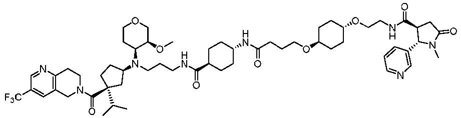 (2S,3S)-N-(2-(((1R,4S)-4-(4-(((1S,4R)-4-((3-(((1R,3S)-3-isopropyl-3-(3-(trifluoromethyl)-5,6,7,8-tetrahydro-1,6-naphthyridine-6-carbonyl)cyclopentyl)((3S,4S)-3-methoxytetrahydro-2H-pyran-4-yl)amino)propyl)carbamoyl)cyclohexyl)amino)-4-oxobutoxy)cyclohexyl)oxy)ethyl)-1-methyl-5-oxo-2-(pyridin-3-yl)pyrrolidine-3-carboxamide | 1081.76 (M+H)⁺ | ¹H NMR (METHANOL-d₄, 400 MHz): δ (ppm) 8.70 (s, 1H), 8.57 (dd, *J* = 4.9, 1.5 Hz, 1H), 8.50 (d, *J* = 2.0 Hz, 1H), 8.09 (br s, 1H), 7.79 (dt, *J* = 7.8, 2.0 Hz, 1H), 7.52 (dd, *J* = 7.6, 5.1 Hz, 1H), 4.82 (d, *J* = 6.8 Hz, 1H), 4.09 (br d, *J =* 12.2 Hz, 2H), 3.84-3.99 (m, 2H), 3.61 (tt, *J* = 11.6, 4.1 Hz, 1H), 3.41-3.57 (m, 5H), 3.33-3.41 (m, 6H, under MeOH peak), 3.20-3.29 (m, 5H), 2.99-3.15 (m, 5H), 2.79 (d, *J* = 9.3 Hz, 2H), 2.66-2.73 (m, 2H), 2.65 (s, 3H), 2.39-2.61 (m, 2H), 2.01-2.26 (m, 7H), 1.64-2.00 (m, 13H), 1.15-1.63 (m, 15H), 0.98 (d, *J =* 6.4 Hz, 3H), 0.78 (d, *J* = 6.4 Hz, 3H). |
| 14 | 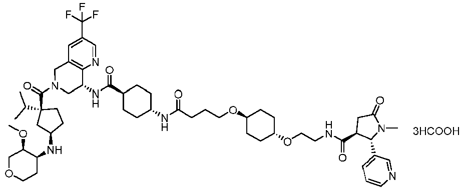 (2S,3S)-N-(2-(((1R,4S)-4-(4-(((1R,4R)-4-(((R)-6-((1S,3R)-1-isopropyl-3-(((3S,4S)-3-methoxytetrahydro-2H-pyran-4-yl)amino)cyclopentane-1-carbonyl)-3-(trifluoromethyl)-5,6,7,8-tetrahydro-1,6-naphthyridin-8-yl)carbamoyl)cyclohexyl)amino)-4-oxobutoxy)cyclohexyl)oxy)ethyl)-1-methyl-5-oxo-2-(pyridin-3-yl)pyrrolidine-3-carboxamide, 3Formic acid salt. | 1039.4 (M+H)⁺. | ¹H NMR (400 MHz, METHANOL-*d*₄) δ ppm 0.84 - 1.03 (m, 6 H) 1.18 - 1.37 (m, 6 H) 1.49 - 1.75 (m, 4 H) 1.76 - 1.90 (m, 4 H) 1.90 - 2.08 (m, 10 H) 2.25 (br t, *J*=7.3 Hz, 4 H) 2.57 (br s, 2 H) 2.66 (s, 3 H) 2.68 - 2.75 (m, 1 H) 2.77 - 2.90 (m, 1 H) 2.97 - 3.17 (m, 1 H) 3.21 - 3.31 (m, 4 H) 3.35 - 3.42 (m, 3 H) 3.43 - 3.59 (m, 12 H) 3.65 (br d, *J*=12 Hz, 2 H) 3.82 - 4.07 (m, 1 H) 4.28 (br d, *J*= 13 Hz, 1 H) 4.38 - 4.49 (m, 1 H) 4.84 (d, *J*=6.9 Hz, 1 H) 5.10 (br s, 1 H) 7.44 - 7.65 (m, 1 H) 7.81 (dt, *J*=8.3, 2.0 Hz, 1 H) 8.13 (s, 1 H) 8.52 (d, *J*=2.5 Hz, 2 H) 8.59 (dd, *J*=4.9, 1.47 Hz, 1 H) 8.83 (s, 1 H). |
| 15 | 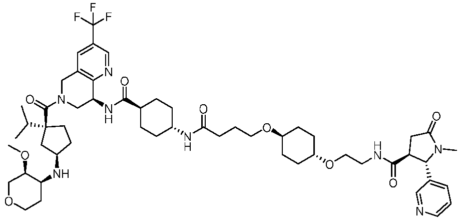 (2S,3S)-N-(2-(((1R,4S)-4-(4-(((1S,4R)-4-(((S)-6-((1S,3R)-1-isopropyl-3-(((3S,4S)-3-methoxytetrahydro-2H-pyran-4-yl)amino)cyclopentane-1-carbonyl)-3-(trifluoromethyl)-5,6,7,8-tetrahydro-1,6-naphthyridin-8-yl)carbamoyl)cyclohexyl)amino)-4-oxobutoxy)cyclohexyl)oxy)ethyl)-1-methyl-5-oxo-2-(pyridin-3-yl)pyrrolidine-3-carboxamide. | 1039.7 (M)⁺. | ¹H NMR (400 MHz, METHANOL-*d*₄) δ ppm 0.80 - 0.90 (m, 3 H) 0.98 (br dd, *J*=13, 5.6 Hz, 3 H) 1.17 - 1.37 (m, 7 H) 1.54 - 1.75 (m, 5 H) 1.78 - 2.03 (m, 13 H) 2.15 - 2.30 (m, 5 H) 2.50 (br d, *J*=8.8 Hz, 1 H) 2.66 (s, 3 H) 2.68 - 2.73 (m, 1 H) 2.78 - 2.88 (m, 2 H) 3.03 - 3.10 (m, 1 H) 3.33 (dt, J=3.3, 1.5 Hz, 9 H) 3.39 (s, 4 H) 3.47 (t, J=5.6 Hz, 5 H) 3.66 (br t, *J=11* Hz, 2 H) 3.91 (br d, *J=11* Hz, 1 H) 4.07 - 4.27 (m, 2 H) 4.73 - 4.86 (m, 2 H) 5.09 (br s, 2 H) 7.54 (dd, *J=7.8,* 4.9 Hz, 1 H) 7.81 (dt, *J*=7.8, 2.0 Hz, 1 H) 8.14 (br s, 1 H) 8.52 (d, *J=*2.0 Hz, 1 H) 8.59 (dd, *J*=4.9, 1.5 Hz, 1 H) 8.80 (d, *J*=7.2 Hz, 1 H). |

### Example 16: N¹-((1R,4s)-4-((3-((1S,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)propyl)carbamoyl)cyclohexyl)-N⁵-(4-((3-((2S,3S)-1-methyl-5-oxo-2-(pyridin-3-yl)pyrrolidine-3-carboxamido)propyl)amino)-4-oxobutyl)glutaramide

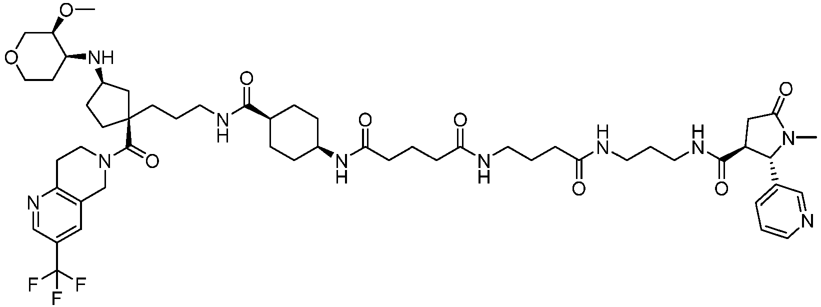

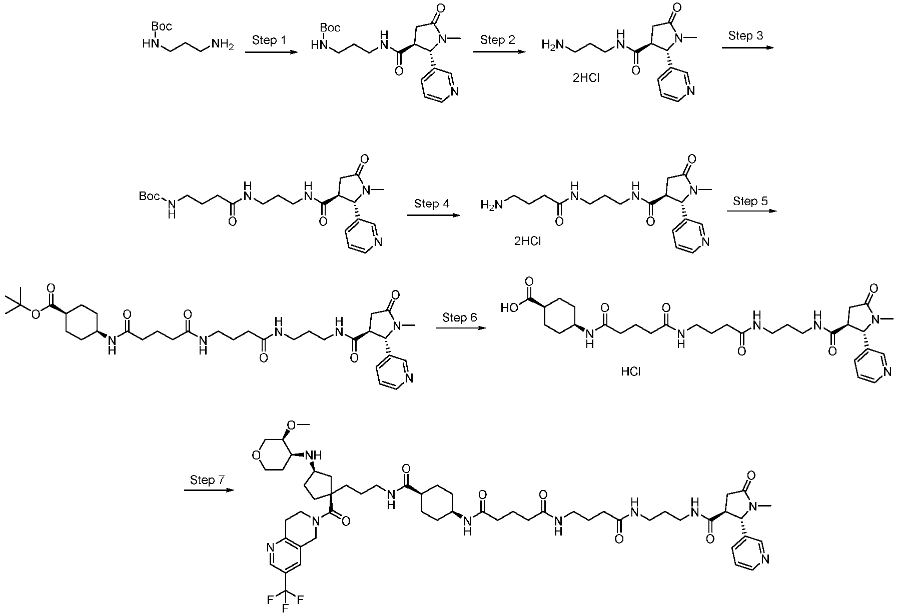

### Step 1: tert-Butyl (3-((2S,3S)-1-methyl-5-oxo-2-(pyridin-3-yl)pyrrolidine-3-carboxamido)propyl)carbamate.

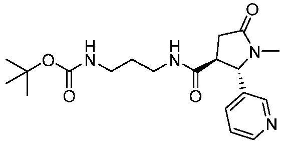

To a mixture of (2*S*,3*S*)-1-methyl-5-oxo-2-(pyridin-3-yl)pyrrolidine-3-carboxylic acid (2000 mg, 9.08 mmol) and commercially-available tert-butyl (3-aminopropyl)carbamate (1741 mg, 9.99 mmol) in dichloromethane (DCM) (30 mL) was added DIPEA (1.740 mL, 9.99 mmol), followed by T3P (50% in ethyl acetate) (5.95 mL, 9.99 mmol). The mixture was stirred at rt for 2 h, was washed with water (10 mL), was dried over Na₂SO₄, and was concentrated under reduced pressure. Purification by normal-phase chromatography eluting with a gradient of 0 to 10 % methanol in dichloromethane (DCM) provided the title compound as a pale-yellow liquid (3430 mg, 8.20 mmol, 90 % yield). LCMS (m/z) 377.1 (M+H)⁺.

### Step 2: (2S,3S)-N-(3-Aminopropyl)-1-methyl-5-oxo-2-(pyridin-3-yl)pyrrolidine-3-carboxamide, 2Hydrochloric acid salt.

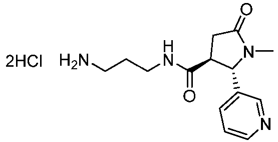

To tert-butyl (3-((2*S*,3*S*)-1-methyl-5-oxo-2-(pyridin-3-yl)pyrrolidine-3-carboxamido)propyl)carbamate (3430 mg, 8.20 mmol) was added 4 N HCl in 1,4-dioxane (6.81 mL, 27.2 mmol). The mixture was stirred for 1 h and was concentrated under reduced pressure to provide the title compound as a white solid (3143 mg, 8.10 mmol, 89 % yield). LCMS (m/z) 277.1 (M+H)⁺.

### Step 3: tert-Butyl (4-((3-((2S,3S)-1-methyl-5-oxo-2-(pyridin-3-yl)pyrrolidine-3-carboxamido)propyl)amino)-4-oxobutyl)carbamate.

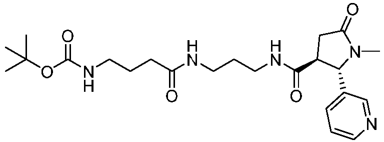

To a mixture of (2S,3S)-N-(3-aminopropyl)-1-methyl-5-oxo-2-(pyridin-3-yl)pyrrolidine-3-carboxamide, 2Hydrochloric acid salt (3143 mg, 9 mmol), 4-((tertbutoxycarbonyl)amino)butanoic acid (1829 mg, 9.00 mmol) in dichloromethane (DCM) (30 mL) was added DIPEA(4.70 mL, 27.0 mmol), followed by the addition of T3P 50% in ethyl acetate (6.43 mL, 10.80 mmol). The mixture was stirred at rt for 2 h and was washed with water (30 mL). The organic phase was dried over Na₂SO₄ and was concentrated under reduced pressure. Purification by normal-phase chromatography eluting with a gradient of 0 to 10 % methanol in dichloromethane (DCM) provided the title compound as a pale-yellow liquid (900 mg, 1.852 mmol, 20.58 % yield) as a pale yellow liquid.to provide the title compound as a pale-yellow liquid (900 mg, 1.852 mmol, 20.58 %). LCMS m/z 462.5 (M+H)⁺.

### Step 4: (2S,3S)-N-(3-(4-Aminobutanamido)propyl)-1-methyl-5-oxo-2-(pyridin-3-yl)pyrrolidine-3-carboxamide, 2Hydrochloric acid salt.

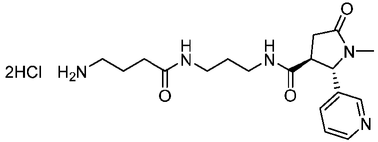

To tert-butyl (4-((3-((2*S*,3*S*)-1-methyl-5-oxo-2-(pyridin-3-yl)pyrrolidine-3-carboxamido)propyl)amino)-4-oxobutyl)carbamate (900 mg, 1.852 mmol, 20.58 % yield) was added 4 N HCl in 1,4-dioxane, the mixture was stirred for 1 h, and was concentrated under reduced pressure to provide the title compound as a white solid (847 mg, 1.852 mmol, 20.58 % yield). LCMS (m/z) 362.1 (M+H)⁺.

### Step 5: tert-Butyl (1s,4s)-4-(5-((4-((3-((2S,3S)-1-methyl-5-oxo-2-(pyridin-3-yl)pyrrolidine-3-carboxamido)propyl)amino)-4-oxobutyl)amino)-5-oxopentanamido)cyclohexane-1-carboxylate.

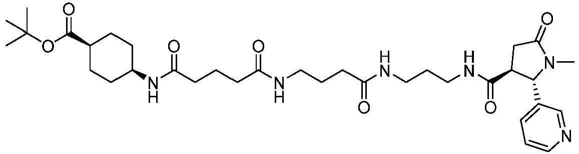

To a mixture of (2*S*,3*S*)-N-(3-(4-aminobutanamido)propyl)-1-methyl-5-oxo-2-(pyridin-3-yl)pyrrolidine-3-carboxamide, 2hydrochloric acid salt (804 mg, 1.852 mmol) and commercially-available dihydro-2H-pyran-2,6(3H)-dione (211 mg, 1.852 mmol) in dichloromethane (DCM) (3 mL) was added DIPEA (1.613 mL, 9.26 mmol) and the mixture was stirred at rt for 10 min. HATU (845 mg, 2.222 mmol) was added, followed by commercially-available (1s,4s)-tert-butyl-4-aminocyclohexanecarboxylate, hydrochloric acid salt (437 mg, 1.852 mmol). The mixture was stirred at rt for 2 h, was washed with water (10 mL), was dried over Na₂SO₄, and was concentrated under reduced pressure. Purification by ISCO CombiFlash^{®} chromatography (24 g Gold column) eluting with a gradient of 0 to 20 % dichloromethane (DCM) in methanol provided the title compound as an off-white solid (1200 mg, 1.644 mmol, 89 % yield). LCMS (m/z) 657.2 (M+H)⁺.

### Step 6: (1s,4s)-4-(5-((4-((3-((2S,3S)-1-Methyl-5-oxo-2-(pyridin-3-yl)pyrrolidine-3-carboxamido)propyl)amino)-4-oxobutyl)amino)-5-oxopentanamido)cyclohexane-1-carboxylic acid, Hydrochloric acid salt.

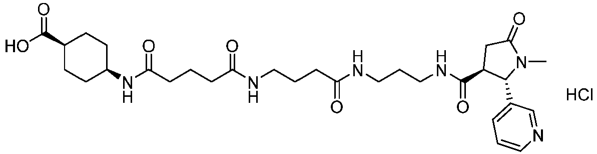

To a solution of (1s,4s, cis)-tert-butyl 4-(5-((4-((3-((2S,3S)-1-methyl-5-oxo-2-(pyridin-3-yl)pyrrolidine-3-carboxamido)propyl)amino)-4-oxobutyl)amino)-5-oxopentanamido)cyclohexanecarboxylate (1200 mg, 1.644 mmol) in Dichloromethane (DCM) (3 mL) was added 4M HCl in Dioxane (3 mL) for 1 h. It was then concentrated under vacuo to give the title compound as a white solid (1155 mg, 1.631 mmol, 88 % yield). LCMS (m/z) 601.1 (M+H)⁺

### Step 7: N¹-((1R,4s)-4-((3-((1S,3R)-3-(((3S,4S)-3-methoxytetrahydro-2H-pyran-4-yl)amino)-1-(3-(trifluoromethyl)-5,6,7,8-tetrahydro-1,6-naphthyridine-6-carbonyl)cyclopentyl)propyl)carbamoyl)cyclohexyl)-N⁵-(4-((3-((2S,3S)-1-methyl-5-oxo-2-(pyridin-3-yl)pyrrolidine-3-carboxamido)propyl)amino)-4-oxobutyl)glutaramide

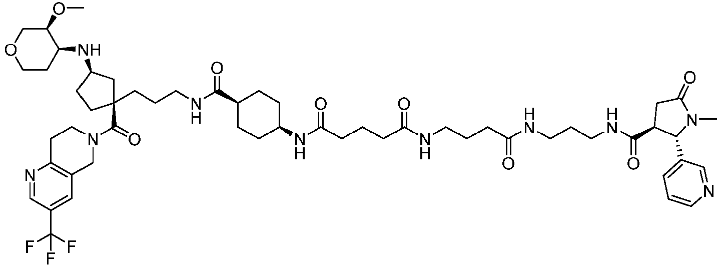

To a solution of ((1*S*,3*R*)-1-(3-aminopropyl)-3-(((3*S*,4*S*)-3-methoxytetrahydro-2H-pyran-4-yl)amino)cyclopentyl)(3-(trifluoromethyl)-7,8-dihydro-1,6-naphthyridin-6(5H)-yl)methanone, 3hydrochloric acid salt (59.4 mg, 0.100 mmol) and (1*s*,4*s*)-4-(5-((4-((3-((2*S*,3*S*)-1-methyl-5-oxo-2-(pyridin-3-yl)pyrrolidine-3-carboxamido)propyl)amino)-4-oxobutyl)amino)-5-oxopentanamido)cyclohexane-1-carboxylic acid (60.1 mg, 0.100 mmol) in dichloromethane (DCM) (2000 µL) were added HATU (57.0 mg, 0.150 mmol) and DIPEA (175 µL, 1.000 mmol) and the mixture was stirred at rt for 1.5 h. Methanol was added and the mixture was concentrated under a steady stream of nitrogen. Purification by MDAP (XSelect^{™} CSH C18 column, 40 mL/min) eluting with a gradient of 15 to 55 % acetonitrile in water containing ammonium bicarbonate (10 mM) and adjusted to pH 10 with ammonia provided the title compound as a white solid (11.1 mg, 9.88 µmol, 9.88 % yield). LCMS (m/z) 1067.7 (M+H)⁺. ¹H NMR (400 MHz, METHANOL-*d*₄) δ 1.38 (br d, J = 8.3 Hz, 3H), 1.52 - 1.69 (m, 6H), 1.70 - 1.84 (m, 7H), 1.85 - 1.96 (m, 2H), 1.96 - 2.10 (m, 2H), 2.13 - 2.29 (m, 6H), 2.51 - 2.63 (m, 1H), 2.67 (s, 2H), 2.68 - 2.77 (m, 1H), 2.80 - 2.91 (m, 1H), 2.98 - 3.06 (m, 1H), 3.07 - 3.29 (m, 8H), 3.31 - 3.35 (m, 14H), 3.36 - 3.52 (m, 5H), 3.88 - 3.96 (m, 2H), 4.01 (br dd, J = 12.2, 5.9 Hz, 2H), 4.17 (br d, J = 11.2 Hz, 1H), 4.84 (d, J = 6.4 Hz, 1H), 7.54 (dd, J = 7.6, 5.1 Hz, 1H), 7.83 (dt, J = 7.9, 1.9 Hz, 1H), 8.06 (br s, 1H), 8.54 (d, J = 1.5 Hz, 1H), 8.58 (dd, J = 4.9, 1.5 Hz, 1H), 8.72 (s, 1H)

### Example 17

### (2S,3S)-N-(2-(2-(2-(3-(((1R,4s)-4-((3-((1S,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)propyl)carbamoyl)cyclohexyl)amino)-3-oxopropoxy)ethoxy)ethoxy)ethyl)-1-methyl-5-oxo-2-(pyridin-3-yl)pyrrolidine-3-carboxamide, Formic acid salt

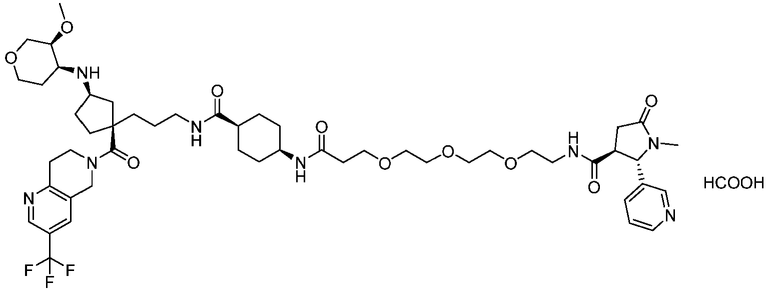

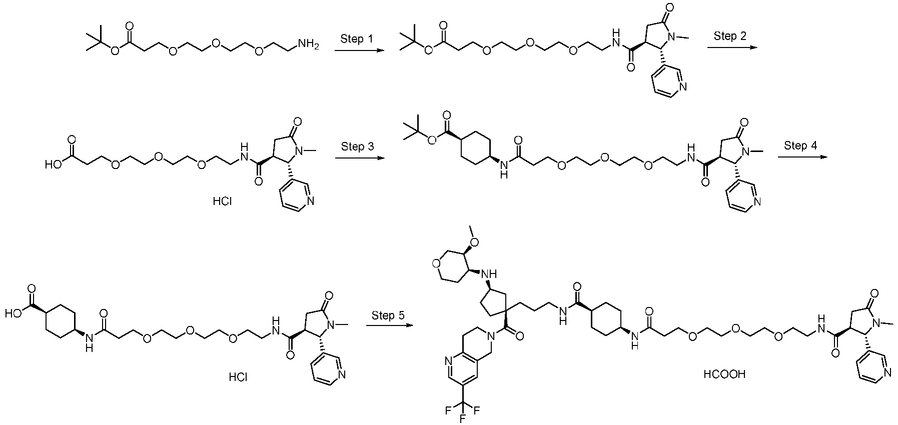

### Step 1: tert-Butyl 1-((2S,3S)-1-methyl-5-oxo-2-(pyridin-3-yl)pyrrolidin-3-yl)-1-oxo-5,8,11-trioxa-2-azatetradecan-14-oate.

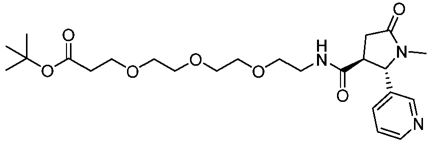

To a mixture of (2*S*,3*S*)-1-methyl-5-oxo-2-(pyridin-3-yl)pyrrolidine-3-carboxylic acid (15.69 g, 71.3 mmol) and commercially-available tert-butyl 3-(2-(2-(2-aminoethoxy)ethoxy)ethoxy)propanoate (19.7638 g, 71.3 mmol) in dichloromethane (DCM) (200 mL) was added HATU (32.5 g, 86 mmol), and triethylamine (25.8 mL, 185 mmol) and the mixture was stirred at rt under a balloon atmosphere of nitrogen for 2 h. Saturated NaHCO₃ was added and the mixture was extracted with dichloromethane (DCM). The combined organic extracts were dried over Na₂SO₄, were filtered, and the filtrate was concentrated. Purification by ISCO CombiFlash^{®} chromatography (330 g silica Gold column, 200 mL/min) eluting with a gradient of 0 to 15 % methanol (containing 10 % NH₄OH) in dichloromethane (DCM) provided the title compound as a yellow oil (31.5 g, 65.7 mmol, 92 % yield). LCMS (m/z) 480.2 (M+H)⁺.

### Step 2: 1-((2S,3S)-1-Methyl-5-oxo-2-(pyridin-3-yl)pyrrolidin-3-yl)-1-oxo-5,8,11-trioxa-2-azatetradecan-14-oic acid, Hydrochloric acid salt

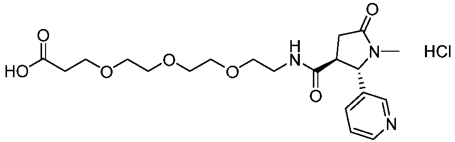

To a mixture of tert-butyl 1-((2*S*,3*S*)-1-methyl-5-oxo-2-(pyridin-3-yl)pyrrolidin-3-yl)-1-oxo-5,8,11-trioxa-2-azatetradecan-14-oate (31 g, 64.6 mmol) in dichloromethane (DCM) (40 mL) at 0 °C was added 4 N HCl n 1,4-dioxane (81 mL, 323 mmol). The mixture was stirred at rt for 2 h and was concentrated to provide the title compound (29 g, 63.1 mmol, 98 % yield). LCMS (m/z) 424.2 (M+H)⁺.

### Step 3: tert-Butyl (1s,4s)-4-(1-((2S,3S)-1-methyl-5-oxo-2-(pyridin-3-yl)pyrrolidin-3-yl)-1-oxo-5,8,11-trioxa-2-azatetradecan-14-amido)cyclohexane-1-carboxylate

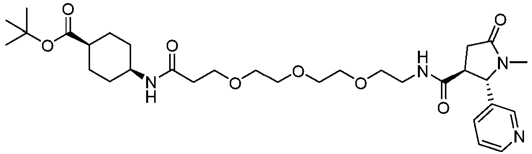

To a mixture of commercially-available tert-butyl (1*s*,4*s*)-4-aminocyclohexane-1-carboxylate, hydrochloric acid salt (2.56 g, 10.87 mmol) and 1-((2*S*,3*S*)-1-methyl-5-oxo-2-(pyridin-3-yl)pyrrolidin-3-yl)-1-oxo-5,8,11-trioxa-2-azatetradecan-14-oic acid, hydrochloric acid salt (5 g, 10.87 mmol) in dichloromethane (DCM) (100 mL) was added triethylamine (7.58 mL, 54.4 mmol) and HATU (4.96 g, 13.05 mmol). The mixture was stirred at rt for 4 h, dichloromethane (DCM) and saturated NaHCO₃ were added, and the aqueous phase was extracted with dichloromethane (DCM). The combined organic phases were dried over Na₂SO₄, were filtered, and the filtrate was concentrated. Purification by ISCO CombiFlash^{®} chromatography (120 g silica Gold column, 60 mL/min) eluting with a gradient of 0 to 15 % methanol (containing 10 % NH₄OH) in dichloromethane (DCM) provided the title compound (5.46 g, 9.03 mmol, 83 % yield). LCMS (m/z) 605.3 (M+H)⁺.

### Step 4: (1s,4s)-4-(1-((2S,3S)-1-Methyl-5-oxo-2-(pyridin-3-yl)pyrrolidin-3-yl)-1-oxo-5,8,11-trioxa-2-azatetradecan-14-amido)cyclohexane-1-carboxylic acid, hydrochloric acid salt

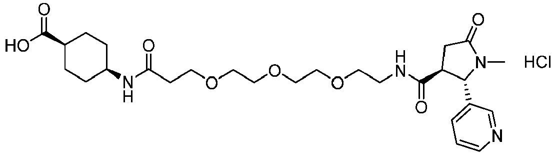

To a mixture of tert-butyl (1*s*,4*s*)-4-(1-((2*S*,3*S*)-1-methyl-5-oxo-2-(pyridin-3-yl)pyrrolidin-3-yl)-1-oxo-5,8,11-trioxa-2-azatetradecan-14-amido)cyclohexane-1-carboxylate (9.32 g, 15.41 mmol) in dichloromethane (DCM) (10 mL) at 0 °C was added 4 N HCl 1,4-dioxane (15.41 mL, 61.6 mmol). The mixture was stirred at rt for 5 h and was concentrated to provide the title compound (10 g, 17.09 mmol, 111 % yield). LCMS (m/z) 549.3 (M+H)⁺.

### Step 5: (2S,3S)-N-(2-(2-(2-(3-(((1R,4s)-4-((3-((1S,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)propyl)carbamoyl)cyclohexyl)amino)-3-oxopropoxy)ethoxy)ethoxy)ethyl)-1-methyl-5-oxo-2-(pyridin-3-yl)pyrrolidine-3-carboxamide, Formic acid salt

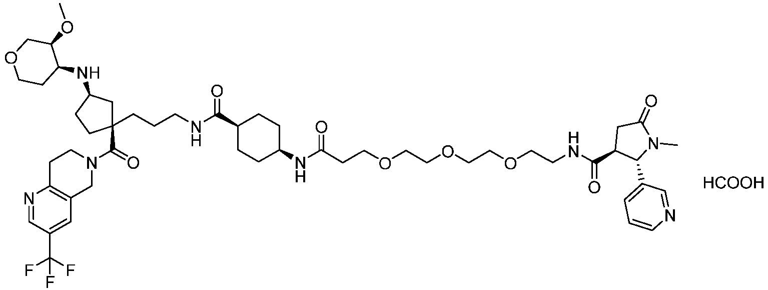

To a solution of ((1*S*,3*R*)-1-(3-aminopropyl)-3-(((3*S*,4*S*)-3-methoxytetrahydro-2H-pyran-4-yl)amino)cyclopentyl)(3-(trifluoromethyl)-7,8-dihydro-1,6-naphthyridin-6(5H)-yl)methanone, 3hydrochloric acid salt (59.4 mg, 0.100 mmol) and (1*s*,4*s*)-4-(1-((2*S*,3*S*)-1-methyl-5-oxo-2-(pyridin-3-yl)pyrrolidin-3-yl)-1-oxo-5,8,11-trioxa-2-azatetradecan-14-amido)cyclohexane-1-carboxylic acid, hydrochloric acid salt (58.5 mg, 0.100 mmol) in dichloromethane (DCM) (2000 µL) was added HATU (57.0 mg, 0.150 mmol) and DIPEA (175 µL, 1.000 mmol) and the mixture was stirred at rt for 1.5 h. Methanol was added and the mixture was concentrated under a steady stream of nitrogen. Purification by MDAP (XSelect^{™} CSH C18 column, 40 mL/min) eluting with a gradient of 5 to 35 % acetonitrile in water containing formic acid (0.1 %) provided the title compound as a white solid (44.5 mg, 0.039 mmol, 39.4 % yield). LCMS (m/z) 1015.7 (M+H)⁺. ¹H NMR (400 MHz, METHANOL-*d*₄) δ ppm 1.29 - 1.50 (m, 2H), 1.52 - 2.05 (m, 13H), 2.22 (br d, J = 5.9 Hz, 3H), 2.31 - 2.41 (m, 1H), 2.42 - 2.50 (m, 2H), 2.51 - 2.62 (m, 1H), 2.65 - 2.68 (m, 3H), 2.68 - 2.77 (m, 1H), 2.83 (d, J = 9.8 Hz, 1H), 2.99 - 3.24 (m, 5H), 3.34 - 3.44 (m, 3H), 3.44 - 3.48 (m, 3H), 3.51 (ddd, J = 5.9, 4.6, 2.7 Hz, 3H), 3.54 - 3.63 (m, 9H), 3.65 - 3.76 (m, 3H), 3.88 - 4.08 (m, 4H), 4.27 (br d, J = 13.2 Hz, 1H), 4.84 (d, J = 6.4 Hz, 1H), 7.54 (dd, J = 7.8, 4.9 Hz, 1H), 7.78 - 7.92 (m, 1H), 8.07 (br s, 1H), 8.51 - 8.61 (m, 2H), 8.74 (s, 1H)

The following compound was prepared with procedures analogous to that described in Example 17:

| **Ex.** | **Compound** | **L C-MS m/z** | **NMR** |
|---|---|---|---|
| 18 | 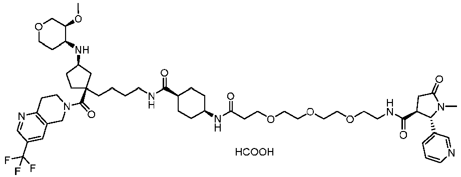 (2S,3S)-N-(2-(2-(2-(3-(((1R,4s)-4-((4-((1S,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)butyl)carbamoyl)cyclohexyl) amino)-3-oxopropoxy)ethoxy)ethoxy)ethyl)-1-methyl-5-oxo-2-(pyridin-3-yl)pyrrolidine-3-carboxamide, Formic acid salt. | 1029.8 (M+H)⁺ | ¹H NMR (400 MHz, METHANOL-*d*₄) δ ppm 1.22 (br s, 2H), 1.34 - 1.51 (m, 1H), 1.51 - 1.65 (m, 5H), 1.65 - 1.93 (m, 10H), 1.95 - 2.12 (m, 2H), 2.16 - 2.34 (m, 4H), 2.42 (br d, J = 6.8 Hz, 1H), 2.48 (t, J = 6.1 Hz, 2H), 2.56 (br s, 1H), 2.67 (s, 3H), 2.68 - 2.77 (m, 1H), 2.81 - 2.90 (m, 1H), 3.07 (ddd, J = 9.4, 7.9, 6.6 Hz, 3H), 3.13 (br s, 2H), 3.34 - 3.44 (m, 3H), 3.45 - 3.49 (m, 3H), 3.49 - 3.54 (m, 3H), 3.54 - 3.65 (m, 11H), 3.73 (br t, |
| | | | J = 6.1 Hz, 3H), 3.93 (br s, 1H), 3.98 - 4.07 (m, 3H), 4.29 (br d, J = 13.2 Hz, 1H), 4.84 (d, J = 6.8 Hz, 1H), 7.55 (dd, J = 7.8, 4.9 Hz, 1H), 7.82 (dt, J = 7.8, 2.0 Hz, 2H), 7.88 (br d, J = 7.3 Hz, 1H), 8.08 (br s, 1H), 8.42 (s, 3H), 8.53 (d, J = 2.0 Hz, 1H), 8.59 (dd, J = 4.9, 1.5 Hz, 1H), 8.74 (s, 1H) |

### Example 19

### (2S,3S)-N-(3-((2-(2-(3-(((1s,4s)-4-((3-((1R,3S)-3-(((3R,4R)-3-Methoxytetrahydro-2H-pyran-4-yl)amino)-1-(3-(trifluoromethyl)-5,6,7,8-tetrahydro-1,6-naphthyridine-6-carbonyl)cyclopentyl)propyl)carbamoyl)cyclohexyl)amino)-3-oxopropoxy)ethoxy)ethyl)amino)-3-oxopropyl)-1-methyl-5-oxo-2-(pyridin-3-yl)pyrrolidine-3-carboxamide

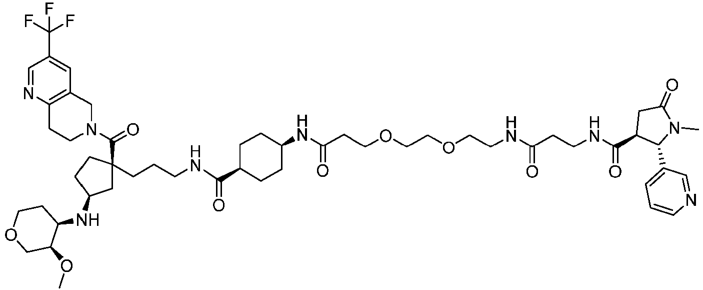

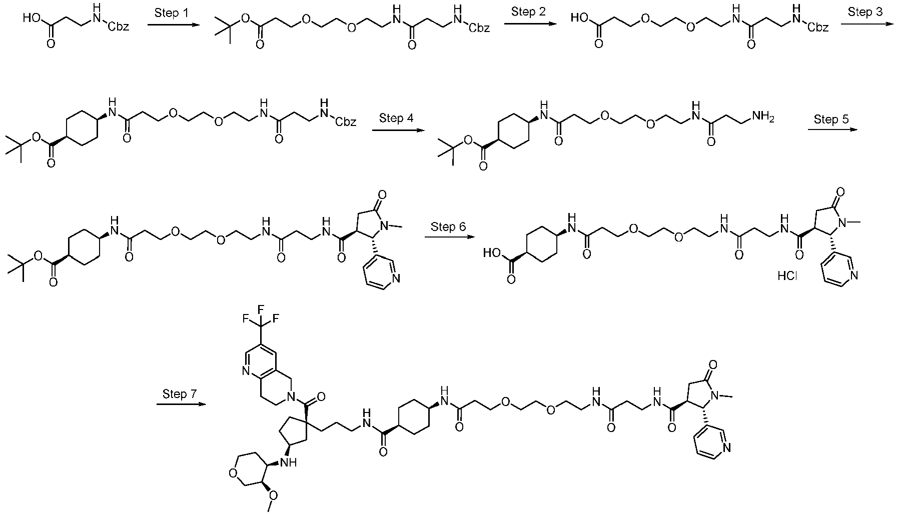

### Step 1: tert-Butyl 3,7-dioxo-1-phenyl-2,11,14-trioxa-4,8-diazaheptadecan-17-oate

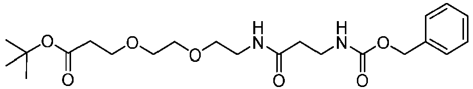

To a mixture of commercially-available tert-butyl 3-(2-(2-aminoethoxy)ethoxy)propanoate (2.5 g, 10.72 mmol) and commercially-available 3-(((benzyloxy)carbonyl)amino)propanoic acid (2.51 g, 11.25 mmol) in dichloromethane (DCM) (35.7 ml) at rt was added DIPEA (5.61 ml, 32.1 mmol) and HATU (4.28 g, 11.25 mmol) and the mixture was stirred under an atmosphere of nitrogen for 20 h. Dichloromethane (DCM) was added and the organic phase was washed with saturated NH₄Cl, with saturated NaHCO₃, and with brine. The organic phase was dried over Na₂SO₄, was filtered, and was concentrated under reduced pressure. Purification by ISCO CombiFlash^{®} Rf chromatography (80g silica column, 60 mL/min) eluting with a gradient of 50 % (5 column volumes), 50 to 90 % (25 column volumes), and 90 % (5 column volumes) ethyl acetate in heptane provided the title compound as a colorless oil (4.69 g, 10.70 mmol, 100 % yield). LCMS (m/z) 383.4 (M+H)⁺.

### Step 2: 3,7-Dioxo-1-phenyl-2,11,14-trioxa-4,8-diazaheptadecan-17-oic acid

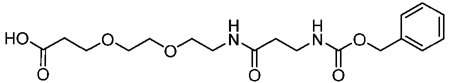

To a suspension of tert-butyl 3,7-dioxo-1-phenyl-2,11,14-trioxa-4,8-diazaheptadecan-17-oate (4.65 g, 10.60 mmol) in 1,4-dioxane (35.3 mL) was added 4 M HCl in 1,4-dioxane (12.60 mL, 50.4 mmol) and the mixture was stirred at rt for 20 h. 4 M HCl in 1,4-dioxane (1mL) was added twice at 2 h intervals and the mixture was concentrated under reduced pressure to provide the title compound as an off-white solid (5.69 g, 13.39 mmol, 126 % yield). LCMS (m/z) 383.4 (M+H)⁺.

### Step 3: tert-Butyl (1s,4s)-4-(3,7-dioxo-1-phenyl-2,11,14-trioxa-4,8-diazaheptadecan-17-amido)cyclohexane-1-carboxylate

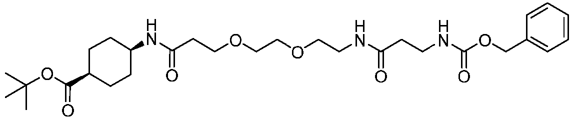

To a suspension of 3,7-dioxo-1-phenyl-2,11,14-trioxa-4,8-diazaheptadecan-17-oic acid (4.05 g, 10.60 mmol) and commercially-available tert-butyl (1s,4s)-4-aminocyclohexane-1-carboxylate, hydrochloric acid salt (2.499 g, 10.60 mmol) in dichloromethane (DCM) (35.3 mL) was added DIPEA (7.59 mL, 43.5 mmol) and HATU (4.23 g, 11.13 mmol) and the mixture was stirred under nitrogen at rt for 20 h. Dichloromethane (DCM) was added and the organic phase was washed with saturated NH₄Cl, with saturated NaHCO₃, and with brine. The organic phase was dried over NaSO₄, was filtered, and was concentrated under reduced pressure. Purification by ISCO Combi Flash^{®} Rf (80 g silica, 60 mL/min) eluting with a stepwise gradient of 0 % (5 column volumes), 0 to 25 % (25 column volumes), and 25 % (5 column volumes) methanol in dichloromethane (DCM) provided the desired product as a clear colorless oil (5.06 g, 8.08 mmol, 76 % yield). LCMS (m/z) 564.5 (M+H)⁺.

### Step 4: tert-Butyl (1s,4s)-4-(3-(2-(2-(3-aminopropanamido)ethoxy)ethoxy)propanamido)cyclohexane-1-carboxylate

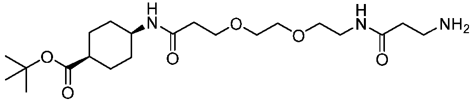

A suspension of tert-butyl (1*s*,4*s*)-4-(2,6-dioxo-1-phenoxy-10,13-dioxa-3,7-diazahexadecan-16-amido)cyclohexane-1-carboxylate (5.0 g, 8.87 mmol) and 10 % Pd-C (0.944 g, 0.887 mmol) in ethyl acetate (29.6 mL) was stirred under a balloon atmosphere of hydrogen at rt for 20 h. 10 % Pd-C (300 mg) was added and the mixture was stirred under a balloon atmosphere of hydrogen for 1 h. The mixture was filtered through Celite^{®} and the filtrate was concentrated under reduced pressure to provide the title compound as a brown oil (3.43 g, 7.19 mmol, 81 % yield). LCMS (m/z) 430.5 (M+H)⁺.

### Step 5: tert-Butyl (1s,4s)-4-(1-((2S,3S)-1-methyl-5-oxo-2-(pyridin-3-yl)pyrrolidin-3-yl)-1,5-dioxo-9,12-dioxa-2,6-diazapentadecan-15-amido)cyclohexane-1-carboxylate

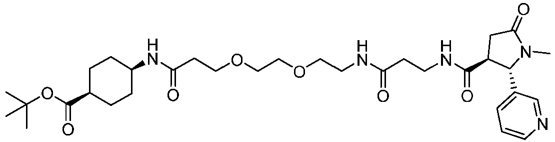

To a suspension of (2S,3S)-1-methyl-5-oxo-2-(pyridin-3-yl)pyrrolidine-3-carboxylic acid (0.872 g, 3.96 mmol) and tert-butyl (1*s*,4*s*)-4-(3-(2-(2-(3-aminopropanamido)ethoxy)ethoxy)propanamido)cyclohexane-1-carboxylate (1.7 g, 3.96 mmol) in dichloromethane (DCM) (13.19 mL) was added DIPEA (2.76 mL, 15.83 mmol) and HATU (1.580 g, 4.16 mmol), and the mixture was stirred at rt for 20 h. Dichloromethane (DCM) was added and the organic phase was washed with saturated NH₄Cl, with saturated NaHCO₃, and with brine. The organic phase was dried over NaSO₄, was filtered, and was concentrated under reduced pressure. Purification by ISCO Combi Flash^{®} Rf (80 g silica, 60 mL/min) eluting with a stepwise gradient of 0 % (5 column volumes), 0 to 25 % (25 column volumes), and 25 % (5 column volumes) methanol in dichloromethane (DCM) provided the title compound as a clear colorless oil (1.74 g, 2.479 mmol, 62.6 % yield). LCMS (m/z) 632.5 (M+H)⁺.

### Step 6: (1s,4s)-4-(1-((2S,3S)-1-Methyl-5-oxo-2-(pyridin-3-yl)pyrrolidin-3-yl)-1,5-dioxo-9,12-dioxa-2,6-diazapentadecan-15-amido)cyclohexane-1-carboxylic acid, Hydrochloric acid salt

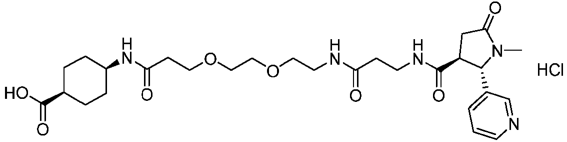

To a suspension of tert-butyl (1*s*,4*s*)-4-(1-((2*S*,3*S*)-1-methyl-5-oxo-2-(pyridin-3-yl)pyrrolidin-3-yl)-1,5-dioxo-9,12-dioxa-2,6-diazapentadecan-15-amido)cyclohexane-1-carboxylate (1.7 g, 2.69 mmol) in 1,4-dioxane (8.97 mL) was added 4 M HCl in 1,4-dioxane (2.018 mL, 8.07 mmol). Acetonitrile (3 mL) was added and the mixture was stirred under an atmosphere of nitrogen at rt for 20 h. 4 M HCl in 1,4-dioxane (3.03 mL, 12.1 mmol) was added and the mixture was stirred for 4 h. 4 M HCl in 1,4-dioxane (6.05 mL, 24.2 mmol) was added, the mixture was stirred at rt for 20 h. The mixture was concentrated under reduced pressure to provide the title compound a sticky, off-white solid (1.88 g, 3.07 mmol, 114 % yield). LCMS (m/z) 576.5 (M+H)⁺.

### Step 7: (2S,3S)-N-(3-((2-(2-(3-(((1S,4s)-4-((3-((1R,3S)-3-(((3R,4R)-3-Methoxytetrahydro-2H-pyran-4-yl)amino)-1-(3-(trifluoromethyl)-5,6,7,8-tetrahydro-1,6-naphthyridine-6-carbonyl)cyclopentyl)propyl)carbamoyl)cyclohexyl)amino)-3-oxopropoxy)ethoxy)ethyl)amino)-3-oxopropyl)-1-methyl-5-oxo-2-(pyridin-3-yl)pyrrolidine-3-carboxamide

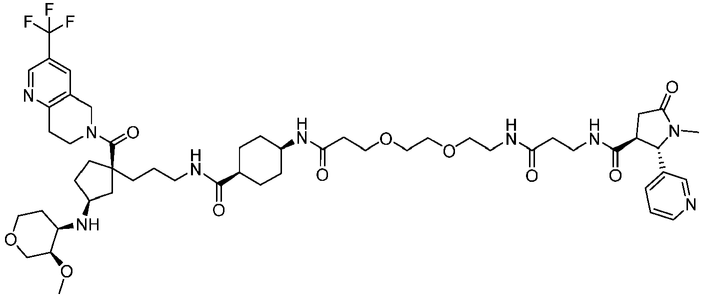

To a solution of ((1*S*,3*R*)-1-(3-aminopropyl)-3-(((3*S*,4*S*)-3-methoxytetrahydro-2H-pyran-4-yl)amino)cyclopentyl)(3-(trifluoromethyl)-7,8-dihydro-1,6-naphthyridin-6(5H)-yl)methanone, 3hydrochloric acid salt (59.4 mg, 0.100 mmol) and (1*s*,4*s*)-4-(1-((2S,3S)-1-methyl-5-oxo-2-(pyridin-3-yl)pyrrolidin-3-yl)-1,5-dioxo-9,12-dioxa-2,6-diazapentadecan-15-amido)cyclohexane-1-carboxylic acid, hydrochloric acid salt (61.2 mg, 0.100 mmol) in dichloromethane (DCM) (2000 µL) was added HATU (57.0 mg, 0.150 mmol) and DIPEA (175 µL, 1.000 mmol) and the mixture was stirred at rt for 1.5 h. Methanol was added and the mixture was concentrated under a steady stream of nitrogen. Purification by MDAP (XSelect^{™} CSH C18 column, 40 mL/min) eluting with a gradient of 15 to 55 % acetonitrile in water containing ammonium bicarbonate (10 mM) and adjusted to pH 10 with ammonia provided the title compound as a white solid (42.3 mg, 0.039 mmol, 38.6 % yield). LCMS (m/z) 1042.7 (M+H)⁺. ¹H NMR (400 MHz, METHANOL-*d*₄) δ ppm 1.29 - 1.48 (m, 3H), 1.53 - 1.84 (m, 12H), 1.93 - 2.09 (m, 2H), 2.14 - 2.29 (m, 2H), 2.40 (td, J = 6.7, 1.7 Hz, 2H), 2.48 (t, J = 6.1 Hz, 2H), 2.53 - 2.64 (m, 1H), 2.64 - 2.74 (m, 4H), 2.79 - 2.91 (m, 2H), 2.97 - 3.19 (m, 5H), 3.34 - 3.48 (m, 11H), 3.52 (q, J = 5.1 Hz, 3H), 3.61 (s, 4H), 3.75 (t, J = 6.1 Hz, 3H), 3.88 - 4.10 (m, 5H), 4.16 (br d, J = 13.7 Hz, 2H), 4.84 (d, J = 6.4 Hz, 1H), 7.54 (dd, J = 7.6, 5.1 Hz, 1H), 7.80 (dt, J = 7.8, 2.0 Hz, 2H), 8.06 (br s, 1H), 8.52 (d, J = 2.0 Hz, 1H), 8.58 (dd, J = 4.9, 2.0 Hz, 1H), 8.72 (s, 1H)

### Example 20

### ((2S,3S)-N-(2-(((1r,4S)-4-(2-(2-(3-((3-((1S,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)propyl)amino)-3-oxopropoxy)ethoxy)ethoxy)cyclohexyl)oxy)ethyl)-1-methyl-5-oxo-2-(pyridin-3-yl)pyrrolidine-3-carboxamide, Formic acid salt

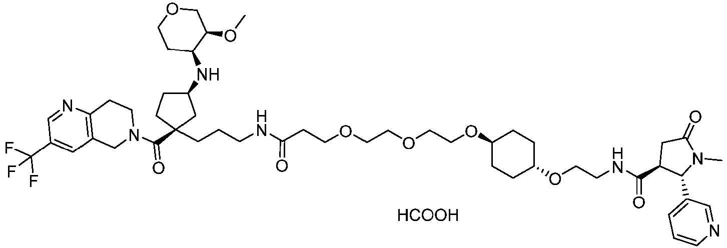

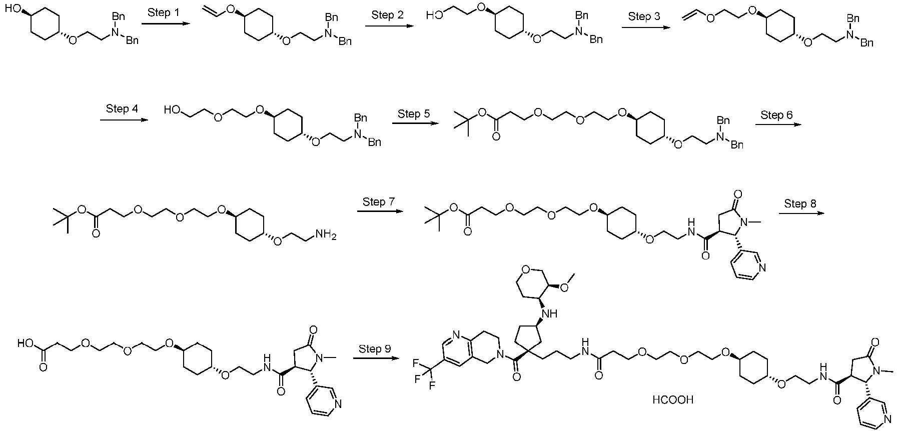

### Step 1: N,N-Dibenzyl-2-(((1r,4r)-4-(vinyloxy)cyclohexyl)oxy)ethan-1-amine.

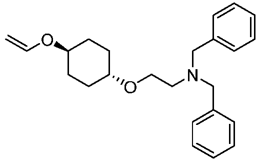

To a mixture of palladium(II) trifluoroacetate (0.154 g, 0.464 mmol) and bathophenanthroline (0.154 g, 0.464 mmol) in ethyl vinyl ether (27.5 mL, 278 mmol) was added DIPEA (1.621 mL, 9.28 mmol) and (1*r*,4*r*)-4-(2-(dibenzylamino)ethoxy)cyclohexan-1-ol (3.15 g, 9.28 mmol) and the mixture was apportioned into two vials. The vials were sealed and were stirred at 75 °C for 3.5 h. Purification by ISCO CombiFlash^{®} chromatography (80 g silica Gold column, 60 mL/min) eluting with a gradient of 0 to 20 % ethyl acetate in heptane provided the title compound (2.3956 g, 6.55 mmol, 70.6 % yield). LCMS (m/z) 366.4 (M+H)⁺.

### Step 2: 2-(((1r,4r)-4-(2-(Dibenzylamino)ethoxy)cyclohexyl)oxy)ethan-1-ol.

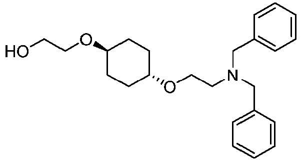

To a solution of N,N-dibenzyl-2-(((1*r*,4*r*)-4-(vinyloxy)cyclohexyl)oxy)ethan-1-amine (2.35 g, 6.43 mmol) in tetrahydrofuran (THF) (50 mL) was added 1 M borane in tetrahydrofuran (THF) (9.64 mL, 9.64 mmol) and the mixture was stirred at rt for 40 min. 5 N sodium hydroxide (12.86 mL, 64.3 mmol) and H₂O₂ (30%) (13.13 mL, 129 mmol) were added and the mixture was stirred at rt for 23 h. The mixture was extracted with ethyl acetate (3 x 30 mL), and the combined organic extracts were washed with brine (30 mL), were dried over MgSO₄, were filtered, and the filtrate was concentrated under reduced pressure. Purification by ISCO CombiFlash^{®} chromatography (40 g silica Gold column, 40 mL/min) eluting with a gradient of 0 to 70 % ethyl acetate in heptane provided the title compound (2.1723 g, 4.86 mmol, 76 % yield). LCMS (m/z) 384.4 (M+H)⁺.

### Step 3: N,N-Dibenzyl-2-(((1r,4r)-4-(2-(vinyloxy)ethoxy)cyclohexyl)oxy)ethan-1-amine.

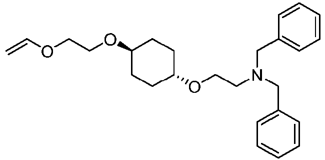

To a mixture of palladium(II) trifluoroacetate (0.081 g, 0.243 mmol) and bathophenanthroline (0.081 g, 0.243 mmol) in ethyl vinyl ether (14.39 mL, 146 mmol) was added DIPEA (0.848 mL, 4.85 mmol) and N,N-dibenzyl-2-(((1*r*,4*r*)-4-(2-(vinyloxy)ethoxy)cyclohexyl)oxy)ethan-1-amine (2.17 g, 4.85 mmol) and the mixture was stirred at 75 °C for 4 h in a sealed vial. The mixture was cooled down to rt, was filtered, and was concentrated under reduced pressure. Purification by ISCO CombiFlash^{®} chromatography (40 g silica Gold column, 40 mL/min) eluting with a gradient of 0 to 30 % ethyl acetate in heptane provided the title compound (1.9085 g, 3.79 mmol, 78 % yield). LCMS (m/z) 410.1 (M+H)⁺.

### Step 4: 2-(2-(((1r,4r)-4-(2-(Dibenzylamino)ethoxy)cyclohexyl)oxy)ethoxy)ethan-1-ol.

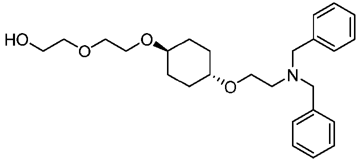

To a solution of N,N-dibenzyl-2-(((1*r*,4*r*)-4-(2-(vinyloxy)ethoxy)cyclohexyl)oxy)ethan-1-amine amine (1.9 g, 3.78 mmol) in tetrahydrofuran (THF) (40 mL) was added borane (1 M in THF) (5.66 mL, 5.66 mmol) and the mixture was stirred at rt for 30 min. 5 N sodium hydroxide (7.55 mL, 37.8 mmol) and H₂O₂ (30%) (7.71 mL, 76 mmol) were added and the mixture was stirred at rt for 19 h. The mixture was extracted with ethyl acetate (3 x 30 mL), and the combined organic extracts were washed with brine (30 mL), were dried over MgSO₄, were filtered, and the filtrate was concentrated under reduced pressure. Purification by ISCO CombiFlash^{®} chromatography (40 g silica Gold column, 40 mL/min) eluting with a gradient of 0 to 80 % ethyl acetate in heptane provided the title compound (0.9820 g, 2.297 mmol, 60.8 % yield). LCMS (m/z) 428.4 (M+H)⁺.

### Step 5: tert-Butyl 3-(2-(2-(((1r,4r)-4-(2-(dibenzylamino)ethoxy)cyclohexyl)oxy)ethoxy)ethoxy)propanoate.

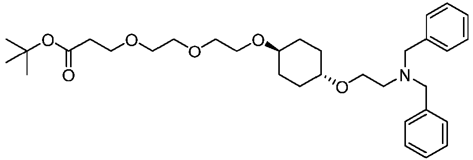

To 2-(2-(((1*r*,4*r*)-4-(2-(Dibenzylamino)ethoxy)cyclohexyl)oxy)ethoxy)ethan-1-ol (970 mg, 2.269 mmol) in dichloromethane (DCM) (16 mL) was added tert-butyl acrylate (0.665 mL, 4.54 mmol), tetrabutylammonium hydrogen sulfate (77 mg, 0.227 mmol), and 5 N sodium hydroxide (0.181 mL, 0.907 mmol). The mixture was stirred at rt for 21 h and was concentrated under reduced pressure. Dichloromethane (DCM) (3 mL) and water (0.181 mL) were added, the mixture was stirred at rt for 23 h, and was concentrated under reduced pressure. Purification by ISCO CombiFlash^{®} chromatography (24 g silica Gold column, 35 mL/min) eluting with a stepwise gradient of 0 to 35 % (15 min) and 35 to 80 % (10 min) ethyl acetate in heptane provided the title compound (0.8950 g, 1.610 mmol, 71.0 % yield). LCMS (m/z) 556.6 (M+H)⁺.

### Step 6: tert-Butyl 3-(2-(2-(((1r,4r)-4-(2-aminoethoxy)cyclohexyl)oxy)ethoxy)ethoxy)propanoate.

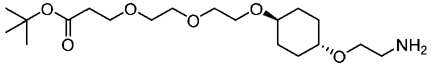

A mixture of tert-butyl 3-(2-(2-(((1*r*,4*r*)-4-(2-(dibenzylamino)ethoxy)cyclohexyl)oxy)ethoxy)ethoxy)propanoate (850 mg, 1.529 mmol) and 10 % Pd/C (163 mg, 0.153 mmol) in methanol (30 mL) was stirred at rt under a hydrogen atmosphere for 4 h. The mixture was filtered through Celite^{®} and was concentrated under reduced pressure to provide the title compound (0.5599 g, 1.491 mmol, 97 % yield). LCMS (m/z) 376.4 (M+H)⁺.

### Step 7: tert-butyl 3-(2-(2-(((1S,4r)-4-(2-((2S,3S)-1-Methyl-5-oxo-2-(pyridin-3-yl)pyrrolidine-3-carboxamido)ethoxy)cyclohexyl)oxy)ethoxy)ethoxy)propanoate

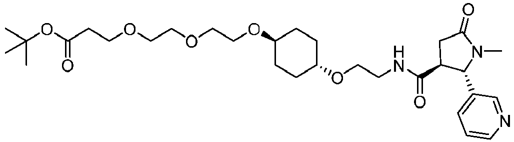

A mixture of tert-butyl 3-(2-(2-(((1*r*,4*r*)-4-(2-aminoethoxy)cyclohexyl)oxy)ethoxy)ethoxy)propanoate (550 mg, 1.465 mmol), (2*S*,3*S*)-1-methyl-5-oxo-2-(pyridin-3-yl)pyrrolidine-3-carboxylic acid (323 mg, 1.465 mmol), DIPEA (0.767 mL, 4.39 mmol) and T3P (50% wt in ethyl acetate) (1.308 mL, 2.197 mmol) was stirred at rt for 93 h. Saturated aqueous NaHCO₃ (5 drops) was added and the mixture was concentrated under reduced pressure. Purification by ISCO CombiFlash^{®} chromatography (24 g silica Gold column, 35 mL/min) eluting with a gradient of 0 to 55 % ethyl acetate in heptane provided the title compound (0.9152 g, 1.381 mmol, 94 % yield). LCMS (m/z) 578.5 (M+H)⁺.

### Step 8: 3-(2-(2-(((1S,4r)-4-(2-((2S,3S)-1-Methyl-5-oxo-2-(pyridin-3-yl)pyrrolidine-3-carboxamido)ethoxy)cyclohexyl)oxy)ethoxy)ethoxy)propanoic acid, hydrochloride

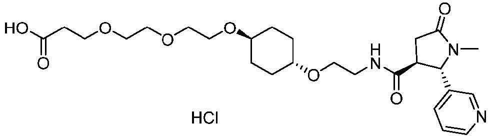

To a solution of tert-butyl 3-(2-(2-(((1S,4r)-4-(2-((2S,3S)-1-Methyl-5-oxo-2-(pyridin-3-yl)pyrrolidine-3-carboxamido)ethoxy)cyclohexyl)oxy)ethoxy)ethoxy)propanoate (0.910 g, 1.374 mmol) in 1,4-Dioxane (2 mL) was added 4 M HCl in 1,4-dioxane (1.717 mL, 6.87 mmol) and the mixture was stirred at rt for 18 h. The mixture was concentrated under reduced pressure to provide the title compound (0.8810 g, 1.085 mmol, 79 % yield). LCMS (m/z) 522.4 (M+H)⁺.

### Step 9: ((2S,3S)-N-(2-(((1r,4S)-4-(2-(2-(3-((3-((1S,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)propyl)amino)-3-oxopropoxy)ethoxy)ethoxy)cyclohexyl)oxy)ethyl)-1-methyl-5-oxo-2-(pyridin-3-yl)pyrrolidine-3-carboxamide, Formic acid salt

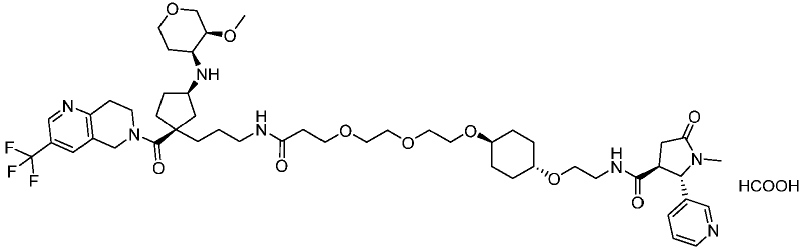

To a solution of tert-butyl (3-((1S,3R)-3-(((3S,4S)-3-methoxytetrahydro-2H-pyran-4-yl)amino)-1-(3-(trifluoromethyl)-5,6,7,8-tetrahydro-1,6-naphthyridine-6-carbonyl)cyclopentyl)propyl)carbamate (55 mg, 0.094 mmol) in 1,4-Dioxane (0.2 mL) was added HCl (4 M in p-dioxane) (0.235 mL, 0.941 mmol). The result reaction mixture was heated at ambient temperature for 20 hrs. The reaction mixture was evaporated down on biotage V10 evaporator under vacuum, dissolved in Dichloromethane (DCM) (0.200 mL) then added HCl (4 M in p-dioxane) (0.235 mL, 0.941 mmol). The result reaction mixture was stirred at ambient temperature for 2 hrs. The reaction mixture was evaporated down on biotage V10 evaporator under vacuum then was added Dichloromethane (DCM) (2 mL), 3-(2-(2-((trans-4-(2-((2S,3S)-1-methyl-5-oxo-2-(pyridin-3-yl)pyrrolidine-3-carboxamido)ethoxy)cyclohexyl)oxy)ethoxy)ethoxy)propanoic acid (76 mg, 0.094 mmol), DIEA (0.099 mL, 0.564 mmol) and HATU (53.7 mg, 0.141 mmol). The result reaction mixture was stirred at ambient temperature for 91 hrs. The reaction was quenched with water (~3 drops), evaporated down on biotage V10 evaporator under vacuum, purified with reverse phase HPLC (HpH) to give the title compound (50.5 mg, 0.049 mmol, 51.9 % yield). 1H NMR (400 MHz, METHANOL-d4) δ ppm 1.13 - 1.44 (m, 7 H), 1.46 - 1.84 (m, 5 H), 1.84 - 2.00 (m, 6 H), 2.16 - 2.24 (m, 2 H), 2.37 (br t, J=6.11 Hz, 2 H), 2.47 - 2.60 (m, 1 H), 2.65 (s, 3 H), 2.78 - 2.85 (m, 1 H), 3.01 - 3.15 (m, 5 H), 3.21 - 3.30 (m, 5 H), 3.32 - 3.41 (m, 5 H), 3.41 - 3.56 (m, 8 H), 3.58 (s, 6 H), 3.69 (t, J=6.11 Hz, 2 H), 3.93 - 4.03 (m, 3 H), 4.24 (br d, J=12.72 Hz, 1 H), 4.82 (d, J=6.85 Hz, 1 H), 7.52 (dd, J=7.83, 4.89 Hz, 1 H), 7.79 (dt, J=8.31, 1.96 Hz, 1 H), 8.06 (br s, 1 H), 8.47 - 8.59 (m, 3 H), 8.72 (s, 1 H); 19F NMR (400 MHz, METHANOL-d4) δ ppm - 63.69; LC/MS (TFA, M+H)+ 989.5 m/z, retention time 0.56 min.

The following compound was prepared with procedures analogous to that described in **Example 20:**

| **Ex.** | **Compound** | **LC-MS m/z** | **NMR** |
|---|---|---|---|
| 21 | 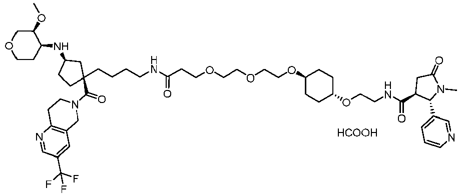 (2S,3S)-N-(2-(((1r,4S)-4-(2-(2-(3-((4-((1S,3R)-3-(((3S,4S)-3-Methoxytetrahydro-2H-pyran-4-yl)amino)-1-(3-(trifluoromethyl)-5,6,7,8-tetrahydro-1,6-naphthyridine-6-carbonyl)cyclopentyl)butyl)amino)-3-oxopropoxy)ethoxy)ethoxy)cyclohexyl)oxy)ethyl)-1-methyl-5-oxo-2-(pyridin-3-yl)pyrrolidine-3-carboxamide, Formic acid salt | 1003.7 (M+H)⁺ | ¹H NMR (400 MHz, METHANOL-*d*₄) δ ppm 1.06 - 1.47 (m, 9 H), 1.52 - 2.00 (m, 11 H), 2.16 - 2.24 (m, 2 H), 2.28 - 2.40 (m, 3 H), 2.43 - 2.59 (m, 1 H), 2.64 (s, 3 H), 2.77 - 2.87 (m, 1 H), 3.00 - 3.16 (m, 5 H), 3.22 - 3.29 (m, 3 H), 3.31 - 3.42 (m, 5 H), 3.42 - 3.54 (m, 8 H), 3.58 (s, 6 H), 3.67 (br t, J=6.36 Hz, 3 H), 3.93 - 4.03 (m, 3 H), 4.24 (br d, J=13.20 Hz, 1 H), 4.81 (d, J=6.36 Hz, 1 H), 7.52 (dd, J=8.31, 4.89 Hz, 1 H), 7.78 (dt, J=8.31, 1.96 Hz, 1 H), 8.06 (br s, 1 H), 8.47 - 8.54 (m, 2 H), 8.56 (dd, J=4.89, 1.47 Hz, 1 H), 8.71 (s, 1 H) |

### Example 22:

### (2S,3S)-N-(1-(3-(4-(6-((1S,3R)-1-Isopropyl-3-(((3S,4S)-3-methoxytetrahydro-2H-pyran-4-yl)amino)cyclopentane-1-carbonyl)-3-(trifluoromethyl)-5,6,7,8-tetrahydro-1,6-naphthyridin-2-yl)butoxy)phenyl)-1-oxo-5,8,11,14,17,20,23,26,29-nonaoxa-2-azahentriacontan-31-yl)-1-methyl-5-oxo-2-(pyridin-3-yl)pyrrolidine-3-carboxamide.

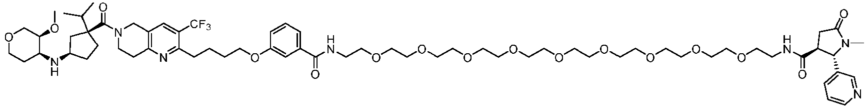

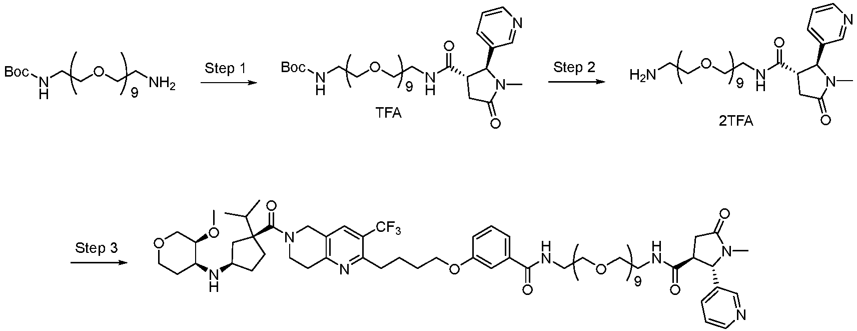

### Step 1: t-Butyl (1-((2S, 3S)-1-methyl-5-oxo-2-(pyridin-3-yl) pyrrolidin-3-yl)-1-oxo-5, 8, 11, 14, 17, 20, 23, 26, 29-nonaoxa-2-aza hentriacontan-31-yl) carbamate, Trifluoroacetic acid salt.

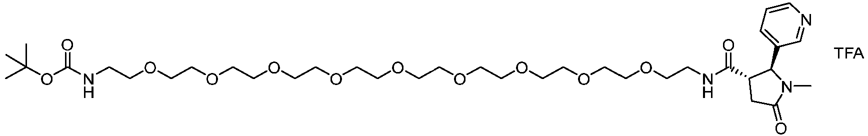

To a solution of (2S, 3S)-1-methyl-5-oxo-2-(pyridin-3-yl)pyrrolidine-3-carboxylic acid (708 mg, 3.21 mmol) and HATU (1345 mg, 3.54 mmol) in N,N-dimethylformamide (DMF) under an atmosphere of nitrogen was added dropwise DIPEA (1.404 mL, 8.04 mmol) and the mixture was stirred for 15 min. t-Butyl (29-amino-3,6,9,12,15,18,21,24,27-nonaoxanonacosyl) carbamate (1969 mg, 3.54 mmol) in N,N-dimethylformamide (DMF) was added and the mixture and stirred at rt for 1.5 h. Purification by ISCO Combiflash^{®} Rf chromatography (C18 100 g column, 60 mL/min) eluting with a gradient of 10 to 100 % acetonitrile in water both containing trifluoracetic acid (0.1 %) provided the title compound (2.8 g, 3.21 mmol, 100 % yield). LCMS (m/z) 759.6 (M+H)⁺.

### Step 2: (2S, 3S)-N-(29-amino-3, 6, 9, 12, 15, 18, 21, 24, 27-nonaoxanonacosyl)-1-methyl-5-oxo-2-(pyridin-3-yl) pyrrolidine-3-carboxamide, 2Trifluoroacetic acid salt.

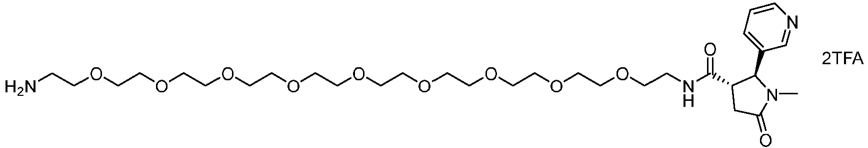

A solution of tert-butyl (1-((2S, 3S)-1-methyl-5-oxo-2-(pyridin-3-yl)pyrrolidin-3-yl)-1-oxo-5,8,11,14,17,20,23,26,29-nonaoxa-2-azahentriacontan-31-yl) carbamate (2.8 g, 3.21 mmol) in dichloromethane (8 mL) and trifluoracetic acid (8.53 mL, 111 mmol) was stirred at 1.5 h and was concentrated under reduced pressure. Purification by ISCO Combiflash^{®} Rf chromatography (C18 100 g column, 60 mL/min) eluting with a gradient of 10 to 100 % acetonitrile in water both containing trifluoracetic acid (0.1 %) provided the title compound (1.32 g, 1.49 mmol, 46.4 % yield). LCMS (m/z) 659.5 (M+H)⁺.

### Step 3: (2S,3S)-N-(1-(3-(4-(6-((1S,3R)-1-Isopropyl-3-(((3S,4S)-3-methoxytetrahydro-2H-pyran-4-yl)amino)cyclopentane-1-carbonyl)-3-(trifluoromethyl)-5,6,7,8-tetrahydro-1,6-naphthyridin-2-yl)butoxy)phenyl)-1-oxo-5,8,11,14,17,20,23,26,29-nonaoxa-2-azahentriacontan-31-yl)-1-methyl-5-oxo-2-(pyridin-3-yl)pyrrolidine-3-carboxamide.

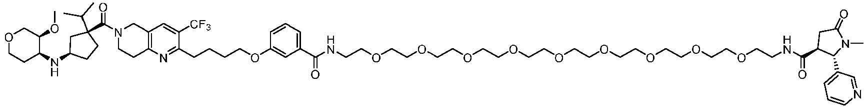

3-(4-(6-((1S,3R)-1-isopropyl-3-(((3S,4S)-3-methoxytetrahydro-2H-pyran-4-yl)amino)cyclopentane-1-carbonyl)-3-(trifluoromethyl)-5,6,7,8-tetrahydro-1,6-naphthyridin-2-yl)butoxy)benzoic acid, 2trifluoroacetic acid salt (1.1 g, 1.236 mmol) was dissolved in DMSO (20 mL) and triethylamine (0.7 ml, 5.02 mmol). A solution of TSTU (0.447 g, 1.483 mmol) in DMSO (2 mL) was added after 5 min and the mixture was stirred for 30 min. (2S,3S)-N-(29-Amino-3,6,9,12,15,18,21,24,27-nonaoxanonacosyl)-1-methyl-5-oxo-2-(pyridin-3-yl)pyrrolidine-3-carboxamide, 2trifluoroacetic acid salt (1.316 g, 1.483 mmol) dissolved in DMSO (4 mL) was added. The mixture was stirred at rt for 1.5 and was acidified using 0.5 N HCl. The mixture was purified by ISCO Combiflash^{®} Rf chromatography (C18 100 g column, 60 mL/min) eluting with a gradient of 0 to 100 % acetonitrile in water both containing trifluoracetic acid (0.1 %). The desired fractions were concentrated under reduced pressure to remove the acetonitrile and pH was adjusted to ~9 using ammonium hydroxide. Purification of the residue by ISCO Combiflash^{®} Rf chromatography (C18 100 g column, 60 mL/min) eluting with a gradient of 0 to 100 % acetonitrile in water containing ammonium hydroxide (0.1 %) to provide the title compound as a clear taffy (817 mg, 0.627 mmol, 50.7 % yield). LCMS (m/z) 652.3 (M/2+H)⁺. ¹H NMR (400 MHz, METHANOL-*d*₄) δ ppm 8.58 (dd, *J*=4.8, 1.5 Hz, 1 H) 8.53 (d, *J*=1.8 Hz, 1 H) 7.97 (br s, 1 H) 7.82 (dt, J=8.0, 1.9 Hz, 1 H) 7.51 - 7.59 (m, 1 H) 7.30 - 7.46 (m, 3 H) 7.09 (ddd, *J*=7.7, 2.6, 1.4 Hz, 1 H) 4.79 - 4.86 (m, 3 H) 4.03 - 4.17 (m, 4 H) 3.84 - 3.95 (m, 2 H) 3.49 - 3.72 (m, 37 H) 3.35 - 3.45 (m, 6 H) 3.12 - 3.20 (m, 1 H) 2.95 - 3.10 (m, 5 H) 2.79 - 2.90 (m, 2 H) 2.68 - 2.76 (m, 1 H) 2.67 (s, 3 H) 2.48 - 2.58 (m, 1 H) 2.27 - 2.41 (m, 1 H) 2.12 - 2.21 (m, 1 H) 1.85 - 1.97 (m, 5 H) 1.57 - 1.77 (m, 4 H) 1.18 - 1.32 (m, 1 H) 0.97 (br d, *J*=6.3 Hz, 3 H) 0.77 - 0.86 (m, 3 H).

### Example 23:

### (2S,3S)-N-(39-(((R)-6-((1S,3R)-1-Isopropyl-3-(((3S,4S)-3-methoxytetrahydro-2H-pyran-4-yl)amino)cyclopentane-1-carbonyl)-3-(trifluoromethyl)-5,6,7,8-tetrahydro-1,6-naphthyridin-8-yl)amino)-39-oxo-3,6,9,12,15,18,21,24,27,30,33,36-dodecaoxanonatriacontyl)-1-methyl-5-oxo-2-(pyridin-3-yl)pyrrolidine-3-carboxamide, 3Formic acid salt.

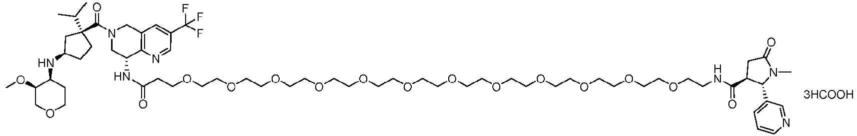

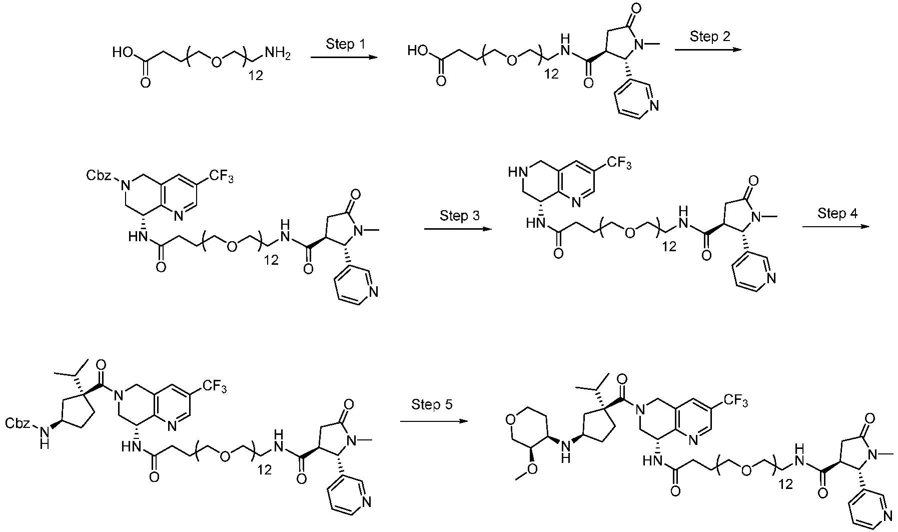

### Step 1: 1-((2S,3S)-1-Methyl-5-oxo-2-(pyridin-3-yl)pyrrolidin-3-yl)-1-oxo-5,8,11,14,17,20,23,26,29,32,35,38-dodecaoxa-2-azahentetracontan-41-oic acid

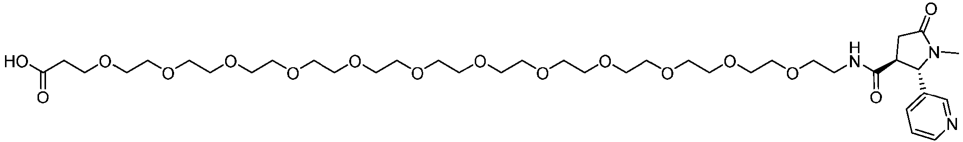

N-methylmorpholine (0.71 mL, 6.46 mmol) and 2-(2,5-dioxopyrrolidin-1-yl)-1,1,3,3-tetramethylisouronium tetrafluoroborate (0.487 g, 1.619 mmol) were added to a solution of (2S,3S)-1-methyl-5-oxo-2-(pyridin-3-yl)pyrrolidine-3-carboxylic acid (0.357 g, 1.619 mmol) in acetonitrile (10 mL). The vial was capped, and the reaction was stirred at rt for 25 min. A partial slurry of commercially available 1-amino-3,6,9,12,15,18,21,24,27,30,33,36-dodecaoxanonatriacontan-39-oic acid (1 g, 1.619 mmol) in acetonitrile (10.00 mL) and dimethyl sulfoxide (DMSO) (6 mL) was added. The reaction was stirred at rt for 1.35 h. It was concentrated to remove the acetonitrile, and water (5 mL) was added. Purification by Combiflash^{®} EZ prep chromatography (C18 50 g Gold column, 40 mL/min) eluting with a gradient of 10 to 70 % acetonitrile in water containing ammonium hydroxide (0.1 %) provided the title compound as a clear pale-yellow oil (0.9445 g, 1.21 mmol, 67.6 % yield). LC-MS *m*/*z* 820.6 (M+H)⁺.

### Step 2: Benzyl (R)-8-(1-((2S,3S)-1-methyl-5-oxo-2-(pyridin-3-yl)pyrrolidin-3-yl)-1-oxo-5,8,11,14,17,20,23,26,29,32,35,38-dodecaoxa-2-azahentetracontan-41-amido)-3-(trifluoromethyl)-7,8-dihydro-1,6-naphthyridine-6(5H)-carboxylate, 2Formic acid salt.

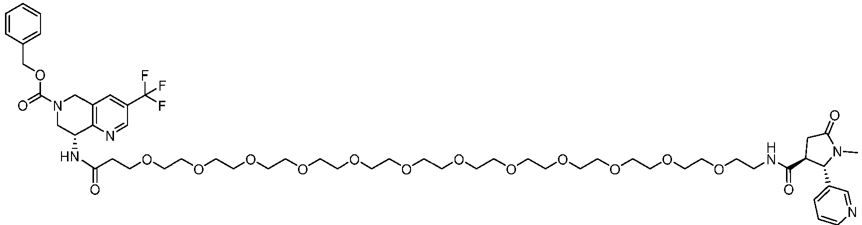

To a stirred solution of 1-((2S,3S)-1-methyl-5-oxo-2-(pyridin-3-yl)pyrrolidin-3-yl)-1-oxo-5,8,11,14,17,20,23,26,29,32,35,38-dodecaoxa-2-azahentetracontan-41-oic acid (116 mg, 0.141 mmol) in N,N-dimethylformamide (DMF) (1.0 mL) was added HATU (53.6 mg, 0.141 mmol) followed by DIPEA (0.067 mL, 0.384 mmol), and the mixture was stirred at rt for 10 min. Benzyl (R)-8-amino-3-(trifluoromethyl)-7,8-dihydro-1,6-naphthyridine-6(5H)-carboxylate (45 mg, 0.128 mmol) was added and the mixture was stirred for 45 min. Purification by reverse-phase HPLC chromatography (C18 Aq 50g Gold column, 40 mL/min) eluting with a gradient of 30 to 60 % acetonitrile in water containing formic acid (0.1 %) provided the title compound as a pale-yellow oil (104.3 mg, 0.084 mmol, 65.4 % yield). LC-MS *m*/*z* 577.3 (M/2+H)⁺.

### Step 3: (2S,3S)-1-Methyl-5-oxo-N-(39-oxo-39-(((R)-3-(trifluoromethyl)-5,6,7,8-tetrahydro-1,6-naphthyridin-8-yl)amino)-3,6,9,12,15,18,21,24,27,30,33,36-dodecaoxanonatriacontyl)-2-(pyridin-3-yl)pyrrolidine-3-carboxamide.

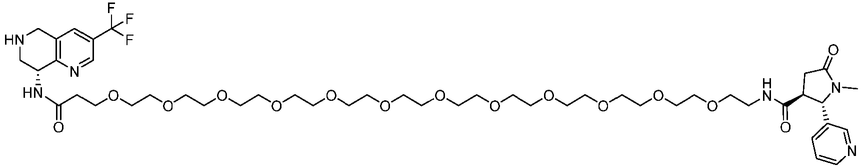

A mixture of benzyl (R)-8-(1-((2S,3S)-1-methyl-5-oxo-2-(pyridin-3-yl)pyrrolidin-3-yl)-1-oxo-5,8,11,14,17,20,23,26,29,32,35,38-dodecaoxa-2-azahentetracontan-41-amido)-3-(trifluoromethyl)-7,8-dihydro-1,6-naphthyridine-6(5H)-carboxylate, 2formic acid salt (104.3 mg, 0.084 mmol) and 20 % palladium hydroxide on carbon (58.8 mg, 0.084 mmol) in methanol (2.0 mL) was sealed in a vial and was purged with nitrogen, then hydrogen from a baloon was bubbled through the mixture for 5 min. The mixture was stirred at RT under a hydrogen baloon for 50 min. The mixture was filtered, the catalyst washed with methanol, and the combined filtrates were concentrated under reduced pressure to provide the title compound as a colorless oil (84 mg, 0.082 mmol, 98 % yield). LC-MS *m*/*z* 1019.1 (M)⁺.

### Step 4: Benzyl ((1R,3S)-3-isopropyl-3-((R)-8-(1-((2S,3S)-1-methyl-5-oxo-2-(pyridin-3-yl)pyrrolidin-3-yl)-1-oxo-5,8,11,14,17,20,23,26,29,32,35,38-dodecaoxa-2-azahentetracontan-41-amido)-3-(trifluoromethyl)-5,6,7,8-tetrahydro-1,6-naphthyridine-6-carbonyl)cyclopentyl)carbamate.

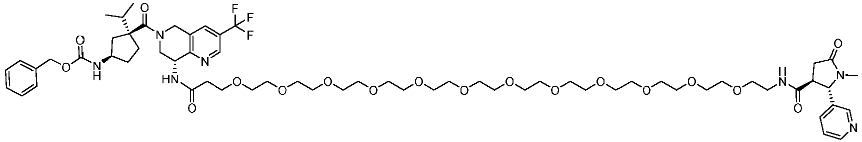

To (1*S*,3*R*)-3-(((benzyloxy)carbonyl)amino)-1-isopropylcyclopentane-1-carboxylic acid (50.3 mg, 0.165 mmol) (freshly dried by toluene azeotrope) was added dichloromethane (DCM) (1.00 mL) at room temperature. HATU (37.6 mg, 0.099 mmol) was added and the mixture was stirred for 1 min before DIPEA (0.115 mL, 0.659 mmol) was added. The mixture was stirred at rt for 18 h. A solution of (2*S*,3*S*)-1-methyl-5-oxo-N-(39-oxo-39-(((*R*)-3-(trifluoromethyl)-5,6,7,8-tetrahydro-1,6-naphthyridin-8-yl)amino)-3,6,9,12,15,18,21,24,27,30,33,36-dodecaoxanonatriacontyl)-2-(pyridin-3-yl)pyrrolidine-3-carboxamide (84 mg, 0.082 mmol) in dichloromethane (DCM) (0.50 mL) was added dropwise at rt and the mixture was stirred at 80 °C for 3 days. The organic solvent was boiled off, and the residue was stirred at 80 °C for 2 h. Another portion of the preactivated acid (prepared as above) was added and the heating continued at 80 °C for 20 h. The vial was cooled, water (0.50 mL) was added, and the heating continued at 100 °C for 2 h. The mixture was partitioned between 1.0 M NaOH and dichloromethane (DCM). The organic phase was dried over Na₂SO₄, was filtered, and was concentrated under reduced pressure. Purification by reverse-phase HPLC chromatography (XSelect^{™} CSH Prep C18 5 µm OBD column, 40 mL/min) eluting with a gradient of 30 to 85 % acetonitrile in water containing ammonium bicarbonate (10 mM) and ammonia hydroxide (0.075 %) provided the title compound as a yellow oil (37.1 mg, 0.028 mmol, 34.5 % yield). LC-MS *m*/*z* 653.8 (M/2)⁺.

### Step 5: (2S,3S)-N-(39-(((R)-6-((1S,3R)-1-Isopropyl-3-(((3S,4S)-3-methoxytetrahydro-2H-pyran-4-yl)amino)cyclopentane-1-carbonyl)-3-(trifluoromethyl)-5,6,7,8-tetrahydro-1,6-naphthyridin-8-yl)amino)-39-oxo-3,6,9,12,15,18,21,24,27,30,33,36-dodecaoxanonatriacontyl)-1-methyl-5-oxo-2-(pyridin-3-yl)pyrrolidine-3-carboxamide, 3Formic acid salt.

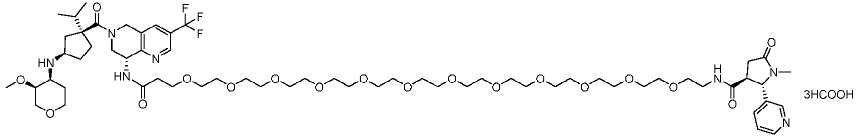

A mixture of benzyl ((1*R*,3*S*)-3-isopropyl-3-((*R*)-8-(1-((2S,3S)-1-methyl-5-oxo-2-(pyridin-3-yl)pyrrolidin-3-yl)-1-oxo-5,8,11,14,17,20,23,26,29,32,35,38-dodecaoxa-2-azahentetracontan-41-amido)-3-(trifluoromethyl)-5,6,7,8-tetrahydro-1,6-naphthyridine-6-carbonyl)cyclopentyl)carbamate (37.1 mg, 0.028 mmol) and 20 % palladium hydroxide on carbon (19.94 mg, 0.028 mmol) follwowed by methanol (1.00 mL) was sealed in a flask and was purged with nitrogen. Hydrogen was bubbled into the mixture for 2 min. The mixture was stirred at rt for 30 min under a balloon atmosphere of hydrogen. The mixture was opened to air, 7.0 M ammonia in methanol (50 mL) was added, the mixture was stirred for 15 min and was filtered. The catalyst was washed with methanol and the combined filtrates were concentrated under reduced pressure. Isopropyl acetate (1.00 mL), dichloromethane (DCM) (0.50 mL), 2-propanol (0.016 mL, 0.213 mmol), and triethylamine (0.012 mL, 0.085 mmol) were added, the mixture was placed into an ice bath for 5 min, and sodium triacetoxyborohydride (39.7 mg, 0.187 mmol) was added followed by dropwise addition of a solution of (*R*)-3-methoxytetrahydro-4H-pyran-4-one (22.17 mg, 0.170 mmol) in isopropyl acetate (1.00 mL). The mixture was stirred for 10 min at 0 °C, was warmed to rt, and was stirred for 1 h. The mixture was diluted with dichloromethane (DCM) and was washed with 1.0 M NaOH. The aqueous phase was extracted with dichloromethane (DCM), the combined organic extracts were dried over Na₂SO₄, were filtered, and the filtrate was concentrated under reduced pressure. Purification by reverse-phase HPLC chromatography (XSelect^{™} CSH Prep C18 5 µm OBD column, 40 mL/min) eluting with a gradient of 15 to 55 % acetonitrile in water containing formic acid (0.1 %) provided the title compound as a colorless foam (4.3 mg, 3.02 µmol, 10.63 % yield). LC-MS *m*/*z* 644.3 (M/2+H)⁺. ¹H NMR (400 MHz, METHANOL-*d*₄) δ ppm 0.84 - 1.06 (range, 6 H) 1.66 (br s, 1 H) 1.81 (br d, *J*=9.3 Hz, 1 H) 2.06 (s, 4 H) 2.14 - 2.29 (m, 2 H) 2.35 (br s, 2 H) 2.58 (br s, 3 H) 2.67 (s, 3 H) 2.68 - 2.75 (m, 2 H) 2.78 - 2.92 (m, 1 H) 3.00 - 3.11 (m, 1 H) 3.45 (s, 3 H) 3.50 - 3.55 (m, 3 H) 3.55 - 3.70 (m, 48 H) 3.82 (br t, *J*=6.1 Hz, 3 H) 4.01 (br dd, *J*=12, 4.7 Hz, 1 H) 4.29 (br d, *J*=14 Hz, 1 H) 4.47 (br d, *J*=14 Hz, 2 H) 5.12 (br s, 2 H) 7.55 (dd, *J*=7.9, 4.9 Hz, 1 H) 7.82 (dt, *J*=7.9, 1.9 Hz, 1 H) 8.15 (s, 1 H) 8.30 (br s, 3 H) 8.53 (br s, 1 H) 8.59 (br d, *J*=4.4 Hz, 1 H) 8.84 (s, 1 H).

The following compound was prepared with procedures analogous to that described in **Example 23:**

| **Ex.** | **Compound** | **LC-MS m/z** | **NMR** |
|---|---|---|---|
| 24 | 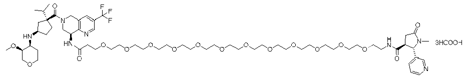 (2*S*,3*S*)-N-(39-(((*S*)-6-((1*S,*3*R*)-1-Isopropyl-3-(((3*S*,4*S*)-3-methoxytetrahydro-2H-pyran-4-yl)amino)cyclopentane-1-carbonyl)-3-(trifluoromethyl)-5,6,7,8-tetrahydro-1,6-naphthyridin-8-yl)amino)-39-oxo-3,6,9,12,15,18,21,24,27,30,33,36-dodecaoxanonatriacontyl)-1-methyl-5-oxo-2-(pyridin-3-yl)pyrrolidine-3-carboxamide, 3Formic acid salt. | 644.7 (M/2+H)⁺ | ¹H NMR (400 MHz, METHANOL-*d*₄) δ ppm 0.85 (br d, *J*=6.9 Hz, 3 H) 1.00 (d, *J*=6.9 Hz, 3 H) 1.31 (s, 1 H) 1.66 (br s, 3 H) 1.91 - 2.02 (m, 1 H) 2.19 - 2.30 (m, 1 H) 2.34 - 2.47 (m, 1 H) 2.57 (br s, 3 H) 2.67 (s, 3 H) 2.69 - 2.76 (m, 1 H) 2.82 - 2.89 (m, 1 H) 3.03 - 3.09 (m, 1 H) 3.36 - 3.45 (m, 8 H) 3.50 - 3.55 (m, 4 H) 3.57 - 3.61 (m, 4 H) 3.61 - 3.68 (m, 44 H) 3.81 (br s, 2 H) 3.93 (br s, 1 H) 4.18 (br d, *J*=14 Hz, 1 H) 4.83 (d, *J*=6.4 Hz, 1 H) 5.07 - 5.26 (m, 2 H) 7.55 (dd, *J*=7.8, 4.9 Hz, 1 H) 7.82 (dt, J=8.3, 2.0 Hz, 1 H) 8.16 (br s, 1 H) 8.53 (d, *J*=2.0 Hz, 1 H) 8.57 (br s, 1 H) 8.59 (dd, *J*=4.9, 1.5 Hz, 1 H) 8.83 (s, 1 H). |

### BIOLOGICAL ASSAYS

Example Compounds 1-24 which are compounds of Formula (I) having a CCR2 binding moiety were tested in various biological assays as described in more detail below.

### EXAMPLE 25: Antibody Dependent Cellular Cytotoxicity (ADCC) Reporter Assay

An antibody dependent cellular cytoxocity reporter assay was conducted using the following four assay components: (i) ARM compound of Formula (I) targeting CCR2 (concentrations ranging from 1 pM to 10 µM) (ii) anti-cotinine antibody having a heavy chain sequence of SEQ ID NO: 11 and a light chain sequence of SEQ ID NO: 12 (rabbit variable region with human IgG1 Fc domain containing a DE mutation (S239D/I332E)) (concentrations ranging from 0.01 µg / mL to 200 µg / mL); (iii) target cells: CHOK1 cells engineered to overexpress either human CCR2 (typically 1000-20,000 cells per well) and (iv) reporter cells: Jurkat cells engineered to express FcyRllla with the reporter gene luciferase under the control of the NFAT promoter (typically 3000-75,000 cells per well). Reagents were combined in a final volume of 20 µL in a 384 - well tissue culture treated plate. All four assay components were incubated together for about 12-18 hours. Thereafter, BioGlo Detection reagent (Promega) was added to the wells to lyse the cells and provide a substrate for the luciferase reporter protein. Luminescence signal was measured on a microplate reader and signal:background was calculated by dividing the signal of a test well by the signal obtained when no heterobivalent compound of Formula (I) was added. EC50 calculations were done using Graphpad Prism Software, specifically a nonlinear regression curve fit ( Y = Bottom + ( Top - Bottom ) / ( 1 + 10 ^ ( ( Log EC50 - X ) * HillSlope ) ) ).

ARMs compounds of Formula (I) of the invention were tested for ADCC activity in the above assay in or more experimental runs and the results are shown in Table 3 below. Potency of the compounds of Formula (I) of the invention is reported as a pEC50 value. The pEC50 value is the negative log of the EC50 value, wherein the EC50 value is the half maximal effective concentration measured in molar (M). For compounds tested in more than one experimental run, the pEC50 value is reported as an average.

### EXAMPLE 26: PBMC Ex Vivo Depletion Assay

A human PBMC (peripheral blood mononuclear cells) monocyte depletion flow cytometry assay was developed to screen compounds in a 384-well plate format. This assay incorporated endogenous natural killer cells to deplete endogenous target monocytes.

To screen compounds in the human PBMC monocyte depletion flow cytometry assay, 11-point dose-response curves were made from 1 mM stocks in neat DMSO by serially diluting an ARM compound of Formula (I) four-fold into a 384-well plate row-wise. These serially diluted compounds were then stamped into a flat bottom polypropylene 384-well plate (Greiner Bio-One) at 100 nL/well. Fifty microliters of 30 µg/mL anti-cotinine antibody having a heavy chain sequence of SEQ ID NO: 11 and a light chain sequence of SEQ ID NO: 12 (rabbit variable region with human IgG1 Fc domain containing a DE mutation (S239D/I332E)) that had been diluted in assay medium (RPMI 1640 (Gibco) containing 10% fetal bovine serum (Gibco), 1 % Glutamax (Gibco) and 1% sodium pyruvate (Corning) was added to the wells of the plate. The plate was centrifuged for 1 min at 500 rpm (revolutions per minute) and then shaken for 5 min at room temperature. The plate was incubated for 15 minutes at 37 °C/5% CO₂.

Frozen PBMC cells (HemaCare BioResearch) were thawed and centrifuged at 1,200 rpm for 4 minutes. The cells were diluted to a cell density of 2 x 10⁶ cells/mL in assay medium. Fifty microliters of the diluted cells were added to the wells of the plate. The plate was centrifuged for 1 min at 500 rpm and incubated for 18-24 hours at 37 °C/5% CO₂.

After 18-24 hours incubation, 2 µL of 6.25 µg human FC block (BD Pharmingen) that was diluted in assay medium was added to the wells of the plate. The plate was centrifuged for 1 minute at 500 rpm and then incubated at room temperature for 15 minutes. Five microliters of 750 nM Helix NP NIR (Biolegend) that had been diluted in assay medium was added to the wells of the plate. Nine microliters of antibody cocktail containing 1/5 diluted fluorescein isothiocyanate (FITC) labeled anti-CD45 antibody (Beckman Coulter), PC7 labeled anti-CD14 antibody (Beckman Coulter) and phyocoerythrin (PE) anti-CD4 antibody (Beckman Coulter) was then added to the wells of the plate. The plate was centrifuged for 1 min at 500 rpm and incubated for 30 minutes at 4-8 °C. Samples from the plate were run on BD FACS Canto II flow cytometer and data was analyzed using FlowJo software.

ARMs compounds of Formula (I) of the invention were tested for the ability to deplete monocytes in the above assay in one or more experimental runs and the results are shown in Table 3 below. Potency of the compounds of the invention is reported as a pEC50 value. The pEC50 value is the negative log of the EC50 value, wherein the EC50 value is the half maximal effective concentration measured in molar (M). For compounds tested in more than one experimental run, the pEC50 value is reported as an average or as the individual values measured.

**Table 3: Results for ADCC Reporter Assay (Example 25) and PBMC Ex Vivo Depletion Assay (Example 26)**

| **Compound Example No.** | **ADCC Reporter Assay pEC50** | **Ex Vivo Depletion Assay pEC50** |
|---|---|---|
| 1 | 8.6 | |
| 2 | 7.9 | |
| 3 | 9.2 | |
| 4 | 9.1 | |
| 5 | 9.8 | |
| 6 | 9.1 | |
| 7 | 8.6 | |
| 8 | 8.8 | |
| 9 | 8.9 | |
| 10 | 8.9 | |
| 11 | 8.6 | |
| 12 | 9.2 | |
| 13 | 9.1 | |
| 14 | >11.7 | |
| 15 | 9.7 | |
| 16 | 9.2 | |
| 17 | 8.2 | |
| 18 | 8.3 | |
| 19 | 8.5 | |
| 20 | 8.4 | |
| 21 | 8.2 | |
| 22 | 8.8 | 9.1 |
| 23 | 9.4 | |
| 24 | 9.0 | |

### EXAMPLE 27: In vivo Depletion Assay

Human CCR2 (hCCR2) knock-in mice (C57 background) were dosed intravenously with a PBS solution containing a compound of Formula (I) to measure depletion of CCR2 expressing cells in peripheral blood. Peripheral blood from IV dosed mice was also analyzed to determine PK properties of the ARM compounds of Formula (I).

Stock solution preparation: Stock solutions of compounds of Formula (I) were prepared at 20 mg/mL and 100 mg/mL in DMSO for PK/PD and dose tolerability studies respectively. Stock solutions were stored at -20°C until further usage.

Formulations preparation: On the day of experiment, the stock solution of the compound of Formula (I) was removed from storage at -20°C and thawed at room temperature. Anti-cotinine antibody having a heavy chain sequence of SEQ ID NO: 13 and a light chain sequence of SEQ ID NO: 14 (rabbit variable region sequence with mouse IgG2a Fc domain), if required was removed from storage at -80°C and thawed at room temperature. Antibody vials were immediately transferred into wet ice after thawing. Compounds of Formula (I) were further diluted in DMSO as per experimental requirements.

Formulation composition: The formulation composition was Saline: DMSO: PBS. Saline was added based on the quantity required and then stock solution of the compound of Formula (I) prepared in DMSO, followed by addition of antibody in PBS. Formulations were incubated at room temperature for 30 minutes before administration to the mouse. DMSO was used at 1 to 2 % (v/v) in the final formulation.

Administration to Animal: Solution formulation of antibody and compound of Formula (I) was injected (bolus injection) to the restrained mouse in the right/left lateral tail vein. Animals were dosed with 0.1 milligram per kilogram (mpk) of the compound of Formula (I) and 10 mpk of the anti-cotinine antibody.

Collection of Blood for PK: Blood was collected at time points 0.25 hour, 2 hours, and 4 hours following administration (50µL/time point) through retro-orbital bleeding under mild isoflurane anesthesia.

Terminal bleeding at end of experiment (48hr): Approximately 250 µL of blood in K₂EDTA tube and approximately 250 µL of blood in SST (serum separation tube) was collected from each mouse through retro-orbital bleeding under deep isoflurane anesthesia. After bleeding, each mouse was sacrificed by cervical dislocation. The blood distribution at termination was determined as follows:
- **For PK:** 50 µL of K₂EDTA blood was transferred to another tube for PK
- **Flow cytometry:** Remaining blood (~200 µL) was used for flow cytometry analysis.
- **For serum collection:** serum separator tubes (BD) were centrifuged at 4°C, 5000 rpm for 15 minutes. Approximately 100 µL serum was separated and stored at -80°C for further usage.
- **Blood drug concentration:** Samples collected at the various timepoints (0.25 hr, 2 hr, 24 and 48 hr) were analyzed to determine blood drug concentration.

### Flow cytometry analysis:

CCR2 depletion in peripheral blood was determined by flow cytometry. Briefly, peripheral blood was collected from all animals at the end of study termination and processed. Blood samples were lysed using 1X RBC Lysis buffer and the resulting cell pellet was washed twice in FACS buffer (HBSS containing 5% FBS). Cells were subjected to Fc blocking using mouse Fc block from Biolegend (Trustain, anti-mouse CD16/32, Cat: 101319) to block non-specific binding followed by surface staining for immune markers (CD45, CD3, CD11b, Ly6C, human CCR2). Surface staining was done for 30 minutes in ice and cells were washed twice to remove unbound antibodies. Cells were stained with 7AAD to determine viability and acquired on BD FACSVerse flow cytometer. Appropriate FMO controls and single-color controls were included in the experiment for gating and compensation respectively.

CCR2+monocytes were gated as CD11b+Ly6C+ Hi, medium and Low populations and treatment induced reduction in CD11b+Ly6c+ Hi cells (i.e., cells with high CCR2 expression) was evaluated. Percent depletion of CD11b+Ly6c+ Hi cells and CCR2 expression for the different treatment groups was determined. The results are shown below in Table 4 and are reported as percent (%) depletion of CCR2 expressing cells.

### Blood drug concentration analysis:

Drug concentration in blood samples was determined by an LC-MS/MS-based bioanalytical method developed at Syngene. Samples were analyzed on Q-Trap, API-5500 LC-MS/MS system coupled with Exion UHPLC system from SCIEX, USA operated in multiple reaction monitoring mode employing electrospray ionization technique in positive polarity. Analyte and internal standard peaks were resolved on Synergi Polar, 75 X 2.0 mm, 4 µ column using mobile phase 10 mM Ammonium acetate in Milli-Q water as phase A and 0.1 % Formic acid in acetonitrile as Phase B. Gradient elution was performed with initial composition 95 % Phase A at 0.0 min, holding it for 0.2 minutes, ramping to 5 % by 1.0 minute, keeping the same for next 0.5 minutes and coming back to 95 % by 1.6 minutes. The total run time was 2 minutes.

Working dilutions for calibration curve and quality control standards were prepared by serially diluting 20 mg/mL stock solution with DMSO. Spiked concentrations for calibration curve in the whole blood ranged from 1 ng/mL to 1000 ng/mL. The working solution of internal standard (Verapamil, 25 ng/mL) was prepared in acetonitrile. 10 µL of the study sample and calibration curve, quality control, and blank whole blood samples were aliquoted in 96 deep well plates for processing. 10 µL of Milli-Q water was added to all the samples and briefly vortexed to initiate complete hemolysis. 10 µL of 20 mM dithiothreitol (DTT) was added to all the samples and incubated for 30 minutes at 37°C. The addition of DTT enhanced the recovery of ARM compounds of Formula (I) from the biological matrix. 300 µL of working internal standard solution was added to all samples except total blank and wash samples, where 300 µL of acetonitrile was added. All the samples were vortex mixed for 5 minutes, followed by centrifugation at 4000 rpm for 10 minutes at 4 °C. Supernatants were transferred to the loading plate and injected 3 µL to LC-MS/MS system for analysis. The results are shown below in Table 4 and are reported as compound exposure 48 hours post-dose.

**Table 4: Results for In Vivo Depletion Assay- % Depletion of CCR2 expressing cells and PK analysis of ARM compounds of Formula (I) after 48 hours exposure**

| **Compound Example No.** | **% Depletion of CCR2 expressing cells** | **Exposure of Compound of Formula (I) 48 hours post-dose (ng/mL)** |
|---|---|---|
| 1 | 91.67 | 55.69 |
| 2 | 97.7 | 53.21 |
| 3 | 98.4 | 31.31 |
| 4 | 99.69 | 26.6 |
| 5 | 98.41 | 95.37 |
| 6 | n.d.¹ | n.d. |
| 7 | 86.93 | 54.35 |
| 8 | 99.22 | 74.92 |
| 9 | 99 | 49.82 |
| 10 | | |
| 11 | 89.3 | 85.07 |
| 12 | 95.4 | 33.3 |
| 13 | n.d. | n.d. |
| 14 | 92.3 | 41.4 |
| 15 | 94.04 | 34.95 |
| 16 | n.d. | n.d. |
| 17 | 96.07 | 28.29 |
| 18 | n.d. | n.d. |
| 19 | 85.39 | 54.19 |
| 20 | 87.3 | 74.63 |
| 21 | n.d. | n.d. |
| 22 | 89 | 15.5 |
| 23 | n.d. | n.d. |
| 24 | n.d. | n.d. |

| | | |
|---|---|---|
| 'n.d.= not determined | | |

### EXAMPLE 28: Binding of Anti-Cotinine Antibody to ARM Compounds of Formula (I) Measured by Surface Plasmon Resonance (SPR)

Anti-cotinine antibodies having a heavy chain sequence of SEQ ID NO: 11 and a light chain sequence of SEQ ID NO: 12 (rabbit variable region with human IgG1 Fc domain containing a DE mutation (S239D/I332E)) were captured on one or more flow cells of a protein A sensor chip (Cytiva) using a Biacore T200 while reserving flow cell 1 as a reference. Following capture, a 3000 second wait step was included to reduce drift during compound analysis. ARM compounds of Formula (I) were then injected at 100 µL/min with 200 and 2000 second association and dissociation times. The entire experiment was run at 37°C with running buffer containing 10 mM HEPES pH 7.4, 150 mM NaCl, 0.005% P20 and 1% DMSO. ARM compounds of Formula (I) were titrated with a top concentration of 200 nM using a 3-fold 5-point dilution series and a corresponding 5-injection buffer cycle was run for blank subtraction. Data were double referenced by subtracting the response of the reference flow cell from that of the antibody-containing flow cell and subsequently subtracting the referenced blank sensorgrams. Following compound analysis, the surface was regenerated using a 30 second injection of pH 1.5 glycine at a flow rate of 30 µL/min after which antibody was re-captured. The experiment was run using Biacore T200 control software and evaluated using Biacore T200 Evaluation software. Curves were fit with a 1:1 kinetic binding model to obtain kₒₙ (1/Ms), k_{off} (1/s), K_{d} (M) determined as k_{off}/kₒₙ, and residence time determined as 1/k_{off}. (s).

Anti-cotinine antibody was tested for binding to ARMs compounds of Formula (I) of the invention in the above assay in or more experimental runs and the results are shown in Table 5 below. Affinity is reported as a pK_{d} value. The pK_{d} value is the negative log of the K_{d} value, wherein the K_{d} value is the dissociation constant measured in molar (M) as noted above. For compounds tested in more than one experimental run, the pK_{d} value is reported as an average.

**Table 5: Binding of Anti-Cotinine Antibody to ARM Compounds of Formula (I) Measured by SPR**

| **Compound Example No.** | **pK_{d} of Antibody for ARM Compound of Formula (I)** |
|---|---|
| 2 | 9.2 |
| 3 | 9.1 |
| 7 | 9.2 |
| 8 | 9.1 |
| 9 | 9.2 |
| 14 | 9.0 |
| 15 | 9.0 |
| 22 | 9.3 |

### EXAMPLE 29: Nicotinic Acetylcholine Receptor Antagonism Assay

Cells expressing human alpha1 nicotinic acetylcholine (nACh) receptor (nAChR) were washed and dissociated using Trypsin containing buffer. Cells were then washed and seeded at a density of 3-5 million cells/mL and allowed to grow for 3 days. Then, 60-80 µL of cells were added to plates and incubated with dose response of an ARM compound of Formula (I) with a top concentration of either 10 µM or 100 µM. The receptor was then stimulated via addition of acetylcholine (ACh) and activation of the receptor was monitored via the IONFlux automated patch clamp system. IonFlux Data Analyzer software was utilized to perform leak subtraction and measure (min) peak current amplitude. These data were then exported to Excel.

Data processing: (1) Current values recorded for vehicle only applications in individual traps/patterns were subtracted from corresponding current amplitude values for control, test article and control+test article. (2) Control response was calculated as % of the average response of the 3rd out of total 3 ACh (10mM) applications alone. In rare situations, if the 3rd ACh response was obscured due to technical artifacts, the 2nd and/or 1st ACh (10mM) application was considered as control. The peak current amplitude generated in the presence of each test article concentration was then used to calculate the % inhibition as compared to control response (10mM ACh alone). These estimates were further corrected for vehicle response and run down corrected by normalizing with time matched control responses (10mM ACh alone). The corrected percentages were then fit with a non-linear function in GraphPad Prism 6 (or later versions) to determine the IC50.

Exclusion criteria: (i) any n's with poor recording quality, (ii) control current amplitude <500pA or (iii) significant outliers, as determined by the Grubbs' test.

ARMs compounds of Formula (I) of the invention were tested for antagonism of the nAChR in the above assay in or more experimental runs and the results are shown in Table 6 below. Antagonistic activity of the compounds of Formula (I) of the invention is reported as an IC50 value (half maximal inhibitory concentration) and the maximum percentage antagonism observed at the highest concentration of compound of Formula (I) tested, as indicated in Table 6. For compounds tested in more than one experimental run, the IC50 value is reported as an average.

**Table 6: Results of nAChR Antagonism Assay**

| **Compound Example No.** | **IC50 (µM)** | **Maximum Concentration of Compound Tested (µM)** | **Maximum Response (%)** |
|---|---|---|---|
| 1 | >100 | 100 | 29 |
| 2 | >100 | 100 | 40 |
| 3 | >100 | 100 | 38 |
| 4 | 100 | 100 | 48 |
| 6 | >100 | 100 | 31 |
| 7 | 94 | 100 | 54 |
| 8 | 100 | 100 | 48 |
| 10 | >100 | 100 | 26 |
| 11 | >100 | 100 | 39 |
| 13 | >100 | 100 | 37 |
| 14 | >100 | 100 | 38 |
| 16 | 71.1 | 100 | 64 |
| 17 | 100 | 100 | 21 |
| 19 | >100 | 100 | 8 |
| 20 | >100 | 100 | 74 |
| 21 | >10 | 10 | 22 |

### EXAMPLE 30: In Vivo Mouse Study (muScreen)

The impact of treatment with CyTaC compounds of formula (I) in combination with anti-cotinine antibody on tumor growth was assessed across multiple in vivo mouse models, including KPC, pan02, b16BL6, H22, RENCA, RM1, B16F10, EMT-6, LLC, CT26, MC38, A20 and Hepa1-6 models.

Dosing and analysis of tumor bearing animals: Tumor cells were injected subcutaneously into the flank of mice (syngeneic with the tumor cells injected). Tumor volume was measured using a digital Vernier caliper, the length (l) and width (w) of the tumor was measured. Tumor volume was calculated using the following formula: Tumor Volume (mm³) = L X W2 / 2, where L = Length (mm); W = Width (mm). Anti-cotinine antibody having a heavy chain sequence of SEQ ID NO: 13 and a light chain sequence of SEQ ID NO: 14 (rabbit variable region sequence with mouse IgG2a Fc domain) and CyTaC compound of Example 22 were diluted in saline and administered intravenously to give the final concentrations of 0.1 or 0.2mpk CyTAC compound and 10mpk Anti-cotinine antibody. Flow cytometry of peripheral blood was conducted as described above in Example 27. For flow cytometry analysis of tumors: Tumor isolation procedures were performed in laminar flow hood following sterile techniques. All dissection tools used during the procedure were sterilized. The tools were kept in 70% ethanol between usage. Mice bearing subcutaneous tumor were humanely euthanized using carbon dioxide asphyxiation, sprayed down with 70% ethanol and then wiped with Betadine to remove loosed fur. The skin of tumor site was lifted up using forceps and cut down with a dissection scissor to expose the subcutaneous tumor. The exposed tumor was cut and removed using a small dissection scissor. The tumor sample was placed in 50 mL falcon adequately filled with RPMI 1640 serum free media and further processed for flow analysis. Antibody staining was conducted as for the samples from blood.

Survival analysis-Animals were humanely euthanized when tumors reached >1500mm³ or ulcerations of the tumor were visible.

The results are shown in **FIGs. 1-6****.**

### EXAMPLE 31: In vivo mouse study- thioglycolate induced peritonitis model

The ability of CyTaC compounds of formula (I) in combination with anti-cotinine antibody to block CCR2-mediated chemotaxis was assessed in a thioglycolate induced peritonitis mouse model to demonstrate the ability of the combination to antagonize CCR2 activity *in vivo.*

Preparation of Thioglycollate Medium (Sigma, B2551-500G): Brewer thioglycollate medium powder (4-6 grams*) was dissolved in 100 mL of MilliQ water and kept in a water bath to completely dissolve the medium. The medium was then autoclaved for 15 minutes at 121 °C. The autoclaved medium was wrapped in aluminium foil and stored at 4-8 °C for two weeks before administration. The aging process during which the medium turns brown is critical for the efficient induction of peritonitis. (*Note: in some studies, 4% w/v thioglycollate medium was used while in others 6% w/v was used).

Disease induction: Mice were injected intraperitoneally (i.p) with the two-week aged thioglycollate medium at a dose of either 1 ml of 4% w/v or 0.7 mL of 6% w/v of thioglycollate medium.

Anti-cotinine antibody having a rabbit variable region sequence with mouse IgG2a Fc domain with ADCC activity disabled through a p-mutation (L234A/L235A) was used for the study. Mice were dosed with anti-cotinine antibody at 10 mpk or 50 mpk and CyTaC compound ranging from 0.001 mpk to 0.5 mpk.

Collection of peritoneal lavage: At different time points (6 hr, 24 hr, 48 and 72 hr) post the thioglycollate administration, peritoneal lavage was carried out on the mice. For peritoneal lavage, the mice were injected i.p with 10 ml of ice cold PBS (with 3% v/v FBS) using a 27g needle. Post injection the abdominal cavity was massaged gently for 2 minutes to dislodge adhering cells into the lavage fluid and make the cell suspension uniform within the abdominal cavity. After completion of the abdominal massage the lavage fluid was collected by aspiration. A 25G needle attached to a 10 mL syringe was placed into the abdominal cavity and the peritoneal fluid was drawn into the syringe by aspiration to the maximum extent possible. The collected peritoneal lavage fluid was kept on ice until TLC and DLC analysis.

Flow cytometry and cytokine analysis: 2 ml of peritoneal lavage was taken for flow cytometry. 1 ml of peritoneal lavage was centrifuged at 10000 rpm at 4°C and supernatant were stored at -80°C for cytokine analysis.

The results are shown in **FIGs. 10A-10B****.**

### EXAMPLE 32: In vivo mouse study- Collagen-induced arthritis (CIA) mouse model

The efficacy of depletion of CCR2 monocytes by combination treatment with a CyTaC compound of formula (I) and an anti-cotinine antibody in a collagen induced model of arthritis was evaluated. The results suggest that combination treatment with a CyTaC compound of formula (I) and an anti-cotinine antibody may have therapeutic benefits for autoimmune disorders.

Preparation of 0.05M Glacial acetic acid: Sterile water for injection (10 mL) was measured and added to a 15 mL falcon tube, and 28.6 µl were removed and replaced with 28.6 µl of Glacial acetic acid.

Preparation of immunization grade type II collagen (CII): Type II Collagen was prepared by adding appropriate quantity of 0.05M Acetic acid solution to make a 4 mg/mL solution of Type II Collagen one day prior to the immunization. The vial was stored at 4°C overnight. On the day of experiment (day 0), the solution was diluted with 0.05M acetic acid (1:1) to obtain a final concentration 2 mg/mL of collagen.

Immunization Procedure on Day 0:
- Emulsion preparation: Collagen (CII) at 2 mg/mL was emulsified with Complete Freund's Adjuvant (CFA) at 2 mg/mL in a ratio of 1:1 for a final concentration of 1 mg/mL of CII and 1 mg/mL of CFA. One-part volume of CFA was added into a sealed 5 mL glass syringe. Then, an equal volume of Collagen (Cll) was gradually added drop wise into the CFA suspension while mixing using a homogeniser at low speed. Once completely mixed, the homogenisation was done a maximum rpm for 2 minutes. The emulsion was then allowed to cool by keeping the syringe in the ice for 5 minutes. The procedure was repeated 3 times to get a uniform emulsion that was stable (non-dispersal when a drop of emulsion is placed on water). The emulsion was transferred from the glass syringe to a 1 mL syringe (BD).
- Administration of emulsion: Animals were lightly anaesthetised using isoflurane. 100 µL emulsion was subcutaneously injected approximately 2 cm from the base of the tail.

Immunization Procedure day 21:
• Emulsion preparation: Collagen (CII) at 2 mg/mL was emulsified with Incomplete Freund's Adjuvant (IFA) in a ratio of 1:1 to for a final concentration of 1 mg/mL of CII. One-part volume of IFA was added into a sealed 5 mL glass syringe. Then an equal volume of Collagen (Cll) was gradually added drop wise into the CFA suspension while mixing using a homogeniser at low speed. Once completely mixed, the homogenisation was done a maximum rpm for 2 minutes. The emulsion was then allowed to cool by keeping the syringe in the ice for 5 minutes. The procedure was repeated 3 times to get a uniform emulsion that was stable (non-dispersal when a drop of emulsion is placed on water). The emulsion was transferred from the glass syringe to a 1 mL syringe (BD).
• Administration of emulsion: Animals were lightly anaesthetised using isoflurane. 100 µL emulsion was subcutaneously injected approximately 1 cm from the base of the tail.
• Scoring: Qualitative scoring for disease severity of paw inflammation was performed using the criteria set forth below in Table 7.

**Table 7: Score Criteria**

| Score | Description |
|---|---|
| 0 | Normal |
| 1 | Mild, but definite redness and swelling of the ankle or wrist, or apparent redness and swelling limited to individual digits, regardless of the number of affected digits |
| 2 | Moderate redness and swelling of ankle of wrist |
| 3 | Severe redness and swelling of the entire paw including digits |
| 4 | Maximally inflamed limb with involvement of multiple joints |

• Treatment initiation: On the day of disease onset (day 26 to day 54)
∘ Dexamethasone (comparator)- 0.1 mg/kg p.o, daily.
∘ Enbrel (comparator)- 10 mg/kg i.p.
∘ Compound of formula (I) (compound of Example 22) + anti-cotinine antibody. - weekly once intraperitoneal.

• Clinical signs: Day 22 (next day after booster dose) to end of experiment (day 54).
• Paw thickness: Using a Mitutoyo micrometre on Day 21 (day of booster dose) base line, Day 36 and Day 54.
• Paw weight: Weight of all the four paws i.e. both hindlimbs and forelimbs excised at the hairline at termination time (day 54)
• Cytokine Analysis: 14 plex mouse Inflammation panel 14 plex in serum and paw homogenate of the forelimbs.
• Cartilage degradation marker in serum: CTXII and COMP using commercially available ELISA kits in the serum obtained on day 54.
• Auto-antibody levels serum: Mouse anti-bovine type II collagen IgG antibody assay using ELISA
• Flow: Peripheral blood - CD45, CD3, CD11b, Ly6c, mCCR2, CD4, PD1, CXCR5
• Flow: Synovial lavage - CD45, CD3, CD11b, Ly6c, mCCR2, CD4, PD1, CXCR5
• Histopathology - hind limb ankle joint was collected in 10% NBF. The joint were decalcified and then stained for cartilage degradation and infiltration.

The results are shown in **FIG. 9****.** In **FIG. 9****,** "sham control" indicates mice not induced with collagen so as not to develop an "arthritic" phenotype; and "pathological control" indicates mice injected with collagen, but did not receive any therapeutic intervention.

### EXAMPLE 33: In vivo mouse study- efficacy of combination treatment with anti-PD-1 or STING agonist

The efficacy of CyTaC compound of Example 22 in combination with anti-cotinine antibody, administered in further combination with anti-PD-1 or STING agonist was evaluated *in vivo.*

### Combination with anti-PD-1

Dosing and analysis of tumor bearing animals: EMT-6 tumor cells were injected subcutaneously into the flank of mice (syngeneic with the tumor cells injected). Tumor volume was measured using a digital Vernier caliper, the length (l) and width (w) of the tumor was measured. Tumor volume was calculated using the following formula: Tumor Volume (mm³) = L X W2 / 2, where L = Length (mm); W = Width (mm). Animals were randomized when tumor volumes were between 50 -100mm³ and treatment began following randomization. Animals were sacrificed with tumor volume exceeded 3000mm³. Anti-cotinine antibody and CyTaC compound of formula (I) (Example 22) were diluted in saline and administered intravenously to give the final concentrations of 0.5mpk CyTAC BIW and 10mpk Anti-cotinine antibody, which was dosed once weekly. Anti-PD1 antibody was dosed at 10mpk once weekly.

The results are shown in **FIGs. 8A-8B****.**

### Combination with STING agonist

The efficacy of CyTaC compound of Example 22 in combination with anti-cotinine antibody, administered in further combination with STING agonist was evaluated *in vivo* in CT26 mouse model, which is a murine colon carcinoma syngeneic mouse model.

Six to eight week old female BALB/c mice were used for this study. Mice were inoculated subcutaneously with 5e4 CT-26 cells on the right hind flank and tumors grew until they reached about 100mm³ as measured by digital calipers and applying the formula tumor volume TV=0.52*L*W² (Day 0). On day 0, mice were randomized by tumor volume into study groups with n=10 mice/group and administered the first dose. Mice were dosed as set forth in the table below. Aspartic acid was administered as vehicle control for STING agonist treatment. Tumor volume and body weight were measured 3x weekly throughout the study and mice reached endpoint when tumor volume was >2000mm³, tumors developed open ulcerations, or mice lost >20% body weight.

| **Treatment 1 (Dose Days)** | **Treatment 2 (Dose Days)** | **Description** |
|---|---|---|
| Aspartic acid (0, 3, 7) | Compound of Example 22 + anti-cotinine antibody (0, 7, 14) | CyTaC + anti-cotinine antibody monotherapy |
| Aspartic acid (0, 3, 7) | Compound of Example 22 + Isotype Ab (0, 7, 14) | CyTaC + isotype antibody monotherapy (control)) |
| STING agonist 10µg (0, 3, 7) | Compound of Example 22 + anti-cotinine antibody (0, 7, 14) | Combination therapy of CyTaC + anti-cotinine antibody with STING agonist |
| STING agonist 10µg (0, 3, 7) | Compound of Example 22 + Isotype Ab (0, 7, 14) | STING agonist + CyTaC + isotype Ab (control) |

The results are shown in **FIG. 7****.**

### SEQUENCE LISTINGS

Heavy chain CDR1 amino acid sequence
   SEQ ID NO: 1
   NYWMS
Heavy chain CDR2 amino acid sequence
   SEQ ID NO: 2
   DIHGNRGFNYHASWAKG
Heavy chain CDR3 amino acid sequence
   SEQ ID NO: 3
   ADDSGSHDI
Light chain CDR1 amino acid sequence
   SEQ ID NO: 4
   QSSQSVYSAKLS
Light chain CDR2 amino acid sequence
   SEQ ID NO: 5
   YGSTLAS
Light chain CDR3 amino acid sequence
   SEQ ID NO: 6
   QGTFYGPDWYFA
Variable heavy chain amino acid sequence
   SEQ ID NO: 7 
EVQLVESGGGLVQPGGSLRLSCAASGFTFSNYWMSWVRQAPGKGLEWVGDIHG
NRGFNYHASWAKGRFTVSRSKNTLYLQMNSLRAEDTAVYYCAKADDSGSHDIW
GQGTLVTVSS
Variable light chain amino acid sequence
   SEQ ID NO: 8 
DIQMTQSPSSLSASVGDRVTITCQSSQSVYSAKLSWYQQKPGKAPKLLIYYGSTLASGVPS
RFSGSGSGTQFTLTISSLQPEDFATYYCQGTFYGPDWYFAFGGGTKVEIK
Heavy chain amino acid sequence
   SEQ ID NO: 9 
EVQLVESGGGLVQPGGSLRLSCAASGFTFSNYWMSWVRQAPGKGLEWVGDIHGNRGFN
YHASWAKGRFTVSRSKNTLYLQMNSLRAEDTAVYYCAKADDSGSHDIWGQGTLVTVSSAS
TKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKKVEPKSCDKTHTCPPCPAPELLGGPDVF
LFPPKPKDTLMISRTPEVTCVVVDVSHEDPEVKFNWYVDGVEVHNAKTKPREEQYNSTYR
VVSVLTVLHQDWLNGKEYKCKVSNKALPAPEEKTISKAKGQPREPQVYTLPPSRDELTKNQ
VSLTCLVKGFYPSDIAVEWESNGQPENNYKTTPPVLDSDGSFFLYSKLTVDKSRWQQGNV
FSCSVMHEALHNHYTQKSLSLSPGK
Light chain amino acid sequence
   SEQ ID NO: 10 
DIQMTQSPSSLSASVGDRVTITCQSSQSVYSAKLSWYQQKPGKAPKLLIYYGST
LASGVPSRFSGSGSGTQFTLTISSLQPEDFATYYCQGTFYGPDWYFAFGGGTKV
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC
Heavy chain amino acid sequence
   SEQ ID NO: 11 
QQQLVESGGR LVTPGGSLTL TCTASGFSLN NYWMSWVRQA PGKGLEWIGD
IHGNRGFNYH ASWAKGRFTV SRTSTTVDLR MTSLTTEDTA IYFCARADDS
GSHDIWGPGT LVTVSSASTK GPSVFPLAPS SKSTSGGTAA LGCLVKDYFP
EPVTVSWNSG ALTSGVHTFP AVLQSSGLYS LSSVVTVPSS SLGTQTYICN
VNHKPSNTKV DKKVEPKSCD KTHTCPPCPA PELLGGPDVF LFPPKPKDTL
MISRTPEVTC VVVDVSHEDP EVKFNWYVDG VEVHNAKTKP REEQYNSTYR
VVSVLTVLHQ DWLNGKEYKC KVSNKALPAP EEKTISKAKG QPREPQVYTL
PPSRDELTKN QVSLTCLVKG FYPSDIAVEW ESNGQPENNY KTTPPVLDSD
GSFFLYSKLT VDKSRWQQGN VFSCSVMHEA LHNHYTQKSL SLSPGK
Light chain amino acid sequence
   SEQ ID NO: 12 
ELDLTQTPSPVSAAVGDTVTINCQSSQSVYSAKLSWYQQKPGQPPKLLIYYGSTLASGVPS
RFKGSGSGTQFSLTISDVQCADAATYYCQGTYYGPDWYFAFGGGTEVVVKRTVAAPSVFI
FPPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC
Heavy chain amino acid sequence
   SEQ ID NO: 13 
QQQLVESGGRLVTPGGSLTLTCTASGFSLNNYWMSWVRQAPGKGLEWIGDIHGNRGFNY
HASWAKGRFTVSRTSTTVDLRMTSLTTEDTAIYFCARADDSGSHDIWGPGTLVTVSSAKTT
APSVYPLAPVCGDTTGSSVTLGCLVKGYFPEPVTLTWNSGSLSSGVHTFPAVLQSDLYTLS
SSVTVTSSTWPSQSITCNVAHPASSTKVDKKIEPRGPTIKPCPPCKCPAPNLLGGPSVFIFPP
KIKDVLMISLSPMVTCVVVDVSEDDPDVQISWFVNNVEVLTAQTQTHREDYNSTLRVVSALP
IQHQDWMSGKEFKCKVNNKALPAPIERTISKPKGSVRAPQVYVLPPPEEEMTKKQVTLTCM
VTDFMPEDIYVEWTNNGKTELNYKNTEPVLDSDGSYFMYSKLRVEKKNWVERNSYSCSVV
HEGLHNHHTTKSFSRTPGK
Light chain amino acid sequence
   SEQ ID NO: 14

ELDLTQTPSPVSAAVGDTVTINCQSSQSVYSAKLSWYQQKPGQPPKLLIYYGSTLASGVPS
RFKGSGSGTQFSLTISDVQCADAATYYCQGTYYGPDWYFAFGGGTEVVVKRADAAPTVSI
FPPSSEQLTSGGASVVCFLNNFYPKDINVKWKIDGSERQNGVLNSWTDQDSKDSTYSMSS
TLTLTKDEYERHNSYTCEATHKTSTSPIVKSFNRNEC

## Claims

1. A compound of Formula (I): 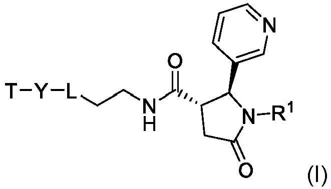
or a pharmaceutically acceptable salt thereof,
wherein:
T is 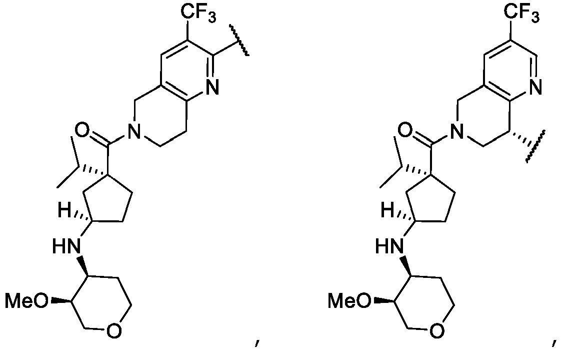 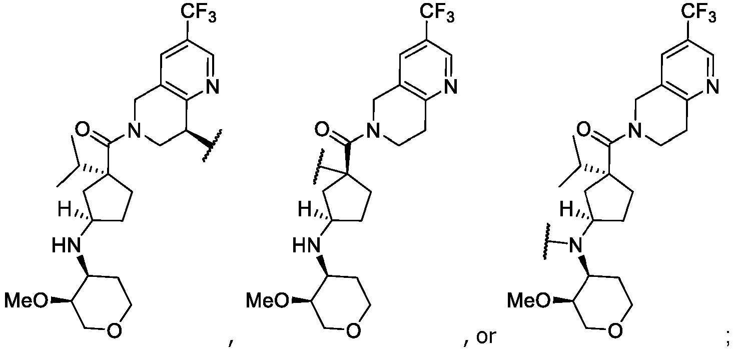
R¹ is C₁₋₄ alkyl or C₃₋₆ cycloalkyl;
L is a divalent linker of Formula (L-a), (L-b), (L-c), (L-d), or (L-e): 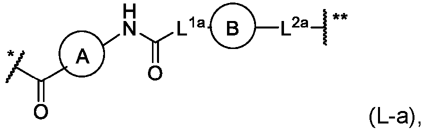 or a stereoisomer thereof,
wherein:
Ring A and Ring B are each independently C₄₋₆ cycloalkylene;
L^{1a} is C₃₋₅ linear alkylene, wherein 1 or 2 methylene units are replaced with -O- or -NR^{a}-;
each R^{a} is independently hydrogen or C₁₋₃ alkyl; and
L^{2a} is -O-, -NHC(O)-, or -CH₂-O-; 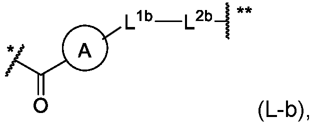 or a stereoisomer thereof, wherein:
Ring A is C₄₋₆ cycloalkylene or C₇₋₉ bridged bicyclic cycloalkylene;
L^{1b} is -CH₂-NH-C(O)-, -NHC(O)-, or -C(O)NH-;
L^{2b} is C₆₋₁₂ linear alkylene, wherein 1, 2, 3, or 4 methylene units are replaced with -O-, -NR^{1b}-, - C(O)NR^{1b}-, or -NR^{1b}C(O)-; or
L^{2b} is 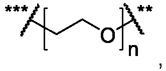 wherein n is 1, 2, 3, or 4, and 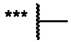 represents a covalent bond to L^{1b}; and
each R^{1b} is independently hydrogen or C₁₋₃ alkyl; 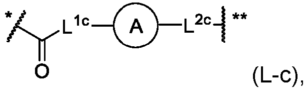 or a stereoisomer thereof, wherein:
L^{1c} is C₂₋₁₀ linear alkylene, wherein 1, 2, or 3 methylene units are replaced with -O-, -NH-, -NHC(O)-, or - C(O)NH-;
Ring A is C₄₋₆ cycloalkylene or C₇₋₉ bridged bicyclic cycloalkylene; and
L^{2c} is -O- or a saturated C₂₋₁₀ linear alkylene, wherein 1, 2, or 3 methylene units are replaced with -O-, -NH-, - NHC(O)-, or -C(O)NH-; 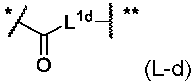 wherein:
L^{1d} is C₁₂₋₃₀ linear alkylene, wherein 1, 2, 3, 4, 5, 6, 7, 8, 9, 10, or 11 methylene units are replaced with -NH-, -O-, -C(O)NH-, -NHC(O)-, or -NHC(O)-NH-; or 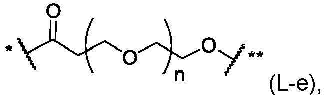 wherein n is an integer of 3 to 50;
wherein each 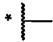 represents a covalent bond to the Y group of Formula (I), or when Y is a bond, a covalent bond to the T group of Formula (I), and each 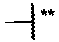 represents a covalent bond to the methylene group of Formula (I); and
Y is a bond or a divalent spacer moiety of one to twelve atoms in length.

2. The compound of claim 1, or a pharmaceutically acceptable salt thereof, wherein R¹ is -CH₃.

3. The compound of claim 1 or 2, or a pharmaceutically acceptable salt thereof, wherein L is a divalent linker of Formula (L-a-i): 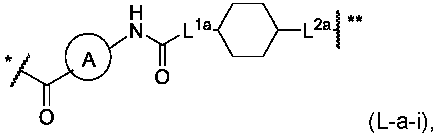 or a stereoisomer thereof, wherein Ring A, L^{1a}, L^{2a}, 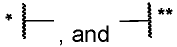 are as defined for Formula (L-a).

4. The compound of any one of the preceding claims, or a pharmaceutically acceptable salt thereof, wherein L is a divalent linker of Formula (L-a-ii): 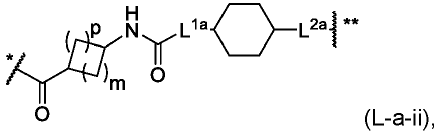 or a stereoisomer thereof, wherein L^{1a}, L^{2a}, 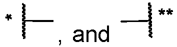 are as defined for Formula (L-a); p is 1 or 2; and m is 1 or 2.

5. The compound of any one of the preceding claims, or a pharmaceutically acceptable salt thereof, wherein L is a divalent linker of Formula (L-a-iii): 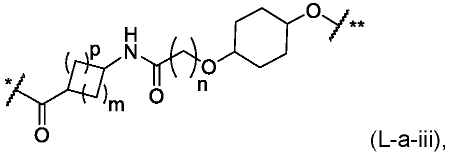 or a stereoisomer thereof, wherein p is 1 or 2; m is 1 or 2; n is 1, 2, or 3; and 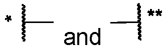 are as defined for Formula (L-a).

6. The compound of any one of claims 1 to 4, or a pharmaceutically acceptable salt thereof, wherein L is a divalent linker of Formula (L-a) selected from the group consisting of:

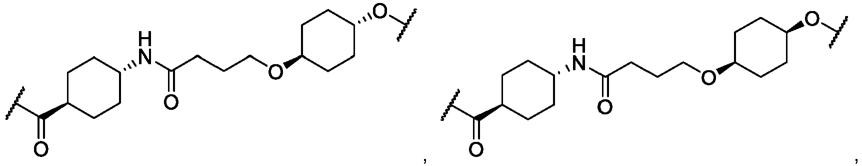

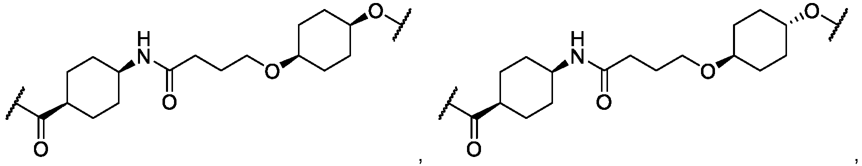

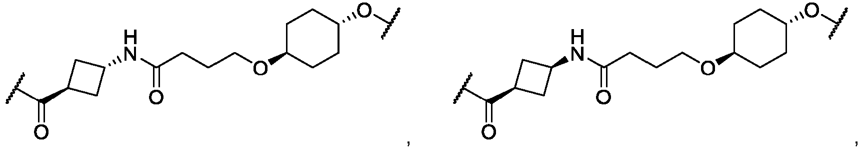

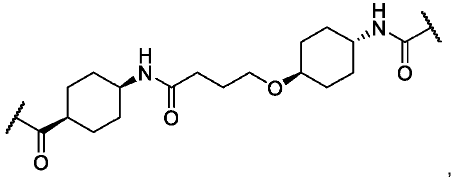

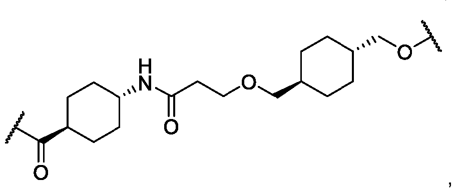

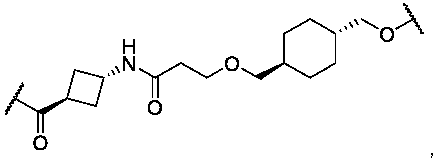

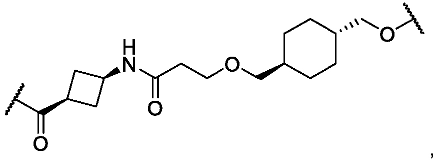

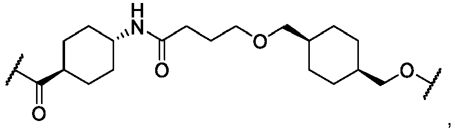

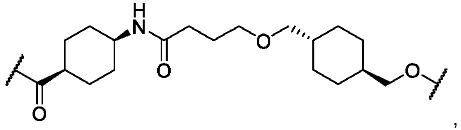

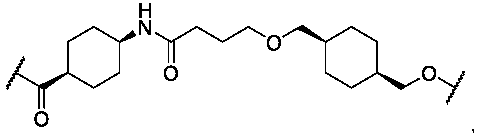

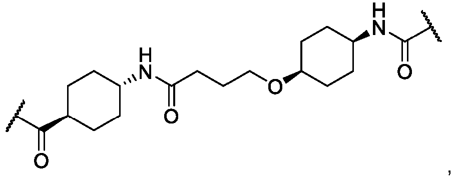

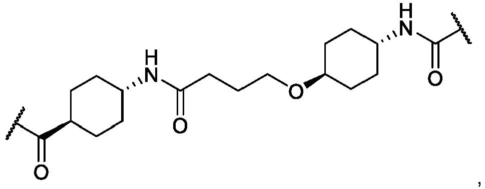

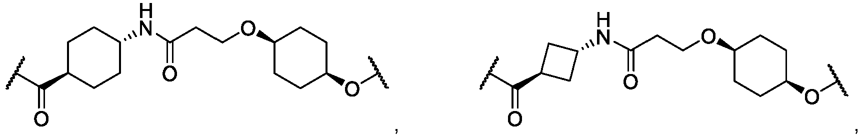

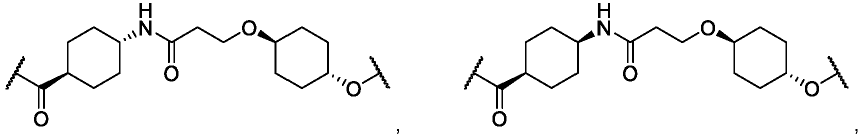

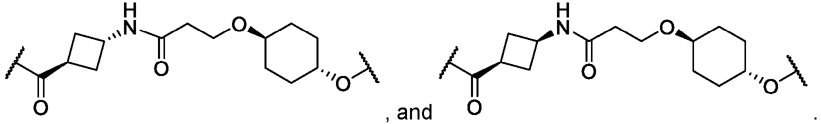

7. The compound of claim 1 or 2, or a pharmaceutically acceptable salt thereof, wherein L is a divalent linker of Formula (L-b-i): 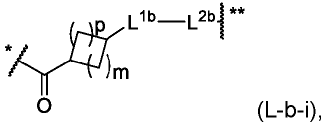 or a stereoisomer thereof, wherein L^{1b}, L^{2b}, 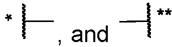 are as defined for Formula (L-b); p is 1 or 2; and m is 1 or 2.

8. The compound of any one of claims 1 to 2, or 7, wherein L is a divalent linker of Formula (L-b) selected from the group consisting of: 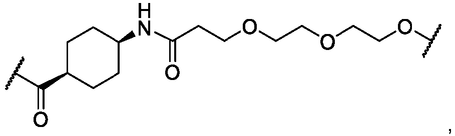 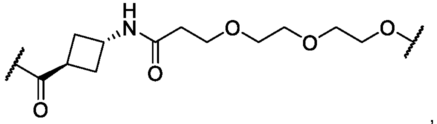 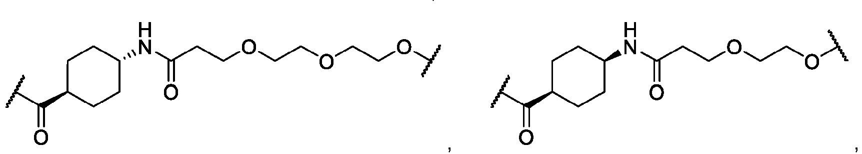 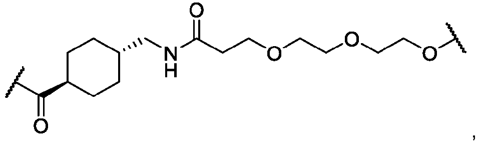 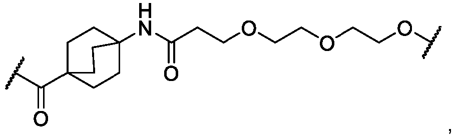 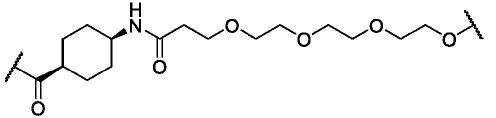 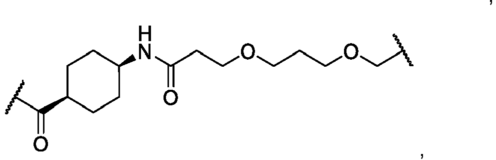 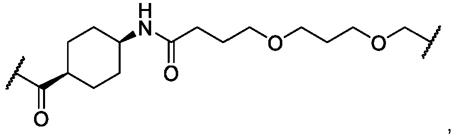 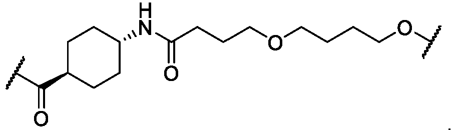 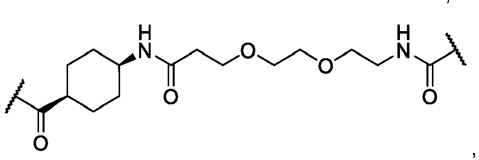 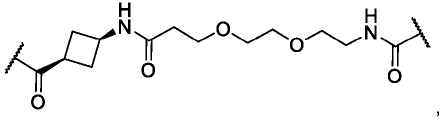 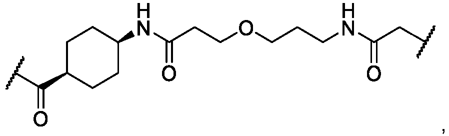 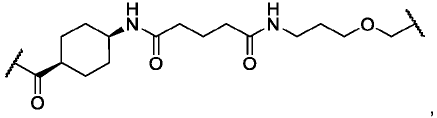 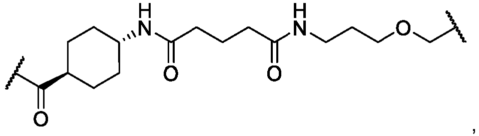 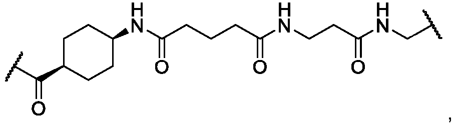 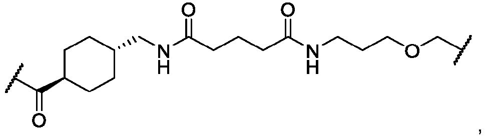 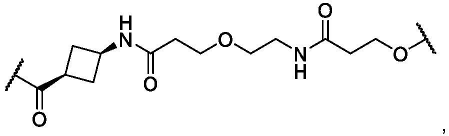 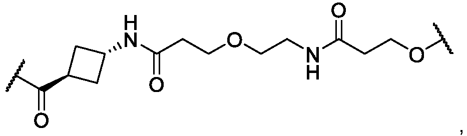 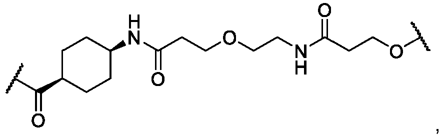 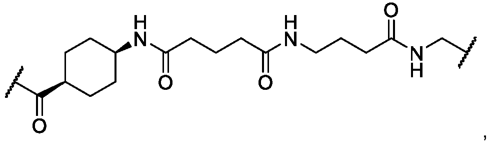 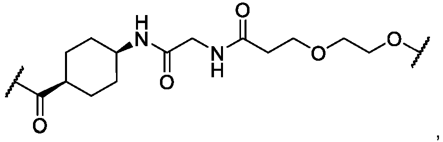 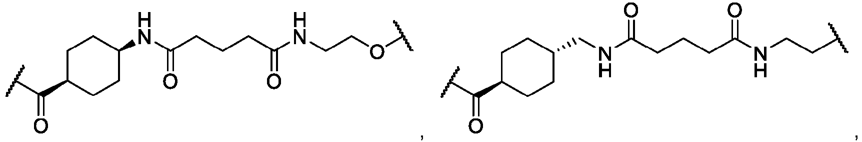 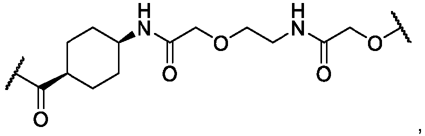 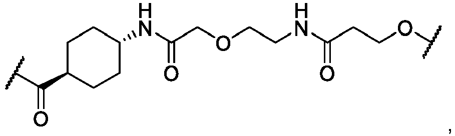 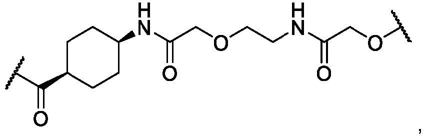 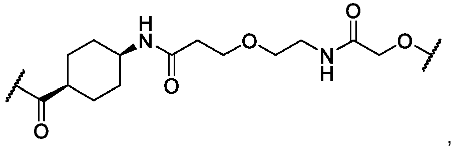 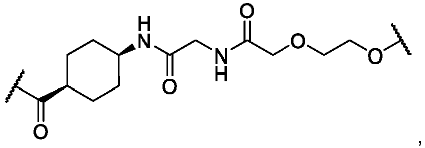 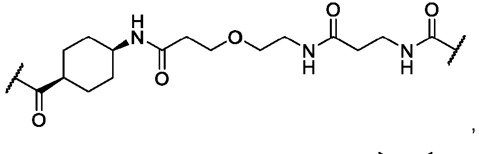 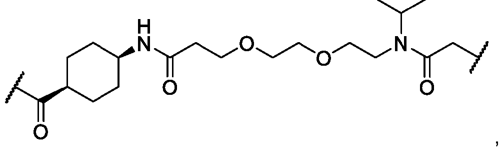 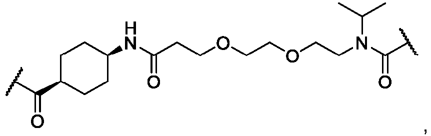 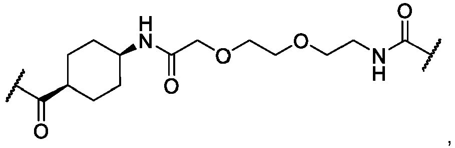 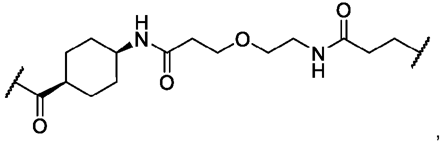 and

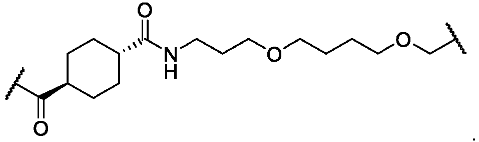

9. The compound of claim 1 or 2, or a pharmaceutically acceptable salt thereof, wherein L is a divalent linker of Formula (L-c-i): 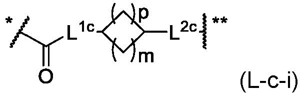 , or a stereoisomer thereof, wherein L^{1c}, L^{2c}, 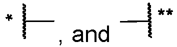 are as defined for Formula (L-c); p is 1 or 2; and m is 1 or 2.

10. The compound of claim 1 or 2, wherein L is a divalent linker of Formula (L-c) selected from the group consisting of:

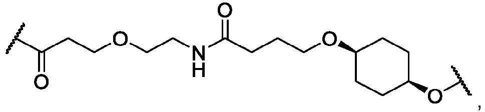

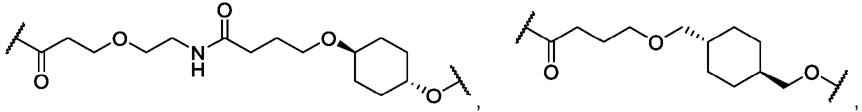

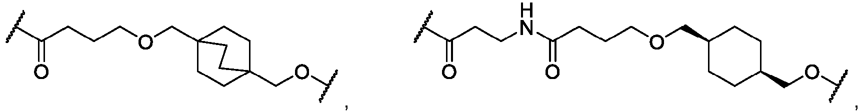

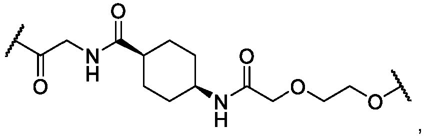

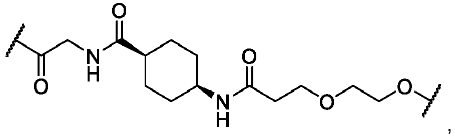

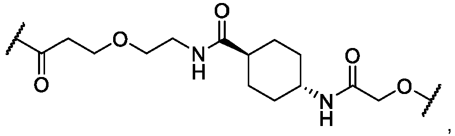

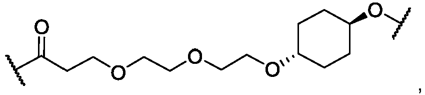

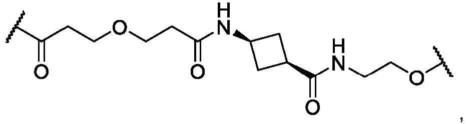

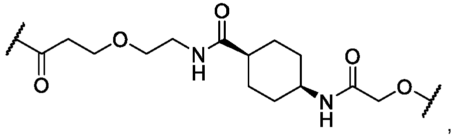

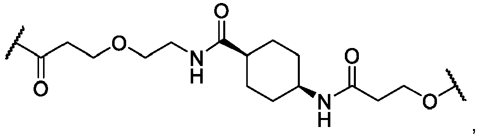

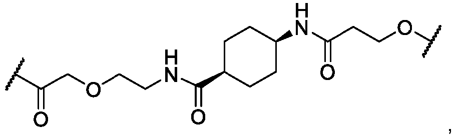

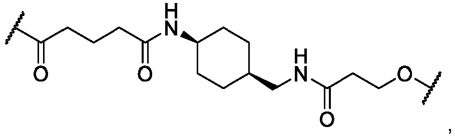

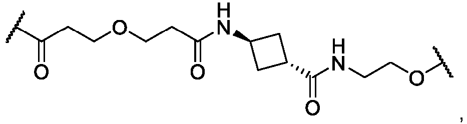

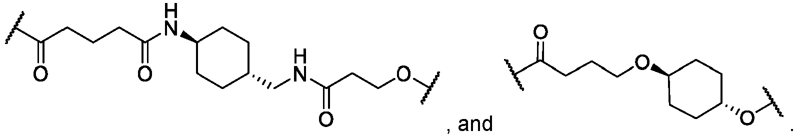

11. The compound of claim 1 or 2, or a pharmaceutically acceptable salt thereof, wherein L is a divalent linker of Formula (L-d) selected from the group consisting of: 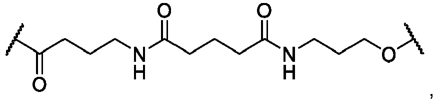 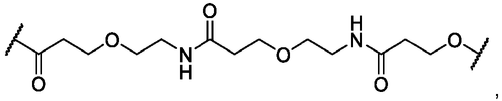 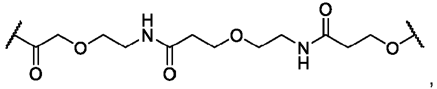 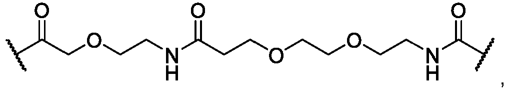 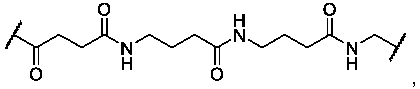 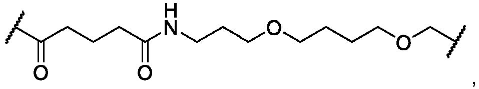 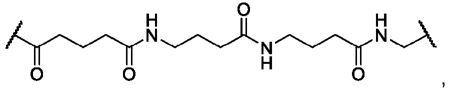 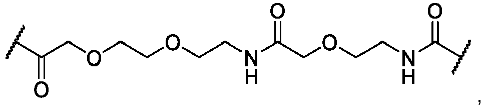 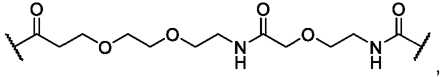 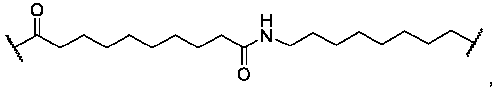 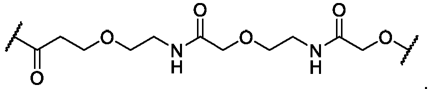 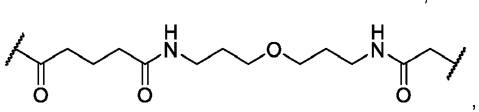 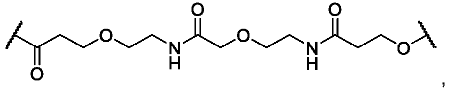 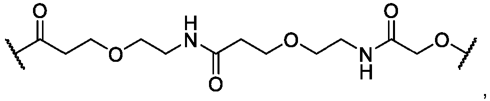 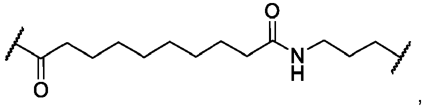 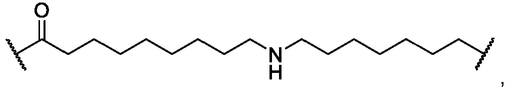 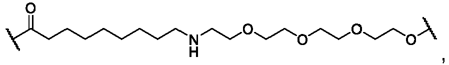 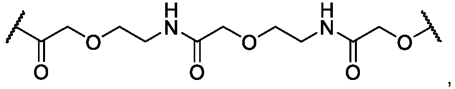 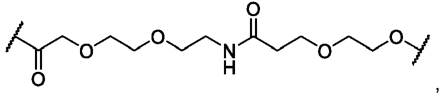 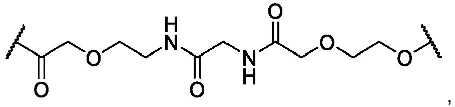 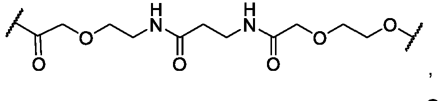 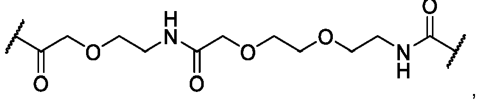 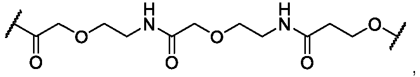 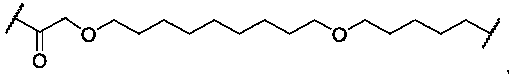 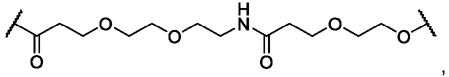 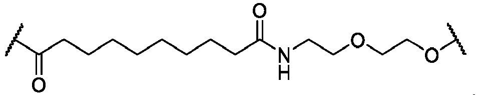 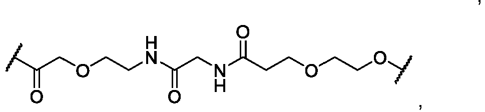 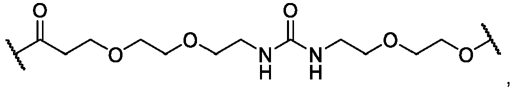 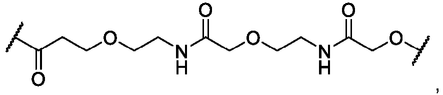 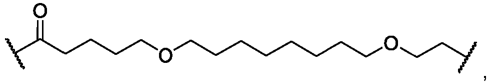 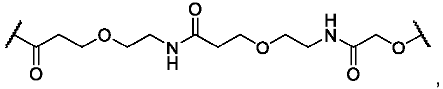 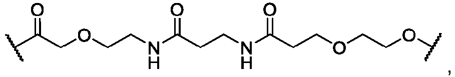 and

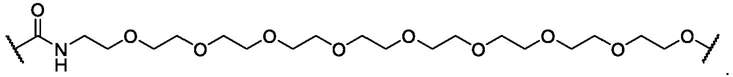

12. The compound of any one of the preceding claims, or a pharmaceutically acceptable salt thereof, wherein Y is selected from a bond; -NH-; -(C₁₋₁₂ alkylene)-, wherein 1, 2, or 3 methylene units are replaced with -O-, -NH-, - C(O)-, -NHC(O)-, -C(O)NH-, -(C₃₋₆ cycloalkylene)-, -(C₃₋₆ cycloalkenylene)-, 3- to 6-membered heterocycloalkylene, arylene, or heteroarylene; or -(C₂₋₁₂ alkenylene)-, wherein 1, 2, or 3 methylene units are replaced with -O-, -NH-, -C(O)-, -NHC(O)-, -C(O)NH-, -(C₃₋₆ cycloalkylene)-, -(C₃₋₆ cycloalkenylene)-, 3- to 6-membered heterocycloalkylene, arylene, or heteroarylene.

13. The compound of any one of claims 1-11, or a pharmaceutically acceptable salt thereof, wherein Y is selected from a bond; -NH-; -(C₁₋₆ alkylene)-O-; -(C₂₋₆ alkenylene)-O-; -(C₁₋₆ alkylene)-C(O)-; -(C₂₋₆ alkenylene)-C(O)-; phenylene; piperidinylene; -(C₁₋₆ alkylene)-O-phenylene-; -(C₂₋₆ alkenylene)-O-piperidinylene; -(C₁₋₅ alkylene)-NH-, wherein 0, 1, or 2 methylene units are replaced with -O-; -NH-(C₁₋₅ alkylene)-NH-; -(C₃₋₆ cycloalkylene)-NH-; -(C₃₋₆ cycloalkenylene)-NH-; or 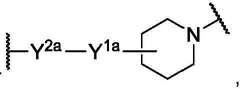 wherein Y^{1a} is a bond, -O-, - NH-, -NHC(O)-, -C(O)NH-, or C₁₋₃ alkylene; and Y^{2a} is a bond, -O-, -NH-, - NHC(O)-, -C(O)NH-, or C₁₋₃ alkylene.

14. The compound of any one of claims 1-11, or a pharmaceutically acceptable salt thereof, wherein Y is selected from the group consisting of:

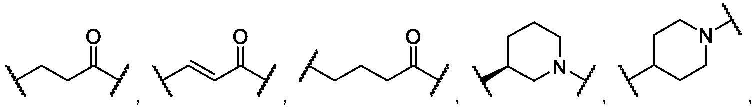

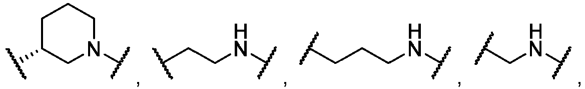

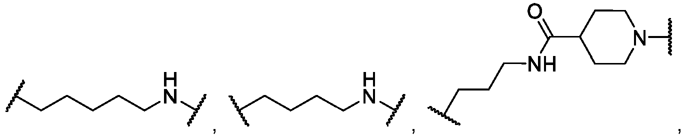

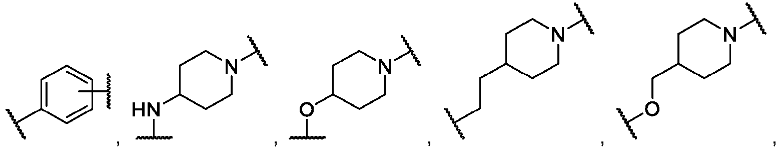

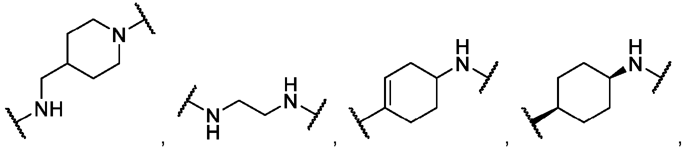

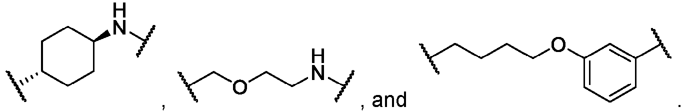

15. The compound of claim 1, wherein the compound is selected from a compound as listed in Table 1, wherein optionally the compound is selected from the group consisting of: 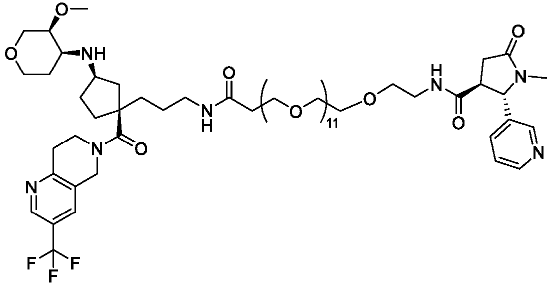 (Example No: 1), 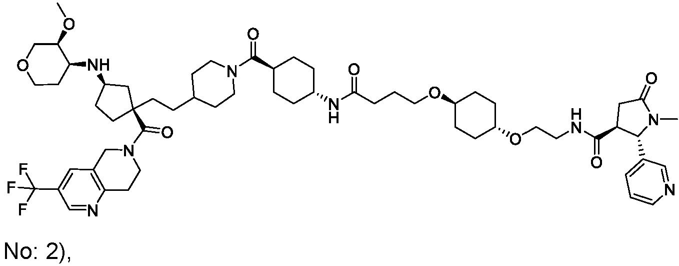 (Example and 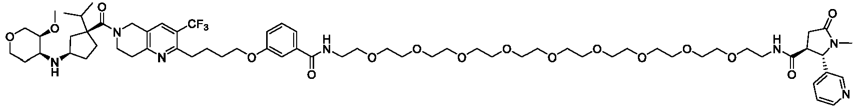 (Example No: 22).

16. A compound of any one of the preceding claims for use with an anti-cotinine antibody, or antigen-binding fragment thereof, to treat and/or prevent a disease or disorder selected from a cancer, an inflammatory disease, an autoimmune disease, a viral infection, or a bacterial infection, wherein optionally (i) the compound and the antibody, or antigen-binding fragment thereof, are administered simultaneously or (ii) the compound and the antibody, or antigen-binding fragment thereof, are administered sequentially.

17. The compound for use according to claim 16, wherein the disease or disorder is mediated by chemokine receptor 2 (CCR2) and/or is associated with CCR2-positive pathogenic cells.

18. The compound for use according to claim 16 or 17, wherein the disease is a cancer that is a solid tumor, wherein optionally
the cancer is selected from non-small cell lung cancer (NSCLC), hepatocellular carcinoma (HCC), colorectal cancer (CRC), cervical squamous cell carcinoma (CESC), head and neck squamous cell carcinoma (HNSC), pancreatic cancer, metastatic castration-resistant prostate cancer (mCRPC), ovarian cancer, bladder cancer, or breast cancer.

19. A compound of any one of claims 1-15 for use with an anti-cotinine antibody, or antigen-binding fragment thereof, for increasing antibody-dependent cell cytotoxicity (ADCC) of C-C motif chemokine receptor 2 (CCR2)-expressing cells, wherein the CCR2-binding moiety of the compound binds the CCR2 expressed on the cells.

20. A compound of any one of claims 1-15 for use with an anti-cotinine antibody, or antigen-binding fragment thereof, for depleting C-C motif chemokine receptor 2 (CCR2)-expressing cells, wherein the CCR2-binding moiety of the compound binds the CCR2 expressed on the cells.

21. The compound for use according to claim 19 or 20, wherein the CCR2-expressing cells are myeloid-derived suppressor cells (MDSCs), T regulatory cells (Tregs), neutrophils, macrophages, B regulatory cells (Bregs), CD8 regulatory cells, (CD8regs), exhausted T cells, or cancer-associated fibroblasts (CAFs).

22. A combination comprising the compound of any one of claims 1 to 15 and an anti-cotinine antibody, or antigen-binding fragment thereof.

23. The compound for use according to any one of claims 16-21 or the combination of claim 22, wherein the anti-cotinine antibody has a heavy chain and a light chain, the heavy chain comprising a CDR1 having SEQ ID NO: 1, a CDR2 having SEQ ID NO: 2, and a CDR3 having SEQ ID NO: 3, and the light chain comprising a CDR1 having SEQ ID NO: 4, a CDR2 having SEQ ID NO: 5, and a CDR3 having SEQ ID NO: 6.

24. The compound for use according to any one of claims 16-21 or the combination of claim 22, wherein the anti-cotinine antibody has a heavy chain and a light chain, the heavy chain comprising a heavy chain variable region (VH) having SEQ ID NO: 7, and the light chain comprising a light chain variable region (VL) having SEQ ID NO: 8, wherein optionally the anti-cotinine antibody has a heavy chain comprising SEQ ID NO: 9 and a light chain comprising SEQ ID NO: 10.

25. The compound for use according to any one of claims 16-21 or the combination of claim 22, wherein the anti-cotinine antibody is of IgG1 isotype comprising a substitution in an Fc region to increase ADCC activity, wherein optionally
the substitution in the Fc region is S239D/I332E, wherein residue numbering is according to the EU Index.

## Patentansprüche

1. Verbindung der Formel (I): 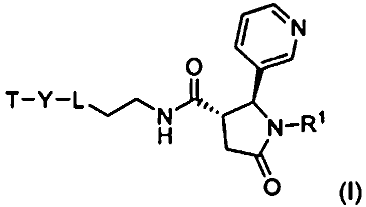 oder ein pharmazeutisch verträgliches Salz davon, wobei:
T is 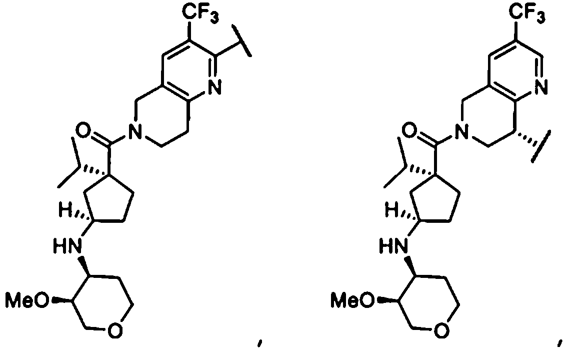 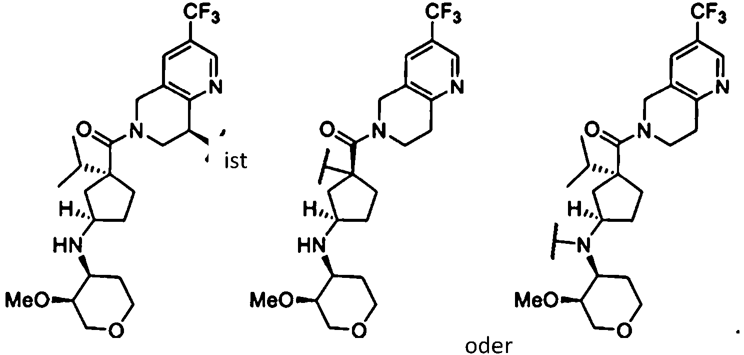
R¹ ist C₁₋₄-Alkyl oder C₃₋₆-Cycloalkyl;
L ein zweiwertiger Linker der Formel (L-a), (L-b), (L-c), (L-d) oder (L-e) ist: 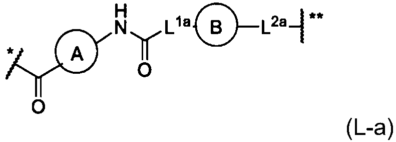 oder ein Stereoisomer davon,
wobei:
Ring A und Ring B jeweils unabhängig voneinander C₄₋₆ - Cycloalkylen sind;
L^{1a} C₃₋₅ lineares Alkylen ist, wobei 1 oder 2 Methyleneinheiten durch -O- oder - N^{Ra}- ersetzt sind;
jedes R^{a} unabhängig voneinander Wasserstoff oder C ₁₋₃ Alkyl ist; und L^{2a} -O-, -NHC(O)- oder -CH₂-O- ist; 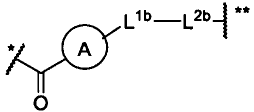 (L-b) oder ein Stereoisomer davon,
wobei:
Ring A C₄₋₆-Cycloalkylen oder C₇₋₉-verbrücktes bicyclisches Cycloalkylen ist; L^{1b} -CH₂-NH-C(O)-, -NHC(O)- oder -C(O)NH- ist;
L^{2b} C₆₋₁₂ lineares Alkylen ist, wobei 1, 2, 3 oder 4 Methyleneinheiten durch -O-, -NR^{1b} -, -C(O)NR^{1b} - oder -NR^{1b} C(O)- ersetzt sind; oder 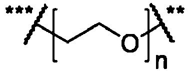 , ist, wobei n 1, 2, 3 oder 4 ist, und 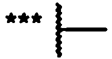 eine kovalente Bindung an L^{1b} darstellt; und jedes R^{1b} unabhängig voneinander Wasserstoff oder C₁₋₃-Alkyl ist; 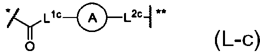 oder ein Stereoisomer davon ist, wobei:
L^{1c} lineares Alkylen ist, wobei 1, 2 oder 3 Methyleneinheiten durch -O-, -NH-, - NHC(O)- oder -C(O)NH- ersetzt sind;
Ring A C₄₋₆₋Cycloalkylen oder C₇₋₉-verbrücktes bicyclisches Cycloalkylen ist; und
L^{2c} -O- oder ein gesättigtes lineares C₂₋₁₀₋Alkylen ist, wobei 1, 2 oder 3 Methyleneinheiten durch -O-, -NH-, -NHC(O)- oder -C(O)NH- ersetzt sind; 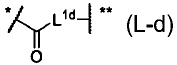 wobei:
L^{1d} C₁₂₋₃₀ lineares Alkylen ist, wobei 1, 2, 3, 4, 5, 6, 7, 8, 9, 10 oder 11 Methyleneinheiten durch -NH-, -O-, -C(O)NH-, -NHC(O)- oder -NHC(O)-NH-ersetzt sind; oder 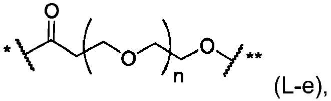 wobei n eine ganze Zahl von 3 bis 50 ist;
wobei jedes 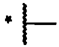 eine kovalente Bindung an die Y-Gruppe der Formel (I) darstellt, oder wenn Y eine Bindung ist, eine kovalente Bindung an die T-Gruppe der Formel (I) darstellt, und jedes 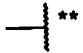 eine kovalente Bindung an die Methylengruppe der Formel (I) darstellt; und
Y eine Bindung oder eine zweiwertige Spacereinheit mit einer Länge von einem bis zwölf Atomen ist.

2. Verbindung nach Anspruch 1 oder ein pharmazeutisch verträgliches Salz davon, wobei R¹ -CH₃ ist.

3. Verbindung nach Anspruch 1 oder 2 oder ein pharmazeutisch verträgliches Salz davon, wobei L ein zweiwertiger Linker der Formel (L-a-i) ist: 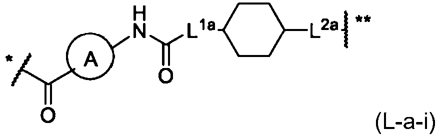 oder ein Stereoisomer davon,
wobei Ring A, L^{1a}, L^{2a}, 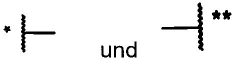 wie für Formel (L-a) definiert sind.

4. Verbindung nach einem der vorstehenden Ansprüche oder ein pharmazeutisch verträgliches Salz davon, wobei L ein zweiwertiger Linker der Formel (L-a-ii) ist: 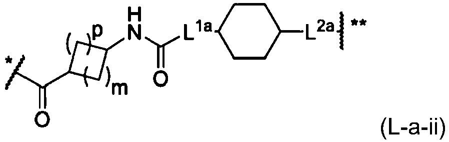 oder ein Stereoisomer davon,
wobei L^{1a} , L^{2a} , 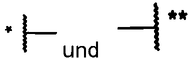 wie für Formel (L-a) definiert sind; p 1 oder 2 ist; und m 1 oder 2 ist.

5. Verbindung nach einem der vorstehenden Ansprüche oder ein pharmazeutisch verträgliches Salz davon, wobei L ein zweiwertiger Linker der Formel (L-a-iii) ist: 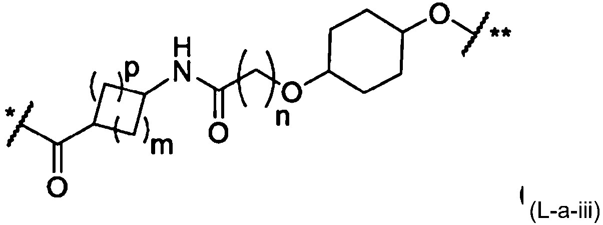 oder ein Stereoisomer davon,
wobei p 1 oder 2 ist; m 1 oder 2 ist; n 1, 2 oder 3 ist; und 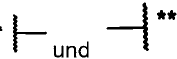 wie für Formel (L-a) definiert sind.

6. Verbindung nach einem der Ansprüche 1 bis 4, oder ein pharmazeutisch verträgliches Salz davon, wobei L ein zweiwertiger Linker der Formel (L-a) ist, ausgewählt aus der Gruppe bestehend aus:

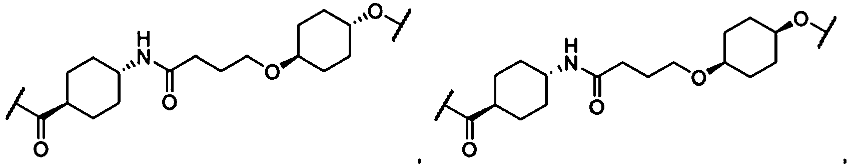

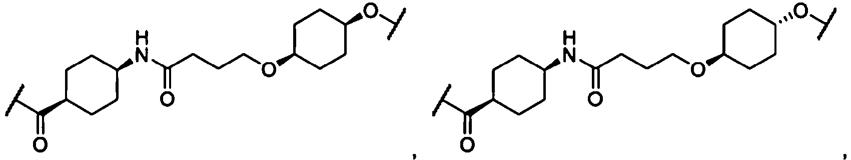

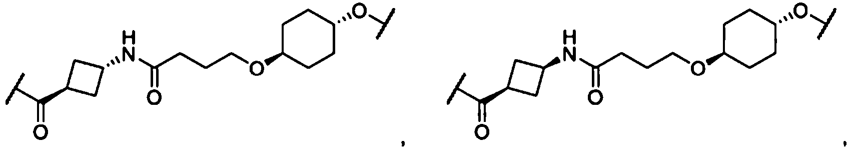

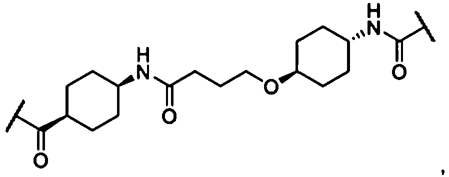

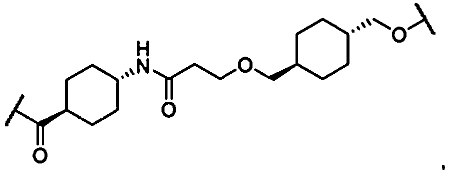

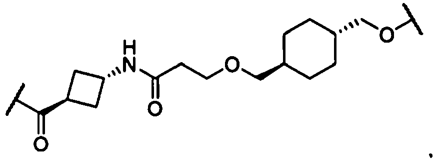

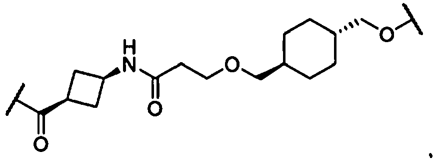

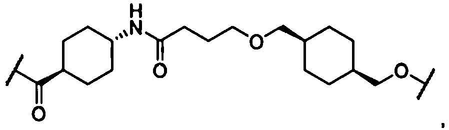

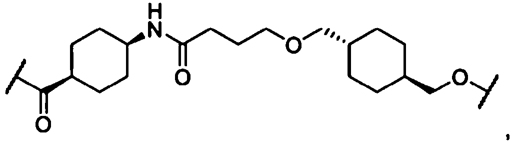

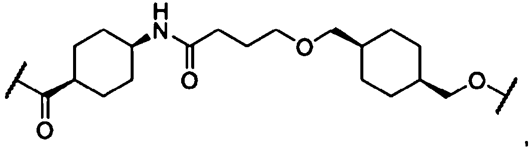

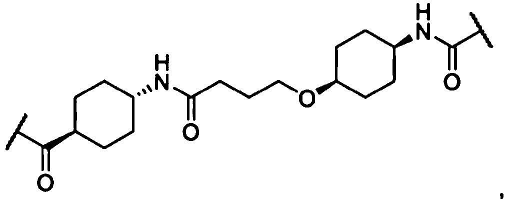

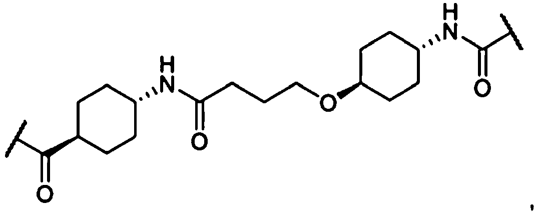

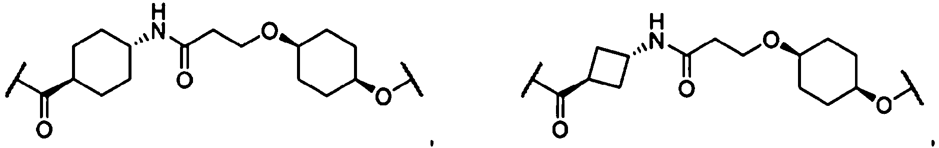

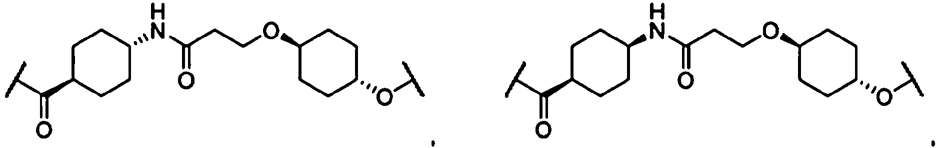

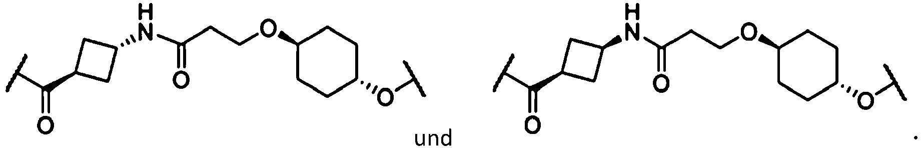

7. Verbindung nach Anspruch 1 oder 2 oder ein pharmazeutisch verträgliches Salz davon, wobei L ein zweiwertiger Linker der Formel (L-b-i) ist: 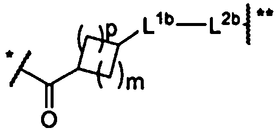
(L-b-i) oder ein Stereoisomer davon,
wobei L^{1b} , L^{2b} , 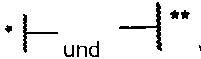 wie für Formel (L-b) definiert sind; p 1 oder 2 ist; und m 1 oder 2 ist.

8. Verbindung nach einem der Ansprüche 1 bis 2 oder 7, wobei L ein zweiwertiger Linker der Formel (L-b) ist, ausgewählt aus der Gruppe bestehend aus: 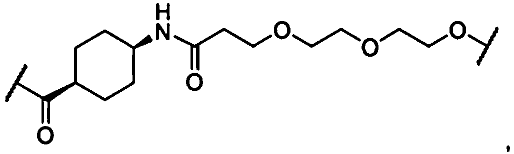 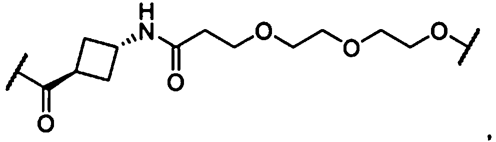 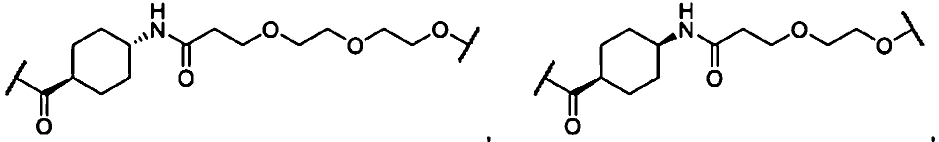 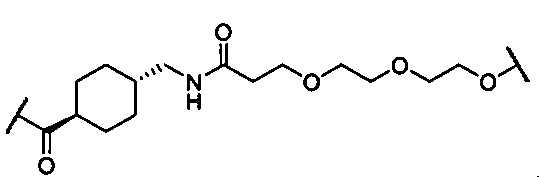 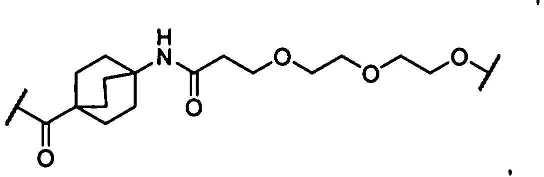 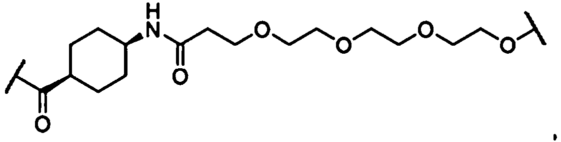 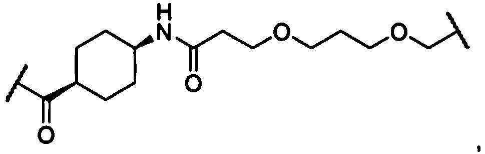 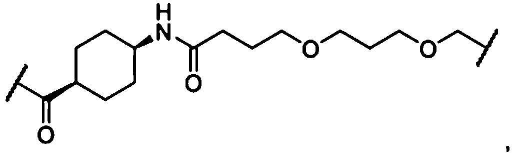 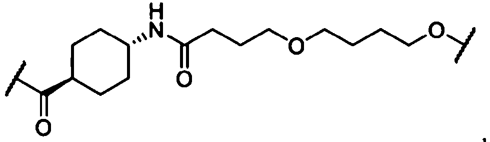 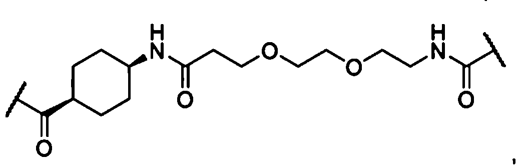 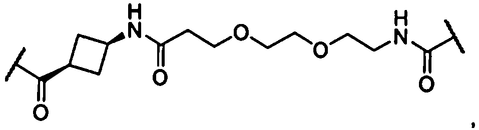 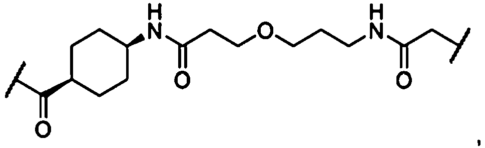 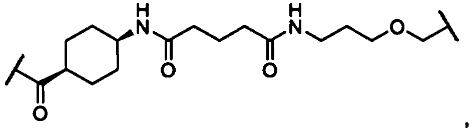 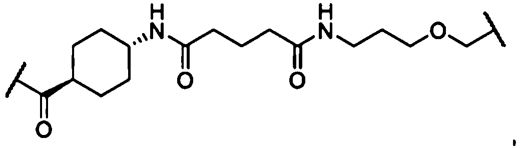 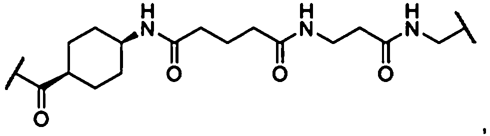 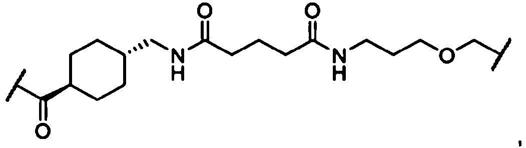 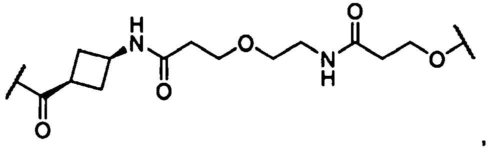 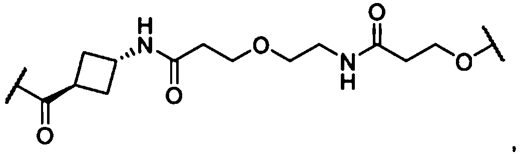 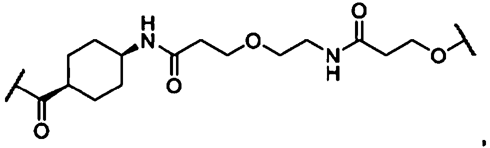 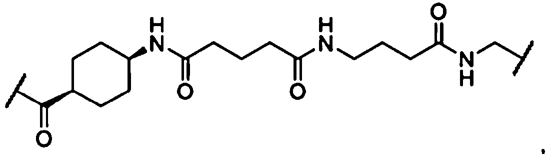 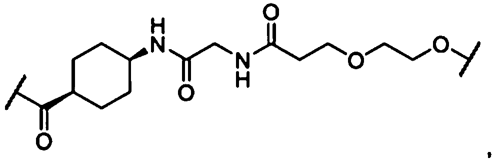 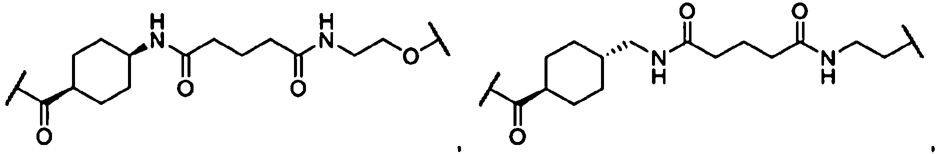 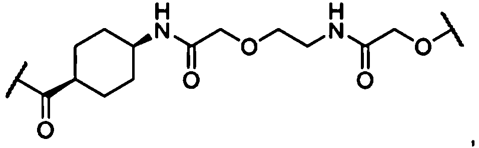 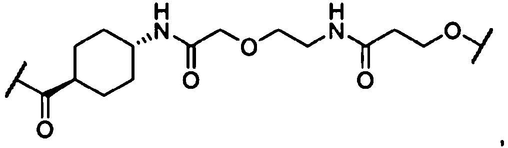 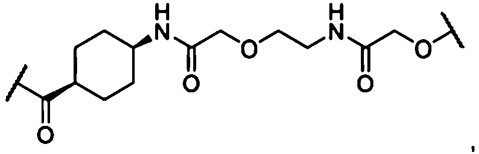 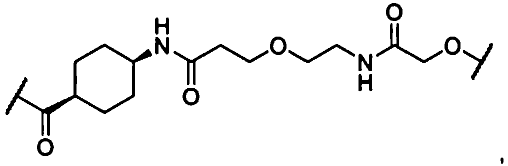 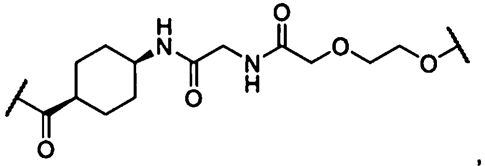 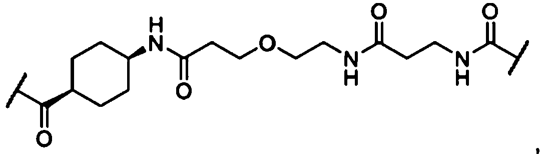 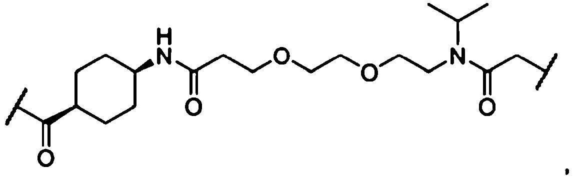 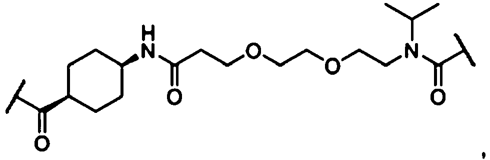 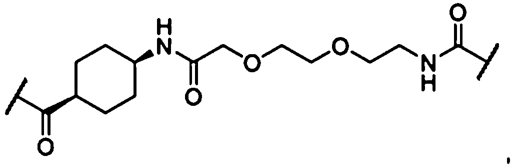 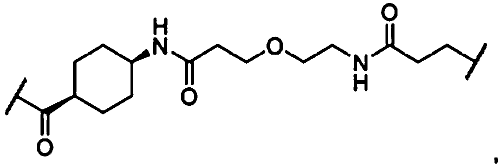 und

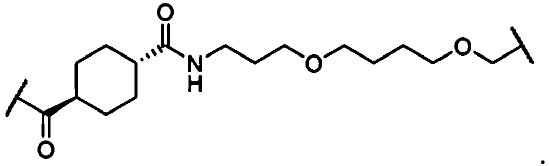

9. Verbindung nach Anspruch 1 oder 2 oder ein pharmazeutisch verträgliches Salz davon, wobei L ein zweiwertiger Linker der Formel (L-c-i) ist: 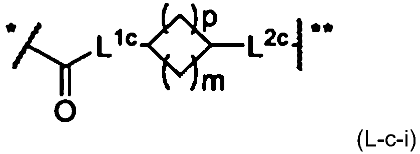 oder ein Stereoisomer davon, wobei L^{1c}, L^{2c}, , 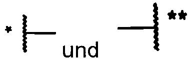 wie für Formel (L-c) definiert sind; p 1 oder 2 ist; und m 1 oder 2 ist.

10. Verbindung nach Anspruch 1 oder 2, wobei L ein zweiwertiger Linker der Formel (L-c) ist, ausgewählt aus der Gruppe bestehend aus:

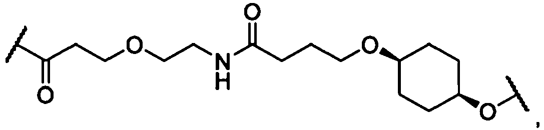

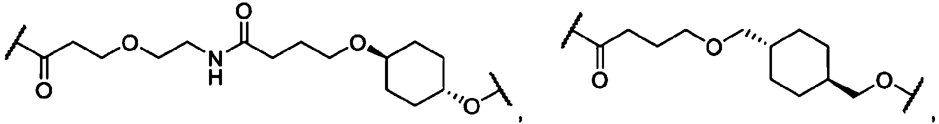

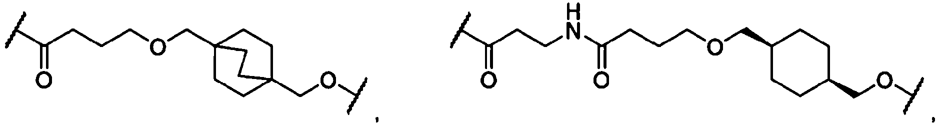

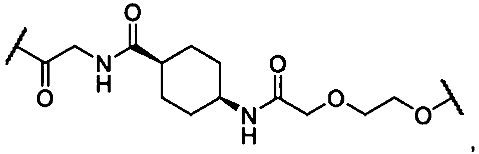

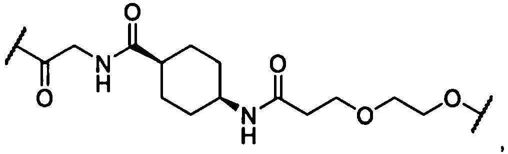

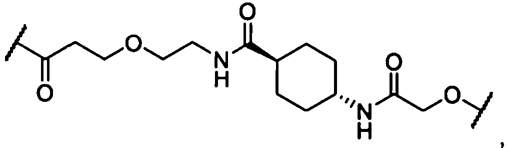

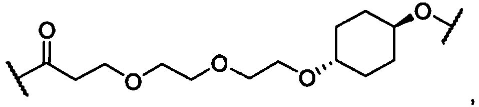

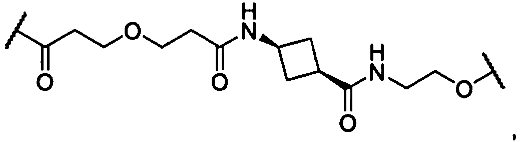

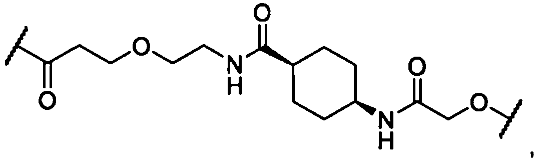

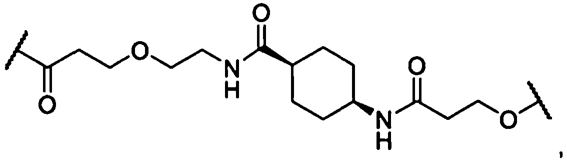

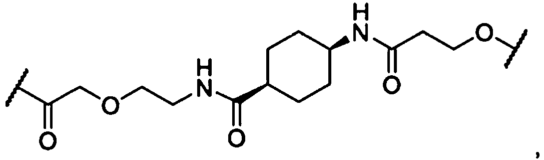

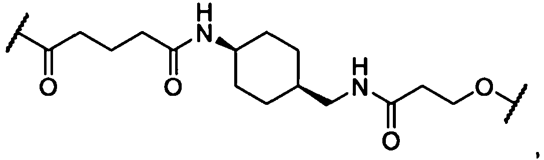

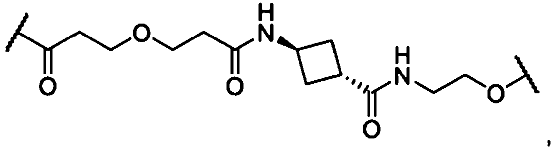

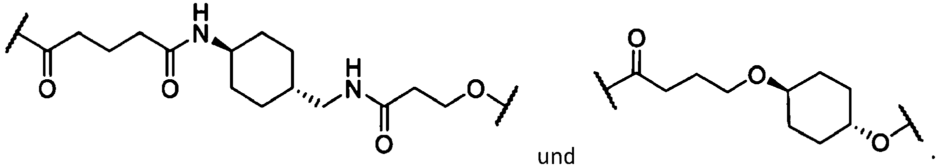

11. Verbindung nach Anspruch 1 oder 2 oder ein pharmazeutisch verträgliches Salz davon, wobei L ein zweiwertiger Linker der Formel (L-d) ist, ausgewählt aus der Gruppe bestehend aus: 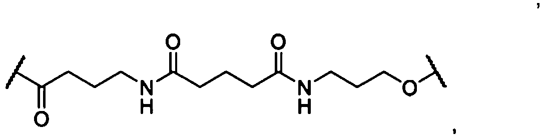 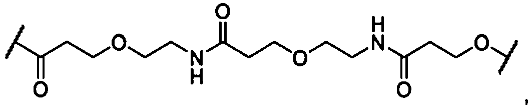 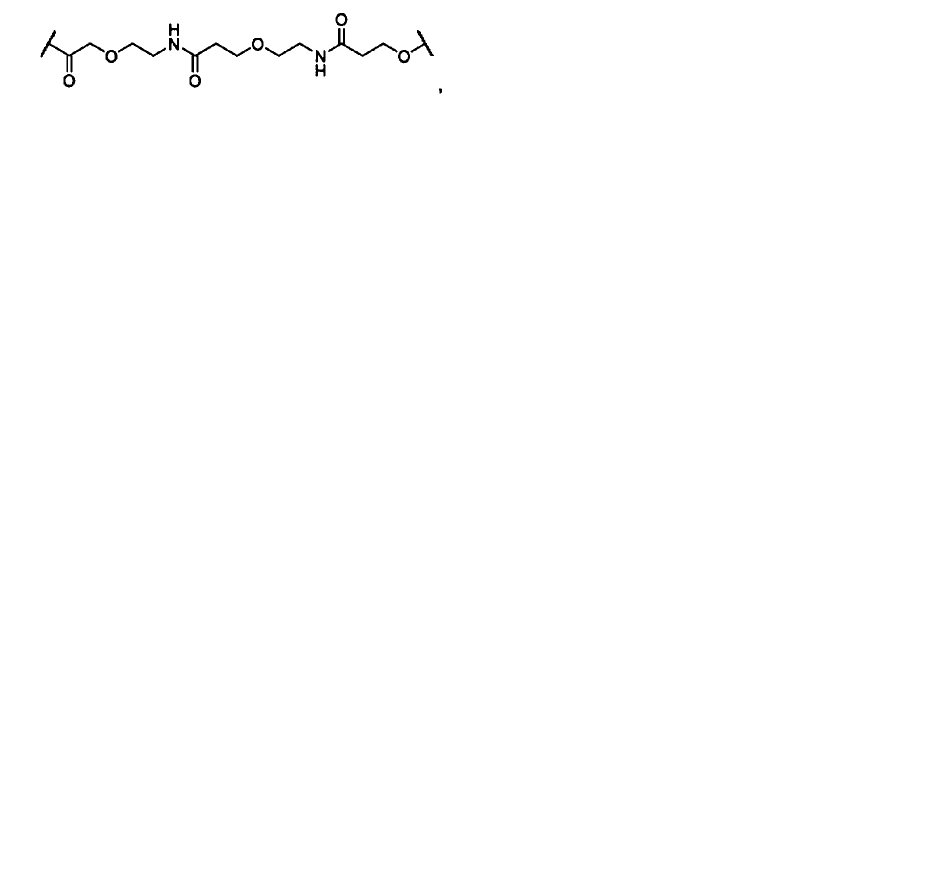 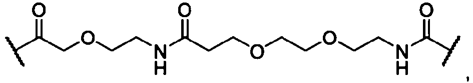 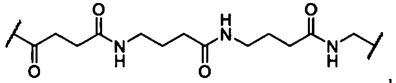 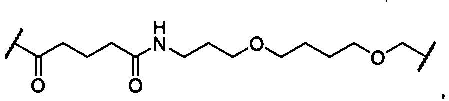 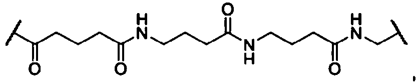 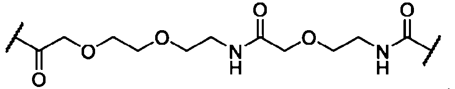 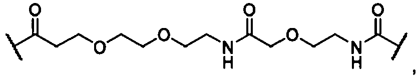 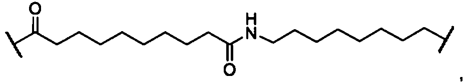 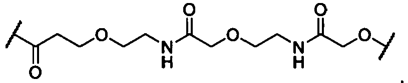 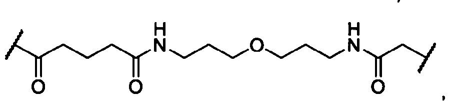 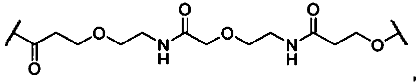 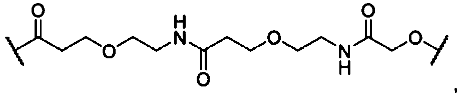 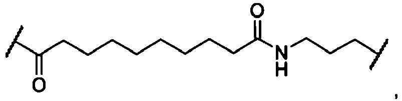 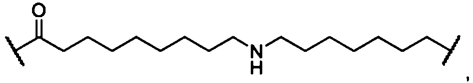 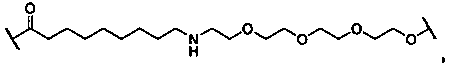 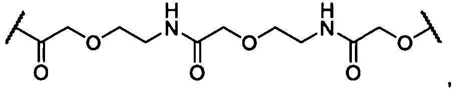 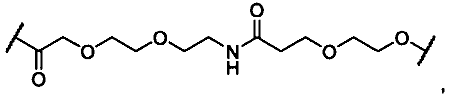 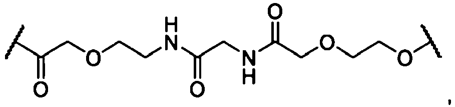 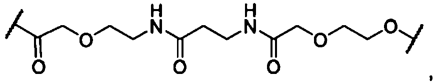 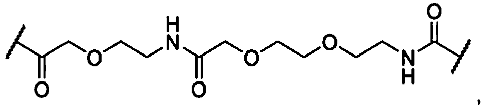 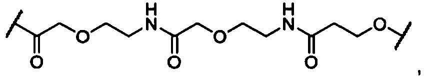 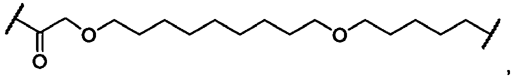 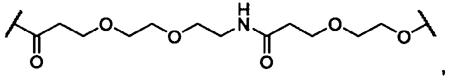 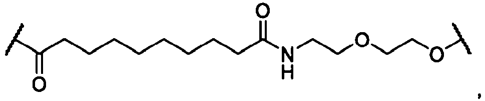 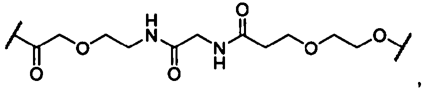 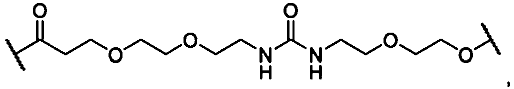 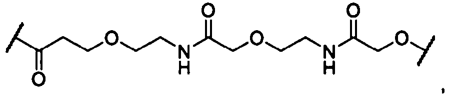 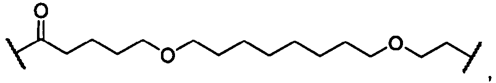 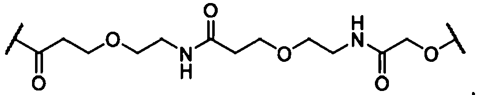 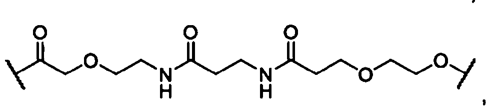 und

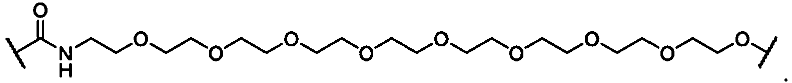

12. Verbindung nach einem der vorstehenden Ansprüche oder ein pharmazeutisch verträgliches Salz davon, wobei Y ausgewählt ist aus einer Bindung; -NH-; -(C₁₋₁₂ Alkylen)-, wobei 1, 2 oder 3 Methyleneinheiten durch -O-, -NH-, -C(O)-, - NHC(O)-, -C(O)NH-, -(C₃₋₆₋Cycloalkylen)-, -(C₃₋₆₋Cycloalkylen)-, 3- bis 6-gliedrigem Heterocycloalkylen, Aryl oder Heteroaryl; oder -(C₂₋₁₂-Alkenylen)-, wobei 1, 2 oder 3 Methyleneinheiten durch -O-, -NH-, -C(O)-, -NHC(O)-, - C(O)NH-, -( C₃₋₆ Cycloalkylen)-, -(C₃₋₆ Cycloalkylen)-, 3- bis 6-gliedrigem Heterocycloalkylen, Aryl oder Heteroaryl ersetzt sind.

13. Verbindung nach einem der Ansprüche 1 bis 11 oder ein pharmazeutisch verträgliches Salz davon, wobei Y ausgewählt ist aus einer Bindung; -NH-; -(C₁₋₆ Alkylen)-O-; -(C₂₋₆ Alkenylen)-O-; -(C₁₋₆ Alkylen)-C(O)-; -( C₂₋₆ -Alkenylen)-C(O)-; Phenylen; Piperidinylen; -(C₁₋₆-A O-Piperidinylen; -(C₁₋₅-Alkylen)-NH-, w 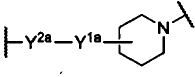 -; -( C₂₋₆ -Alkenylen)-nyleneinheiten durch -O- ersetzt sind; -NH-(C₁₋₅-Alkylen)-NH-; -(C₃₋₆-Cycloalkylen)-NH-; -(C₃₋₆ Cycloalkenylen)-NH-; oder, wobei Y^{1a} eine Bindung, -O-, -NH-, - NHC(0)-, -C(O)NH- oder C₁₋₃-Alkylen ist; und Y^{2a} eine Bindung, -O-, -NH-, - NHC(O)-, -C(O)NH- oder C₁₋₃-Alkylen ist.

14. Verbindung nach einem der Ansprüche 1 bis 11 oder ein pharmazeutisch verträgliches Salz davon, wobei Y ausgewählt ist aus der Gruppe bestehend aus:

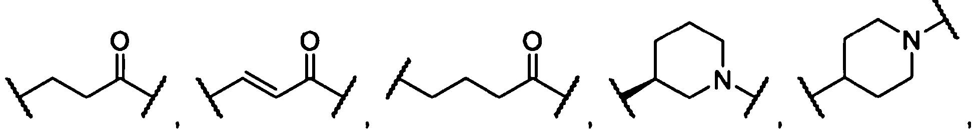

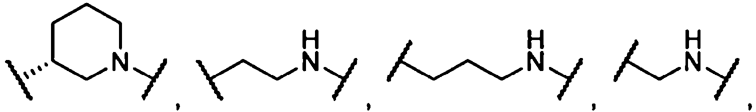

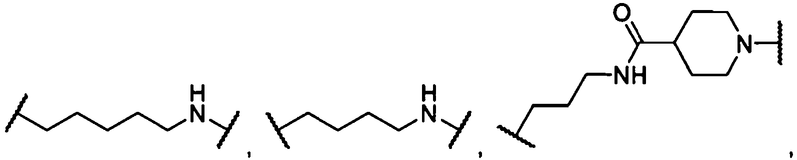

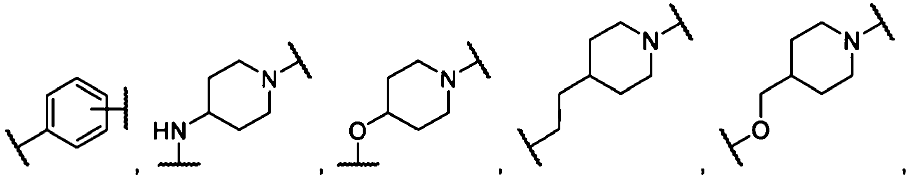

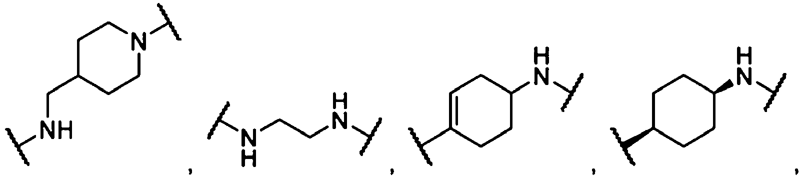

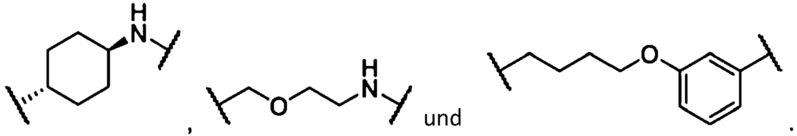

15. Verbindung nach Anspruch 1, wobei die Verbindung aus einer in Tabelle 1 aufgeführten Verbindung ausgewählt ist, wobei optional die Verbindung aus der Gruppe ausgewählt ist, bestehend aus: (Beispiel Nr.: 1), 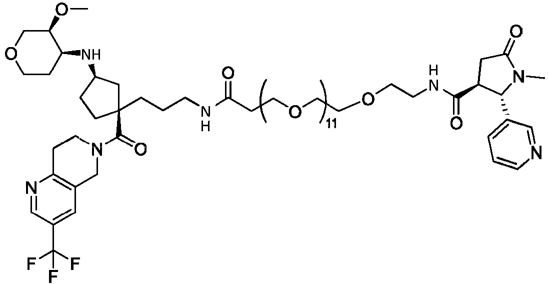 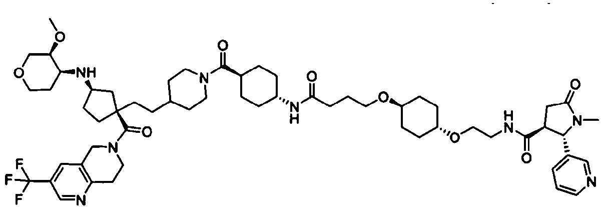 (Beispiel Nr.: 2), und 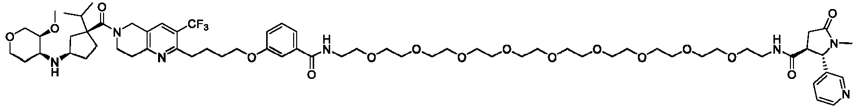 (Beispiel Nr.: 22).

16. Verbindung nach einem der vorstehenden Ansprüche zur Verwendung mit einem Anti-Cotinin-Antikörper oder einem Antigen-bindenden Fragment davon zur Behandlung und/oder Vorbeugung einer Krankheit oder Störung, ausgewählt aus Krebs, einer entzündlichen Erkrankung, einer Autoimmunerkrankung, einer Virusinfektion oder einer bakteriellen Infektion, wobei optional (i) die Verbindung und der Antikörper oder das Antigen-bindende Fragment davon gleichzeitig verabreicht werden oder (ii) die Verbindung und der Antikörper oder das Antigen-bindende Fragment davon nacheinander verabreicht werden.

17. Verbindung zur Verwendung nach Anspruch 16, wobei die Krankheit oder Störung durch den Chemokinrezeptor 2 (CCR2) vermittelt wird und/oder mit CCR2-positiven pathogenen Zellen assoziiert ist.

18. Verbindung zur Verwendung nach Anspruch 16 oder 17, wobei die Krankheit ein Krebs ist, der ein solider Tumor ist, wobei optional
der Krebs ausgewählt ist aus nicht-kleinzelligem Lungenkrebs (NSCLC), Leberzellkarzinom (HCC), Darmkrebs (CRC), Plattenepithelkarzinom des Gebärmutterhalses (CESC), Plattenepithelkarzinom des Kopfes und Halses (HNSC), Bauchspeicheldrüsenkrebs, metastasiertem kastrationsresistentem Prostatakrebs (mCRPC), Eierstockkrebs, Blasenkrebs oder Brustkrebs.

19. Verbindung nach einem der Ansprüche 1 bis 15 zur Verwendung mit einem Anti-Cotinin-Antikörper oder einem Antigen-bindenden Fragment davon zur Erhöhung der antikörperabhängigen zellulären Zytotoxizität (ADCC) von C-C-Motiv-Chemokinrezeptor-2 (CCR2)-exprimierenden Zellen, wobei die CCR2-bindende Einheit der Verbindung an das auf den Zellen exprimierte CCR2 bindet.

20. Verbindung nach einem der Ansprüche 1 bis 15 zur Verwendung mit einem Anti-Cotinin-Antikörper oder einem Antigen-bindenden Fragment davon zum Abbau von C-C-Motiv-Chemokinrezeptor-2 (CCR2)-exprimierenden Zellen, wobei die CCR2-bindende Einheit der Verbindung an das auf den Zellen exprimierte CCR2 bindet.

21. Verbindung zur Verwendung nach Anspruch 19 oder 20, wobei die CCR2-exprimierenden Zellen myeloide Suppressorzellen (MDSCs), regulatorische T-Zellen (Tregs), Neutrophile, Makrophagen, regulatorische B-Zellen (Bregs), regulatorische CD8-Zellen (CD8regs), erschöpfte T-Zellen oder Krebsassoziierte Fibroblasten (CAFs) sind.

22. Kombination, umfassend die Verbindung nach einem der Ansprüche 1 bis 15 und einen Anti-Cotinin-Antikörper oder ein Antigen-bindendes Fragment davon.

23. Verbindung zur Verwendung nach einem der Ansprüche 16 bis 21 oder der Kombination nach Anspruch 22, wobei der Anti-Cotinin-Antikörper eine schwere Kette und eine leichte Kette aufweist, wobei die schwere Kette eine CDR1 mit SEQ ID NO: 1, eine CDR2 mit der SEQ ID NO: 2 und eine CDR3 mit der SEQ ID NO: 3 umfasst, und die leichte Kette eine CDR1 mit der SEQ ID NO: 4, eine CDR2 mit der SEQ ID NO: 5 und eine CDR3 mit der SEQ ID NO: 6 umfasst.

24. Verbindung zur Verwendung nach einem der Ansprüche 16 bis 21 oder der Kombination nach Anspruch 22, wobei der Anti-Cotinin-Antikörper eine schwere Kette und eine leichte Kette aufweist, wobei die schwere Kette eine variable Region (VH) der schweren Kette mit der SEQ ID NO: 7 umfasst und die leichte Kette eine variable Region (VL) der leichten Kette mit der SEQ ID NO: 8 umfasst, wobei der Anti-Cotinin-Antikörper optional eine schwere Kette mit SEQ ID NO: 9 und eine leichte Kette mit SEQ ID NO: 10 umfasst.

25. Verbindung zur Verwendung nach einem der Ansprüche 16 bis 21 oder der Kombination nach Anspruch 22, wobei der Anti-Cotinin-Antikörper vom IgG1-Isotyp ist und eine Substitution in einer Fc-Region umfasst, um die ADCC-Aktivität zu erhöhen, wobei optional die Substitution in der Fc-Region S239D/I332E ist, wobei die Nummerierung der Reste gemäß dem EU-Index erfolgt.

## Revendications

1. Composé de formule (I) : 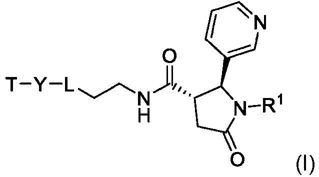
ou son sel pharmaceutiquement acceptable,
dans lequel :
T est 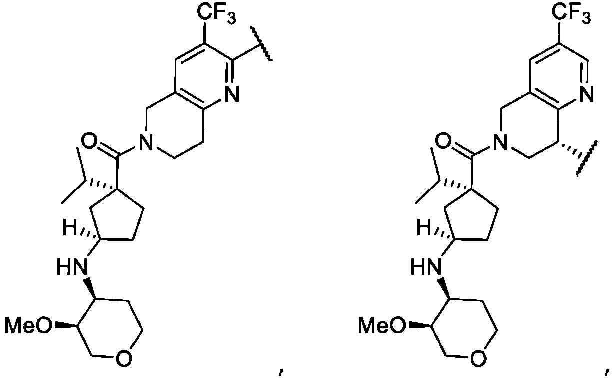 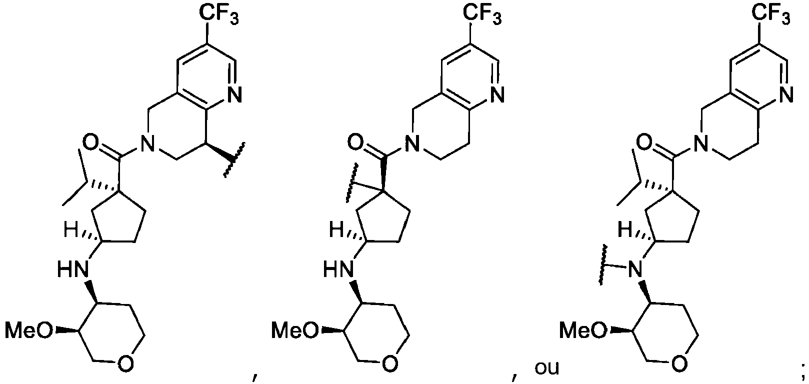
R¹ est un alkyle en C₁ à C₄ ou un cycloalkyle en C₃ à C₆ ;
L est un lieur divalent de formule (L-a), (L-b), (L-c), (L-d), ou (L-e) : 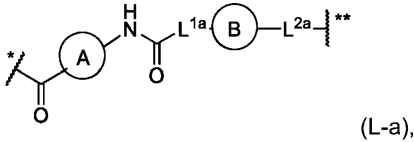 ou son stéréoisomère, dans lequel :
le cycle A et le cycle B sont chacun indépendamment un cycloalkylène en C₄ à C₆ ;
L^{1a} est un alkylène linéaire en C₃ à C₅, dans lequel 1 ou 2 unités méthylène sont remplacées par -O- ou -NR^{a}- ;
chaque R^{a} est indépendamment un atome d'hydrogène ou un alkyle en C₁ à C₃ ; et
L^{2a} est -O-, -NHC(O)- ou -CH₂-O- ; 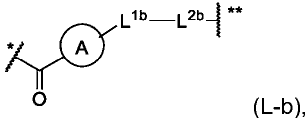 ou son stéréoisomère, dans lequel :
le cycle A est un cycloalkylène en C₄ à C₆ ou un cycloalkylène bicyclique ponté en C₇ à C₉ ;
L^{1b} est -CH₂-NH-C(O)-, -NHC(O)- ou -C(O)NH- ;
L^{2b} est un alkylène linéaire en C₆ à C₁₂, dans lequel 1, 2, 3 ou 4 unités méthylène sont remplacées par -O-, -NR^{1b}-, -C(O)NR^{1b}- ou - NR^{1b}C(O)- ; ou
L^{2b} est, 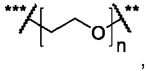 dans lequel n est 1, 2, 3 ou 4, et 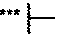 représente une liaison covalente à L^{1b} ; et
chaque R^{1b} est indépendamment un atome d'hydrogène ou un alkyle en C₁ à C₃ ; 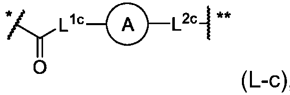 ou son stéréoisomère, dans lequel :
L^{1c} est un alkylène linéaire en C₂ à C₁₀, dans lequel 1, 2 ou 3 unités méthylène sont remplacées par -O-, -NH-, -NHC(O)- ou - C(O)NH- ;
le cycle A est un cycloalkylène en C₄ à C₆ ou un cycloalkylène bicyclique ponté en C₇ à C₉ ; et
L^{2c} est -O- ou un alkylène linéaire en C₂ à C₁₀ saturé, dans lequel 1, 2 ou 3 unités méthylène sont remplacées par -O-, -NH-, - NHC(O)- ou -C(O)NH- ; 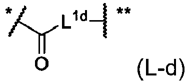 dans lequel :
L^{1d} est un alkylène linéaire en C₁₂ à C₃₀, dans lequel 1, 2, 3, 4, 5, 6, 7, 8, 9, 10 ou 11 unités méthylène sont remplacées par -NH-, - O-, -C(O)NH-, -NHC(O)- ou -NHC(O)-NH- ; ou 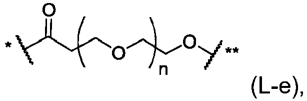 dans lequel n est un nombre entier compris entre 3 et 50 ;
dans lequel chaque 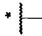 représente une liaison covalente au groupe Y de formule (I), ou lorsque Y est une liaison, une liaison covalente au groupe T de formule (I), et chaque 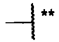 représente une liaison covalente au groupe méthylène de formule (I) ; et
Y est une liaison ou un groupe fonctionnel espaceur divalent d'une longueur d'un à douze atomes.

2. Composé de la revendication 1, ou son sel pharmaceutiquement acceptable, dans lequel R¹ est -CH₃.

3. Composé de la revendication 1 ou 2, ou son sel pharmaceutiquement acceptable, dans lequel L est un lieur divalent de formule (L-a-i) : 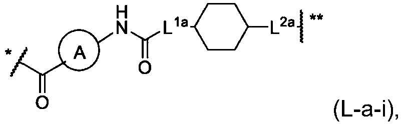 ou son stéréoisomère,
dans lequel le cycle A, L^{1a}, L^{2a}, 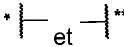 sont tels que définis pour la formule (L-a).

4. Composé de l'une quelconque des revendications précédentes, ou son sel pharmaceutiquement acceptable, dans lequel L est un lieur divalent de formule (L-a-ii) : 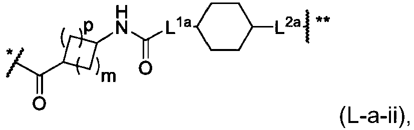 ou son stéréoisomère,
dans lequel L^{1a}, L^{2a}, 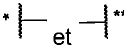 sont tels que définis pour la formule (L-a) ; p est 1 ou 2 ; et m est 1 ou 2.

5. Composé de l'une quelconque des revendications précédentes, ou son sel pharmaceutiquement acceptable, dans lequel L est un lieur divalent de formule (L-a-iii) : 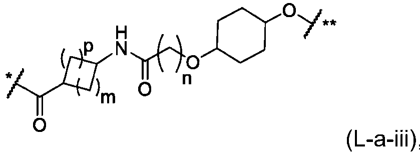 ou son stéréoisomère,
dans lequel p est 1 ou 2 ; m est 1 ou 2 ; n est 1, 2 ou 3 ; et 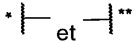 sont tels que définis pour la formule (L-a).

6. Composé de l'une quelconque des revendications 1 à 4, ou son sel pharmaceutiquement acceptable, dans lequel L est un lieur divalent de formule (L-a) choisi dans le groupe constitué par :

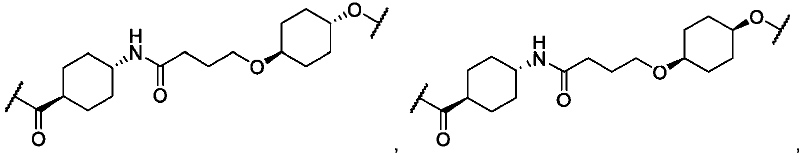

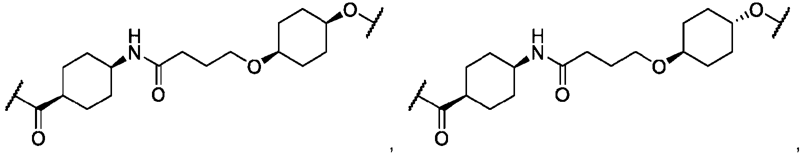

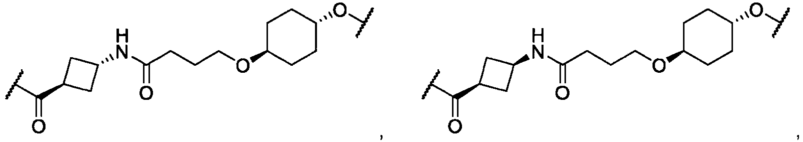

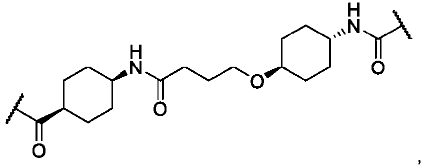

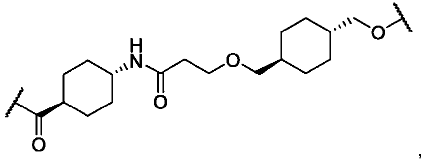

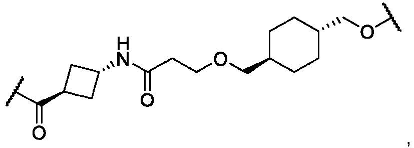

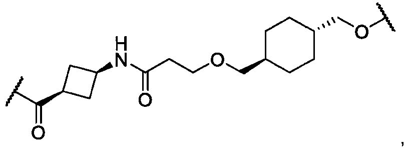

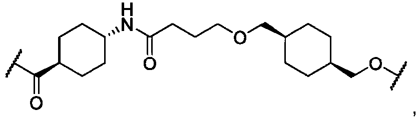

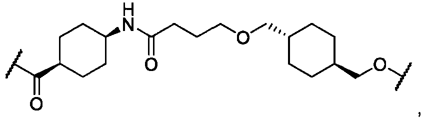

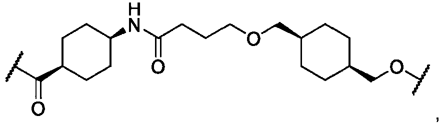

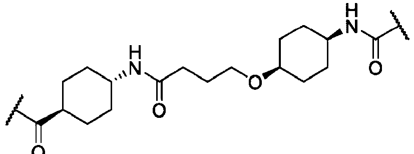

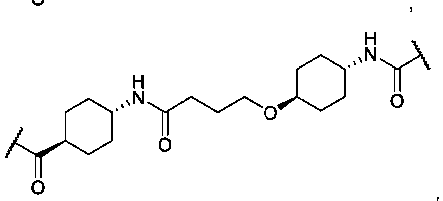

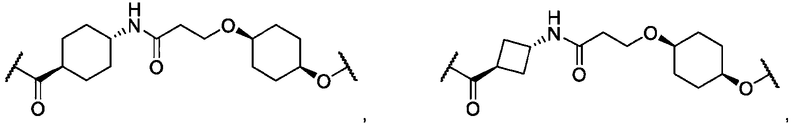

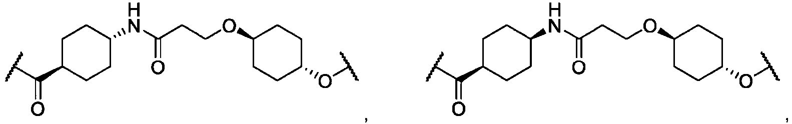

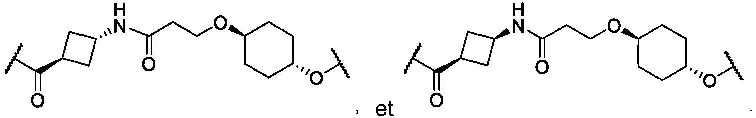

7. Composé de la revendication 1 ou 2, ou son sel pharmaceutiquement acceptable, dans lequel L est un lieur divalent de formule (L-b-i) : 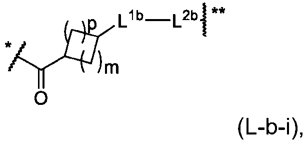 ou son stéréoisomère,
dans lequel L^{1b}, L^{2b}, 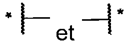 sont tels que définis pour la formule (L-b) ; p est 1 ou 2 ; et m est 1 ou 2.

8. Composé de l'une quelconque des revendications 1 à 2 ou 7, dans lequel L est un lieur divalent de formule (L-b) choisi dans le groupe constitué par : 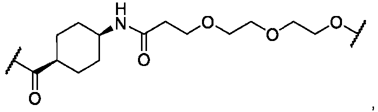 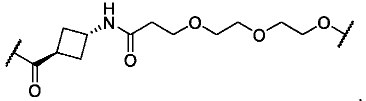 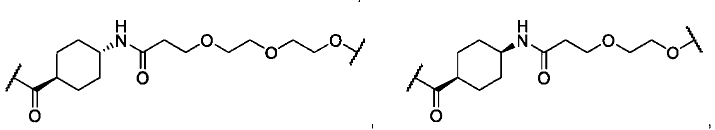 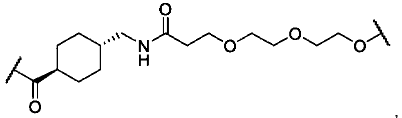 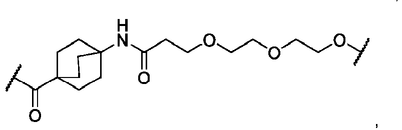 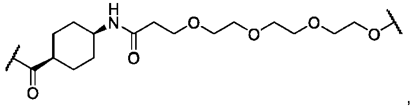 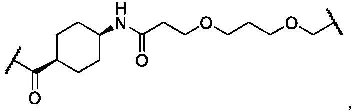 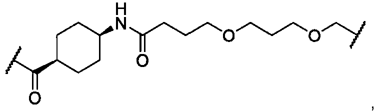 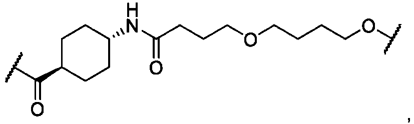 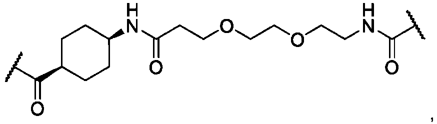 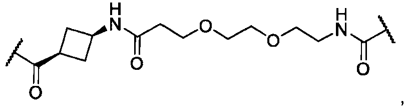 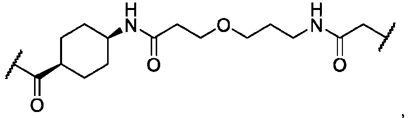 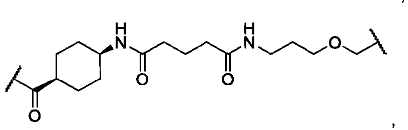 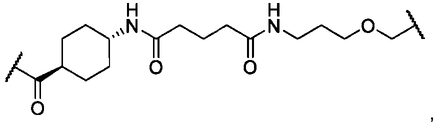 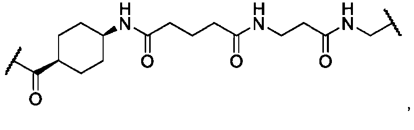 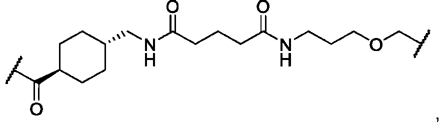 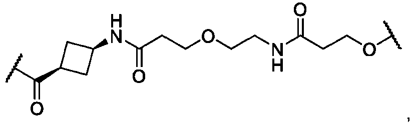 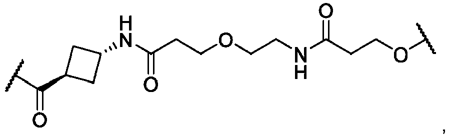 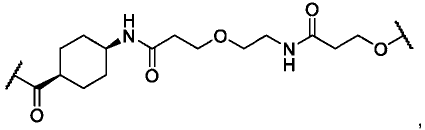 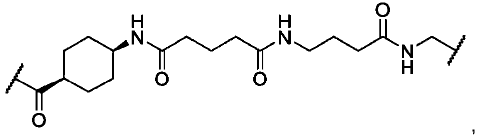 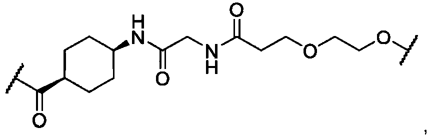 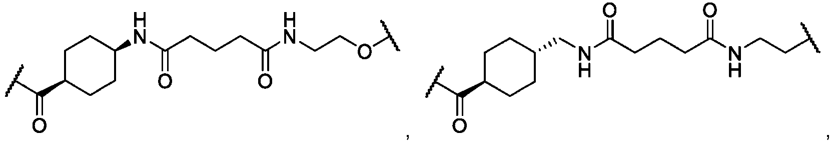 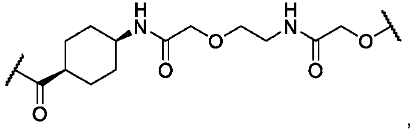 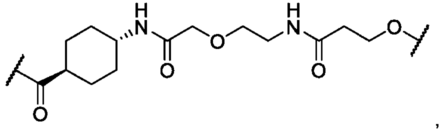 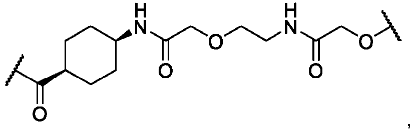 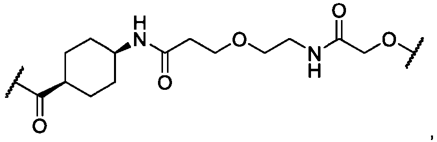 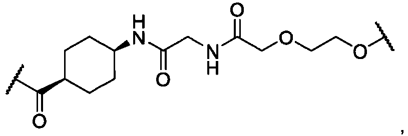 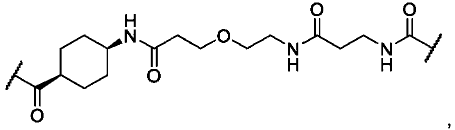 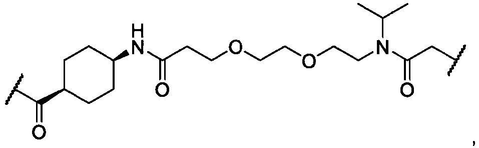 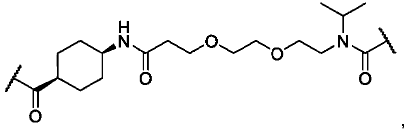 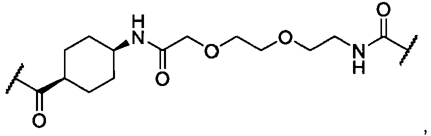 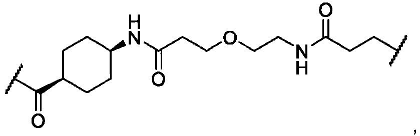 et

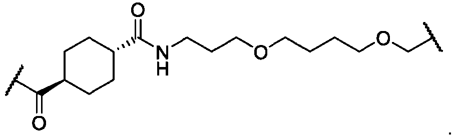

9. Composé de la revendication 1 ou 2, ou son sel pharmaceutiquement acceptable, dans lequel L est un lieur divalent de formule (L-c-i) : 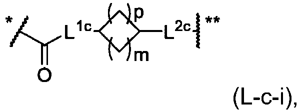 ou son stéréoisomère,
dans lequel L^{1c}, L^{2c}, 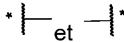 sont tels que définis pour la formule (L-c) ; p est 1 ou 2 ; et m est 1 ou 2.

10. Composé de la revendication 1 ou 2, dans lequel L est un lieur divalent de formule (L-c) choisi dans le groupe constitué par :

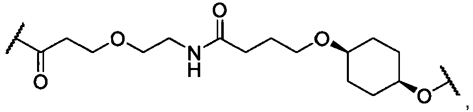

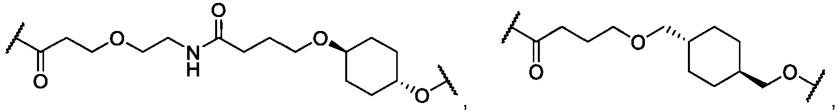

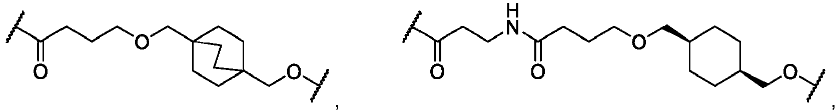

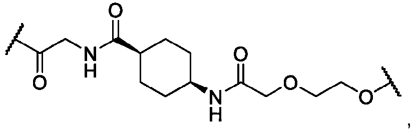

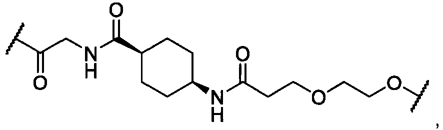

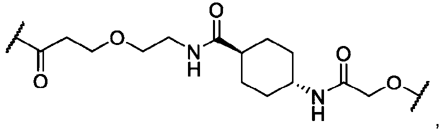

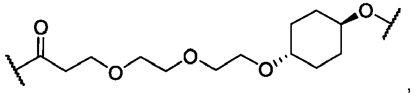

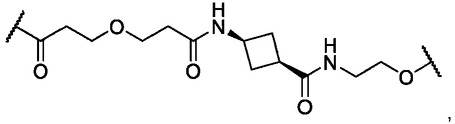

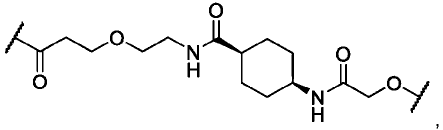

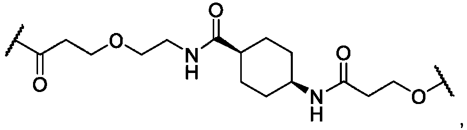

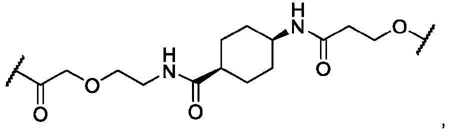

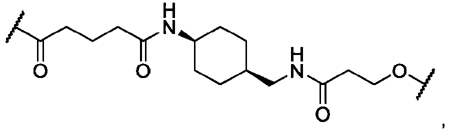

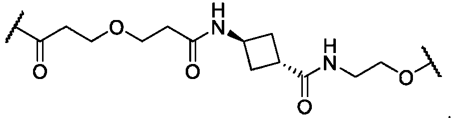

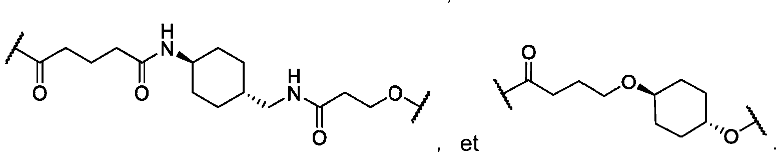

11. Composé de la revendication 1 ou 2, ou son sel pharmaceutiquement acceptable, dans lequel L est un lieur divalent de formule (L-d) choisi dans le groupe constitué par : 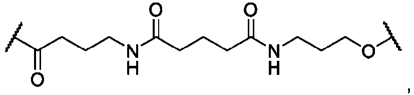 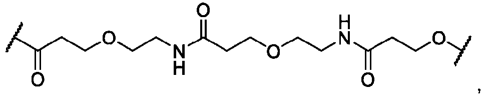 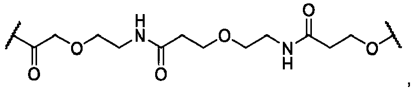 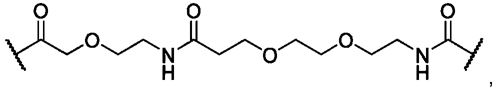 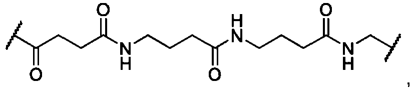 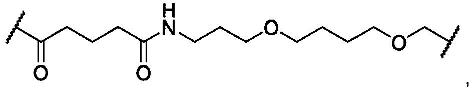 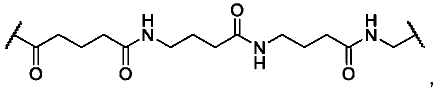 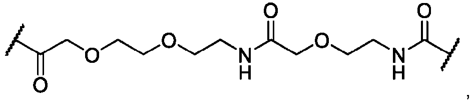 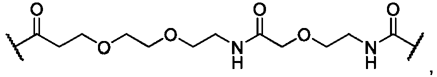 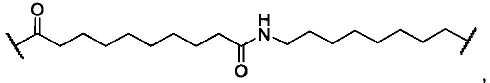 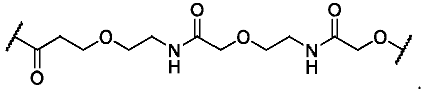 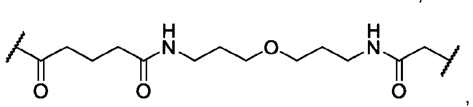 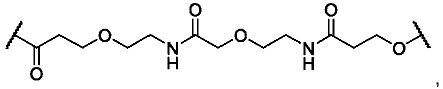 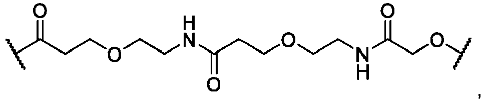 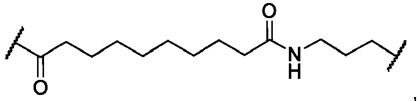 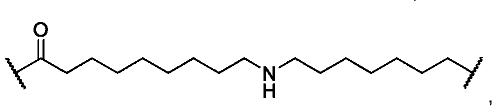 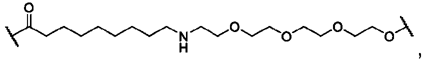 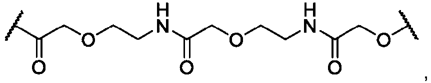 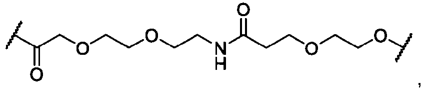 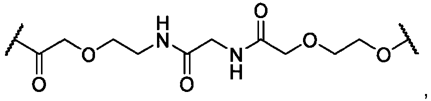 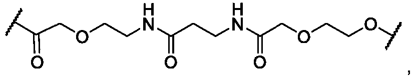 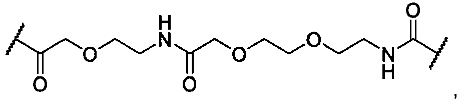 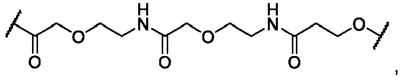 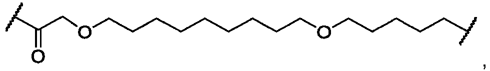 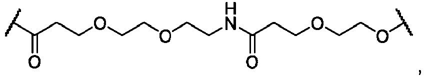 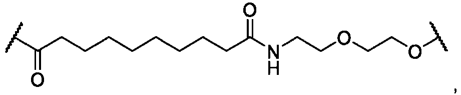 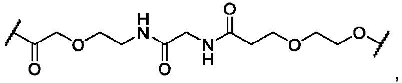 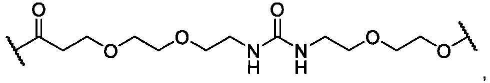 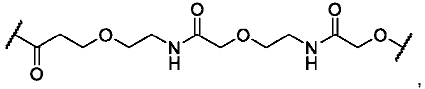 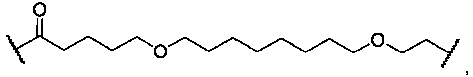 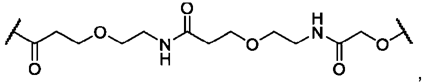 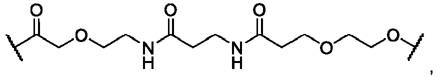 et

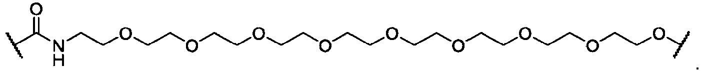

12. Composé de l'une quelconque des revendications précédentes, ou son sel pharmaceutiquement acceptable, dans lequel Y est choisi parmi une liaison ; -NH- ; - (alkylène en C₁ à C₁₂)-, dans lequel 1, 2 ou 3 unités méthylène sont remplacées par -O-, - NH-, -C(O)-, -NHC(O)-, -C(O)NH-, -(cycloalkylène en C₃ à C₆)-, -(cycloalcénylène en C₃ à C₆)-, hétérocycloalkylène à 3 à 6 chaînons, arylène ou hétéroarylène ; ou -(alcénylène en C₂ à C₁₂)-, dans lequel 1, 2 ou 3 unités méthylène sont remplacées par -O-, -NH-, -C(O)-, -NHC(O)-, -C(O)NH-, -(cycloalkylène en C₃ à C₆)-, -(cycloalcénylène en C₃ à C₆)-, hétérocycloalkylène à 3 à 6 chaînons, arylène ou hétéroarylène.

13. Composé de l'une quelconque des revendications 1 à 11, ou son sel pharmaceutiquement acceptable, dans lequel Y est choisi parmi une liaison ; -NH- ; - (alkylène en C₁ à C₆)-O- ; -(alcénylène en C₂ à C₆)-O- ; -(alkylène en C₁ à C₆)-C(O)- ; - (alcénylène en C₂ à C₆)-C(O)- ; phénylène ; pipéridinylène ; -(alkylène en C₄ à C₆)-O-phénylène- ; -(alcénylène en C₂ à C₆)-O-pipéridinylène ; -(alkylène en C₄ à C₅)-NH-, dans lequel 0, 1 ou 2 unités méthylène sont remplacées par -O- ; -NH-(alkylène en C₁ à C₆)-NH- ; -(cycloalkylène en C₃ à C₆)-NH- ; -(cycloalcénylène en C₃ à C₆)-NH- ; ou 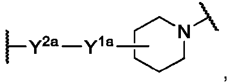 dans lequel Y^{1a} est une liaison, -O-, -NH-, -NHC(O)-, -C(O)NH-, ou un alkylène en C₁ à C₃ ; et Y^{2a} est une liaison, -O-, -NH-, -NHC(O)-, -C(O)NH-, ou un alkylène en C₁ à C₃.

14. Composé de l'une quelconque des revendications 1 à 11, ou son sel pharmaceutiquement acceptable, dans lequel Y est choisi dans le groupe constitué par :

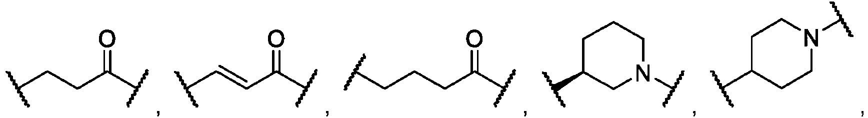

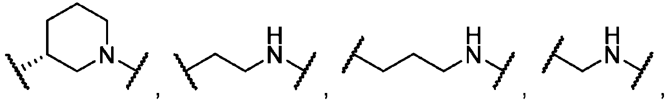

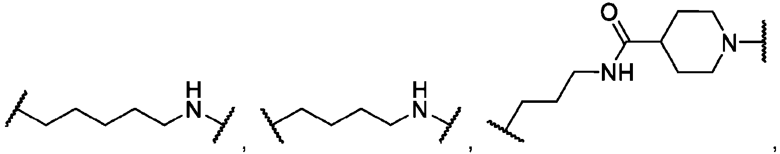

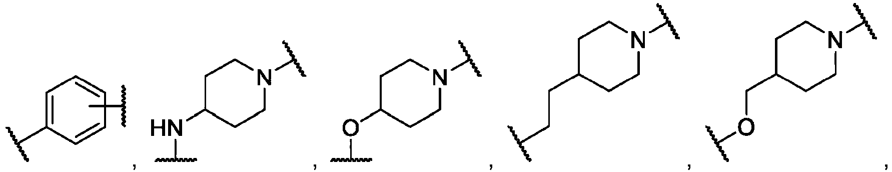

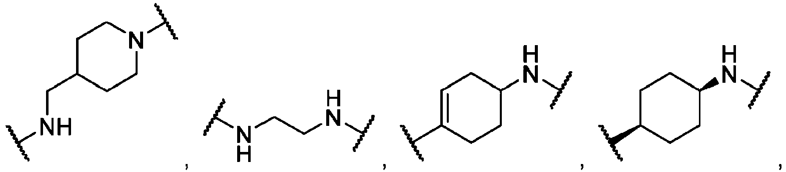

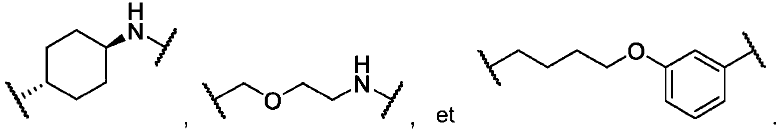

15. Composé de la revendication 1, dans lequel le composé est choisi parmi un composé tel qu'énuméré dans le tableau 1, dans lequel, éventuellement
le composé est choisi dans le groupe constitué par : 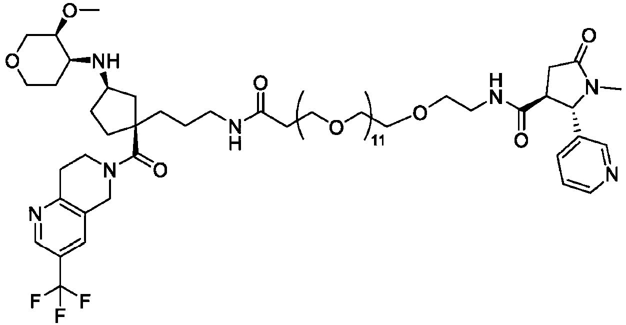 (Exemple n° : 1), 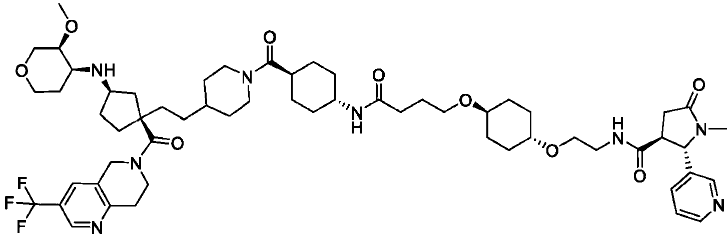 (Exemple n° : 2), et 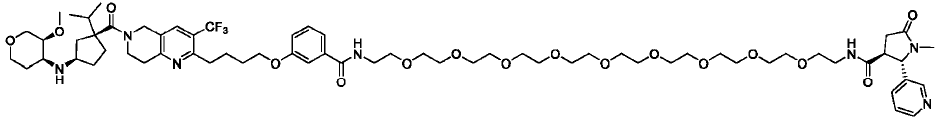 (Exemple n° : 22).

16. Composé de l'une quelconque des revendications précédentes pour une utilisation avec un anticorps anti-cotinine, ou son fragment de liaison à l'antigène, pour traiter et/ou prévenir une maladie ou un trouble choisi parmi un cancer, une maladie inflammatoire, une maladie auto-immune, une infection virale ou une infection bactérienne, dans lequel, éventuellement (i) le composé et l'anticorps, ou son fragment de liaison à l'antigène, sont administrés simultanément ou (ii) le composé et l'anticorps, ou son fragment de liaison à l'antigène, sont administrés séquentiellement.

17. Composé pour une utilisation de la revendication 16, dans lequel la maladie ou le trouble est médié par le récepteur 2 de la chimiokine (CCR2) et/ou est associé à des cellules pathogènes CCR2-positives.

18. Composé pour une utilisation de la revendication 16 ou 17, dans lequel la maladie est un cancer qui est une tumeur solide, dans lequel, éventuellement,
le cancer est choisi parmi le cancer du poumon non à petites cellules (NSCLC), le carcinome hépatocellulaire (CHC), le cancer colorectal (CCR), le carcinome épidermoïde cervical (CEC), le carcinome épidermoïde de la tête et du cou (CECHC), le cancer du pancréas, le cancer de la prostate métastatique résistant à la castration (mCRPC), le cancer de l'ovaire, le cancer de la vessie ou le cancer du sein.

19. Composé de l'une quelconque des revendications 1 à 15, pour une utilisation avec un anticorps anti-cotinine, ou son fragment de liaison à l'antigène, pour augmenter la cytotoxicité cellulaire dépendante des anticorps (ADCC) des cellules exprimant le récepteur 2 de la chimiokine à motif C-C (CCR2), dans lequel le groupe fonctionnel de liaison au CCR2 du composé se lie au CCR2 exprimé sur les cellules.

20. Composé de l'une quelconque des revendications 1 à 15, pour une utilisation avec un anticorps anti-cotinine, ou son fragment de liaison à l'antigène, pour épuiser les cellules exprimant le récepteur 2 de la chimiokine à motif C-C (CCR2), dans lequel le groupe fonctionnel de liaison au CCR2 du composé se lie au CCR2 exprimé sur les cellules.

21. Composé pour une utilisation de la revendication 19 ou 20, dans lequel les cellules exprimant le CCR2 sont des cellules suppressives dérivées des myéloïdes (MDSC), des cellules T régulatrices (Treg), des neutrophiles, des macrophages, des cellules B régulatrices (Breg), des cellules CD8 régulatrices (CD8reg), des cellules T épuisées, ou des fibroblastes associés au cancer (CAF).

22. Combinaison comprenant le composé de l'une quelconque des revendications 1 à 15 et un anticorps anti-cotinine, ou son fragment de liaison à l'antigène.

23. Composé pour une utilisation selon l'une quelconque des revendications 16 à 21 ou combinaison de la revendication 22, dans lequel/laquelle l'anticorps anti-cotinine a une chaîne lourde et une chaîne légère, la chaîne lourde comprenant un CDR1 ayant le SEQ ID n° : 1, un CDR2 ayant le SEQ ID n° : 2 et un CDR3 ayant le SEQ ID n° : 3, et la chaîne légère comprenant un CDR1 ayant le SEQ ID n° : 4, un CDR2 ayant le SEQ ID n° : 5 et un CDR3 ayant le SEQ ID n° : 6.

24. Composé pour une utilisation selon l'une quelconque des revendications 16 à 21 ou combinaison de la revendication 22, dans lequel/laquelle l'anticorps anti-cotinine a une chaîne lourde et une chaîne légère, la chaîne lourde comprenant une région variable de chaîne lourde (VH) ayant le SEQ ID n° : 7, et la chaîne légère comprenant une région variable de chaîne légère (VL) ayant le SEQ ID n° : 8, dans lequel, éventuellement, l'anticorps anti-cotinine a une chaîne lourde comprenant le SEQ ID n° : 9 et une chaîne légère comprenant le SEQ ID n° : 10.

25. Composé pour une utilisation selon l'une quelconque des revendications 16 à 21 ou combinaison de la revendication 22, dans lequel/laquelle l'anticorps anti-cotinine est de l'isotype IgG 1 comprenant une substitution dans une région Fc pour augmenter l'activité ADCC, dans laquelle, éventuellement,
la substitution dans la région Fc est S239D/1332E, dans laquelle la numérotation des résidus est conforme à l'index UE.
